(19) Europäisches Patentamt
European Patent Office
Office européen des brevets

(11) EP 2 389 922 A1

(12) **EUROPEAN PATENT APPLICATION**

(43) Date of publication:
**30.11.2011 Bulletin 2011/48**

(51) Int Cl.:
***A61K 8/42*** *(2006.01)* ***A61Q 19/08*** *(2006.01)*
***C07C 271/34*** *(2006.01)*

(21) Application number: **10163832.8**

(22) Date of filing: **25.05.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Symrise AG**
**37603 Holzminden (DE)**

(72) Inventors:
- **Küper, Thomas**
  **48734, Reken (DE)**
- **Oertling, Heiko**
  **37671, Holzminden (DE)**
- **Grune, Tilman**
  **10787, Berlin (DE)**
- **Saathoff, Matthias**
  **26607, Hamburg (DE)**
- **Herrmann, Martina**
  **31789, Hameln (DE)**
- **Betke, Julia**
  **32694, Dörentrup (DE)**
- **Gömann, Claudia**
  **37640, Golmbach (DE)**
- **Brodhage, Rahim**
  **37671, Höxter (DE)**
- **Meyer, Imke**
  **37619, Bodenwerder (DE)**

(74) Representative: **Eisenführ, Speiser & Partner**
**Am Kaffee-Quartier 3**
**28217 Bremen (DE)**

(54) **Cyclohexyl carbamate compounds as anti-ageing actives**

(57) The present invention relates to the cosmetic, dermatological or therapeutic use of compounds of formula (I) given below, in particular as anti-ageing actives.

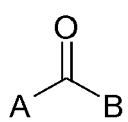

(I)

wherein
A denotes

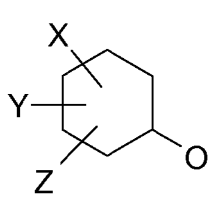

,

wherein X, Y and Z independently of one another denote hydrogen, C1-C4-alkyl or C2-C4-alkenyl,
wherein optionally two of the radicals X, Y and Z are covalently bonded to one another under formation of a bicyclic ring system, in such a bicyclic ring system two of the radicals X, Y and Z together preferably form a radical having 1 to 4 carbon atoms, preferably a hydrocarbon radical having 1 to 3 carbon atoms,
B denotes $NR^1R^2$, wherein
$R^1$ denotes hydrogen or an organic radical having 1 to 14 carbon atoms,
$R^2$ denotes an organic radical having 1 to 14 carbon atoms,
and
wherein optionally $R^1$ and $R^2$ are covalently bonded to one another, preferably so that B is a 3 to 8 membered ring.

The invention further relates to corresponding methods and to compositions and cosmetic, dermatological or pharmaceutical preparations (compositions) comprising one or more compounds of formula (I) and one or more further anti-ageing actives. The invention further relates to compounds of formula (I) as a drug, their use for the preparation of a pharmaceutical composition and to certain novel compounds of formula (I).

EP 2 389 922 A1

## Description

The present invention relates to the cosmetic, dermatological or pharmaceutical (therapeutic) use of cyclohexyl carbamate compounds of formula (I) given below, in particular as agents which enable prolongation of the life span of a cell, preferably of a human skin cell and/or a human neuronal cell, by inhibition of cellular senescence, i.e. the compounds of formula (I) act as anti-ageing actives. The invention further relates to corresponding methods and to compositions and cosmetic, dermatological or pharmaceutical preparations (compositions) comprising one or more compounds of formula (I) and one or more further anti-ageing actives. The invention further relates to compounds of formula (I) as a drug, their use for the preparation of a pharmaceutical composition and to certain novel compounds of formula (I).

The mechanism of ageing is generally divided into two different parts. The first part is called intrinsic ageing caused by factors derived from within the body. The second part is classified as extrinsic ageing provoked by environmental factors. During the process of ageing several impairments can be observed, e.g. a decline in metabolic activity, a lower rate of cell division, a reduced DNA repair capacity, stiffening of vessel walls and an impaired peripheral blood circulation. All these aspects are closely linked to each other. However, the ageing process advances gender specifically due to the different hormonal structure.

For skin ageing the following physiological changes can be observed, for example: in the epidermis the reduction of mitosis rate of basal cells results in a thinner epidermis; in addition, a high water loss due to diffusion, a reduced elasticity, a rougher external structure and wrinkle formation are generally observed.

The dermis is impaired by less fibroblasts with reduced activity; further, a reduced production of tropocollagen, hyaluronic acid and elastin are observed as well as loss of elasticity, reduced sebum production and reduced perspiration.

For the subcutis changes in the palisade structure of feminine skin can be observed. All actions in the epidermis, the dermis and subcutis are reflected in the appearance of the stratum corneum. The skin looks thinner, dry and rough like parchment. For these reasons, an anti-ageing product should ideally focus on all skin layers and their changes accordingly and should be used as early as possible.

One field of skin ageing relates to the so called destructive modifications. These modifications target intracellular and extracellular proteins resulting in the loss of their biological function. Examples for destructive modifications are AGE-modifications (Advanced Glycation Endproducts) and carbonylations. AGE-modifications are based on the reaction between sugars and amino acid residues leading to crosslinked proteins. This is known for extracellular proteins like collagen as well as intracellular proteins like vimentin. The crosslinking of collagen results in increased stiffening of the skin whereas the glycation of vimentin is accompanied by the loss of contractile capacity of fibroblasts.

One group of intrinsic factors consists of matrix metalloproteinases (MMPs). MMPs are enzymes which cleave structural proteins in biological tissue. One of these proteins is collagen, the major constituent of the extracellular matrix of the skin. Collagen builds fibres in the skin and is therefore important for skin structure and firmness. For example, MMP-1 cleaves collagen resulting in the degradation of collagen. An activation of the proteasome results therefore in less MMP-1 and less collagen degradation, see e.g. J. Biol. Chem, 284(44), 30076-30086.

In aged skin, the amount of MMPs is increased resulting in fragmented collagen and finally in wrinkle formation. A prominent example for extrinsic ageing is the induction of MMPs by UV light, also called photo-ageing. There are several strategies to prevent the fragmentation of collagen. MMP action and/or amount can be reduced by the application of respective inhibitors. Furthermore, the induction of the skin's own MMP inhibitors, TIMPs (tissue inhibitor of metalloproteinases), protects against collagen degradation. In concert with MMP inhibition, the stimulation of collagen synthesis provides a powerful tool for repair of damaged tissue. Finally, sun protection by UV filters is an effective strategy for protection against photo-ageing.

Another constituent of the extracellular matrix is hyaluronic acid which belongs to the family of glucosaminoglycans (GAGs, also: glycosaminoglycans). This substance is composed of repeated disaccharide units, namely D-glucuronic acid and D-N-acetylglucosamine, building a complex polymer of different sizes. GAGs are major constituents of the extracellular matrix and are known for their extremely high water binding capacity. Two types of GAGs are known, acidic GAGs like hyaluronic acid, which is mainly involved in the preservation of the tissue hydration, and neutral GAGs like Chondroitin sulphate, which are mainly involved in intracellular signaling, cell communication in the dermal-epidermal-junction (DEJ) and stabilization of DEJ.

The age-induced loss of hyaluronic acid is therefore accompanied by a tremendous loss of tissue volume leading to sagging skin. This process is enhanced by the age-dependent decline of other GAG's like dermatan sulphate. Hallmarks of aged skin are also found in the upper epidermis: aged skin is marked by weakened epidermal barrier permeability, leading to dry skin. The permeability barrier is located in the horny layer and consists of lipid bilayers surrounding the corneocytes.

Ceramides, cholesterol and fatty acids are the main lipid constituents of the epidermal barrier significantly reduced in aged skin. Therefore, topical application of (pseudo-)ceramides, combined with free fatty acids and cholesterol helps to prevent and to repair dry skin.

Another reason for the dryness of aged skin is the reduced amount of the skin's own moisturizers (natural

moisturizing factor, NMF).

A significant trigger for ageing is the development of reactive oxygen species (ROS). Two major sources are known for the formation of ROS. The first source is the extrinsic ageing factor UV-light which induces ROS formation like hydroxyl radicals, superoxides, peroxides and other reactive species which can virtually damage all kinds of macromolecules. Reactive oxygen damage the proteasome resulting in an impaired activity, see for example J. Biol. Chem, 284(44), 30076-30086.

The second intrinsic source for free radicals is the mitochondrial electron transport chain. The mitochondrium is the cellular power station generating ATP, which is the driving force for nearly every biological function in the cellular metabolism. Although the mitochondrial electron transport chain works with a high efficacy, the machinery is not perfect and misdirected electrons result in the production of toxic free radical superoxides and therewith in oxidative stress. The most potent cellular defence mechanism to scavenge free radicals is the glutathione system combined with enzymes that dissipate free radicals, namely catalase and superoxide dismutase. Even small doses of UV or other stress factors override these protective systems. This is the point where topical antioxidant application becomes important to prevent oxidative damage.

Another strategy to reduce the mitochondria-based oxidative stress is the theory of calorie restriction. There, reducing the input of calories may result in a lower activity of the metabolism and a decreased ROS production. A common diet-restriction regimen used in experimental animals is a 30% reduction in food intake resulting in a significantly prolonged healthy lifespan.

A class of enzymes called sirtuins (sirtuin proteins) was shown to be upregulated in mammals when set under calorie restriction and is thought to be an important player promoting longevity. In consequence, stimulation of sirtuins (SIRTs), namely of SIRT1, has recently become an important target for anti-ageing actives.

The sirtuins post-translationally modulate the function of many cellular proteins that undergo reversible acetylation-deacetylation cycles. These modifications affect physiological responses that have implications for ageing in humans. In mammals there are seven members of the sirtuin family, SIRT 1-7, of which SIRT1 is the best studied protein. The protein SIRT1 is an $NAD^+$-dependent deacetylase catalyzing the reaction in which an acetyl group is removed from lysine side chains of proteins or peptides cleaving $NAD^+$, which acts as a co-substrate. This results in the deacetylated protein 2'-O-acetyl-ADP-ribose and nicotinamide.

The polyphenol Resveratrol was shown to increase the deacetylase activity of certain sirtuin proteins thereby extending lifespan of yeast by 70% and fish by 60%.

Some examples for the SIRT1 deacetylation substrates include PGC1$\alpha$, FOXO, p53, and the p65 subunit of the nuclear factor-$\kappa$B. These targets belong to the central regulators of the cellular metabolism, energy expenditure, inflammation and stress response. Overexpression of SIRT1 results therefore in the stimulation of mitochondrial biogenesis and metablic rate as well as the modulation of insulin sensitivity.

Furthermore, in white adipose tissue, an increase in SIRT1 protein level or enzyme activity decreases adipogenesis and lipid stores. The anti-inflammatory effect of SIRT is based on the suppression of NF-$\kappa$B activity or through cyclooxgenase 2 inhibition. Excessive activation of NF-$\kappa$B was reported to be present in aged skin, linking inflammation to ageing. One result of NF-$\kappa$B activation is the expression of destructive matrix metalloproteinases leading to fragmented collagen observed in skin of elderly people. An additional key factor for the anti-ageing properties of SIRT1 is the activation of the heat shock response. The heat shock factors (i.e. HSP70) are essential for protecting cells from protein-damaging stress associated with misfolded proteins. This chaperone activity regulates protein homeostasis. Recent studies were also able to link SIRT1 to ageing diseases of the heart as well while overexpression of SIRT 1 showed an attenuation of cardiac hypertrophy, apoptosis, fibrosis, cardiac dysfunction and expression of senescence marker. Furthermore it indirectly supports cardiac function by deacetylating the eNOS enzyme. SIRT1 was shown to increase upon colorie restriction in rodent and human tissue like white adipose, liver, skeletal muscle, brain and kidney. Additionally, small molecule activators of SIRT1 replicate signalling pathways triggered by calorie restriction in vivo. Cosmetic activators of SIRT1 provide therefore benefits as anti-ageing ingredient while acting as cell life prolongators and energy stimulators. Activators of SIRT1 will therefore be beneficial as anti-wrinkle agents and cellular protectors in general. Furthermore these activators prevent inflammation induced ageing (inflammageing) by reducing proinflammatory cytokines leading to an even skin tone and reduced pigment spots.

One of the most disturbing signs of ageing is the loss of even skin tone due to an irregular distribution of collagen, haemoglobin and the formation of age spots. The underlying mechanism of the development of age spots is still unclear, however the current opinion is that the dark spots are composed of accumulated melanin and lipofuscin. In addition, age spots appear as invaginations of the basal membrane and a striking increase in the number of dermal papillae combined with irregular arranged melanin clusters was observed. Furthermore, the formation of age spots goes along with the upregelation of genes described for inflammatory response. The oldest approach to lighten age spots is the topical application of substances that bleach melanin. An alternative strategy for the lightening of age spots is the inhibition of the enzyme tyrosinase which is essential for the synthesis of new melanin. Additionally, the prevention of melanocyte proliferation can be a useful strategy to reduce the amount of melanin produced.

The proteasome degrades damaged, misfolded, oxidized or unnecessary proteins. Seeing that, the proteasome is among other things involved in life span regulation of proteins, cell cycle control, gene expression, (oxidative) stress, immune response and carcino-genesis. Specifically, the protasome is responsible for cell clearance of abnormal, denatured or in general damaged proteins as well as for the regulated degradation of short-lived proteins. Efficient protein degradation is one of the major factors that contribute to the retention of cellular homeostatic balance.

The 20S proteasome is a cylindric structure, with a molecular weight of 700 kDa, an approximately diameter of 12 nm and a length of 17 nm, which consists of 4 rings. The two outer rings are made of 7 different $\alpha$-subunits. The other 2 rings build the middle structure of the 20S proteasome and consist of 7 diverse $\beta$-subunits. The outer $\alpha$-rings control the access of 20S-proteasome substrates into the inner proteolytic chamber, which is made up by the two $\beta$-rings. This proteolytic activity could be divided into three different parts: 1. Peptidyl-glutamyl-peptide-hydrolysing activity (caspase like activity); 2. Trypsin like activity and 3. Chymotrypsin like activity.

Products of the proteasomal degradation are oligopeptides, generally with a length of 8-10 amino acids. In contrast to the 26S proteasome there is no need for polyubiquitination to degrade proteins by the 20S proteasome. Furthermore, degradation by the 20S proteasome is ATP-independent.

In response to the skin the proteasome has an important role in ageing / photo-ageing. The meaning of photo-ageing is skin ageing after (long term) chronic exposure to UVA and UVB light. Following UV energy absorption there are different changes in the skin, including production of free radicals, modified proteins and 4-hydroxy-2-nonenal (HNE). These products affect the gene expression in skin cells and lead to an increase of matrix metalloproteinases (MMPs) and a decrease of their inhibitors (TIMPs) which results in a reduction of collagen and therefore in wrinkle formation. A drastic decline in proteasome activity and a simultaneous accumulation of modified and ubiquitinated proteins after UV irradiation in human keratinocytes has been reported. Today it is proven that there is a decline of proteasome activity and expression in human keratinocytes and fibroblasts during ageing. It is evident that this down regulation of proteasome activity is a result of protein aggregation. In a circuit, shrinking of proteasome activity leads to new protein aggregates and non degraded polyubiquitinated proteins, which in turn affect the proteasome again. Based on these findings, skin ageing is linked to proteasome dysfunction and preservation of "normal" proteasome function allays skin ageing.

SDS and some fatty acids have been reported to stimulate proteasome activities in the test tube by favoring the open confirmation of the proteasome. Recently, isolated oleuropein, the most abundant of the phenolic compounds in Olea europaea leaf extract, olive oil and olives was shown to have stimulatory impact on proteasome activities. Furthermore, a phaeodactylum algae extract was reported to stimulate proteasome activity (WO 02/080876) as well as Palmitoyl Isoleucin.

Besides the stimulation of proteasome activity by stimulation of one or more of the enzymatic activities, the upregulation of the proteasomal proteins itself is a potent method to induce the proteasomal degradation. This is of special interest regarding the fact that not only proteasomal activity declines in elderly people but also the expression of proteasomal subunits. Overexpression of proteasomal subunits in primary human embryonic fibroblasts and the accompanied increased rate of lipolysis were shown to extend the lifespan of these cells by 15-20%.

Moreover, it was shown that restoration of the normal level of proteasome subunits in aged human fibroblasts reduces the level of ageing biomarkers. A decline of proteasome activities has been revealed in human primary cultures undergoing replicative senescence, whereas proteasome inhibition in young cells induces premature senescence.

Maintenance of cellular homeostasis influences the rate of ageing and is determined by several factors, including efficient proteolysis of damaged proteins. Protein degradation is predominantly catalyzed by the proteasome. Specifically, the proteasome is responsible for cell clearance of abnormal, denatured or in general damaged proteins as well as for the regulated degradation of short-lived proteins. As the proteasome has an impaired function during ageing, actives that restore its function are needed. Furthermore, UV irradiation impairs proteasomal function, thereby enhancing the cellular deposition of toxic proteins. Importantly, centenarians show a very high activity of the proteasome and are a good example of healthy ageing.

The activation of the proteasome is also linked to age-associated diseases, especially the neurodegenerative diseases in which misfolded proteins aggregate. Proteasome activators enhance the survival of Huntington's disease neuronal model cells and the development of Alzheimer's disease and Parkinson's disease is partly based on an inefficient proteasomal clearance.

Skin-lightening active ingredients intervene in one form or another in melanin metabolism or catabolism. Melanin pigments, which are normally brown to black in colour, are formed in the melanocytes of the skin, transferred to the keratinocytes and give the skin or hair its colour. In mammals, the brown-black eumelanins are primarily formed from hydroxy-substituted aromatic amino acids such as L-tyrosine and L-DOPA, the yellow to red pheomelanins additionally from sulfur-containing molecules (Cosmetics & Toiletries 1996, 111 (5), 43-51).

Skin-lightening agents are used for various reasons: if for some reason the melaninforming melanocytes in human skin are not evenly distributed, pigment spots occur which are either lighter or darker than the surrounding skin area. To overcome this problem, skin lightening agents are sold which at least partially help to balance out such pigment spots. In addition, many people have a need to lighten their naturally dark skin colour or to prevent skin pigmentation.

This requires very safe and effective skin and hair lightening agents.

One area of application in this regard is the therapeutic treatment of melanin-induced pigmentation disorders such as hyperpigmentations (e.g. scar hyperpigmentations, post-traumatic drug-induced hyperpigmentations, post-inflammatory hyperpigmentations induced by phototoxic reactions, ephelides).

Furthermore, UV-absorbing substances are also used to protect against the increase in skin pigmentation caused by UV light. UV absorbers do not bring about a true lightening of the skin but merely inhibit the increase in skin pigmentation caused by UV light.

US 4,959,393 discloses 4-alkyl-resorcinols as skin lightening agents. WO 2004/105736 teaches certain diphenylmethane-derivatives as skin lightening agents.

Hydroquinone, hydroquinone derivatives such as e.g. arbutin, vitamin C, derivatives of ascorbic acid such as e.g. ascorbyl palmitate, kojic acid and derivatives of kojic acid such as e.g. kojic acid dipalmitate, are used in particular in commercial cosmetic or therapeutic skin lightening preparations.

The object of the present invention was to provide effective agents which enable prolongation of the life span of a cell, preferably of a human skin cell and/or a human neuronal cell, by inhibition of cellular senescence. Preferably, said agents should achieve said effect either via proteasomal clearance in a cell or via stimulation of proteasome activity and/or proteasome expression activation (Route (A)) or via stimulation of activity and/or expression of one or more sirtuin proteins (sirtuins) (Route (B)), in particular of SIRT1. More preferably, the agents should exhibit activity along both pathways, i.e. achieve the desired effect by exhibiting activity in Route (A) and in Route (B).

It has surprisingly been found that this object can be achieved by using compounds of formula (I) or a cosmetically acceptable salt of a compound of formula (I) or a mixture containing two or more of these compounds or the salts thereof

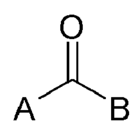

(I)

wherein
A denotes

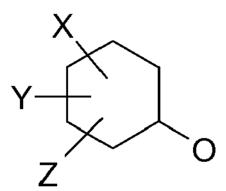

,

wherein X, Y and Z independently of one another denote hydrogen, C1-C4-alkyl or C2-C4-alkenyl,
wherein optionally two of the radicals X, Y and Z are covalently bonded to one another under formation of a bicyclic ring system, in such a bicyclic ring system two of the radicals X, Y and Z together preferably form a radical having 1 to 4 carbon atoms, preferably a hydrocarbon radical having 1 to 3 carbon atoms,
B denotes $NR^1R^2$, wherein
$R^1$ denotes hydrogen or an organic radical having 1 to 14 carbon atoms,
$R^2$ denotes an organic radical having 1 to 14 carbon atoms,
and
wherein optionally $R^1$ and $R^2$ are covalently bonded to one another, preferably so that B is a 3 to 8 membered ring.

The compounds of formula (I) thus are cyclohexyl carbamates (Carb-I)

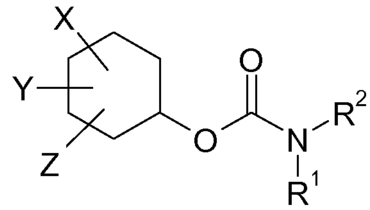

(Carb-I)

wherein $R^1$, $R^2$ and X, Y and Z have the meaning indicated hereinbefore or hereinafter.

As common in the art, in the context of the present invention, the substituents X, Y, and Z can in each case occupy - as indicated in the different structural formulae - any position in the cyclohexyl ring, i.e. in ipso, ortho, meta or para position to the cyclohexyl-carbon atom bonded to the oxygen of group A.

It is thus evident that two of the substituents X, Y, and Z - with exception of the ipso-position - can be bonded to the same carbon atom of the cyclohexyl ring of group A.

Surprisingly, it was found that the compounds of formula (I) not only stimulate SIRT1 expression, but also lead to an increase of proteasome activity and/or proteasome expression, thereby leading to proteasomal clearance in a cell. Thus it was found that compounds of formula (I), in particular the preferred and the particularly preferred compounds of formula (I) mentioned hereinbelow, exhibit activity along both pathways mentioned above, i.e. achieve the desired effects by exhibiting activity in Route (A) and/or in Route (B).

No mention or suggestion is made in the prior art of a cosmetic or therapeutic use of compounds of formula (I) as anti-ageing agents or of compounds of formula (I) having anti-ageing action.

The compounds of formula (I) show pronounced prolongation of the life span of a cell, preferably of a human skin cell and/or a human neuronal cell, by inhibition of cellular senescence (i.e. senescence of said cell), in particular by

- proteasomal clearance, and/or
- stimulation or increase of proteasome activity and/or proteasome expression, and/or
- stimulation of activity and/or expression of one or more sirtuin proteins (sirtuins), preferably of SIRT1.

The term "anti-ageing effect" as used in the present text primarily releates to the prolongation of the life span of a cell, preferably of a human skin cell and/or a human neuronal cell, by inhibition of cellular senescence.

In particular, the term "anti-ageing effect" relates to one, several or all of the following effects and activities:

- proteasomal clearance in a cell, and/or
- stimulation or increase of proteasome activity and/or proteasome expression, and/or
- stimulation of activity and/or expression of one or more sirtuin proteins, preferably of SIRT1.

In further aspects, the term "anti-ageing effect" relates to one, several or all of the following effects and activities:

- skin anti-ageing effects, preferably an activity against wrinkles, age spots, photo-ageing, hyperpigmentation, inflammation-induced ageing and/or an uneven skin tone,
  and/or
- an activity against an age-associated disease, preferably an activity against a neurodegenerative disease, in particular Alzheimer's disease, Parkinson's disease and/or Huntington's disease,
  and/or
- an activity for increasing the insulin sensitivity,
  and/or
  an activity for the cosmetic (non-therapeutic) or therapeutic
- increase of insulin sensitivity, and/or
- reduction of proinflammatory cytokines, and/or

- prevention, treatment or reduction of oxidative stress, and/or
- induction of cellular antioxidant capacity, and/or
- stimulation of cellular energy metabolism, and/or
- stimulation of mitochondrial protein expression, and/or
- inhibition of UV-induced erythema, and/or
- inhibition of DNA-damage and/or induction of DNA-damage repair systems, and/or
- induction of cellular proliferation, and/or
- reduction and/or degradation of damaged proteins in a cell, preferably in a human skin cell and/or a human neuronal cell, and/or
- inhibition of collagen-degradation, preferably by reducing the MMP-level in the skin.

Further, the compounds of formula (I) are used in accordance with the present invention

(i) for the cosmetic prevention, treatment or reduction of skin ageing effects, in particular wrinkles, age spots, photo-ageing and inflammation-induced ageing,
and/or

(ii) as anti-ageing active, preferably as cosmetic anti-ageing active, in particular as anti-wrinkle active, as active against inflammation-induced ageing, as active against photo-ageing and/or as skin tone regulator (i.e. active leading to an even skin tone),
and/or
for the cosmetic (non-therapeutic) or therapeutic

- increase of insulin sensitivity, and/or
- reduction of proinflammatory cytokines, and/or
- prevention, treatment or reduction of oxidative stress, and/or
- induction of cellular antioxidant capacity, and/or
- stimulation of cellular energy metabolism, and/or
- stimulation of mitochondrial protein expression, and/or
- inhibition of UV-induced erythema, and/or
- inhibition of DNA-damage and/or induction of DNA-damage repair systems, and/or
- induction of cellular proliferation, and/or
- reduction and/or degradation of damaged proteins in a cell, preferably in a human skin cell and/or a human neuronal cell, and/or
- inhibition of collagen-degradation, preferably by reducing the MMP-level in the skin.

The invention therefore also relates to cosmetic or pharmaceutical preparations (compositions) comprising one or more compounds of formula (I) or a salt thereof, in an effective quantity for achieving one or more (as defined above).

The compounds of formula (I) structurally belong to the group of cyclohexyl carbamates. Various of these compounds have been described in the prior art.

The compounds according to the invention of formula (I) may exist in different isomeric forms and may be used in the context of the present invention in all their isomeric forms, i.e. - depending on their structure - as enantiomers, diastereomers, syn-/anti-isomers, cis-/trans-isomers, epimers as well as (E)-/(Z)-isomers. The compounds of formula (I) can be used in the context of the present invention in the form of the pure stereoisomeric form or in the form of any mixture of stereoisomers. The compounds of formula (I) can also be used in the context of the present invention in the form of the pure enantiomers or in the form of any mixture of enantiomers, in the latter case racemates being preferred.

As common in the art, a "flat" structural formula, i.e. a graphical formula which does not convey any stereochemical information and gives no concrete information about the three-dimensional structure thereof, relates to and

includes all stereoisomers of said structural formula. For the sake of clarity, carbamates of "flat" structural formula (I) thus include all stereoisomeric forms thereof.

As common in the art, in the context of the present invention, abbreviations for certain chemical groups are used, for example Me = methyl, Et = ethyl, Pr = propyl, Bu = butyl, Ph = phenyl.

For the sake of clarity, it is emphasized that the present invention does not relate to substances as such or mixtures of substances as such which have been described in the prior art.

Various compounds of formula (I) and more specifically several of formulae (Carb-II-R1H) and (M-X) as defined below, have been described in the literature.

Also, several compounds of formulae (I), (Carb-II) and (Carb-II-R1H) (each as defined herein) in which X, Y, and Z each denote H have been disclosed in the prior art.

Further, several compounds of formulae (I), (Carb-II), (Carb-II-R1H) and (M-X) (each as defined herein) wherein $R^2$ denotes phenyl or naphthyl have been described in the prior art.

Additionally, some bicyclic carbamates of formulae (Carb-II) and (Carb-II-R1H) as defined below wherein two of the radicals X, Y and Z are covalently bonded to one another under formation of a bicyclic ring system in which $R^2$ contains a -COOH and/or a =CH2 group have been described in the prior art.

Bioorganic & Medicinal Chemistry Letters (2005), 15(9), 2209-2213 describes

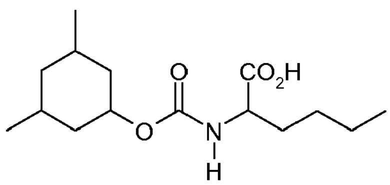 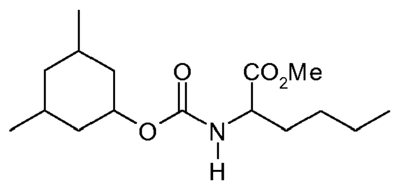

Organic Preparations and Procedures International (2004), 36(2), 141-149 discloses

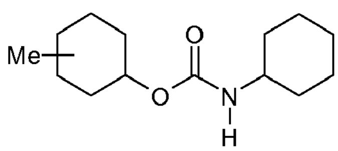, i.e. 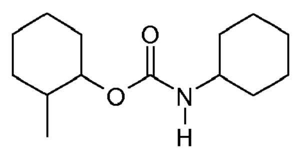

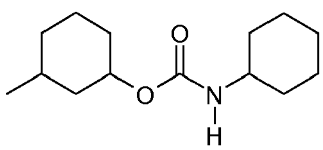 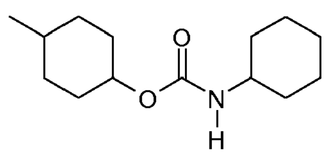

JP06-072036-A discloses

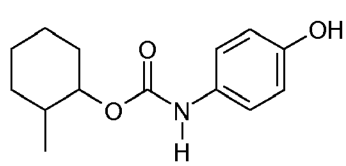

US 5,260,474 mentions

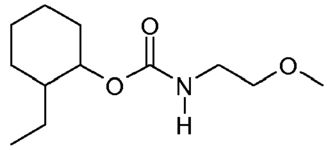

Doklady - Akademiya Nauk Azerbaidzhanskoi SSR (1980), 36(2), 63-66 describes

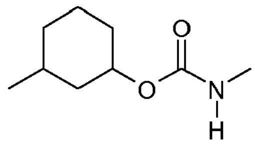

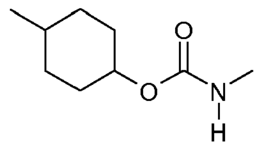

DE 20 500 87 discloses

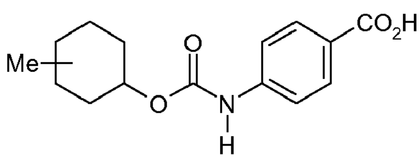

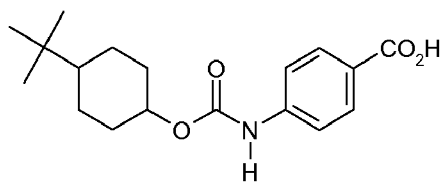

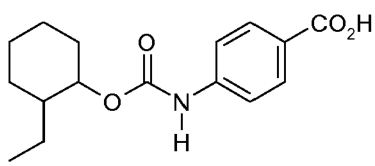

Journal of Agricultural and Food Chemistry (1967), 15(6), 1022-1029 describes

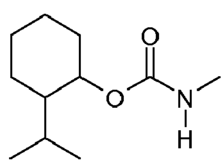

Izvestiya Akademii Nauk SSSR, Seriya Khimicheskaya (1966), (5), 922-924 discloses

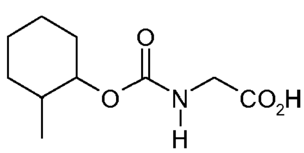

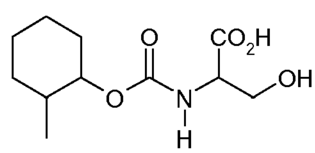

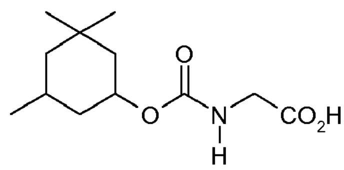

FR 1 401 219 mentions

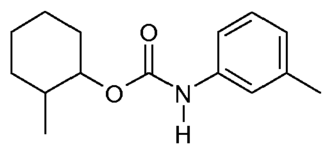

Collection of Czechoslovak Chemical Communications (1965), 30(2), 585-598 and 599-604 describe

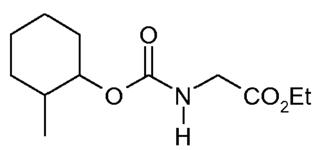

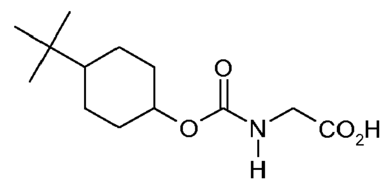

Annales Pharmaceutiques Francaises (1958), 16, 408-13 and Journal of Organic Chemistry (1958), 23, 1590-1591 disclose

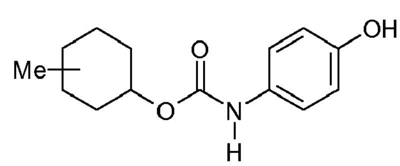

Annales Pharmaceutiques Francaises (1958), 16, 408-13 mentions

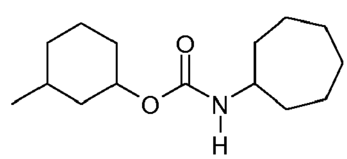

Azarbaycan Neft Tasarrufati (1933), (No. 3), 66-75 discloses

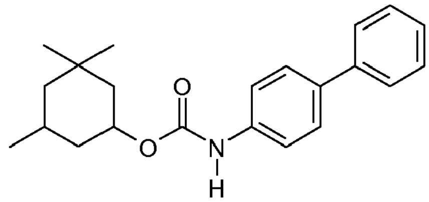

J. Org. Chem. 1981, 46, 2804-2806 discloses

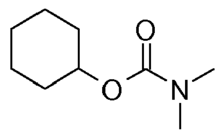

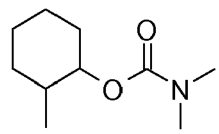

WO 2004/089880 discloses

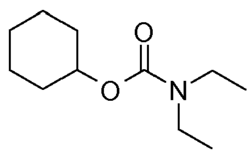

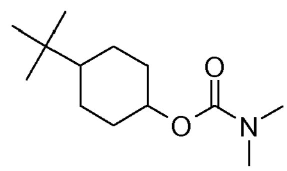

Biochemical Pharmacology 1961, 8, 179-191 describes

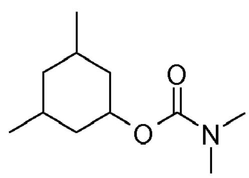

Synthetic Communications 2001, 31(24), 3759-3773 discloses

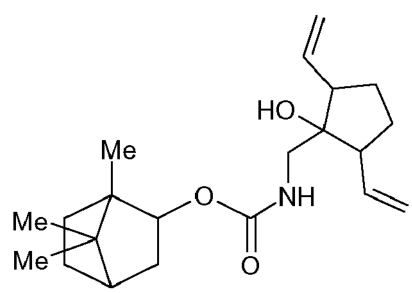

Journal of Medicinal Chemistry 1983, 26(9), 1215-18 discloses

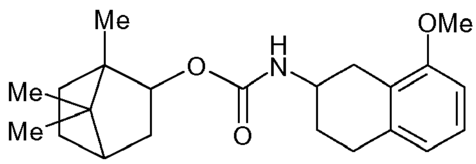

Journal of Chromatography 1982, 239, 227-31 discloses

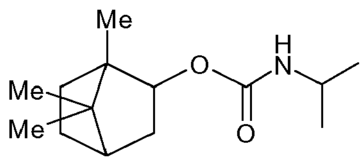

Ecotoxicology and Environmental Safety 2008, 71(3), 889-894 discloses

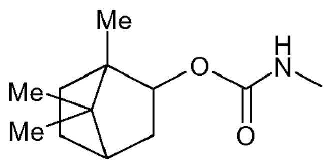

Chirality 2010, 22(2), 267-274 discloses

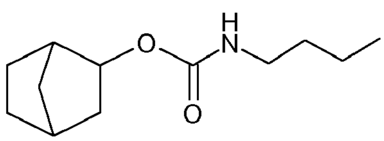

Synthesis 1989, (2), 131-132 discloses

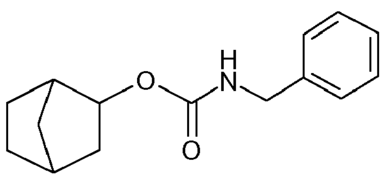

Various menthyl carbamates of formula (I) have been described in the prior art.
EP 2 135 516 discloses several neomenthyl-carbamates as umami-flavor substances, inter alia the following:

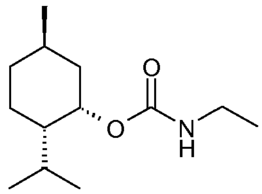

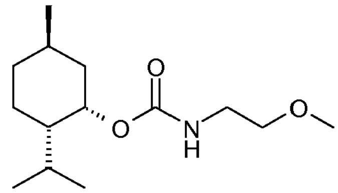

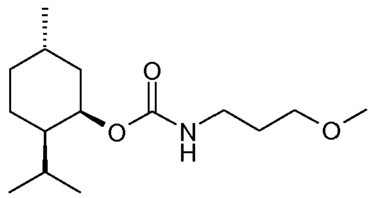

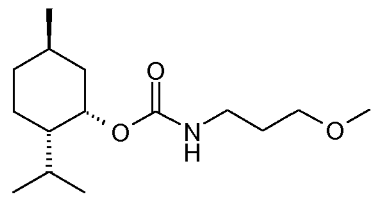

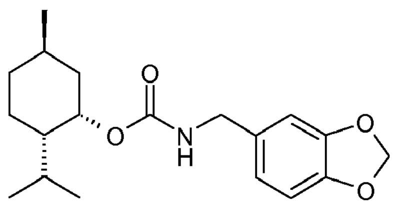

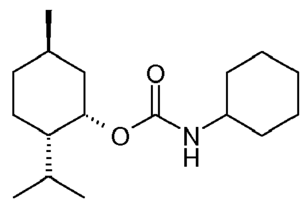

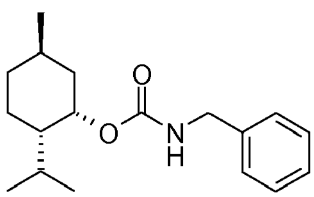

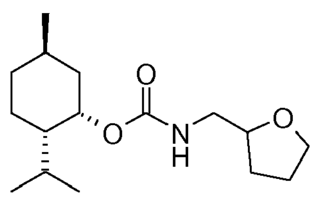

WO 2004/000023 describes the following l-menthyl-carbamates as insect repellents:

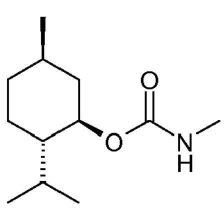

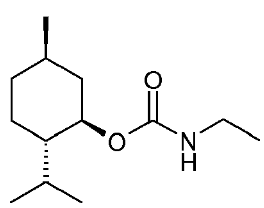

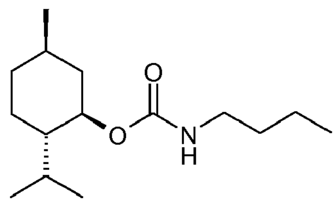

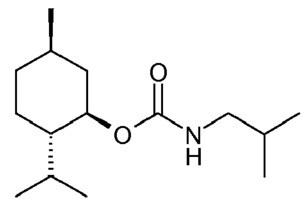

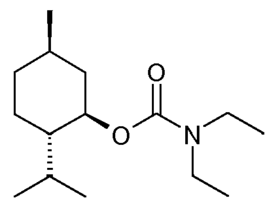

J. Org. Chem. 1999, 7921 - 7928 dislcoses N,N-diethyl (-)-(1R)-menthyl carbamate:

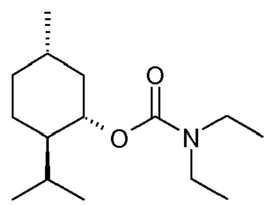

Polish Journal of Chemistry (1996), 70(3), 310-19 describes

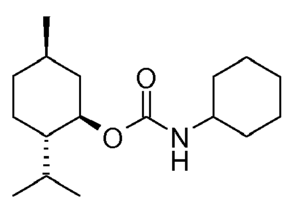

Advanced Synthesis & Catalysis (2008), 350(9), 1235-1240 and Organic & Biomolecular Chemistry (2005), 3 (15), 2741-2749 disclose benzyl-carbamic acid (1R,2S,5R) / (1S,2R,5S)-2-isopropyl-5-methyl-cyclohexyl ester, respectively

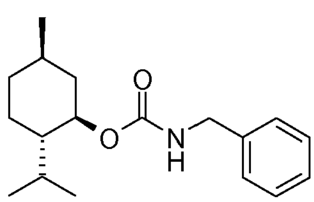

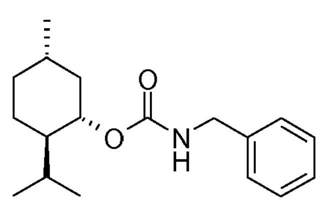

US 5,703,123 discloses the following formula which does not convey any stereochemical information:

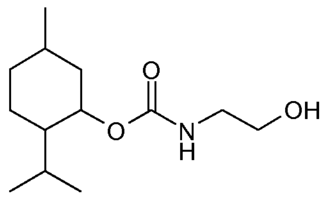

DE 1300725 discloses the following carbamate without including any stereochemical information:

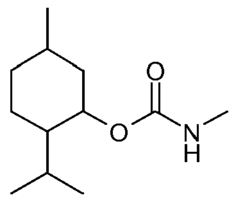

US 6,150,415 discloses the following carbamate without including any stereochemical information:

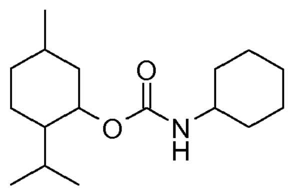

Angewandte Chemie (1982), 94(9), 709-710 describes all eight different enantiomers of isopropyl-carbamic acid 2-isopropyl-5-methyl-cyclohexyl ester:

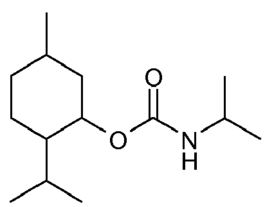

WO 2004/033422 relates to compounds inhibiting fatty acid amide hydrolase (FAAH). Methods are described therein to control appetite and treat appetite disorders by administering FAAH inhibitors, thereby reducing body fat or body weight. The only specific compounds disclosed in WO 2004/033422 of relevance in the context of the present invention are the following:

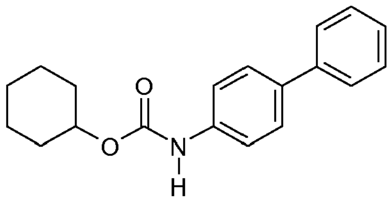
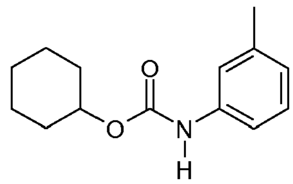
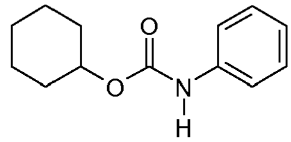

EP 1 284 145 describes the use of N-2-(3,4-dihydroxyphenyl)ethyl-substituted carbonic acid derivatives as radical scavengers and antioxidants. EP 1 284 145 further describes cosmetic preparations containing said carbonic acid derivatives. The effect of these compounds on the metabolism of fat cells or the body weight of humans was not investigated there. The only explicitly mentioned compound in EP 1 284 145 of relevance in view of formula (I) of the present invention is *N*-[2-(3,4-dihydroxyphenyl)ethyl-*O*-(1*R*,3*R*,4*S*)-menthyl]carbamate. According to EP 1 284 145, the cosmetic or dermatologic preparations described therein may additionally comprise cosmetic substances, by way of example kojic acid, hydroquinone, arbutin, hyaluronic acid, vitamin A, vitamin C, tocopherol, carnosine and UV filters may be mentioned.

In a preferred embodiment, a cosmetic or pharmaceutical preparation according to the present invention is free of *N*-[2-(3,4-dihydroxyphenyl)ethyl-*O*-(1*R*,3*R*,4*S*)-menthyl]carbamate. In another preferred embodiment, compounds of formula (I) according to the present invention, more specifically compounds of formulae (Carb-II-R1H), are excluded in which $R^2$ denotes a 2-(3,4-dihydroxyphenyl)ethyl - radical. In another preferred embodiment, cosmetic or pharmaceutical preparations according to the present invention are free of compounds of formula (I) according to the present invention, more specifically free of compounds of formula (Carb-II-R1H), in which $R^2$ denotes a 2-(3,4-dihydroxyphenyl)ethyl - radical.

WO 01/98235 describes the use of *N*-3,4-dihydroxybenzyl-substituted carbonic acid derivatives as radical scavengers and antioxidants. WO 01/98235 further describes cosmetic preparations containing said carbonic acid derivatives. The effect of these compounds on the metabolism of fat cells or the body weight of humans was not investigated there. The only explicitly mentioned compound in WO 01/98235 of relevance in view of formula (I) of the present invention is *N*-(3,4-dihydroxybenzyl)-*O*-(1*R*,3*R*,4*S*)-menthyl]carbamate. According to WO 01/98235, the cosmetic or dermatologic preparations described therein may additionally comprise cosmetic substances, by way of example kojic acid, hydroquinone, arbutin, hyaluronic acid, vitamin A, vitamin C, tocopherol, carnosine and UV filters may be mentioned.

In a preferred embodiment, a cosmetic or pharmaceutical preparation according to the present invention is free of *N*-(3,4-dihydroxybenzyl)-*O*-(1*R*,3*R*,4*S*)-menthyl]carbamate. In another preferred embodiment, compounds of formula (I) according to the present invention, more specifically compounds of formula (Carb-II-R1H), are excluded in which $R^2$ denotes a 3,4-dihydroxybenzyl - radical. In another preferred embodiment, cosmetic or pharmaceutical preparations according to the present invention are free of compounds of formula (I) according to the present invention, more specifically free of compounds of formula (Carb-II-R1H), in which $R^2$ denotes a radical containing a 3,4-dihydroxybenzyl - group.

In a preferred embodiment, compounds of formula (I) according to the present invention, more specifically of compounds of formula (Carb-II-R1H), are excluded in which $R^2$ denotes a 3,4-dihydroxyphenyl - group or a radical containing a 3,4-dihydroxyphenyl - group. In another preferred embodiment, cosmetic or pharmaceutical preparations according to the present invention are free of compounds of formula (I) according to the present invention, more specifically free of compounds of formula (Carb-II-R1H), in which $R^2$ denotes a radical containing a 3,4-dihydroxyphenyl - group.

In another preferred embodiment, compounds of formula (I) according to the present invention, more specifically compounds of formula (Carb-II-R1H), are excluded in which $R^2$ denotes a radical containing a dihydroxyphenyl - group. In another preferred embodiment, cosmetic or pharmaceutical preparations according to the present invention are free of compounds of formula (I) according to the present invention, more specifically free of compounds of formula (Carb-II-R1H), in which $R^2$ denotes a dihydroxyphenyl - group or a radical containing a dihydroxyphenyl - group.

There is no indication hitherto that the compounds used in accordance with the present invention are suitable for the cosmetic (non-therapeutic) or therapeutic prolongation of the life span of a cell, preferably of a human skin cell and/or a human neuronal cell, by inhibition of cellular senescence.

Compounds of formula (I) and preparations (compositions) according to the invention, comprising one or more compounds of formula (I) influence cellular senescence and thereby act as anti-ageing actives in the sense of one or more of the anti-ageing effects indicated above.

The term "influencing" in this context means that activation, of Route (A) and/or (B) is observed, i.e. that proteasomal clearance in a cell, stimulation of proteasome activity and/or proteasome expression activation (Route (A))

or stimulation of activity and/or expression of one or more sirtuin proteins (sirtuins) (Route (B)) is observed.

In accordance with the present invention, one or more anti-ageing effects are achieved by a preparation (composition) containing one or more compounds of formula (I) by influencing the above described Routes (A) and/or (B), preferably by influencing both above described Routes (A) and (B).

Thus, preferred compounds of formula (I) exhibit an activity in the sense of the present invention corresponding to Route (A) and Route (B).

To determine whether a compound exhibits an activity in the sense of the present invention corresponding to Route (B), it is tested whether a compound exhibits stimulation activity in a SIRT1-assay, preferably said test is performed in accordance with Example 2 given below.

To determine whether a compound exhibits an activity in the sense of the present invention corresponding to Route (A), it is tested whether a compound exhibits proteasomal stimulating activity, preferably based on the measurement of the 20S core proteasome activity. Said tests are preferably performed using isolated proteasome, preferably in addition the proteasome activity using cell lysates is determined. Preferably, said test is performed in accordance with Examples 3.1 given below, preferably in addition a test in accordance with Example 3.2 given below is performed.

In the context of the present invention, a cosmetic use or a cosmetic method is free of any therapeutic (side) effects.

In the context of the present invention, a therapeutic or pharmaceutical use or method is considered as medical treatment, optionally with cosmetic (side) effects.

The compounds according to the invention of formula (I), depending on the meaning of X, Y, Z, $R^1$ and $R^2$, may exist in different stereoisomeric forms and may be used in the context of the present invention as stereoisomers, enantiomers, diastereomers, syn-/anti-isomers, endo-/exo-isomers, cis-/trans-isomers or epimers. The compounds of formula (I) can be used in the context of the present invention in the form of the pure cis- or trans-, syn- or anti-diastereomer or in the form of any mixture of diastereomers. The compounds of formula (I) can also be used in the context of the present invention in the form of the pure enantiomers or in the form of any mixture of enantiomers, in the latter case racemates being preferred.

In case $R^1$ does not denote hydrogen, $R^1$ and $R^2$ independently of one another preferably denote an optionally substituted radical selected from the group consisting of alkyl, heteroalkyl, cycloalkyl, cycloalkylalkyl, alkenyl, cycloalkenyl, cycloalkenylalkyl, alkynyl, cycloalkylalkynyl, aryl, heteroaryl, arylalkyl, cycloalkylaryl, cycloalkenylaryl, cycloalkylheteroaryl, heterocycloalkylaryl, heterocycloalkenylaryl, heterocycloalkenylheteroaryl and heteroarylalkyl.

In case $R^1$ does not denote hydrogen, $R^1$ and $R^2$ independently of one another more preferably denote an optionally substituted radical $C_1$-$C_{14}$-alkyl, $C_1$-$C_{14}$-heteroalkyl, $C_3$-$C_{14}$-cycloalkyl, $C_4$-$C_{14}$-cycloalkylalkyl, $C_2$-$C_{14}$-alkenyl, $C_3$-$C_{14}$-cycloalkenyl, $C_4$-$C_{14}$-cycloalkenylalkyl, $C_2$-$C_{14}$-alkynyl, $C_5$-$C_{14}$-cycloalkylalkynyl, $C_3$-$C_{14}$-aryl, $C_2$-$C_{14}$-heteroaryl, $C_4$-$C_{14}$-arylalkyl, $C_8$-$C_{14}$-cycloalkylaryl, $C_8$-$C_{14}$-cycloalkenylaryl, $C_5$-$C_{14}$-cycloalkylheteroaryl, $C_8$-$C_{14}$-heterocycloalkylaryl, $C_8$-$C_{14}$-heterocycloalkenylaryl, $C_8$-$C_{14}$-heterocycloalkenylheteroaryl and $C_3$-$C_{14}$-heteroarylalkyl.

Heteroalkyl, heteroaryl, cycloalkylheteroaryl, heterocycloalkylaryl, heterocycloalkenylaryl, heterocycloalkenylheteroaryl and heteroarylalkyl radicals in the context of the present invention preferably contain at least one heteroatom, optionally up to four heteroatoms, selected independently from the group consisting of O, S and/or N. Preferred are heteroalkyl, heteroaryl, cycloalkylheteroaryl, heterocycloalkylaryl, heterocycloalkenylaryl, heterocycloalkenylheteroaryl and heteroarylalkyl radicals containing one, two or three heteroatoms, selected independently from the group consisting of O, S and/or N.

Preferably, substituents X, Y, and Z in each case occupy any desired position in the cyclohexyl ring in ortho, meta or para position to the cyclohexyl-carbon atom bonded to the oxygen of the carbamate group. Thus, preferably A denotes

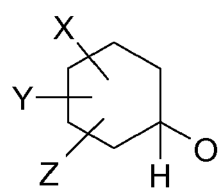

,

wherein X, Y and Z have the meaning indicated hereinbefore or hereinafter.

The corresponding preferred compounds of formula (I) are cyclohexyl carbamates of formula (Carb-II):

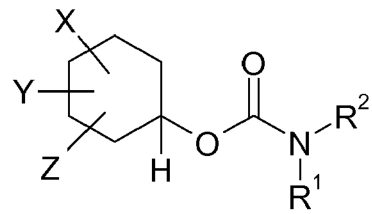

(Carb-II)

wherein $R^1$, $R^2$ and X, Y and Z have the meaning indicated hereinbefore or hereinafter.

Substituents X, Y and Z independently of one another preferably denote hydrogen, methyl, ethyl, n-propyl, isopropyl, n-butyl, sec.-butyl, isobutyl, tert.-butyl, ethenyl, prop-2-en-1-yl, prop-1-en-1-yl, prop-1-en-2-yl, but-1-en-1-yl, but-1-en-2-yl, but-1-en-3-yl, but-2-en-1-yl, but-3-en-1-yl, but-2-en-2-yl, 2-methylprop-1-en-1-yl, 2-methylprop-2-en-1-yl.

In a preferred embodiment, substituents X, Y and Z independently of one another denote hydrogen or C1-C4-alkyl. In another preferred embodiment, at least one of the substituents X, Y and Z denotes C1-C4-alkyl, i.e. at least one of the substituents X, Y and Z does not denote hydrogen.

In another preferred embodiment, two of the substituents X, Y and Z independently of one another denote hydrogen or C1-C4-alkyl and at least one of the substituents X, Y and Z denotes C1-C4-alkyl.

In one embodiment, in preferred compounds of formulae (I), (Carb-I) and (Carb-II) $R^1$ denotes hydrogen. In our investigations, these compounds were generally found to have good to excellent activity and efficacy regarding the anti-ageing effects described herein.

Thus, in one embodiment, more preferred compounds of formula (I) are cyclohexyl carbamates of formula (Carb-II-R1H):

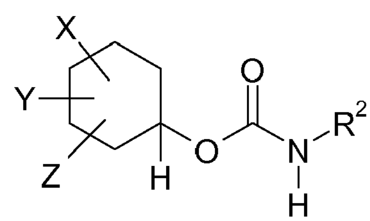

(Carb-II-R1H)

wherein X, Y and Z have the meaning indicated hereinbefore or hereinafter.

In preferred compounds of formulae (I), (Carb-I), (Carb-II) and (Carb-II-R1H) (as defined below), $R^2$ denotes an organic radical having 1 to 12 carbon atoms, preferably an organic radical having 1 to 10 carbon atoms, more preferably an organic radical having 1 to 8 carbon atoms.

In more preferred compounds of formulae (I), (Carb-I), (Carb-II) and (Carb-II-R1H), $R^2$ denotes an optionally substituted radical $C_1$-$C_{10}$-alkyl, $C_1$-$C_{10}$-heteroalkyl, $C_3$-$C_{10}$-cycloalkyl, $C_4$-$C_{10}$-cydoalkylalkyl, $C_2$-$C_{10}$-alkenyl, $C_3$-$C_{10}$-cycloalkenyl, $C_4$-$C_{10}$-cycloalkenylalkyl, $C_2$-$C_{10}$-alkynyl, $C_5$-$C_{10}$-cycloalkylalkynyl, $C_3$-$C_{10}$-aryl, $C_2$-$C_{10}$-heteroaryl, $C_4$-$C_{10}$-arylalkyl, $C_8$-$C_{10}$-cycloalkylaryl, $C_8$-$C_{10}$-cycloalkenylaryl, $C_5$-$C_{10}$-cycloalkylheteroaryl, $C_8$-$C_{10}$-heterocycloalkylaryl, $C_8$-$C_{10}$-heterocycloalkenylaryl, $C_8$-$C_{10}$-heterocycloalkenylheteroaryl and $C_3$-$C_{10}$-heteroarylalkyl.

In most preferred compounds of formulae (I), (Carb-I), (Carb-II) and (Carb-II-R1H), $R^2$ denotes an optionally substituted radical chosen from the group consisting of $C_1$-$C_8$-alkyl, $C_3$-$C_8$-cycloalkyl $C_4$-$C_{12}$-cycloalkylalkyl, $C_2$-$C_8$-alkenyl, $C_3$-$C_8$-cycloalkenyl, $C_4$-$C_8$-cycloalkenylalkyl, $C_3$-$C_8$-aryl, $C_2$-$C_8$-heteroaryl, $C_4$-$C_8$-arylalkyl, $C_5$-$C_8$-cycloalkylheteroaryl and $C_4$-$C_8$-heteroarylalkyl.

If the radicals $R^1$ and/or $R^2$ are substituted, $R^1$ and/or $R^2$ each may contain one or more heteroatoms, preferably independently selected from the group consisting of O, S, N, Si and F. If the heteroatoms are selected from the group consisting of O, S and N, the radicals $R^1$ and/or $R^2$ each preferably contain one, two or three heteroatoms selected independently from the group consisting of O, S and/or N.

If the radicals $R^1$ and/or $R^2$ are substituted the following substituents are preferred:

hydroxyl,

fluoride,

$C_1$-$C_8$-alkyl, preferably methyl, ethyl, n-propyl, iso-propyl, n-butyl, iso-butyl, tert- butyl,

$C_3$-$C_{12}$-cycloalkyl, preferably cyclopropyl, cyclopentyl, cyclohexyl, cyclooctyl, cyclododecyl,

$C_2$-$C_8$-alkynyl, preferably ethynyl, propynyl,

$C_1$-$C_8$-perfluoroalkyl, preferably trifluoromethyl, nonafluorobutyl,

$C_1$-$C_8$-alkoxy, preferably methoxy, ethoxy, n-propoxy, iso-propoxy, n-butoxy, iso-butoxy, tert-butoxy,

$C_3$-$C_8$-cycloalkoxy, preferably $C_3$-cycloalkoxy, $C_5$-cycloalkoxy, $C_6$-cycloalkoxy, $C_8$-cycloalkoxy,

$C_1$-$C_{10}$-alkoxyalkyl, in which 1 to 3 $CH_2$ groups are replaced by oxygen, preferably $-[-O-CH_2-CH_2-]_v-Q$ or $-[-O-CH_2-CHMe-]_v-Q$, wherein Q is OH or $CH_3$ and wherein v denotes an integer from 1 to 3,

$C_1$-$C_4$-acyl, preferably acetyl,

$C_1$-$C_4$-acetal, preferably dimethylacetal, diethylacetal or a methylenedioxy group $-O-CH_2-O-$.

$C_1$-$C_4$-carboxyl, preferably $CO_2Me$, $CO_2Et$, $CO_2$iso-Pr, $CO_2$tert-Bu,

$C_1$-$C_4$-acyloxy, preferably acetyloxy,

$Si_1$-$Si_{10}$-silyl, and

$Si_1$-$Si_{30}$-siloxy or polysiloxy.

Preferred cosmetically or pharmaceutically acceptable salts of compounds of formula (I) are those in which the one or more counterions (counteracting cation) is selected from the group consisting of $Na^+$, $K^+$, $NH_4^+$, trialkylammonium $NHR^i{}_3^+$, $Ca^{2+}$, $Mg^{2+}$, $Zn^{2+}$ and $Al^{3+}$.

In trialkylammonium $NHR^i{}_3^+$, preferably each $R^i$ independently of the other radicals $R^i$ denotes an alkyl group having 1 to 30 C-atoms, preferably having 4 to 22 C-atoms.

Particular preferred counterions are $Na^+$, $K^+$, $NH_4^+$, $Ca^{2+}$ and/or $Mg^{2+}$.

In case two different compounds of formula (I) are used as a mixture, generally the ratio by weight of the two compounds is chosen in the range of from 10 : 1 to 1 : 10, preferably in the range of from 5 : 1 to 1 : 5, more preferably in the range of from 3 : 1 to 1 : 3, the counterion, if present, not being included in the case of salts.

In the context of the present invention, a wavy line in structural formulae means that the double bond can be in the (E) or (Z) configuration.

Further, preferred compounds or a cosmetically acceptable salt thereof are those of formula (Carb-II)

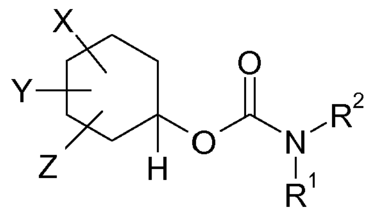

(Carb-II)

wherein
$R^1$ denotes H, C1-C8-alkyl or C2-C8-alkenyl,
$R^2$ denotes a radical having 1 to 14 carbon atoms, wherein $R^2$ consists of carbon, hydrogen and optionally oxygen and optionally silicon,
wherein optionally $R^1$ and $R^2$ are covalently bonded to one another such that $NR^1R^2$ together denote a radical, selected from the group consisting of

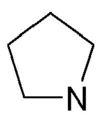 , 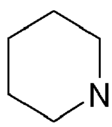 , 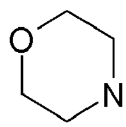 and 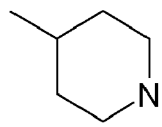 ,

X, Y and Z independently of one another denote hydrogen, C1-C4-alkyl or C2-C4-alkenyl,
wherein optionally two of the radicals X, Y and Z are covalently bonded to one another under formation of a bicyclic ring system, in such a bicyclic ring system two of the radicals X, Y and Z together preferably form a radical having 1 to 4 carbon atoms, preferably a hydrocarbon radical having 1 to 3 carbon atoms,
wherein the compound of formula (Carb-II) contains a maximum number of 24 carbon atoms and has a molecular weight of at most 500 g/mol, preferably a molecular weight of at most 450 g/mol.

Preferred compounds of formulae (I), (Carb-I), (Carb-II) and (Carb-II-R1H) are those in which A denotes a radical chosen from the following list "**CyO**":

| | | | |
|---|---|---|---|
| 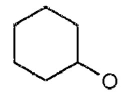 | 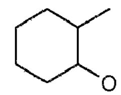 | 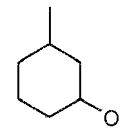 | 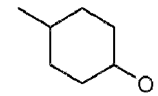 |
| AA | AB | AC | AD |
| 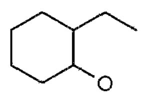 | 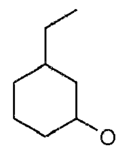 | 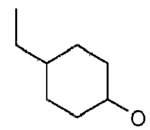 | 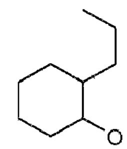 |
| AE | AF | AG | AH |
| 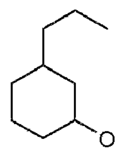 | 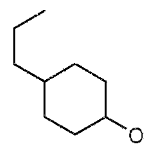 | 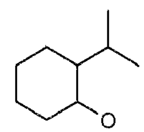 | 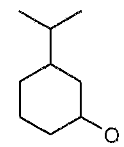 |
| AI | AJ | AK | AL |
| 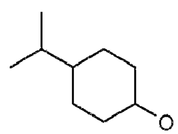 | 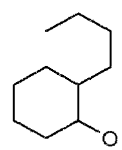 | 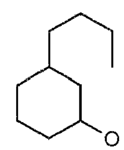 | 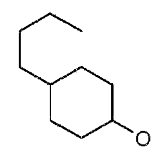 |
| AM | AN | AO | AP |
| 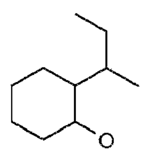 | 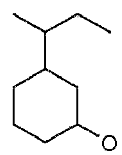 | 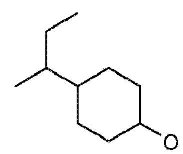 | 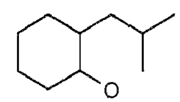 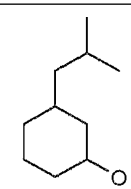 |
| AQ | AR | AS | AT AU |

(continued)

| | | | | |
|---|---|---|---|---|
| 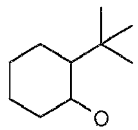 | 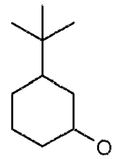 | 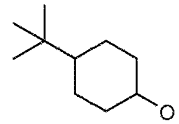 | 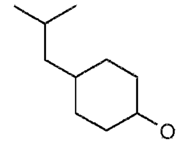 | |
| AV | AW | AX | AY | |
| 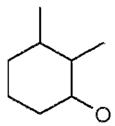 | 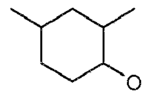 | 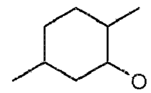 | 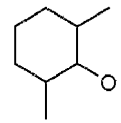 | 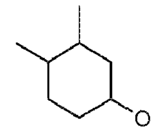 |
| AZ | BA | BB | BC | BD |
| 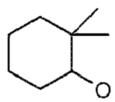 | 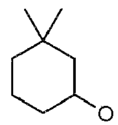 | 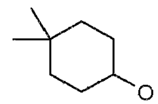 | | 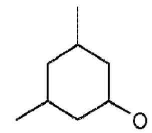 |
| BE | BF | BG | | BH |
| 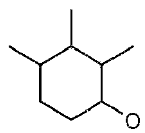 | 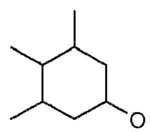 | 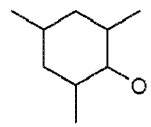 | 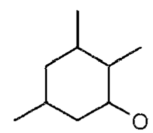 | 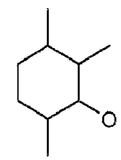 |
| BI | BJ | BK | BL | BM |
| 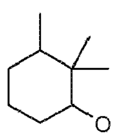 | 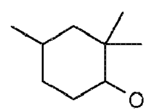 | 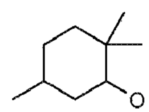 | 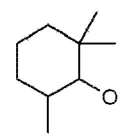 | 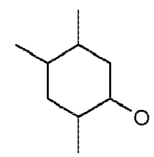 |
| BN | BO | BP | BQ | BR |

(continued)

| | | | |
|---|---|---|---|
| 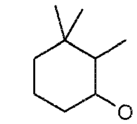 | 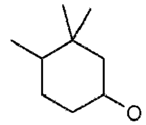 | 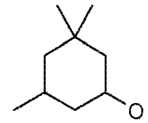 | 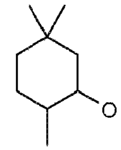 |
| BS | BT | BU | BV |
| 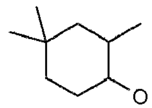 | 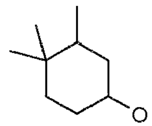 | 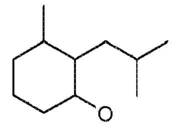 | 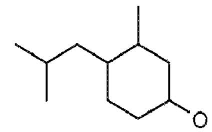 |
| BW | BX | BY | BZ |
| 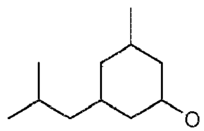 | 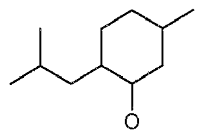 | 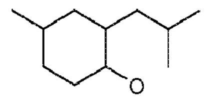 | 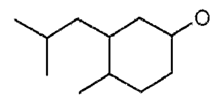 |
| CA | CB | CC | CD |
| 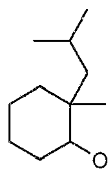 | 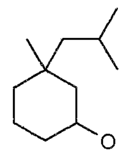 | 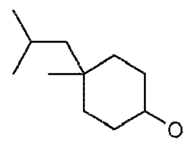 | |
| CE | CF | CG | |
| 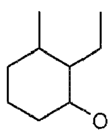 | 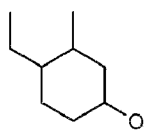 | 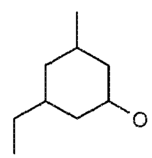 | 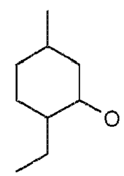 |
| CH | CI | CJ | CK |

(continued)

| | | | |
|---|---|---|---|
| 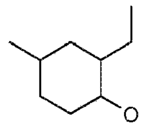 | 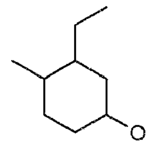 | 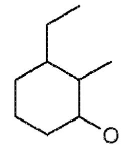 | 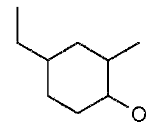 |
| CL | CM | CN | CO |
| 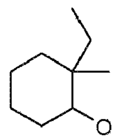 | 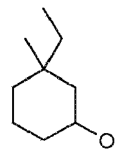 | 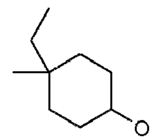 | |
| CP | CQ | CR | |
| 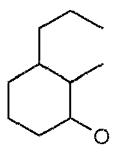 | 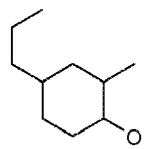 | 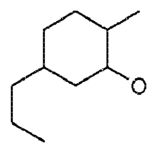 | 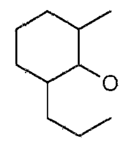 |
| cs | CT | CU | CV |
| 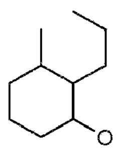 | 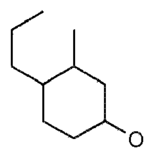 | 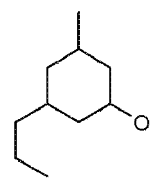 | 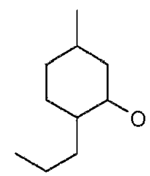 |
| CW | CX | CY | CZ |
| 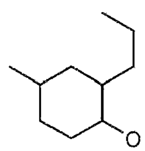 | 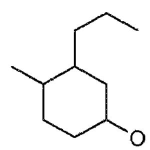 | 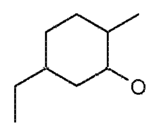 | 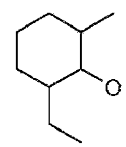 |
| DA | DB | DC | DD |

(continued)

| | | | |
|---|---|---|---|
| 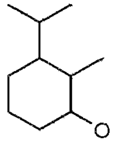 | 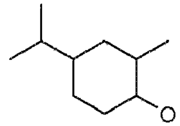 | 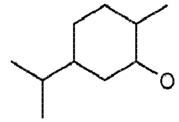 | 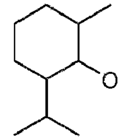 |
| DE | DF | DG | DH |
| 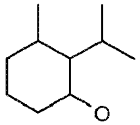 | 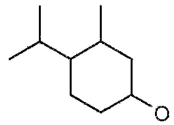 | 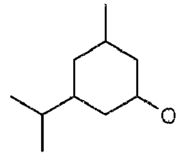 | |
| DI | DJ | DK | |
| 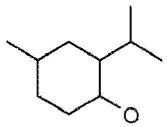 | 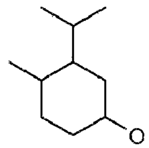 | | |
| DL | DM | | |
| 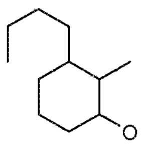 | 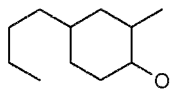 | 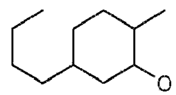 | 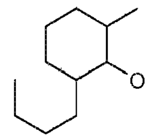 |
| DN | DO | DP | DQ |
| 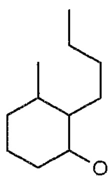 | 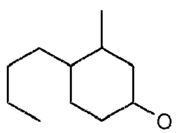 | 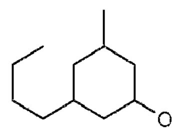 | 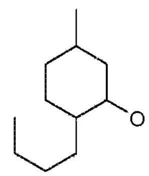 |
| DR | DS | DT | DU |

(continued)

| | | | |
|---|---|---|---|
| 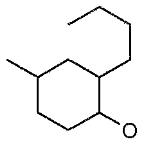 | 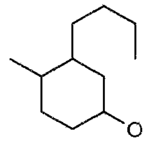 | | |
| DV | DW | | |
| 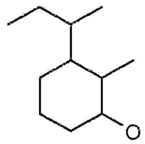 | 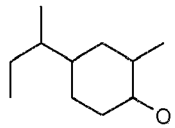 | 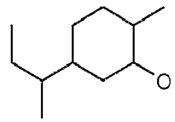 | 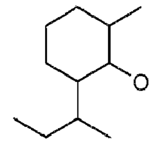 |
| DX | DY | DZ | EA |
| 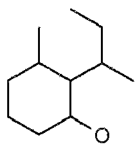 | 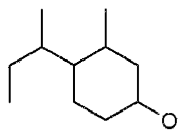 | 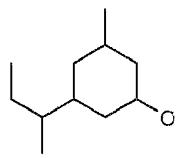 | 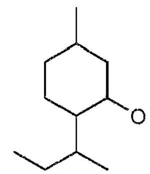 |
| EB | EC | ED | EE |
| 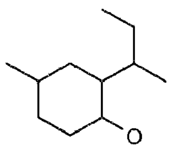 | 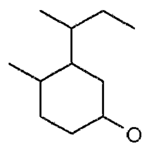 | | |
| EF | EG | | |
| 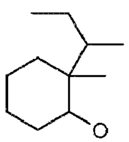 | 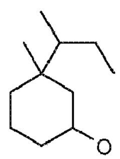 | 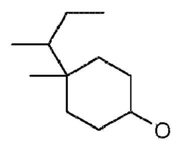 | 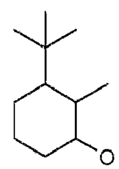 |
| EH | EI | EJ | EK |

(continued)

| | | | |
|---|---|---|---|
| 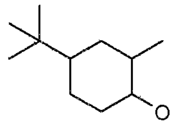 | 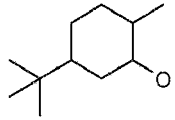 | 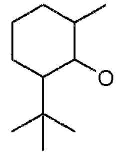 | 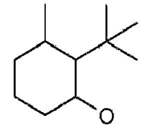 |
| EL | EM | EN | EO |
| 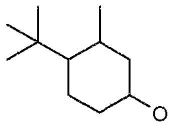 | 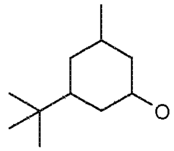 | 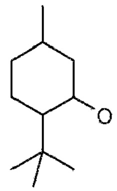 | 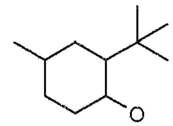 |
| EP | EQ | ER | ES |
| 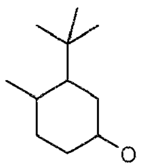 | 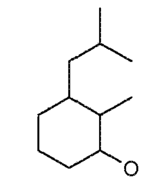 | 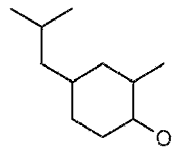 | 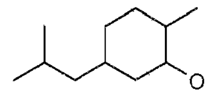 |
| ET | EU | EV | EW |
| 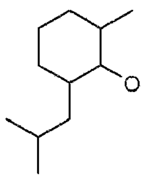 | 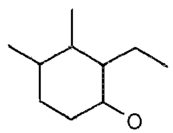 | 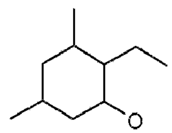 | |
| EX | EY | EZ | |
| 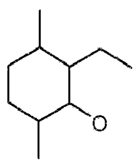 | 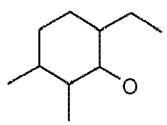 | 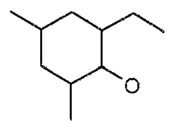 | 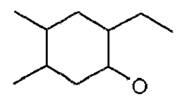 |

(continued)

| FA | FB | FC | FD |
|---|---|---|---|
| 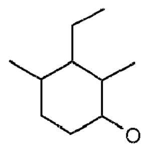 | 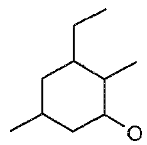 | 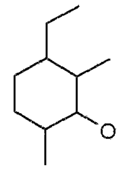 | 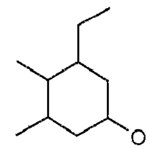 |
| FE | FF | FG | FH |
| 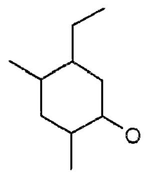 | | | 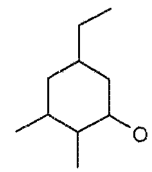 |
| FI | | | FJ |
| 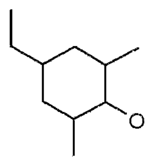 | 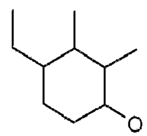 | 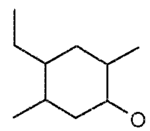 | 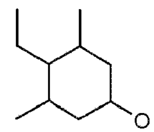 |
| FK | FL | FM | FN |
| 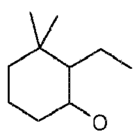 | 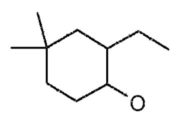 | 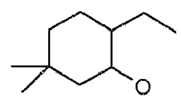 | 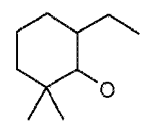 |
| FO | FP | FQ | FR |
| 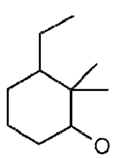 | 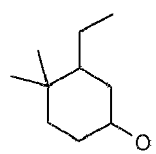 | 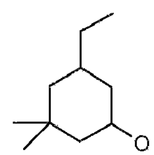 | 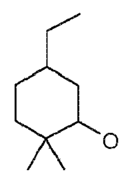 |
| FS | FT | FU | FV |

(continued)

| | | | |
|---|---|---|---|
| 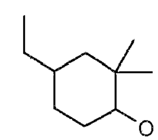 | 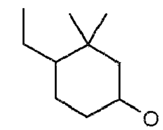 | | |
| FW | FX | | |
| 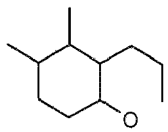 | 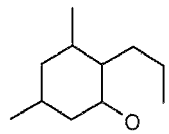 | 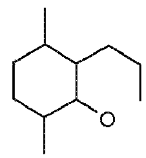 | 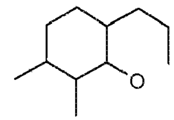 |
| FY | FZ | GA | GB |
| 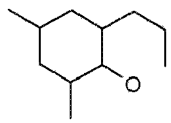 | | | 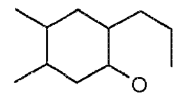 |
| GC | | | GD |
| 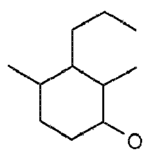 | 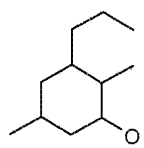 | 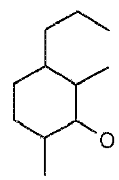 | 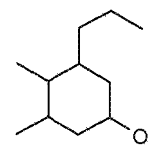 |
| GE | GF | GG | GH |
| 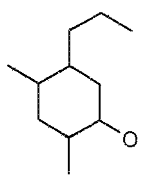 | | | 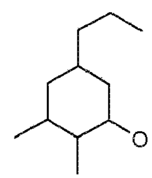 |
| GI | | | GJ |

(continued)

| | | | |
|---|---|---|---|
| 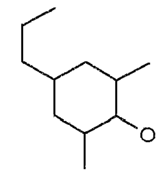 | 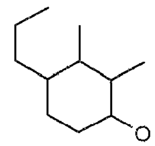 | 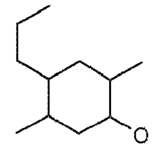 | 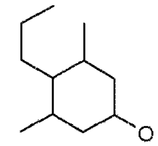 |
| GK | GL | GM | GN |
| 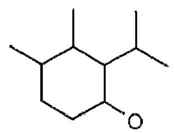 | 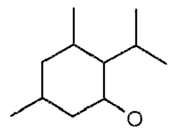 | 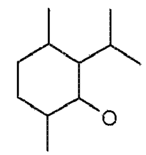 | 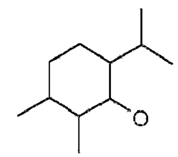 |
| GO | GP | GQ | GR |
| 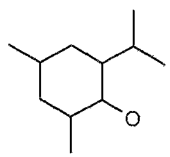 | | | 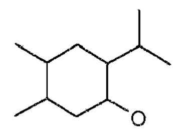 |
| GS | | | GT |
| 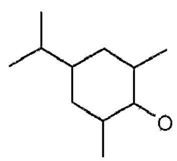 | 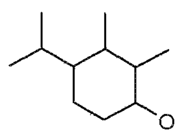 | 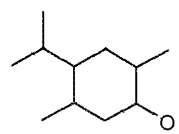 | 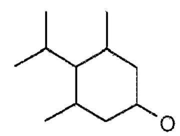 |
| GU | GV | GW | GX |
| 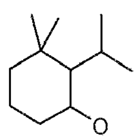 | 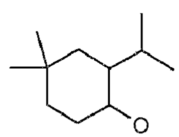 | 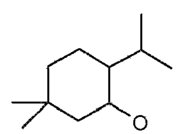 | 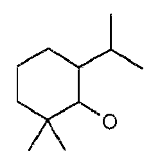 |
| GY | GZ | HA | HB |

(continued)

| | | | |
|---|---|---|---|
| 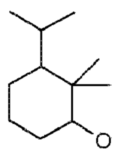 | 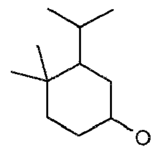 | 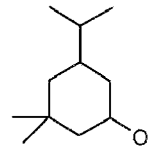 | 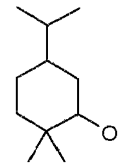 |
| HC | HD | HE | HF |
| 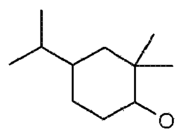 | 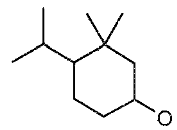 | | |
| HG | HH | | |
| 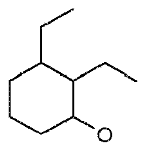 | 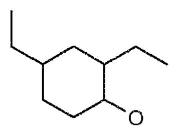 | 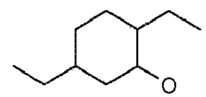 | 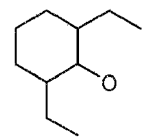 |
| HI | HJ | HK | HL |
| 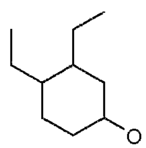 | 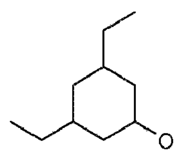 | 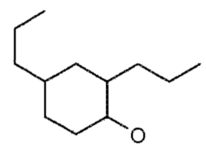 | 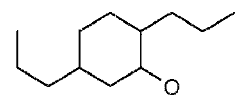 |
| HM | HN | HO | HP |
| 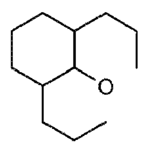 | 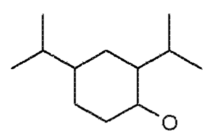 | 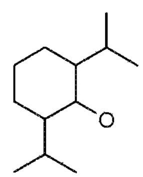 | |
| HQ | HR | HS | |

(continued)

| | | | |
|---|---|---|---|
| | 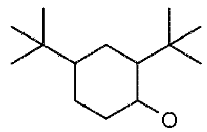 | 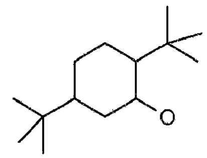 | 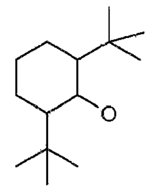 |
| | HT | HU | HV |
| 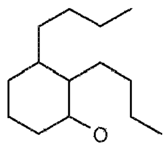 | 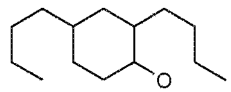 | 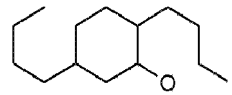 | |
| HW | HX | HY | |
| 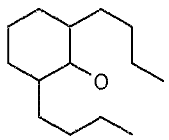 | 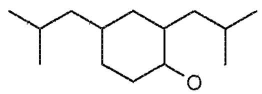 | 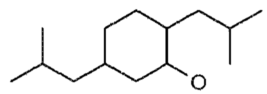 | |
| HZ | IA | IB | |
| 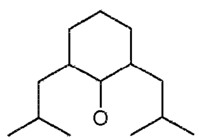 | 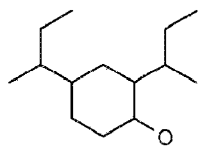 | 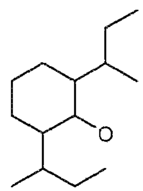 | |
| IC | ID | IE | |
| 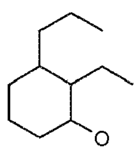 | 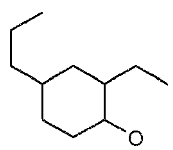 | 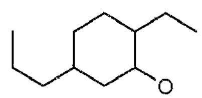 | 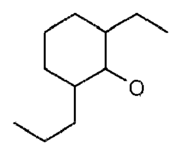 |
| IF | IG | IH | II |

(continued)

| | | | |
|---|---|---|---|
| 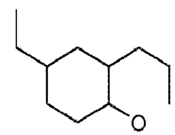 | 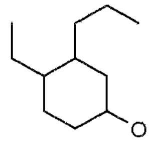 | | |
| IJ | IK | | |
| 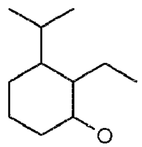 | 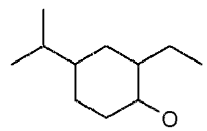 | 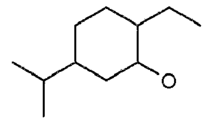 | 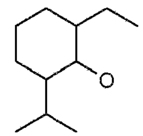 |
| IL | IM | IN | IO |
| | 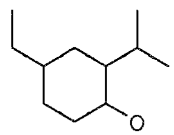 | 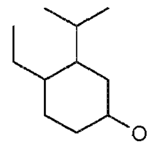 | |
| | IP | IQ | |
| 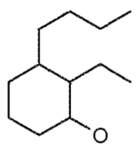 | 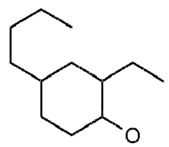 | 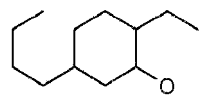 | 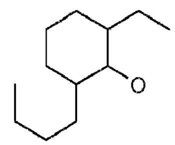 |
| IR | IS | IT | IU |
| 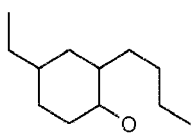 | 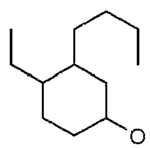 | 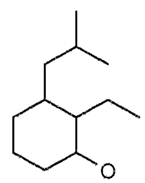 | 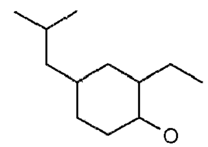 |
| IV | IW | IX | IY |

(continued)

| | | | |
|---|---|---|---|
| 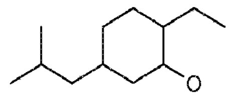 | 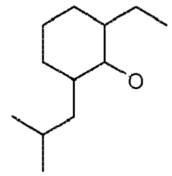 | 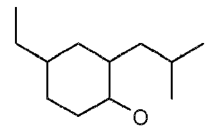 | 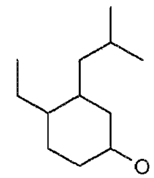 |
| IZ | JA | JB | JC |
| 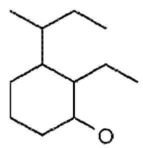 | 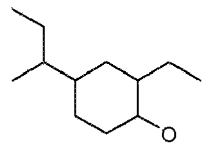 | 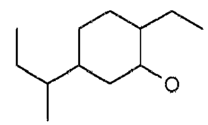 | 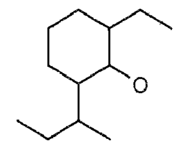 |
| JD | JE | JF | JG |
| 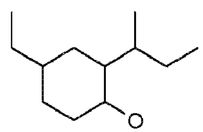 | 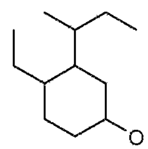 | | |
| JH | JI | | |
| | 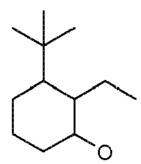 | 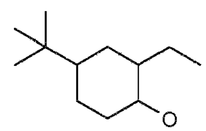 | 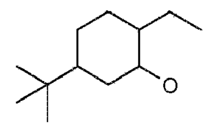 |
| | JJ | JK | JL |
| 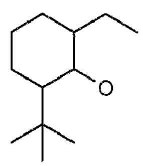 | 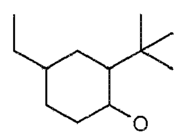 | 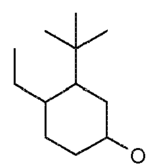 | |
| JM | JN | JO | |

(continued)

| | | | |
|---|---|---|---|
| 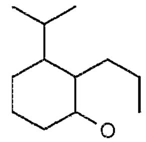 | 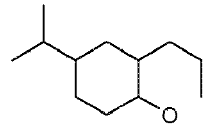 | 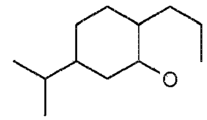 | 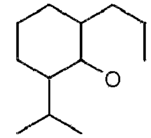 |
| JP | JQ | JR | JS |
| 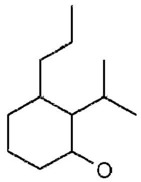 | 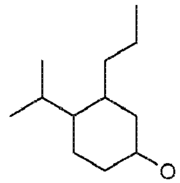 | 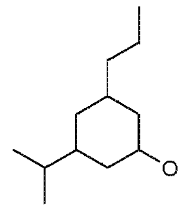 | 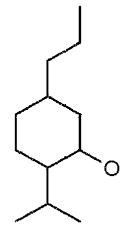 |
| JT | JU | JV | JW |
| 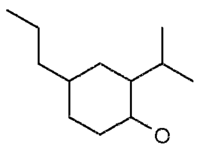 | 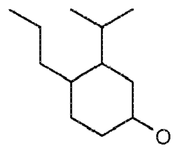 | | |
| JX | JY | | |
| 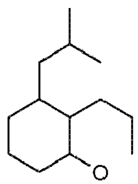 | 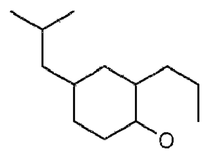 | 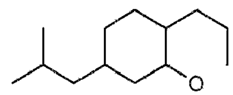 | 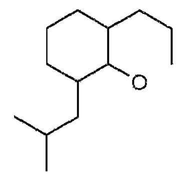 |
| JZ | KA | KB | KC |

(continued)

| | | | |
|---|---|---|---|
| 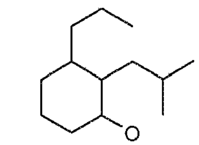 | 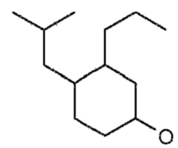 | 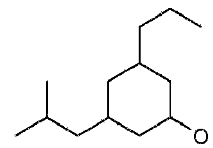 | 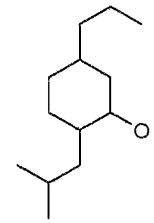 |
| KD | KE | KF | KG |
| 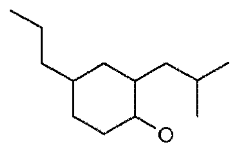 | 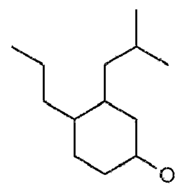 | 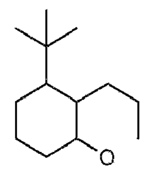 | 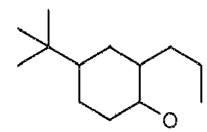 |
| KH | KI | KJ | KK |
| 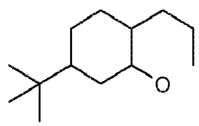 | 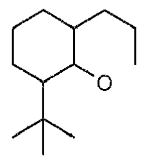 | 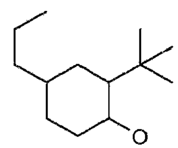 | 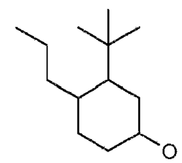 |
| KL | KM | KN | KO |
| 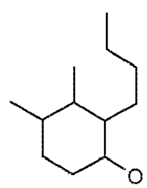 | 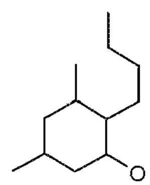 | 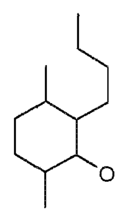 | 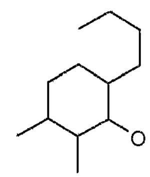 |
| KP | KQ | KR | KS |

(continued)

| | | | |
|---|---|---|---|
| 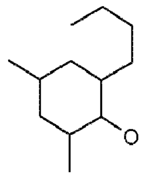 | 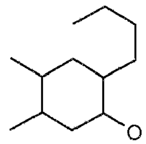 | | |
| KT | KU | | |
| 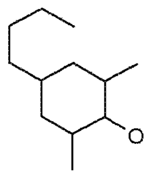 | 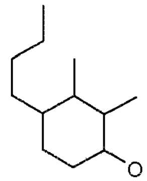 | 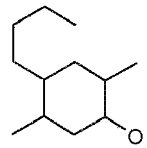 | 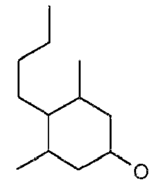 |
| KV | KW | KX | KY |
| 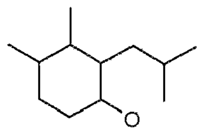 | 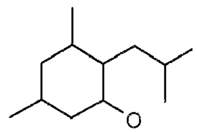 | 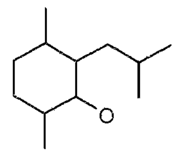 | 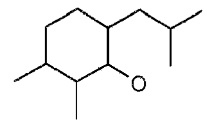 |
| KZ | LA | LB | LC |
| 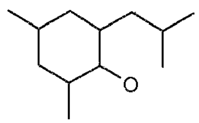 | 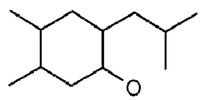 | 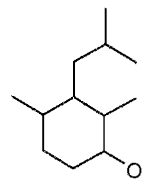 | 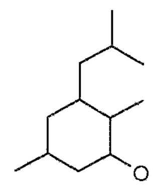 |
| LD | LE | LF | LG |

(continued)

| | | | |
|---|---|---|---|
| 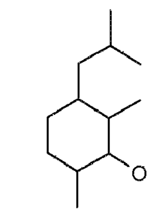 | 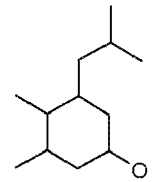 | 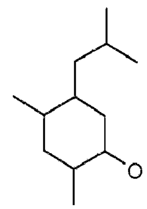 | 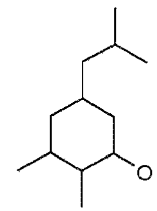 |
| LH | LI | LJ | LK |
| 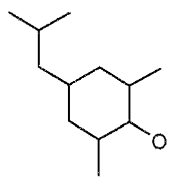 | 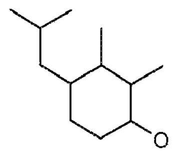 | 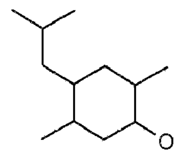 | 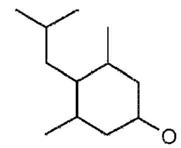 |
| LL | LM | LN | LO |
| 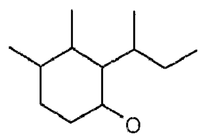 | 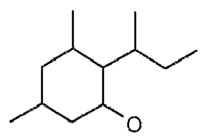 | 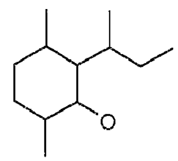 | 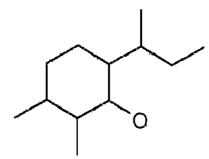 |
| LP | LQ | LR | LS |
| 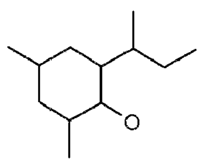 | 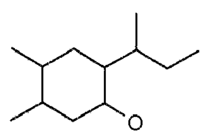 | 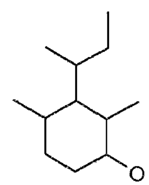 | 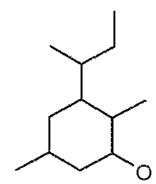 |
| LT | LU | LV | LW |

(continued)

| | | | |
|---|---|---|---|
| 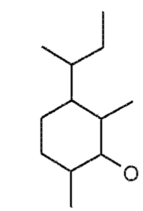 | 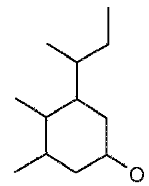 | 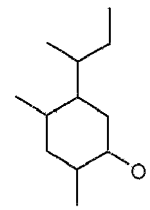 | 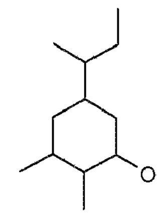 |
| LX | LY | LZ | MA |
| 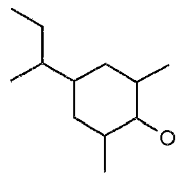 | 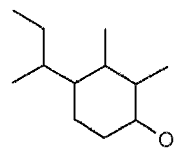 | 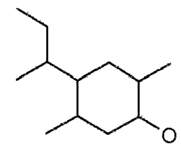 | 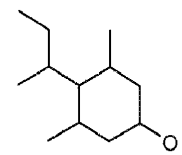 |
| MB | MC | MD | ME |
| 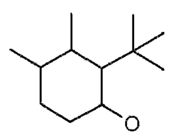 | 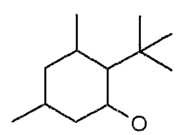 | 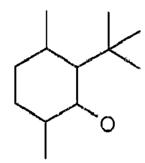 | 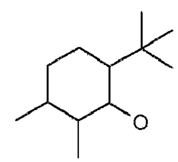 |
| MF | MG | MH | MI |
| 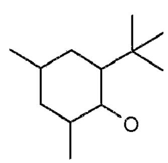 | 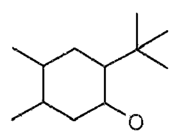 | 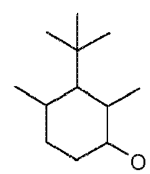 | 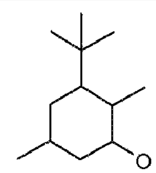 |
| MJ | MK | ML | MM |

(continued)

| | | | |
|---|---|---|---|
| 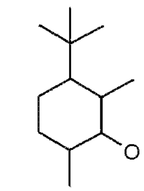 | 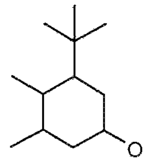 | 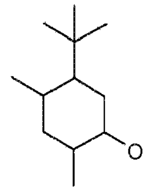 | 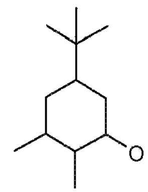 |
| MN | MO | MP | MQ |
| 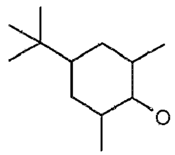 | 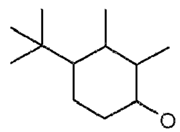 | 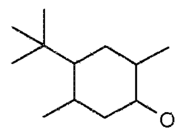 | 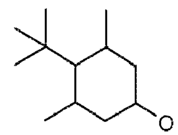 |
| MR | MS | MT | MU |
| 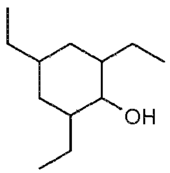 | 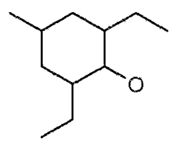 | 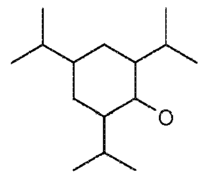 | 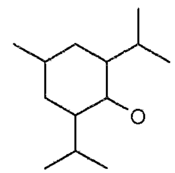 |
| MV | MW | MX | MY |
| 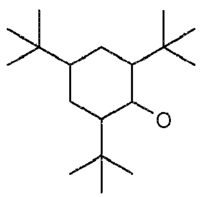 | 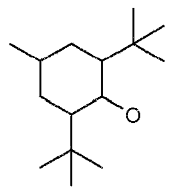 | | |
| MZ | NA | | |
| 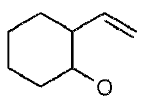 | 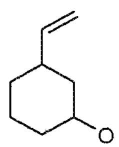 | 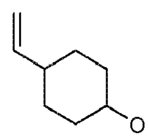 | |

(continued)

| | | | |
|---|---|---|---|
| NB | NC | ND | |
| 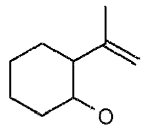 | 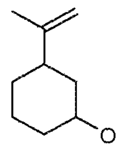 | 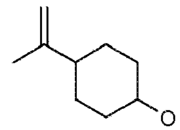 | |
| NE | NF | NG | |
| 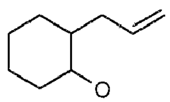 | 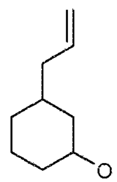 | 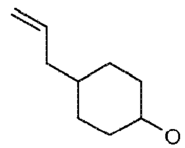 | 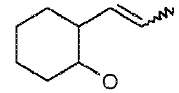 |
| NH | NI | NJ | NK |
| 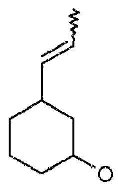 | 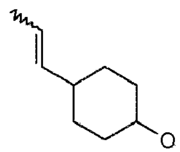 | | |
| NL | NM | | |
| 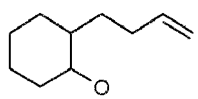 | 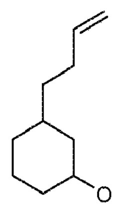 | 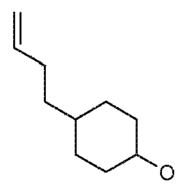 | 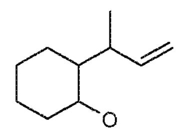 |
| NN | NO | NP | NQ |

(continued)

| | | | |
|---|---|---|---|
| 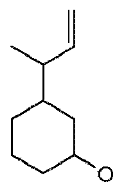 | 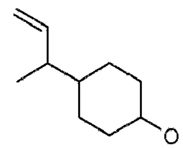 | 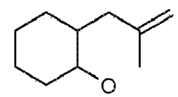 | 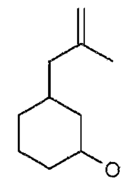 |
| NR | NS | NT | NU |
| 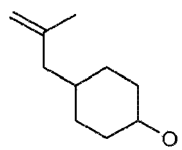 | 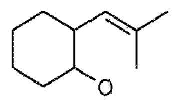 | 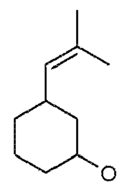 | 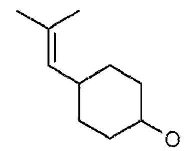 |
| NV | NW | NX | NY |
| 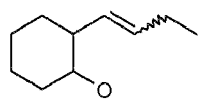 | 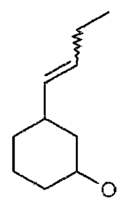 | 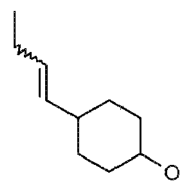 | |
| NZ | OA | OB | |
| 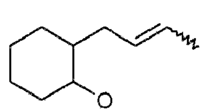 | 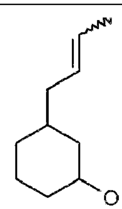 | 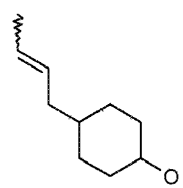 | |
| OC | OD | OE | |

(continued)

| | | | |
|---|---|---|---|
| 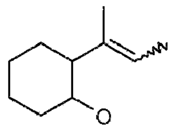 | 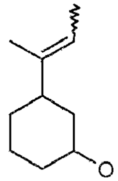 | 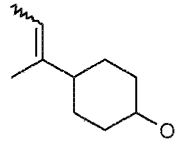 | |
| OF | OG | OH | |
| 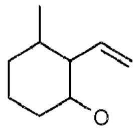 | 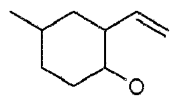 | 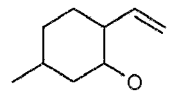 | 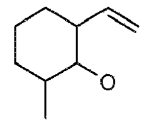 |
| OI | OJ | OK | OL |
| 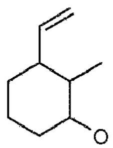 | 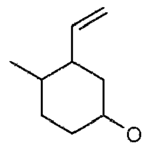 | 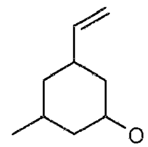 | 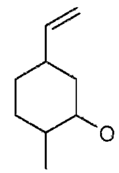 |
| OM | ON | OO | OP |
| 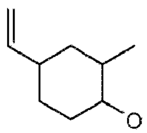 | 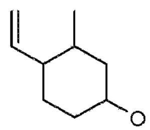 | 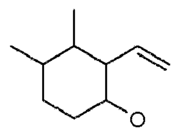 | 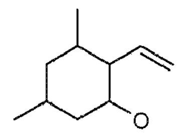 |
| OQ | OR | OS | OT |
| 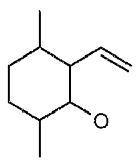 | 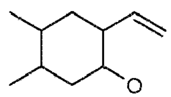 | 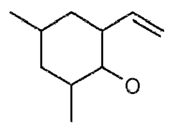 | 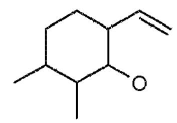 |
| OU | OV | OW | OX |

(continued)

| | | | |
|---|---|---|---|
| 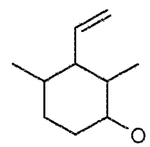 | 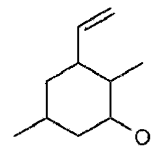 | 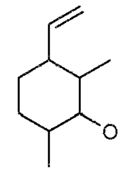 | 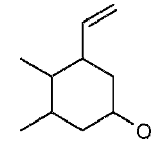 |
| OY | OZ | PA | PB |
| 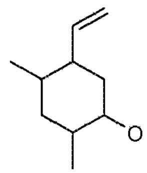 | | 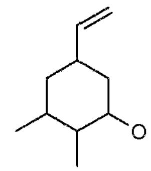 | 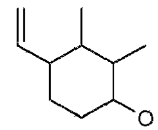 |
| PC | | PD | PE |
| 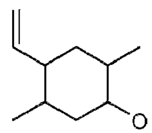 | 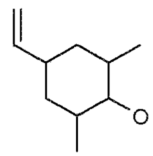 | 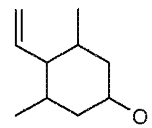 | |
| PF | PG | PH | |
| 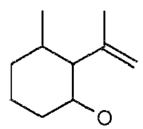 | 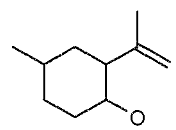 | 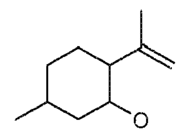 | 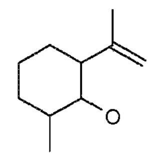 |
| PI | PJ | PK | PL |
| 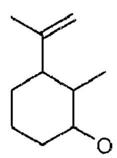 | 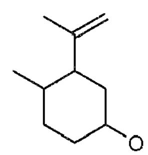 | 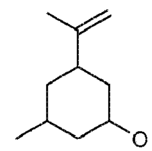 | 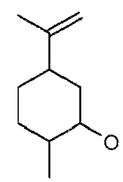 |
| PM | PN | PO | PP |

(continued)

| | | | |
|---|---|---|---|
| 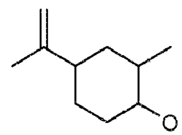 | 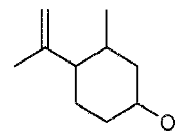 | | 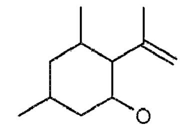 |
| PQ | PR | PS | PT |
| 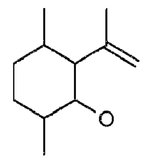 | 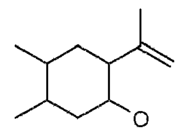 | 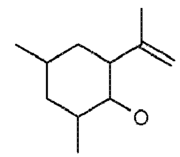 | 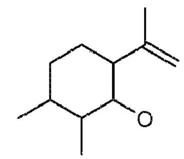 |
| PU | PV | PW | PX |
| 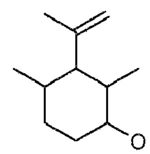 | 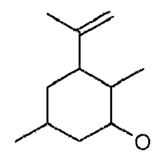 | 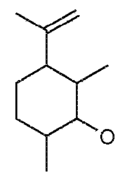 | 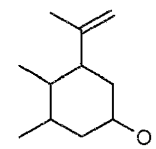 |
| PY | PZ | QA | QB |
| 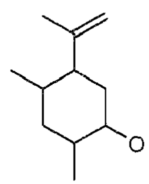 | | 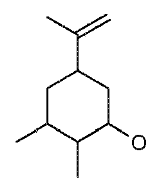 | 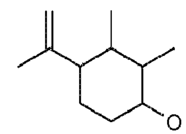 |
| QC | | QD | QE |
| 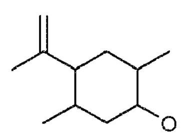 | 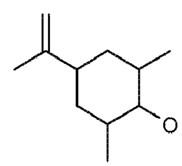 | 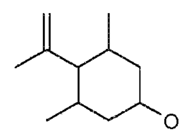 | |
| QF | QG | QH | |

(continued)

| | | | |
|---|---|---|---|
| 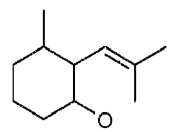 | 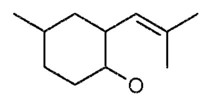 | 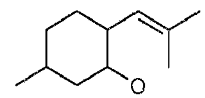 | 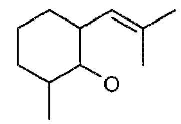 |
| QI | QJ | QK | QL |
| 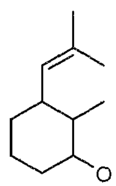 | 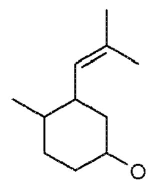 | 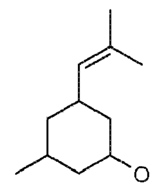 | 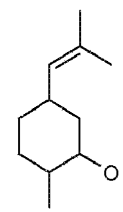 |
| QM | QN | QO | QP |
| 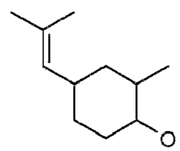 | 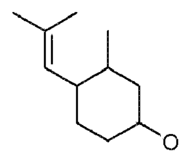 | 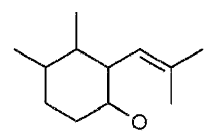 | 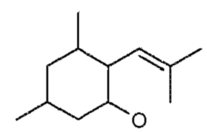 |
| QQ | QR | QS | QT |
| 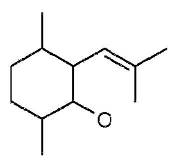 | 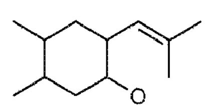 | 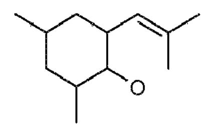 | 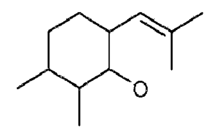 |
| QU | QV | QW | QX |
| 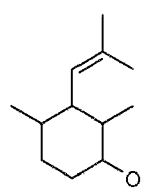 | 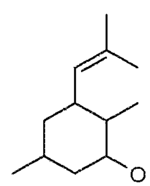 | 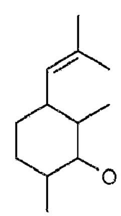 | 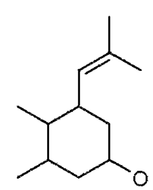 |

(continued)

| | | | |
|---|---|---|---|
| QY | QZ | RA | RB |
| 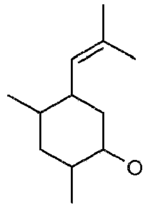 | | 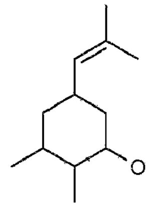 | 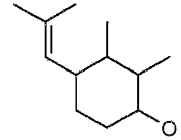 |
| RC | | RD | RE |
| 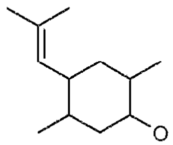 | 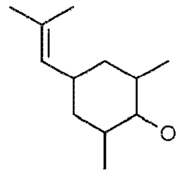 | 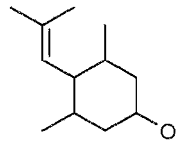 | |
| RF | RG | RH | |
| 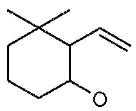 | 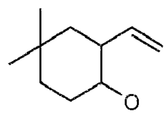 | 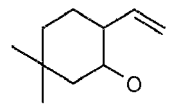 | 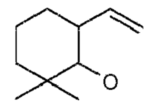 |
| RI | RJ | RK | RL |
| 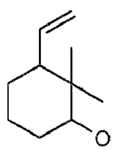 | 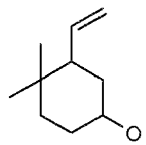 | 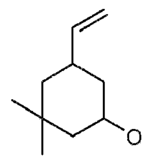 | 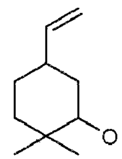 |
| RM | RN | RO | RP |
| 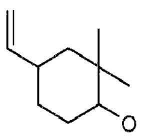 | 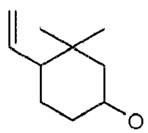 | | |
| RQ | RR | | |

(continued)

| | | | |
|---|---|---|---|
| 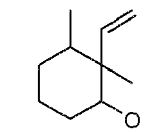 | 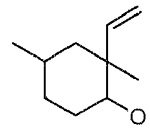 | 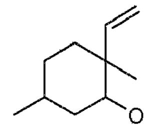 | 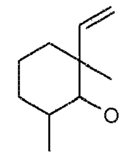 |
| RS | RT | RU | RV |
| 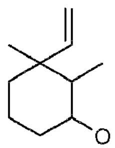 | 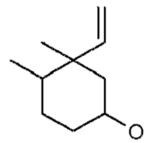 | 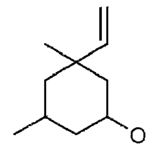 | 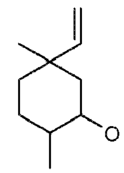 |
| RW | RX | RY | RZ |
| 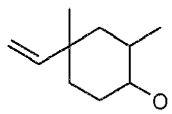 | 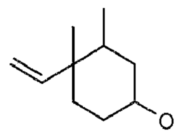 | | |
| SA | SB | | |
| 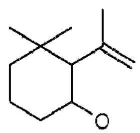 | 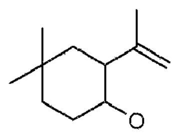 | 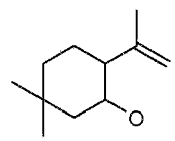 | 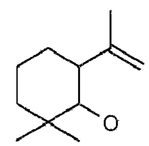 |
| SC | SD | SE | SF |
| 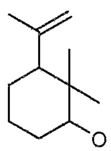 | 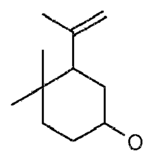 | 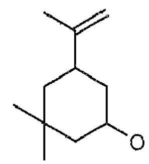 | 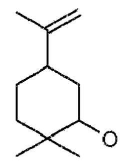 |
| SG | SH | SI | SJ |

(continued)

| | | | |
|---|---|---|---|
| 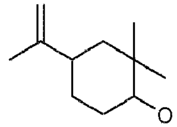 | 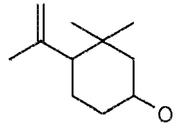 | | |
| SK | SL | | |
| 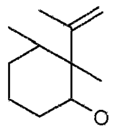 | 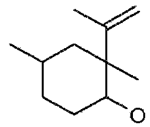 | 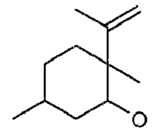 | 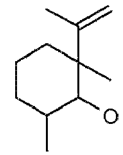 |
| SM | SN | SO | SP |
| 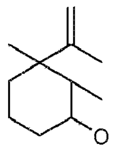 | 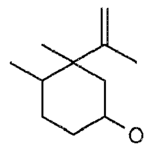 | 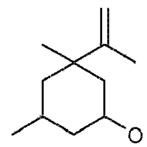 | 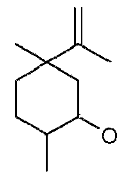 |
| SQ | SR | SS | ST |
| 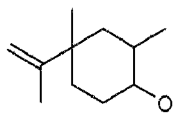 | 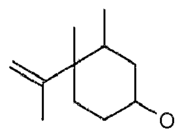 | | |
| SU | SV | | |
| 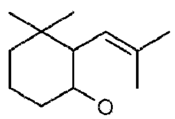 | 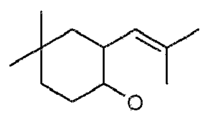 | 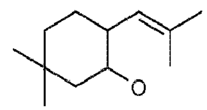 | 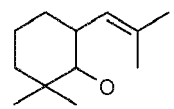 |
| SW | SX | SY | SZ |

(continued)

| | | | |
|---|---|---|---|
| 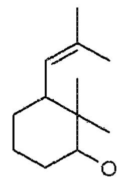 | 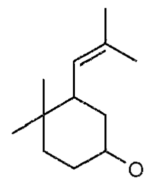 | 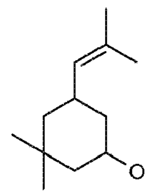 | 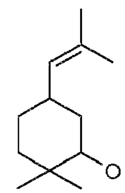 |
| TA | TB | TC | TD |
| 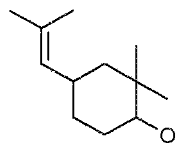 | 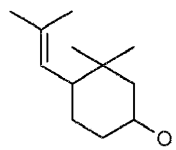 | | |
| TE | TF | | |
| 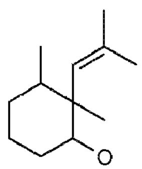 | 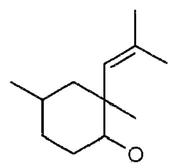 | 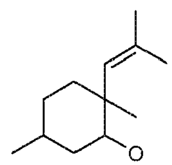 | 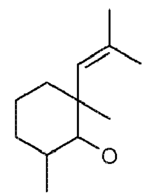 |
| TG | TH | TI | TJ |
| 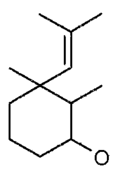 | 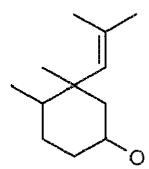 | 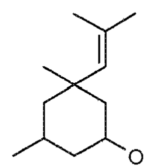 | 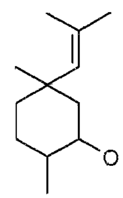 |
| TK | TL | TM | TN |
| 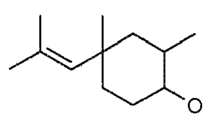 | 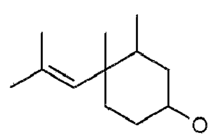 | | |

(continued)

| | | | |
|---|---|---|---|
| TO | TP | | |
| 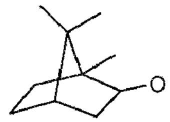 | 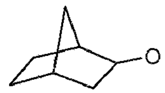 | 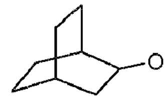 | 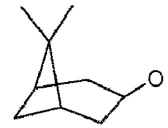 |
| TQ | TR | TS | TT |
| 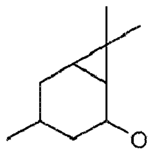 | 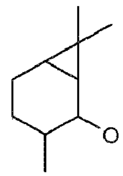 | 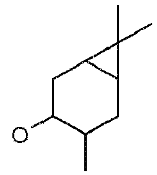 | 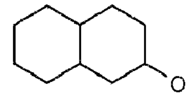 |
| TU | TV | TW | TX |
| 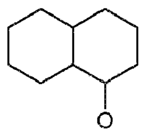 | 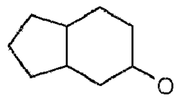 | 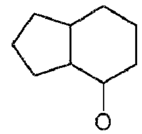 | |
| TY | TZ | UA | |

Preferred compounds of formulae (I), (Carb-I), (Carb-II) and (Carb-II-R1H) are those in which B denotes $NR^1R^2$, wherein preferably $R^1$ denotes hydrogen, and wherein $NR^2$ is a radical chosen from the following list "**N**":

| | | | |
|---|---|---|---|
| 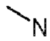 | 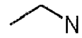 | 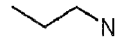 | 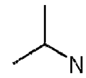 |
| 1 | 2 | 3 | 4 |
| 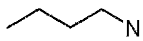 | 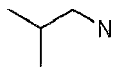 | 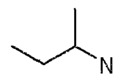 | 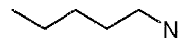 |
| 5 | 6 | 7 | 8 |
| 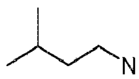 | 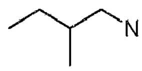 | 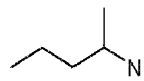 | 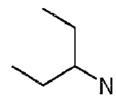 |
| 9 | 10 | 11 | 12 |
| 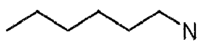 | 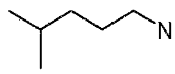 | 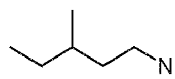 | 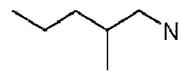 |
| 13 | 14 | 15 | 16 |
| 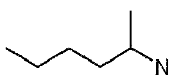 | 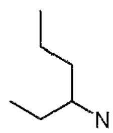 | 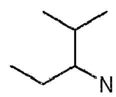 | 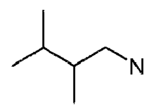 |
| 17 | 18 | 19 | 20 |
| 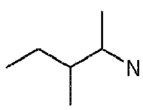 | 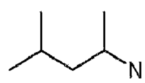 | 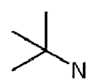 | 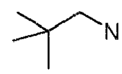 |
| 21 | 22 | 23 | 24 |
| 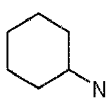 | 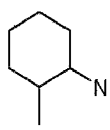 | 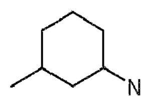 | 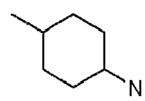 |
| 25 | 26 | 27 | 28 |
| 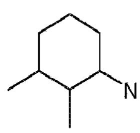 | 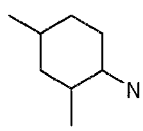 | 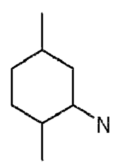 | 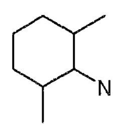 |
| 29 | 30 | 31 | 32 |
| 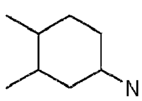 | 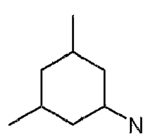 | | |
| 33 | 34 | | |

(continued)

| | | | |
|---|---|---|---|
| 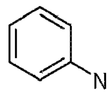 | 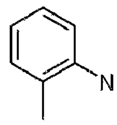 | 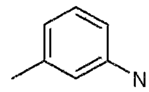 | 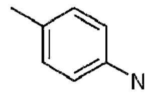 |
| 35 | 36 | 37 | 38 |
| 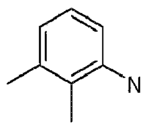 | 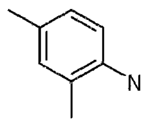 | 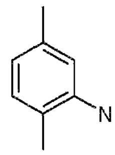 | 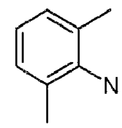 |
| 39 | 40 | 41 | 42 |
| 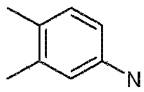 | 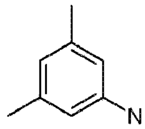 | | |
| 43 | 44 | | |
| 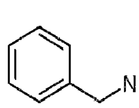 | 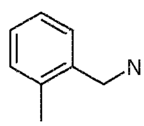 | 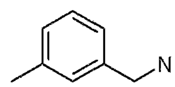 | 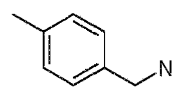 |
| 45 | 46 | 47 | 48 |
| 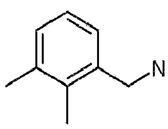 | 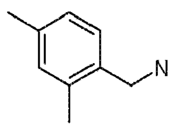 | 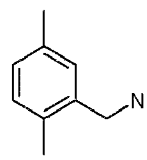 | 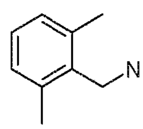 |
| 49 | 50 | 51 | 52 |
| 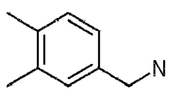 | 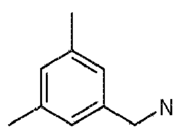 | | |
| 53 | 54 | | |
| 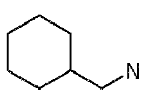 | 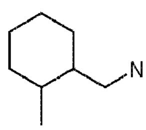 | 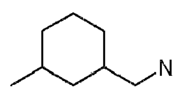 | 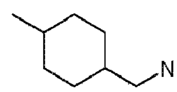 |
| 55 | 56 | 57 | 58 |
| 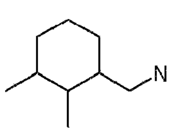 | 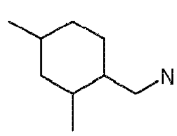 | 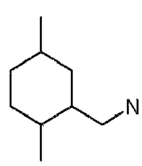 | 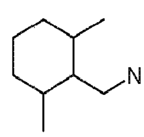 |
| 59 | 60 | 61 | 62 |

(continued)

| | | | |
|---|---|---|---|
| 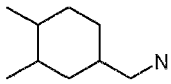 | 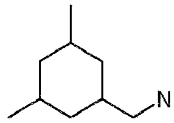 | | |
| 63 | 64 | | |
| 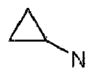 | 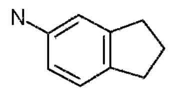 | 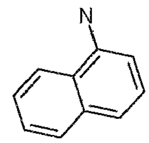 | 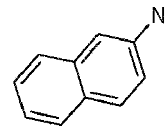 |
| 65 | 66 | 67 | 68 |
| 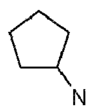 | 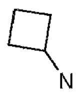 | 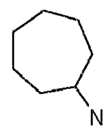 | 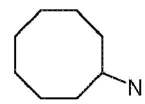 |
| 69 | 70 | 71 | 72 |
| 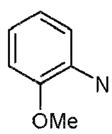 | 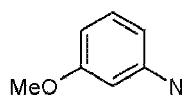 | 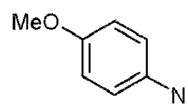 | |
| 73 | 74 | 75 | |
| 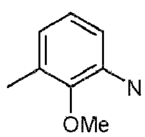 | 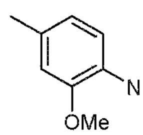 | 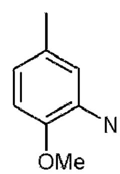 | 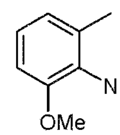 |
| 76 | 77 | 78 | 79 |
| 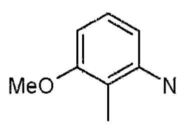 | 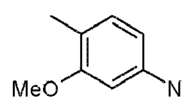 | 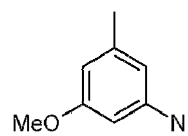 | 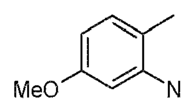 |
| 80 | 81 | 82 | 83 |
| 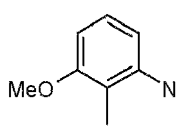 | 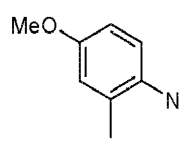 | 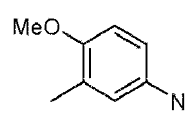 | |
| 84 | 85 | 86 | |
| 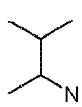 | 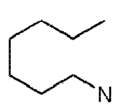 | 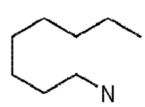 | 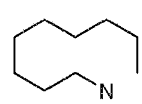 |
| 87 | 88 | 89 | 90 |
| 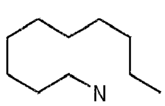 | 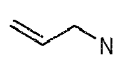 | 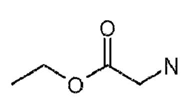 | 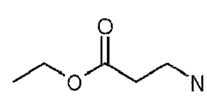 |

(continued)

| 91 | 92 | 93 | 94 |
|---|---|---|---|
| 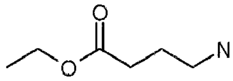 | 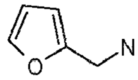 | 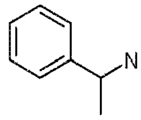 | 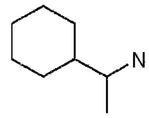 |
| 95 | 96 | 97 | 98 |
| 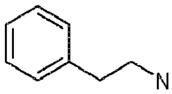 | 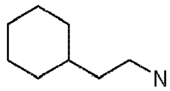 | 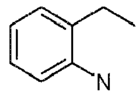 | 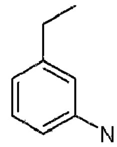 |
| 99 | 100 | 101 | 102 |
| 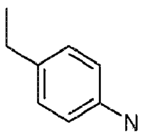 | 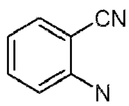 | 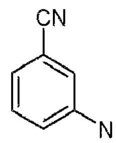 | 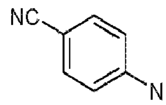 |
| 103 | 104 | 105 | 106 |
| 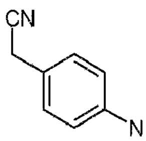 | 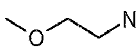 | 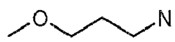 | 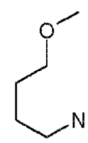 |
| 107 | 108 | 109 | 110 |
| 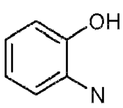 | 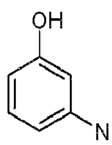 | 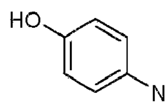 | 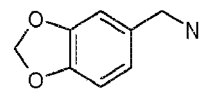 |
| 111 | 112 | 113 | 114 |
| 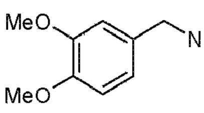 | 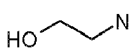 | 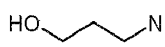 | 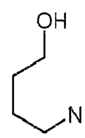 |
| 115 | 116 | 117 | 118 |
| 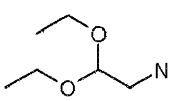 | 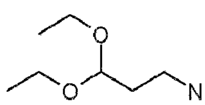 | 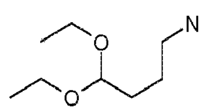 | 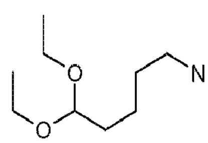 |
| 119 | 120 | 121 | 122 |
| 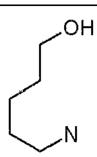 | 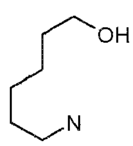 | 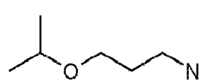 | 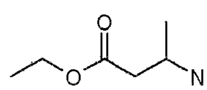 |
| 123 | 124 | 125 | 126 |

(continued)

| | | | |
|---|---|---|---|
| 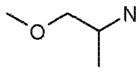 | 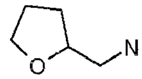 | 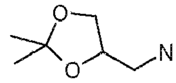 | 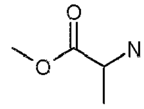 |
| 127 | 128 | 129 | 130 |
| 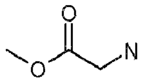 | 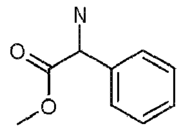 | 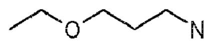 | 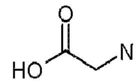 |
| 131 | 132 | 133 | 134 |
| 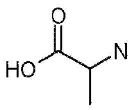 | 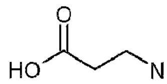 | 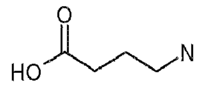 | 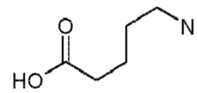 |
| 135 | 136 | 137 | 138 |
| 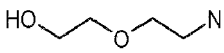 | 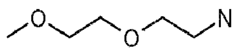 | 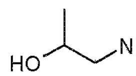 | 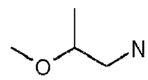 |
| 139 | 140 | 141 | 142 |
| 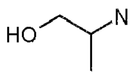 | 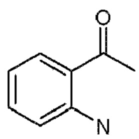 | 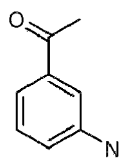 | 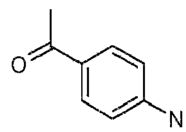 |
| 143 | 144 | 145 | 146 |
| 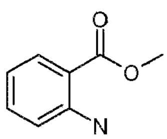 | 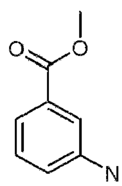 | 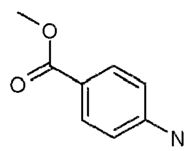 | 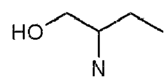 |
| 147 | 148 | 149 | 150 |
| 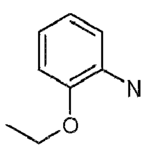 | 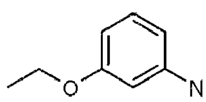 | 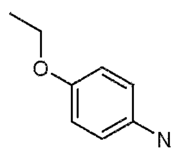 | 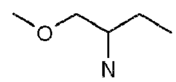 |
| 151 | 152 | 153 | 154 |

Preferred compounds of formulae (I), (Carb-I) and (Carb-II) are those in which **B** denotes $NR^1R^2$, wherein $NR^1R^2$ is a radical chosen from the following list "**D**":

| | | | |
|---|---|---|---|
| 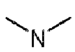 | 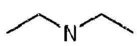 | 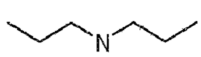 | 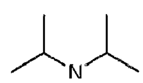 |
| 201 | 202 | 203 | 204 |

(continued)

| | | | |
|---|---|---|---|
| 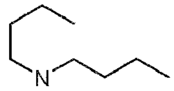 | 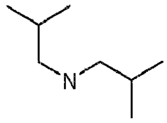 | 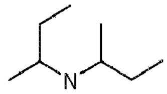 | 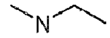 |
| 205 | 206 | 207 | 208 |
| 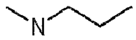 | 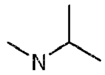 | 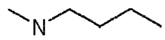 | 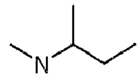 |
| 209 | 210 | 211 | 212 |
| 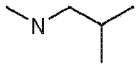 | 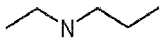 | 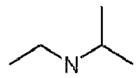 | 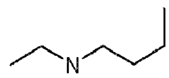 |
| 213 | 214 | 215 | 216 |
| 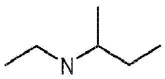 | 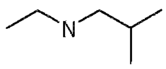 | 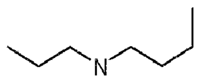 | 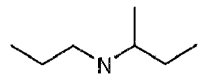 |
| 217 | 218 | 219 | 220 |
| 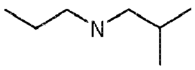 | 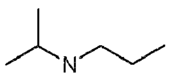 | 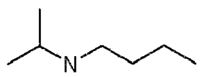 | 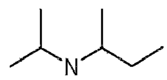 |
| 221 | 222 | 223 | 224 |
| 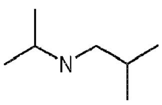 | 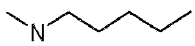 | 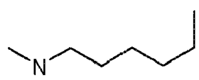 | 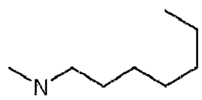 |
| 225 | 226 | 227 | 228 |
| 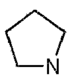 | 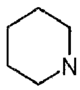 | 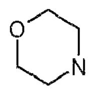 | 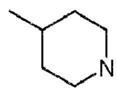 |
| 229 | 230 | 231 | 232 |

The **CyO-N-code** as defined and used hereinafter specifies a single individual compound of formula (Carb-II-R1H) in accordance with the present invention. A specific compound is defined by the CyO-N-code by selecting a radical from list "CyO" as substituent A in formula (I) and selecting in substituent B a radical from list "N" as group $NR^2$, whereby $R^1$ of substituent B denotes hydrogen.

By way of example, said CyO-N-code is illustrated by the following compounds:

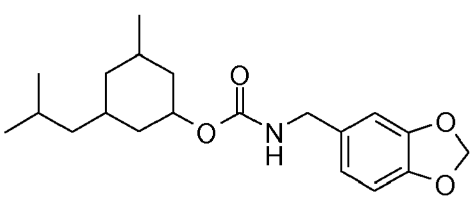

CyO-N-code: **CA114**

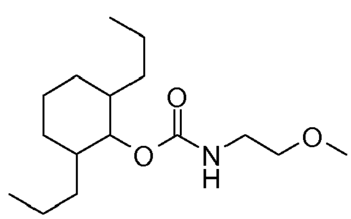

CyO-N-code: **HQ108**

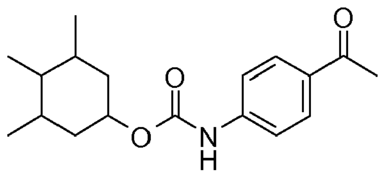

CyO-N-code: **BJ146**

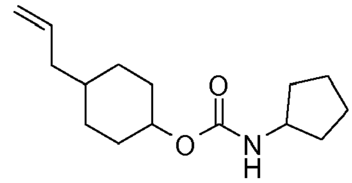

CyO-N-code: **NJ69**

The **CyO-D-code** as defined and used hereinafter specifies a single individual compound of formula (Carb-II) in accordance with the present invention. A specific compound is defined by the CyO-D-code by selecting a radical from list "CyO" as substituent A in formula (I) and selecting from list "D" as group substituent B in formula (I).
By way of example, said CyO-D-code is illustrated by the following compounds:

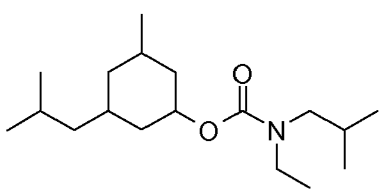

CyO-D-code: **CA218**

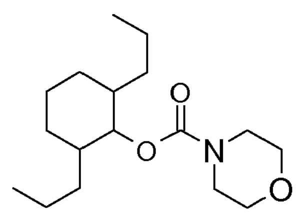

CyO-D-code: **HQ231**

Further preferred compounds of formula (I) are those in which B denotes $NHR^2$, wherein $NR^2$ corresponds to radical 1 of list "**N**", above, and A of formula (I) denotes one radical selected from list "**CyO**", above.
Further preferred compounds of formula (I) are those in which B denotes $NHR^2$, wherein $NR^2$ corresponds to radical 2 of list "**N**", above, and A of formula (I) denotes one radical selected from list "**CyO**", above.
Further preferred compounds of formula (I) are those in which B denotes $NHR^2$, wherein $NR^2$ corresponds to radical **3** of list **"N",** above, and A of formula (I) denotes one radical selected from list **"CyO",** above.
Further preferred compounds of formula (I) are those in which B denotes $NHR^2$, wherein $NR^2$ corresponds to radical **4** of list **"N",** above, and A of formula (I) denotes one radical selected from list **"CyO",** above.
Further preferred compounds of formula (I) are those in which B denotes $NHR^2$, wherein $NR^2$ corresponds to radical **5** of list **"N",** above, and A of formula (I) denotes one radical selected from list **"CyO",** above.
Further preferred compounds of formula (I) are those in which B denotes $NHR^2$, wherein $NR^2$ corresponds to radical **6** of list **"N",** above, and A of formula (I) denotes one radical selected from list **"CyO",** above.
Further preferred compounds of formula (I) are those in which B denotes $NHR^2$, wherein $NR^2$ corresponds to radical **7** of list **"N",** above, and A of formula (I) denotes one radical selected from list **"CyO",** above.
Further preferred compounds of formula (I) are those in which B denotes $NHR^2$, wherein $NR^2$ corresponds to radical **8** of list **"N",** above, and A of formula (I) denotes one radical selected from list **"CyO",** above.
Further preferred compounds of formula (I) are those in which B denotes $NHR^2$, wherein $NR^2$ corresponds to radical 9 of list **"N",** above, and A of formula (I) denotes one radical selected from list **"CyO",** above.
Further preferred compounds of formula (I) are those in which B denotes $NHR^2$, wherein $NR^2$ corresponds to radical **10** of list **"N",** above, and A of formula (I) denotes one radical selected from list **"CyO",** above.
Further preferred compounds of formula (I) are those in which B denotes $NHR^2$, wherein $NR^2$ corresponds to radical **11** of list **"N",** above, and A of formula (I) denotes one radical selected from list **"CyO",** above.
Further preferred compounds of formula (I) are those in which B denotes $NHR^2$, wherein $NR^2$ corresponds to radical **12** of list **"N",** above, and A of formula (I) denotes one radical selected from list **"CyO",** above.
Further preferred compounds of formula (I) are those in which B denotes $NHR^2$, wherein $NR^2$ corresponds to radical **13** of list **"N",** above, and A of formula (I) denotes one radical selected from list **"CyO",** above.
Further preferred compounds of formula (I) are those in which B denotes $NHR^2$, wherein $NR^2$ corresponds to radical **14** of list **"N",** above, and A of formula (I) denotes one radical selected from list **"CyO",** above.

Further preferred compounds of formula (I) are those in which B denotes $NHR^2$, wherein $NR^2$ corresponds to radical **15** of list **"N",** above, and A of formula (I) denotes one radical selected from list **"CyO",** above.

Further preferred compounds of formula (I) are those in which B denotes $NHR^2$, wherein $NR^2$ corresponds to radical **16** of list **"N",** above, and A of formula (I) denotes one radical selected from list **"CyO",** above.

Further preferred compounds of formula (I) are those in which B denotes $NHR^2$, wherein $NR^2$ corresponds to radical **17** of list **"N",** above, and A of formula (I) denotes one radical selected from list **"CyO",** above.

Further preferred compounds of formula (I) are those in which B denotes $NHR^2$, wherein $NR^2$ corresponds to radical **18** of list **"N",** above, and A of formula (I) denotes one radical selected from list **"CyO",** above.

Further preferred compounds of formula (I) are those in which B denotes $NHR^2$, wherein $NR^2$ corresponds to radical **19** of list **"N",** above, and A of formula (I) denotes one radical selected from list **"CyO",** above.

Further preferred compounds of formula (I) are those in which B denotes $NHR^2$, wherein $NR^2$ corresponds to radical **20** of list **"N",** above, and A of formula (I) denotes one radical selected from list **"CyO",** above.

Further preferred compounds of formula (I) are those in which B denotes $NHR^2$, wherein $NR^2$ corresponds to radical **21** of list **"N",** above, and A of formula (I) denotes one radical selected from list **"CyO",** above.

Further preferred compounds of formula (I) are those in which B denotes $NHR^2$, wherein $NR^2$ corresponds to radical **22** of list **"N",** above, and A of formula (I) denotes one radical selected from list **"CyO",** above.

Further preferred compounds of formula (I) are those in which B denotes $NHR^2$, wherein $NR^2$ corresponds to radical **23** of list **"N",** above, and A of formula (I) denotes one radical selected from list **"CyO",** above.

Further preferred compounds of formula (I) are those in which B denotes $NHR^2$, wherein $NR^2$ corresponds to radical **24** of list **"N",** above, and A of formula (I) denotes one radical selected from list **"CyO",** above.

Further preferred compounds of formula (I) are those in which B denotes $NHR^2$, wherein $NR^2$ corresponds to radical **25** of list **"N",** above, and A of formula (I) denotes one radical selected from list **"CyO",** above.

Further preferred compounds of formula (I) are those in which B denotes $NHR^2$, wherein $NR^2$ corresponds to radical **26** of list **"N",** above, and A of formula (I) denotes one radical selected from list **"CyO",** above.

Further preferred compounds of formula (I) are those in which B denotes $NHR^2$, wherein $NR^2$ corresponds to radical **27** of list **"N",** above, and A of formula (I) denotes one radical selected from list **"CyO",** above.

Further preferred compounds of formula (I) are those in which B denotes $NHR^2$, wherein $NR^2$ corresponds to radical **28** of list **"N",** above, and A of formula (I) denotes one radical selected from list **"CyO",** above.

Further preferred compounds of formula (I) are those in which B denotes $NHR^2$, wherein $NR^2$ corresponds to radical **29** of list **"N",** above, and A of formula (I) denotes one radical selected from list **"CyO",** above.

Further preferred compounds of formula (I) are those in which B denotes $NHR^2$, wherein $NR^2$ corresponds to radical **30** of list **"N",** above, and A of formula (I) denotes one radical selected from list **"CyO",** above.

Further preferred compounds of formula (I) are those in which B denotes $NHR^2$, wherein $NR^2$ corresponds to radical **31** of list **"N",** above, and A of formula (I) denotes one radical selected from list **"CyO",** above.

Further preferred compounds of formula (I) are those in which B denotes $NHR^2$, wherein $NR^2$ corresponds to radical **32** of list **"N",** above, and A of formula (I) denotes one radical selected from list **"CyO",** above.

Further preferred compounds of formula (I) are those in which B denotes $NHR^2$, wherein $NR^2$ corresponds to radical **33** of list **"N",** above, and A of formula (I) denotes one radical selected from list **"CyO",** above.

Further preferred compounds of formula (I) are those in which B denotes $NHR^2$, wherein $NR^2$ corresponds to radical **34** of list **"N",** above, and A of formula (I) denotes one radical selected from list **"CyO",** above.

Further preferred compounds of formula (I) are those in which B denotes $NHR^2$, wherein $NR^2$ corresponds to radical **35** of list **"N",** above, and A of formula (I) denotes one radical selected from list **"CyO",** above.

Further preferred compounds of formula (I) are those in which B denotes $NHR^2$, wherein $NR^2$ corresponds to radical **36** of list **"N",** above, and A of formula (I) denotes one radical selected from list **"CyO",** above.

Further preferred compounds of formula (I) are those in which B denotes $NHR^2$, wherein $NR^2$ corresponds to radical **37** of list **"N",** above, and A of formula (I) denotes one radical selected from list **"CyO",** above.

Further preferred compounds of formula (I) are those in which B denotes $NHR^2$, wherein $NR^2$ corresponds to radical **38** of list **"N",** above, and A of formula (I) denotes one radical selected from list **"CyO",** above.

Further preferred compounds of formula (I) are those in which B denotes $NHR^2$, wherein $NR^2$ corresponds to radical **39** of list **"N",** above, and A of formula (I) denotes one radical selected from list **"CyO",** above.

Further preferred compounds of formula (I) are those in which B denotes $NHR^2$, wherein $NR^2$ corresponds to radical **40** of list **"N",** above, and A of formula (I) denotes one radical selected from list **"CyO",** above.

Further preferred compounds of formula (I) are those in which B denotes $NHR^2$, wherein $NR^2$ corresponds to radical **41** of list **"N",** above, and A of formula (I) denotes one radical selected from list **"CyO",** above.

Further preferred compounds of formula (I) are those in which B denotes $NHR^2$, wherein $NR^2$ corresponds to radical **42** of list **"N",** above, and A of formula (I) denotes one radical selected from list **"CyO",** above.

Further preferred compounds of formula (I) are those in which B denotes $NHR^2$, wherein $NR^2$ corresponds to radical **43** of list **"N",** above, and A of formula (I) denotes one radical selected from list **"CyO",** above.

Further preferred compounds of formula (I) are those in which B denotes $NHR^2$, wherein $NR^2$ corresponds to radical **44** of list **"N",** above, and A of formula (I) denotes one radical selected from list **"CyO",** above.
Further preferred compounds of formula (I) are those in which B denotes $NHR^2$, wherein $NR^2$ corresponds to radical **45** of list **"N",** above, and A of formula (I) denotes one radical selected from list **"CyO",** above.
Further preferred compounds of formula (I) are those in which B denotes $NHR^2$, wherein $NR^2$ corresponds to radical **46** of list **"N",** above, and A of formula (I) denotes one radical selected from list **"CyO",** above.
Further preferred compounds of formula (I) are those in which B denotes $NHR^2$, wherein $NR^2$ corresponds to radical **47** of list **"N",** above, and A of formula (I) denotes one radical selected from list **"CyO",** above.
Further preferred compounds of formula (I) are those in which B denotes $NHR^2$, wherein $NR^2$ corresponds to radical **48** of list **"N",** above, and A of formula (I) denotes one radical selected from list **"CyO",** above.
Further preferred compounds of formula (I) are those in which B denotes $NHR^2$, wherein $NR^2$ corresponds to radical **49** of list **"N",** above, and A of formula (I) denotes one radical selected from list **"CyO",** above.
Further preferred compounds of formula (I) are those in which B denotes $NHR^2$, wherein $NR^2$ corresponds to radical **50** of list **"N",** above, and A of formula (I) denotes one radical selected from list **"CyO",** above.
Further preferred compounds of formula (I) are those in which B denotes $NHR^2$, wherein $NR^2$ corresponds to radical **51** of list **"N",** above, and A of formula (I) denotes one radical selected from list **"CyO",** above.
Further preferred compounds of formula (I) are those in which B denotes $NHR^2$, wherein $NR^2$ corresponds to radical **52** of list **"N",** above, and A of formula (I) denotes one radical selected from list **"CyO",** above.
Further preferred compounds of formula (I) are those in which B denotes $NHR^2$, wherein $NR^2$ corresponds to radical **53** of list **"N",** above, and A of formula (I) denotes one radical selected from list **"CyO",** above.
Further preferred compounds of formula (I) are those in which B denotes $NHR^2$, wherein $NR^2$ corresponds to radical **54** of list **"N",** above, and A of formula (I) denotes one radical selected from list **"CyO",** above.
Further preferred compounds of formula (I) are those in which B denotes $NHR^2$, wherein $NR^2$ corresponds to radical **55** of list "**N**", above, and A of formula (I) denotes one radical selected from list "**CyO**", above.
Further preferred compounds of formula (I) are those in which B denotes $NHR^2$, wherein $NR^2$ corresponds to radical **56** of list "**N**", above, and A of formula (I) denotes one radical selected from list "**CyO**", above.
Further preferred compounds of formula (I) are those in which B denotes $NHR^2$, wherein $NR^2$ corresponds to radical **57** of list **"N",** above, and A of formula (I) denotes one radical selected from list **"CyO",** above.
Further preferred compounds of formula (I) are those in which B denotes $NHR^2$, wherein $NR^2$ corresponds to radical **58** of list **"N",** above, and A of formula (I) denotes one radical selected from list **"CyO",** above.
Further preferred compounds of formula (I) are those in which B denotes $NHR^2$, wherein $NR^2$ corresponds to radical **59** of list **"N",** above, and A of formula (I) denotes one radical selected from list **"CyO",** above.
Further preferred compounds of formula (I) are those in which B denotes $NHR^2$, wherein $NR^2$ corresponds to radical **60** of list **"N",** above, and A of formula (I) denotes one radical selected from list **"CyO",** above.
Further preferred compounds of formula (I) are those in which B denotes $NHR^2$, wherein $NR^2$ corresponds to radical **61** of list **"N",** above, and A of formula (I) denotes one radical selected from list **"CyO",** above.
Further preferred compounds of formula (I) are those in which B denotes $NHR^2$, wherein $NR^2$ corresponds to radical **62** of list **"N",** above, and A of formula (I) denotes one radical selected from list **"CyO",** above.
Further preferred compounds of formula (I) are those in which B denotes $NHR^2$, wherein $NR^2$ corresponds to radical **63** of list **"N",** above, and A of formula (I) denotes one radical selected from list **"CyO",** above.
Further preferred compounds of formula (I) are those in which B denotes $NHR^2$, wherein $NR^2$ corresponds to radical 64 of list "N", above, and A of formula (I) denotes one radical selected from list "**CyO**", above.
Further preferred compounds of formula (I) are those in which B denotes $NHR^2$, wherein $NR^2$ corresponds to radical **65** of list "**N**", above, and A of formula (I) denotes one radical selected from list "**CyO**", above.
Further preferred compounds of formula (I) are those in which B denotes $NHR^2$, wherein $NR^2$ corresponds to radical **66** of list **"N",** above, and A of formula (I) denotes one radical selected from list **"CyO",** above.
Further preferred compounds of formula (I) are those in which B denotes $NHR^2$, wherein $NR^2$ corresponds to radical **67** of list **"N",** above, and A of formula (I) denotes one radical selected from list **"CyO",** above.
Further preferred compounds of formula (I) are those in which B denotes $NHR^2$, wherein $NR^2$ corresponds to radical **68** of list **"N",** above, and A of formula (I) denotes one radical selected from list **"CyO",** above.
Further preferred compounds of formula (I) are those in which B denotes $NHR^2$, wherein $NR^2$ corresponds to radical **69** of list **"N",** above, and A of formula (I) denotes one radical selected from list **"CyO",** above.
Further preferred compounds of formula (I) are those in which B denotes $NHR^2$, wherein $NR^2$ corresponds to radical **70** of list **"N",** above, and A of formula (I) denotes one radical selected from list **"CyO",** above.
Further preferred compounds of formula (I) are those in which B denotes $NHR^2$, wherein $NR^2$ corresponds to radical **71** of list **"N",** above, and A of formula (I) denotes one radical selected from list **"CyO",** above.
Further preferred compounds of formula (I) are those in which B denotes $NHR^2$, wherein $NR^2$ corresponds to radical **72** of list **"N",** above, and A of formula (I) denotes one radical selected from list **"CyO",** above.

Further preferred compounds of formula (I) are those in which B denotes $NHR^2$, wherein $NR^2$ corresponds to radical **73** of list **"N",** above, and A of formula (I) denotes one radical selected from list **"CyO",** above.
Further preferred compounds of formula (I) are those in which B denotes $NHR^2$, wherein $NR^2$ corresponds to radical **74** of list **"N",** above, and A of formula (I) denotes one radical selected from list **"CyO",** above.
Further preferred compounds of formula (I) are those in which B denotes $NHR^2$, wherein $NR^2$ corresponds to radical **75** of list **"N",** above, and A of formula (I) denotes one radical selected from list **"CyO",** above.
Further preferred compounds of formula (I) are those in which B denotes $NHR^2$, wherein $NR^2$ corresponds to radical **76** of list **"N",** above, and A of formula (I) denotes one radical selected from list **"CyO",** above.
Further preferred compounds of formula (I) are those in which B denotes $NHR^2$, wherein $NR^2$ corresponds to radical **77** of list **"N",** above, and A of formula (I) denotes one radical selected from list **"CyO",** above.
Further preferred compounds of formula (I) are those in which B denotes $NHR^2$, wherein $NR^2$ corresponds to radical **78** of list **"N",** above, and A of formula (I) denotes one radical selected from list **"CyO",** above.
Further preferred compounds of formula (I) are those in which B denotes $NHR^2$, wherein $NR^2$ corresponds to radical **79** of list **"N",** above, and A of formula (I) denotes one radical selected from list **"CyO",** above.
Further preferred compounds of formula (I) are those in which B denotes $NHR^2$, wherein $NR^2$ corresponds to radical **80** of list **"N",** above, and A of formula (I) denotes one radical selected from list **"CyO",** above.
Further preferred compounds of formula (I) are those in which B denotes $NHR^2$, wherein $NR^2$ corresponds to radical **81** of list **"N",** above, and A of formula (I) denotes one radical selected from list **"CyO",** above.
Further preferred compounds of formula (I) are those in which B denotes $NHR^2$, wherein $NR^2$ corresponds to radical **82** of list **"N",** above, and A of formula (I) denotes one radical selected from list **"CyO",** above.
Further preferred compounds of formula (I) are those in which B denotes $NHR^2$, wherein $NR^2$ corresponds to radical **83** of list **"N",** above, and A of formula (I) denotes one radical selected from list **"CyO",** above.
Further preferred compounds of formula (I) are those in which B denotes $NHR^2$, wherein $NR^2$ corresponds to radical **84** of list **"N",** above, and A of formula (I) denotes one radical selected from list **"CyO",** above.
Further preferred compounds of formula (I) are those in which B denotes $NHR^2$, wherein $NR^2$ corresponds to radical **85** of list **"N",** above, and A of formula (I) denotes one radical selected from list **"CyO",** above.
Further preferred compounds of formula (I) are those in which B denotes $NHR^2$, wherein $NR^2$ corresponds to radical **86** of list **"N",** above, and A of formula (I) denotes one radical selected from list **"CyO",** above.
Further preferred compounds of formula (I) are those in which B denotes $NHR^2$, wherein $NR^2$ corresponds to radical **87** of list **"N",** above, and A of formula (I) denotes one radical selected from list **"CyO",** above.
Further preferred compounds of formula (I) are those in which B denotes $NHR^2$, wherein $NR^2$ corresponds to radical **88** of list **"N",** above, and A of formula (I) denotes one radical selected from list **"CyO",** above.
Further preferred compounds of formula (I) are those in which B denotes $NHR^2$, wherein $NR^2$ corresponds to radical **89** of list **"N",** above, and A of formula (I) denotes one radical selected from list **"CyO",** above.
Further preferred compounds of formula (I) are those in which B denotes $NHR^2$, wherein $NR^2$ corresponds to radical **90** of list **"N",** above, and A of formula (I) denotes one radical selected from list **"CyO",** above.
Further preferred compounds of formula (I) are those in which B denotes $NHR^2$, wherein $NR^2$ corresponds to radical **91** of list **"N",** above, and A of formula (I) denotes one radical selected from list **"CyO",** above.
Further preferred compounds of formula (I) are those in which B denotes $NHR^2$, wherein $NR^2$ corresponds to radical **92** of list **"N",** above, and A of formula (I) denotes one radical selected from list **"CyO",** above.
Further preferred compounds of formula (I) are those in which B denotes $NHR^2$, wherein $NR^2$ corresponds to radical **93** of list **"N",** above, and A of formula (I) denotes one radical selected from list **"CyO",** above.
Further preferred compounds of formula (I) are those in which B denotes $NHR^2$, wherein $NR^2$ corresponds to radical **94** of list **"N",** above, and A of formula (I) denotes one radical selected from list **"CyO",** above.
Further preferred compounds of formula (I) are those in which B denotes $NHR^2$, wherein $NR^2$ corresponds to radical **95** of list **"N",** above, and A of formula (I) denotes one radical selected from list **"CyO",** above.
Further preferred compounds of formula (I) are those in which B denotes $NHR^2$, wherein $NR^2$ corresponds to radical **96** of list **"N",** above, and A of formula (I) denotes one radical selected from list **"CyO",** above.
Further preferred compounds of formula (I) are those in which B denotes $NHR^2$, wherein $NR^2$ corresponds to radical **97** of list **"N",** above, and A of formula (I) denotes one radical selected from list **"CyO",** above.
Further preferred compounds of formula (I) are those in which B denotes $NHR^2$, wherein $NR^2$ corresponds to radical **98** of list **"N",** above, and A of formula (I) denotes one radical selected from list **"CyO",** above.
Further preferred compounds of formula (I) are those in which B denotes $NHR^2$, wherein $NR^2$ corresponds to radical **99** of list **"N",** above, and A of formula (I) denotes one radical selected from list **"CyO",** above.
Further preferred compounds of formula (I) are those in which B denotes $NHR^2$, wherein $NR^2$ corresponds to radical **100** of list **"N",** above, and A of formula (I) denotes one radical selected from list **"CyO",** above.
Further preferred compounds of formula (I) are those in which B denotes $NHR^2$, wherein $NR^2$ corresponds to radical **101** of list **"N",** above, and A of formula (I) denotes one radical selected from list **"CyO",** above.

Further preferred compounds of formula (I) are those in which B denotes $NHR^2$, wherein $NR^2$ corresponds to radical **102** of list **"N",** above, and A of formula (I) denotes one radical selected from list **"CyO",** above.
Further preferred compounds of formula (I) are those in which B denotes $NHR^2$, wherein $NR^2$ corresponds to radical **103** of list **"N",** above, and A of formula (I) denotes one radical selected from list **"CyO",** above.
Further preferred compounds of formula (I) are those in which B denotes $NHR^2$, wherein $NR^2$ corresponds to radical **104** of list **"N",** above, and A of formula (I) denotes one radical selected from list **"CyO",** above.
Further preferred compounds of formula (I) are those in which B denotes $NHR^2$, wherein $NR^2$ corresponds to radical **105** of list **"N",** above, and A of formula (I) denotes one radical selected from list **"CyO",** above.
Further preferred compounds of formula (I) are those in which B denotes $NHR^2$, wherein $NR^2$ corresponds to radical **106** of list **"N",** above, and A of formula (I) denotes one radical selected from list **"CyO",** above.
Further preferred compounds of formula (I) are those in which B denotes $NHR^2$, wherein $NR^2$ corresponds to radical **107** of list **"N",** above, and A of formula (I) denotes one radical selected from list **"CyO",** above.
Further preferred compounds of formula (I) are those in which B denotes $NHR^2$, wherein $NR^2$ corresponds to radical **108** of list **"N",** above, and A of formula (I) denotes one radical selected from list **"CyO",** above.
Further preferred compounds of formula (I) are those in which B denotes $NHR^2$, wherein $NR^2$ corresponds to radical **109** of list **"N",** above, and A of formula (I) denotes one radical selected from list **"CyO",** above.
Further preferred compounds of formula (I) are those in which B denotes $NHR^2$, wherein $NR^2$ corresponds to radical **110** of list **"N",** above, and A of formula (I) denotes one radical selected from list **"CyO",** above.
Further preferred compounds of formula (I) are those in which B denotes $NHR^2$, wherein $NR^2$ corresponds to radical **111** of list **"N",** above, and A of formula (I) denotes one radical selected from list **"CyO",** above.
Further preferred compounds of formula (I) are those in which B denotes $NHR^2$, wherein $NR^2$ corresponds to radical **112** of list **"N",** above, and A of formula (I) denotes one radical selected from list **"CyO",** above.
Further preferred compounds of formula (I) are those in which B denotes $NHR^2$, wherein $NR^2$ corresponds to radical **113** of list **"N",** above, and A of formula (I) denotes one radical selected from list **"CyO",** above.
Further preferred compounds of formula (I) are those in which B denotes $NHR^2$, wherein $NR^2$ corresponds to radical **114** of list **"N",** above, and A of formula (I) denotes one radical selected from list **"CyO",** above.
Further preferred compounds of formula (I) are those in which B denotes $NHR^2$, wherein $NR^2$ corresponds to radical **115** of list **"N",** above, and A of formula (I) denotes one radical selected from list **"CyO",** above.
Further preferred compounds of formula (I) are those in which B denotes $NHR^2$, wherein $NR^2$ corresponds to radical **116** of list **"N",** above, and A of formula (I) denotes one radical selected from list **"CyO",** above.
Further preferred compounds of formula (I) are those in which B denotes $NHR^2$, wherein $NR^2$ corresponds to radical **117** of list **"N",** above, and A of formula (I) denotes one radical selected from list **"CyO",** above.
Further preferred compounds of formula (I) are those in which B denotes $NHR^2$, wherein $NR^2$ corresponds to radical **118** of list **"N",** above, and A of formula (I) denotes one radical selected from list **"CyO",** above.
Further preferred compounds of formula (I) are those in which B denotes $NHR^2$, wherein $NR^2$ corresponds to radical **119** of list **"N",** above, and A of formula (I) denotes one radical selected from list **"CyO",** above.
Further preferred compounds of formula (I) are those in which B denotes $NHR^2$, wherein $NR^2$ corresponds to radical **120** of list **"N",** above, and A of formula (I) denotes one radical selected from list **"CyO",** above.
Further preferred compounds of formula (I) are those in which B denotes $NHR^2$, wherein $NR^2$ corresponds to radical **121** of list **"N",** above, and A of formula (I) denotes one radical selected from list **"CyO",** above.
Further preferred compounds of formula (I) are those in which B denotes $NHR^2$, wherein $NR^2$ corresponds to radical **122** of list **"N",** above, and A of formula (I) denotes one radical selected from list **"CyO",** above.
Further preferred compounds of formula (I) are those in which B denotes $NHR^2$, wherein $NR^2$ corresponds to radical **123** of list **"N",** above, and A of formula (I) denotes one radical selected from list **"CyO",** above.
Further preferred compounds of formula (I) are those in which B denotes $NHR^2$, wherein $NR^2$ corresponds to radical **124** of list **"N",** above, and A of formula (I) denotes one radical selected from list **"CyO",** above.
Further preferred compounds of formula (I) are those in which B denotes $NHR^2$, wherein $NR^2$ corresponds to radical **125** of list **"N",** above, and A of formula (I) denotes one radical selected from list **"CyO",** above.
Further preferred compounds of formula (I) are those in which B denotes $NHR^2$, wherein $NR^2$ corresponds to radical **126** of list **"N",** above, and A of formula (I) denotes one radical selected from list **"CyO",** above.
Further preferred compounds of formula (I) are those in which B denotes $NHR^2$, wherein $NR^2$ corresponds to radical **127** of list **"N",** above, and A of formula (I) denotes one radical selected from list **"CyO",** above.
Further preferred compounds of formula (I) are those in which B denotes $NHR^2$, wherein $NR^2$ corresponds to radical **128** of list **"N",** above, and A of formula (I) denotes one radical selected from list **"CyO",** above.
Further preferred compounds of formula (I) are those in which B denotes $NHR^2$, wherein $NR^2$ corresponds to radical **129** of list **"N",** above, and A of formula (I) denotes one radical selected from list **"CyO",** above.
Further preferred compounds of formula (I) are those in which B denotes $NHR^2$, wherein $NR^2$ corresponds to radical **130** of list **"N",** above, and A of formula (I) denotes one radical selected from list **"CyO",** above.

Further preferred compounds of formula (I) are those in which B denotes $NHR^2$, wherein $NR^2$ corresponds to radical **131** of list **"N",** above, and A of formula (I) denotes one radical selected from list **"CyO",** above.
Further preferred compounds of formula (I) are those in which B denotes $NHR^2$, wherein $NR^2$ corresponds to radical **132** of list **"N",** above, and A of formula (I) denotes one radical selected from list **"CyO",** above.
Further preferred compounds of formula (I) are those in which B denotes $NHR^2$, wherein $NR^2$ corresponds to radical **133** of list **"N",** above, and A of formula (I) denotes one radical selected from list **"CyO",** above.
Further preferred compounds of formula (I) are those in which B denotes $NHR^2$, wherein $NR^2$ corresponds to radical **134** of list **"N",** above, and A of formula (I) denotes one radical selected from list **"CyO",** above.
Further preferred compounds of formula (I) are those in which B denotes $NHR^2$, wherein $NR^2$ corresponds to radical **135** of list **"N",** above, and A of formula (I) denotes one radical selected from list **"CyO",** above.
Further preferred compounds of formula (I) are those in which B denotes $NHR^2$, wherein $NR^2$ corresponds to radical **136** of list **"N",** above, and A of formula (I) denotes one radical selected from list **"CyO",** above.
Further preferred compounds of formula (I) are those in which B denotes $NHR^2$, wherein $NR^2$ corresponds to radical **137** of list **"N",** above, and A of formula (I) denotes one radical selected from list **"CyO",** above.
Further preferred compounds of formula (I) are those in which B denotes $NHR^2$, wherein $NR^2$ corresponds to radical **138** of list **"N",** above, and A of formula (I) denotes one radical selected from list **"CyO",** above.
Further preferred compounds of formula (I) are those in which B denotes $NHR^2$, wherein $NR^2$ corresponds to radical **139** of list **"N",** above, and A of formula (I) denotes one radical selected from list **"CyO",** above.
Further preferred compounds of formula (I) are those in which B denotes $NHR^2$, wherein $NR^2$ corresponds to radical **140** of list **"N",** above, and A of formula (I) denotes one radical selected from list **"CyO",** above.
Further preferred compounds of formula (I) are those in which B denotes $NHR^2$, wherein $NR^2$ corresponds to radical **141** of list **"N",** above, and A of formula (I) denotes one radical selected from list **"CyO",** above.
Further preferred compounds of formula (I) are those in which B denotes $NHR^2$, wherein $NR^2$ corresponds to radical **142** of list **"N",** above, and A of formula (I) denotes one radical selected from list **"CyO",** above.
Further preferred compounds of formula (I) are those in which B denotes $NHR^2$, wherein $NR^2$ corresponds to radical **143** of list **"N",** above, and A of formula (I) denotes one radical selected from list **"CyO",** above.
Further preferred compounds of formula (I) are those in which B denotes $NHR^2$, wherein $NR^2$ corresponds to radical **144** of list **"N",** above, and A of formula (I) denotes one radical selected from list **"CyO",** above.
Further preferred compounds of formula (I) are those in which B denotes $NHR^2$, wherein $NR^2$ corresponds to radical **145** of list **"N",** above, and A of formula (I) denotes one radical selected from list **"CyO",** above.
Further preferred compounds of formula (I) are those in which B denotes $NHR^2$, wherein $NR^2$ corresponds to radical **146** of list **"N",** above, and A of formula (I) denotes one radical selected from list **"CyO",** above.
Further preferred compounds of formula (I) are those in which B denotes $NHR^2$, wherein $NR^2$ corresponds to radical **147** of list **"N",** above, and A of formula (I) denotes one radical selected from list **"CyO",** above.
Further preferred compounds of formula (I) are those in which B denotes $NHR^2$, wherein $NR^2$ corresponds to radical **148** of list **"N",** above, and A of formula (I) denotes one radical selected from list **"CyO",** above.
Further preferred compounds of formula (I) are those in which B denotes $NHR^2$, wherein $NR^2$ corresponds to radical **149** of list **"N",** above, and A of formula (I) denotes one radical selected from list **"CyO",** above.
Further preferred compounds of formula (I) are those in which B denotes $NHR^2$, wherein $NR^2$ corresponds to radical **150** of list **"N",** above, and A of formula (I) denotes one radical selected from list **"CyO",** above.
Further preferred compounds of formula (I) are those in which B denotes $NHR^2$, wherein $NR^2$ corresponds to radical **151** of list **"N",** above, and A of formula (I) denotes one radical selected from list **"CyO",** above.
Further preferred compounds of formula (I) are those in which B denotes $NHR^2$, wherein $NR^2$ corresponds to radical **152** of list **"N",** above, and A of formula (I) denotes one radical selected from list **"CyO",** above.
Further preferred compounds of formula (I) are those in which B denotes $NHR^2$, wherein $NR^2$ corresponds to radical **153** of list **"N",** above, and A of formula (I) denotes one radical selected from list **"CyO",** above.
Further preferred compounds of formula (I) are those in which B denotes $NHR^2$, wherein $NR^2$ corresponds to radical **154** of list **"N",** above, and A of formula (I) denotes one radical selected from list **"CyO",** above.
However, in the context of the present invention, and depending on the circumstances, each individual compound of the compounds of formula (Carb-II-R1H), in particular those defined by the CyO-N-code, may for technical or non-technical reasons, as the case may be, in some embodiments be more preferred or less preferred than other compounds of formula (Carb-II-R1H), in particular those defined by the CyO-N-code. Thus, in some cases, compounds of formula (Carb-II-R1H) as defined by the CyO-N-code do not necessarily share the same level of preference.
Further preferred compounds of formula (I) are those in which B denotes $NR^1R^2$, wherein $NR^1R^2$ corresponds to radical **201** of list **"D",** above, and A of formula (I) denotes one radical selected from list **"CyO",** above.
Further preferred compounds of formula (I) are those in which B denotes $NR^1R^2$, wherein $NR^1R^2$ corresponds to radical **202** of list **"D",** above, and A of formula (I) denotes one radical selected from list **"CyO",** above.
Further preferred compounds of formula (I) are those in which B denotes $NR^1R^2$, wherein $NR^1R^2$ corresponds

to radical **203** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **204** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **205** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **206** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **207** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **208** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **209** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **210** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **211** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **212** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **213** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **214** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **215** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **216** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **217** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **218** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **219** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **220** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **221** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **222** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **223** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **224** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **225** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **226** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **227** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **228** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **229** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **230** of list **"D",** above, and A of formula (I) denotes one radical selected from list **"CyO",** above.

Further preferred compounds of formula (I) are those in which B denotes $NR^1R^2$, wherein $NR^1R^2$ corresponds to radical **231** of list **"D",** above, and A of formula (I) denotes one radical selected from list **"CyO",** above.

Further preferred compounds of formula (I) are those in which B denotes $NR^1R^2$, wherein $NR^1R^2$ corresponds

to radical **232** of list **"D",** above, and A of formula (I) denotes one radical selected from list **"CyO",** above.

However, in the context of the present invention, and depending on the circumstances, each individual compound of the compounds of formula (Carb-II), in particular those defined by the CyO-D-code, may for technical or non-technical reasons, as the case may be, in some embodiments be more preferred or less preferred than other compounds of formula (Carb-II), in particular those defined by the CyO-D-code. Thus, in some cases, compounds defined by the CyO-D-code do not necessarily share the same level of preference.

Several compounds of formula (I), in particular the preferred compounds according to the present invention, are identified and referred to using an arbitrary internal reference-numbering system of the type "BIO", followed by a four-digit number.

In a preferred embodiment, preferred cyclohexyl carbamates of formula (Carb-II) are those wherein
$R^1$ denotes an alkyl radical having 1 to 8 carbon atoms, preferably an alkyl radical having 1 to 4 carbon atoms and
X, Y, Z and $R^2$ have the (preferred or particularly preferred) meaning given hereinbefore or hereinafter.

In a preferred embodiment, preferred N,N-dialkyl-cyclohexyl carbamates of formula (Carb-II) are the following:

| Reference-number | Chemical Name | Structure | CyO-D-Code |
|---|---|---|---|
| BIO1692 | N,N-Diethyl-carbamic acid 2,3,6-trimethyl-cyclohexyl ester | 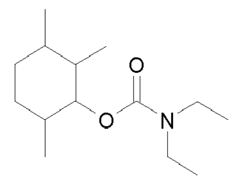 | **BM202** |
| BIO1694 | N,N-Diethyl-carbamic acid 2-isopropyl-cyclohexyl ester | 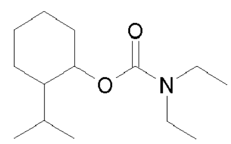 | **AK202** |
| BIO1691 | N,N-Diethyl-carbamic acid 3,5-dimethyl-cyclohexyl ester | 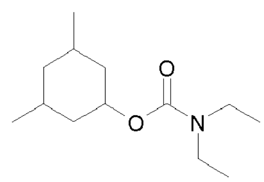 | **BH202** |

In another preferred embodiment, compounds of formulae (I), (Carb-I), (Carb-II) and (Carb-II-R1H), X, Y and Z each denote hydrogen.

Such cyclohexyl carbamates are derived from unsubstituted cyclohexanols, thus to compounds of formula (I) in which A denotes:

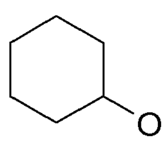

.

A particularly preferred cyclohexyl carbamate, derived from unsubstituted cyclohexanol is:

BIO1741: Phenyl-carbamic acid cyclohexyl ester (corresponding to CyO-N-code AA35)

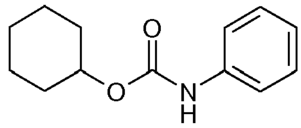

In another preferred embodiment, preferred compounds of formulae (I), (Carb-I), (Carb-II) and (Carb-II-R1H), are those in which X denotes C1-C4-alkyl or C2-C4-alkenyl and Y and Z both denote hydrogen.

Such cyclohexyl carbamates are derived from monosubstituted cyclohexanols, thus to compounds of formula (I) in which A denotes

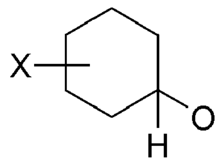,

wherein X has the meaning given above.

Preferably, X denotes C1-C4-alkyl, more preferably X denotes methyl, isopropyl or tert.-butyl.

Most preferably A denotes

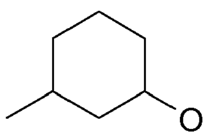, 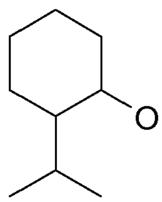 or 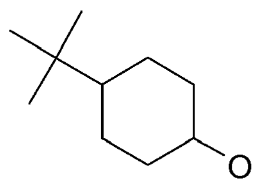.

Particularly preferred cyclohexyl carbamates of formula (Carb-II-R1H), derived from monosubstituted cyclohexanols, are the following:

| Reference-number | Chemical Name | Structure | CyO-N-Code |
|---|---|---|---|
| BIO1825 | p-Tolyl-carbamic acid 3-methyl-cyclohexyl ester | 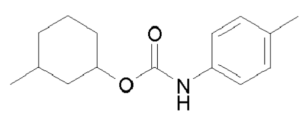 | **AC38** |
| BIO1841 | Butyl-carbamic acid 2-isopropyl-cyclohexyl ester | 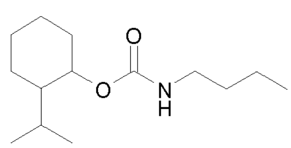 | **AK5** |

(continued)

| Reference-number | Chemical Name | Structure | CyO-N-Code |
|---|---|---|---|
| BIO1824 | p-Tolyl-carbamic acid 2-isopropyl-cyclohexyl ester | 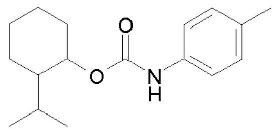 | **AK38** |
| BIO1744 | (2-Methoxy-phenyl)-carbamic acid 2-isopropyl-cyclohexyl ester | 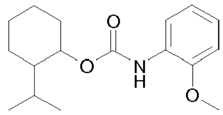 | **AK73** |
| BIO1690 | (2-Methyl-cyclohexyl)-carbamic acid 4-tert-butyl-cyclohexyl ester | 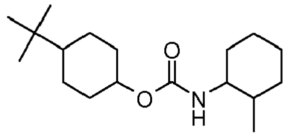 | **AX26** |
| BIO1707 | (4,4-Diethoxy-butyl)-carbamic acid 4-propyl-cyclohexyl ester | 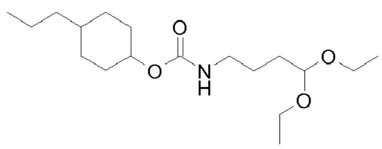 | **AJ121** |
| BIO1646 | (2-Hydroxy-phenyl)-carbamic acid 2-isopropyl-cyclohexyl ester | 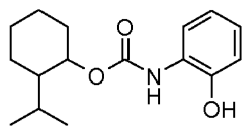 | **AK111** |
| BIO1740 | Phenyl-carbamic acid 2-tert-butyl-cyclohexyl ester | 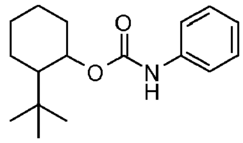 | **AV35** |
| BIO1552 | Ethyl-carbamic acid 2-isopropyl-cyclohexyl ester | 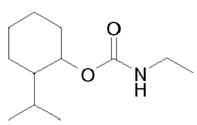 | **AK2** |
| BIO1851 | Hexyl-carbamic acid 2-isopropyl-cyclohexyl ester | 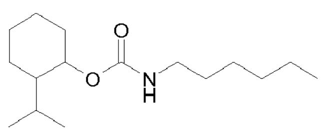 | **AK13** |

(continued)

| Reference-number | Chemical Name | Structure | CyO-N-Code |
|---|---|---|---|
| BIO1821 | Butyl-carbamic acid 3-methyl-cyclohexyl ester | 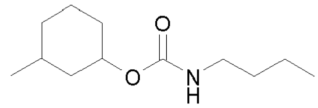 | **AC5** |
| BIO1828 | Butyl-carbamic acid 2-methyl-cyclohexyl ester | 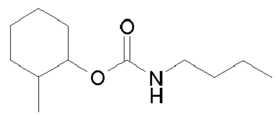 | **AB5** |
| BIO1747 | Cyclohexyl-carbamic acid 4-tert-butyl-cyclohexyl ester | 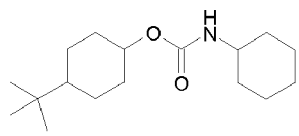 | **AX25** |
| BIO1748 | Benzyl-carbamic acid 2-isopropyl-cyclohexyl ester | 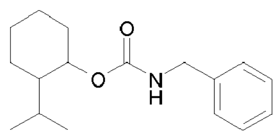 | **AK45** |
| BIO1696 | Ethyl-carbamic acid 2-methyl-cyclohexyl ester | 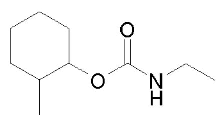 | **AB2** |

The (preferred) compounds of formula (I) derived from monosubstituted cyclohexanols, in particular those explicitly listed above, were particularly active regarding the anti-ageing effects to be achieved in the context of the present invention.

In another preferred embodiment, preferred compounds of formulae (I), (Carb-I), (Carb-II) and (Carb-II-R1H), are those in which X and Y independently of one another denote C1-C4-alkyl or C2-C4-alkenyl and Z denotes hydrogen.

Such cyclohexyl carbamates are derived from disubstituted cyclohexanols, thus to compounds of formula (I) in which A denotes

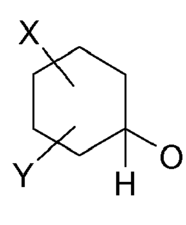

,

wherein X and Y have the meaning given above.

Preferably, X and Y independently of one another denote C1-C4-alkyl, more preferably methyl, isopropyl or tert.-butyl. In a preferred embodiment, X or Y denotes methyl.

More preferably, X and Y independently of one another denote methyl or isopropyl, most preferably A denotes

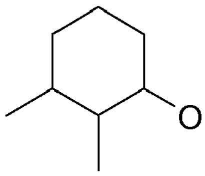, 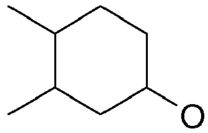, 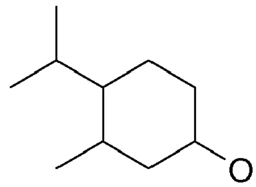,

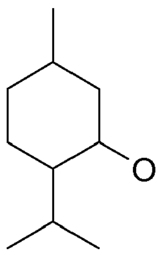, 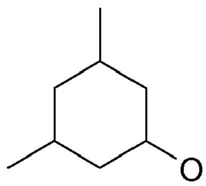 or 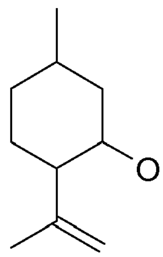.

In one embodiment of the present invention, particularly preferred cyclohexyl carbamates of formula (Carb-II-R1H), derived from disubstituted cyclohexanols other than menthol (see formula (M-X), below), are the following:

| Reference-number | Chemical Name | Structure | CyO-N-Code |
|---|---|---|---|
| BIO1561 | Ethyl-carbamic acid 3,5-dimethyl-cyclohexyl ester | 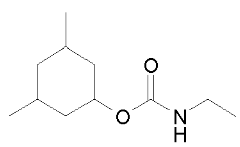 | **BH2** |
| BIO1822 | p-Tolyl-carbamic acid 3,5-dimethyl-cyclohexyl ester | 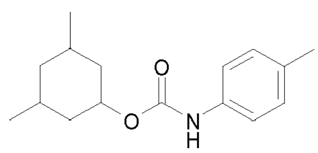 | **BH38** |
| BIO1840 | Butyl-carbamic acid 3,5-dimethyl-cyclohexyl ester | 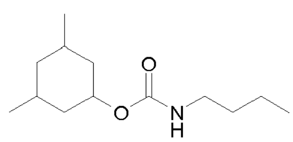 | **BH5** |
| BIO1685 | Phenyl-carbamic acid 3,5-dimethyl-cyclohexyl ester | 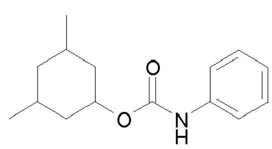 | **BH35** |

(continued)

| Reference-number | Chemical Name | Structure | CyO-N-Code |
|---|---|---|---|
| BIO1643 | (2-Hydroxy-phenyl)-carbamic acid 2,3-dimethyl-cyclohexyl ester | 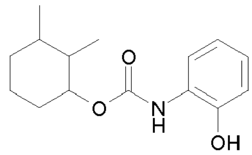 | **AZ111** |
| BIO1842 | Butyl-carbamic acid 2,3-dimethyl-cyclohexyl ester | 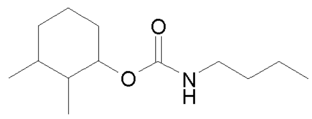 | **AZ5** |
| BIO1615 | Butyl-carbamic acid 2-isopropenyl-5-methyl-cyclohexyl ester | 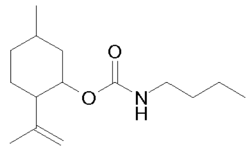 | **PK5** |
| BIO1551 | Ethyl-carbamic acid 2-isopropenyl-5-methyl-cyclohexyl ester | 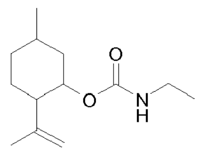 | **PK2** |
| BIO1743 | Cyclohexyl-carbamic acid 3,5-dimethyl-cyclohexyl ester | 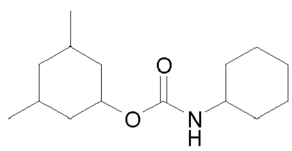 | **BH25** |
| BIO1745 | Benzyl-carbamic acid 3,5-dimethyl-cyclohexyl ester | 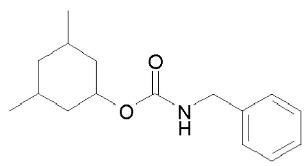 | **BH45** |
| BIO1823 | (4-Ethyl-phenyl)-carbamic acid 3,5-dimethyl-cyclohexyl ester | 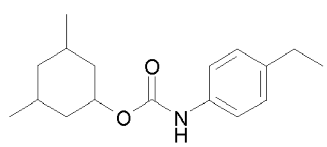 | **BH103** |
| BIO1582 | Ethyl-carbamic acid 3,4-dimethyl-cyclohexyl ester | 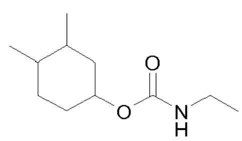 | **BD2** |

(continued)

| Reference-number | Chemical Name | Structure | CyO-N-Code |
|---|---|---|---|
| BIO1827 | Butyl-carbamic acid 3,4-dimethyl-cyclohexyl ester | 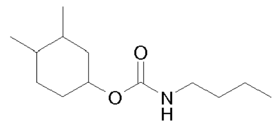 | **BD5** |
| BIO1845 | sec-Butyl-carbamic acid 2,3-dimethyl-cyclohexyl ester | 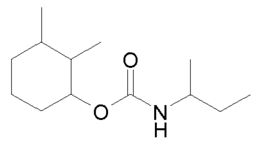 | **AZ7** |
| BIO1581 | Ethyl-carbamic acid 2,3-dimethyl-cyclohexyl ester | 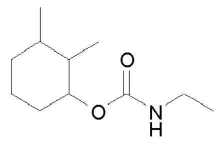 | **AZ2** |
| BIO1560 | Ethyl-carbamic acid 4-isopropyl-3-methyl-cyclohexyl ester | 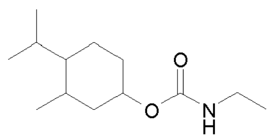 | **DJ2** |
| BIO1645 | (2-Hydroxy-phenyl)-carbamic acid 3,5-dimethyl-cyclohexyl ester | 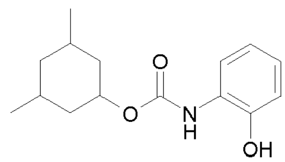 | **BH111** |

The (preferred) compounds of formula (I) derived from disubstituted cyclohexanols, in particular those explicitly listed above, were particularly active regarding the anti-ageing effects to be achieved in the context of the present invention.

The compounds of formula (I) inter alia include menthyl carbamates of formula (M-X)

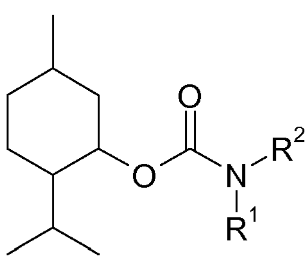

(M-X)

wherein $R^1$ and $R^2$ have the meaning given hereinbefore or hereinafter.

In a preferred embodiment of the present invention, compounds of formula (I) are menthyl carbamates of formula (M-X) wherein $R^1$ and $R^2$ mutually independently have the (preferred) meaning given hereinbefore or hereinafter.

For the sake of clarity, it is noted that menthyl-carbamates of formula (M-X) include all stereoisomeric forms thereof, i.e. the menthyl-, neomenthyl-, isomenthyl- and neoisomenthyl-carbamates, including their respective enantiomeric forms.

The menthyl carbamates of formula (M-X) are derived from 2-isopropyl-5-methylcyclohexanol (p-menthan-3-ol) which has three asymmetric carbon atoms in its cyclohexane ring and occurs as four pairs of enantiomers.

These isomers can be illustrated by the following formulae, showing one enantiomer each of the four diastereomers.

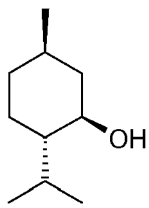

(-)-Menthol (IIa)

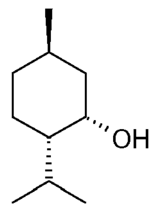

(+)-Neomenthol (IIb)

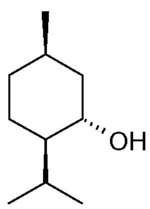

(+)-Isomenthol (IIc)

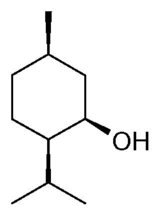

(+)-Neoisomenthol (IId)

The enantiomers (IIa) to (IId) and their optical antipodes may, for example, be obtained by hydrogenation of thymol (e.g. WO 2004/018398 and the references cited therein) or via cyclization of citronellal to the corresponding isopulegol-isomers and subsequent hydrogenation. The menthol isomers can be separated via accurate distillation (for more details on manufacturing and separation of menthol isomers see "Common Fragrance and Flavor Materials", 4th Edition, Wiley-VCH, Weinheim 2001, 52-55).

Structurally derived from the enantiomers (IIa) to (IId) and their optical antipodes are the following compounds of formula (I). Compounds (Ia) are derived from (-)-menthol (IIa), compounds (ent-Ia) are derived from (+)-menthol, compounds (Ib) are derived from (+)-neomenthol (IIb), compounds (ent-Ib) are derived from (-)-neomenthol and so forth.

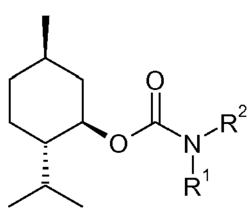

(Ia)

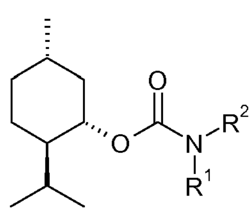

(ent-Ia)

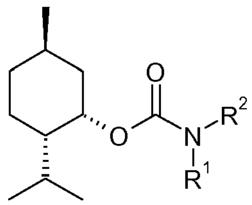

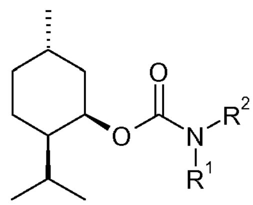

(Ib) (ent-Ib)

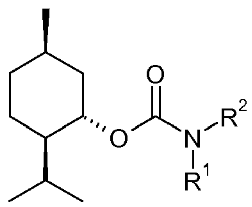

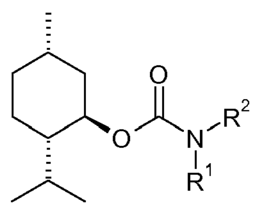

(Ic) (ent-Ic)

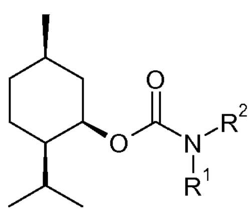

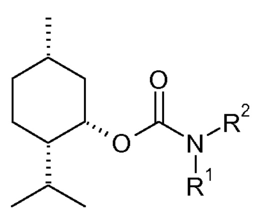

(Id) (ent-Id)

wherein $R^1$ and $R^2$ in each formula (Ia), (ent-Ia), (Ib), (ent-Ib), (Ic), (ent-Ic), (Id) and (ent-Id) mutually independently have the (preferred) meaning given hereinbefore or hereinafter.

Further preferred compounds of formula (I) are those of formula (Ia) wherein $R^1$ denotes hydrogen and wherein $NR^2$ of formula (I) denotes one radical selected from list **"N",** above.

Further preferred compounds of formula (I) are those of formula (ent-Ia) wherein $R^1$ denotes hydrogen and wherein $NR^2$ of formula (I) denotes one radical selected from list **"N",** above.

Further preferred compounds of formula (I) are those of formula (Ib) wherein $R^1$ denotes hydrogen and wherein $NR^2$ of formula (I) denotes one radical selected from list **"N",** above.

Further preferred compounds of formula (I) are those of formula (ent-Ib) wherein $R^1$ denotes hydrogen and wherein $NR^2$ of formula (I) denotes one radical selected from list **"N",** above.

Other preferred compounds of formula (I) are those of formula (Ic) wherein $R^1$ denotes hydrogen and wherein $NR^2$ of formula (I) denotes one radical selected from list **"N",** above.

Other preferred compounds of formula (I) are those of formula (ent-Ic) wherein $R^1$ denotes hydrogen and

wherein $NR^2$ of formula (I) denotes one radical selected from list **"N",** above.

Other preferred compounds of formula (I) are those of formula (Id) wherein $R^1$ denotes hydrogen and wherein $NR^2$ of formula (I) denotes one radical selected from list **"N",** above.

Other preferred compounds of formula (I) are those of formula (ent-Id) wherein $R^1$ denotes hydrogen and wherein $NR^2$ of formula (I) denotes one radical selected from list **"N",** above.

However, in the context of the present invention, and depending on the circumstances, each individual compound of the preferred compounds of formula (M-X) indicated above wherein $R^1$ denotes hydrogen and wherein $NR^2$ is a radical chosen from list **"N",** above, may for technical or non-technical reasons, as the case may be, in some embodiments be more preferred or less preferred than other preferred compounds. Thus, in some cases said compounds do not necessarily share the same level of preference.

Further preferred compounds of formula (I) are those of formula (Ia) wherein $NR^1R^2$ is a radical chosen from list **"D",** above.

Further preferred compounds of formula (I) are those of formula (ent-Ia) wherein $NR^1R^2$ is a radical chosen from list **"D",** above.

Further preferred compounds of formula (I) are those of formula (Ib) wherein $NR^1R^2$ is a radical chosen from list **"D",** above.

Further preferred compounds of formula (I) are those of formula (ent-Ib) wherein $NR^1R^2$ is a radical chosen from list **"D",** above.

Other preferred compounds of formula (I) are those of formula (Ic) wherein $NR^1R^2$ is a radical chosen from list **"D",** above.

Other preferred compounds of formula (I) are those of formula (ent-Ic) wherein $NR^1R^2$ is a radical chosen from list **"D",** above.

Other preferred compounds of formula (I) are those of formula (Id) wherein $NR^1R^2$ is a radical chosen from list **"D",** above.

Other preferred compounds of formula (I) are those of formula (ent-Id) wherein $NR^1R^2$ is a radical chosen from list **"D",** above.

However, in the context of the present invention, and depending on the circumstances, each individual compound of the preferred compounds of formula (M-X) indicated above wherein $NR^1R^2$ is a radical chosen from list **"D"**, above, may for technical or non-technical reasons, as the case may be, in some embodiments be more preferred or less preferred than other preferred compounds. Thus, in some cases said compounds do not necessarily share the same level of preference.

Due to their good to excellent activity and efficacy regarding the anti-ageing effects to be achieved in the context of the present invention, preferred compounds of formula (M-X) in accordance with the present invention are selected from the group consisting of:

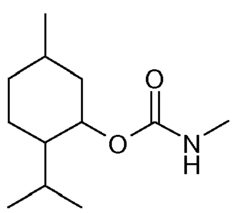

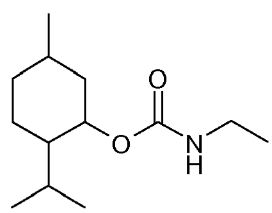

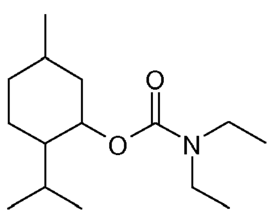

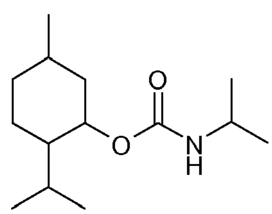

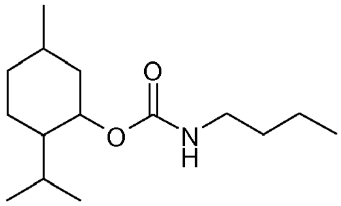
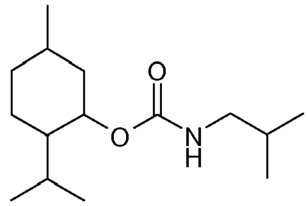

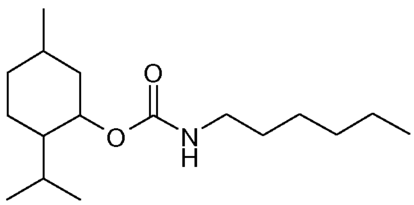
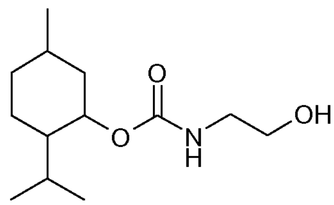

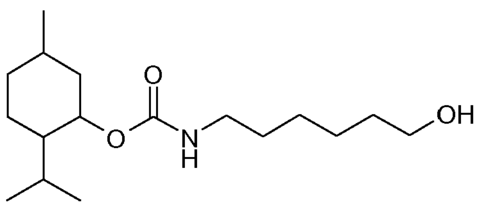
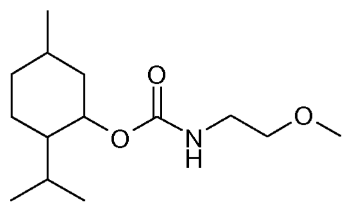

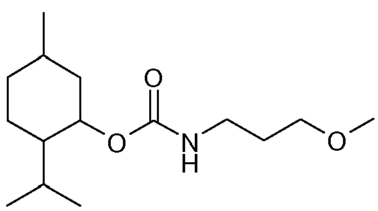
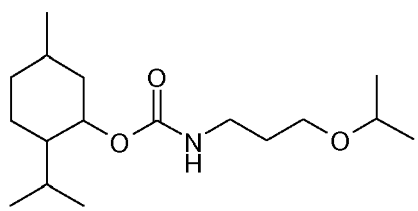

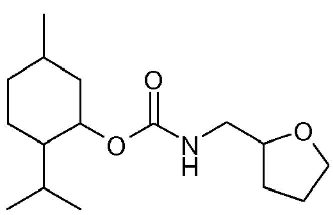
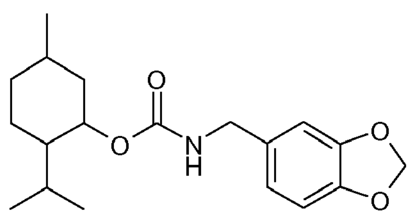

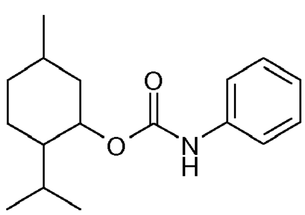
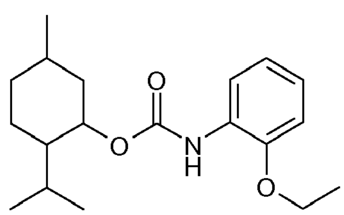

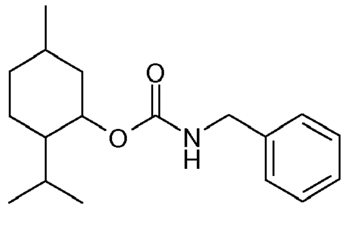

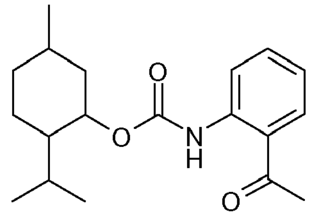

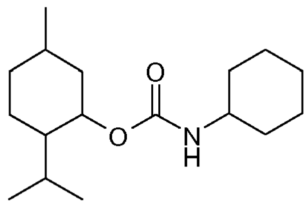

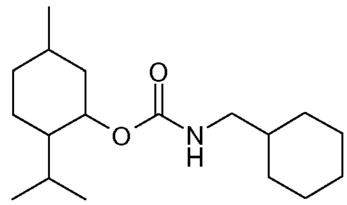

Of said compounds, those corresponding to formulae (Ia), (ent-Ia), (Ib) or (ent-Ib) or a respective racemic mixture thereof are more preferred.

Due to their higher activity regarding the anti-ageing effects to be achieved in the context of the present invention, more preferred menthyl carbamates of formula (M-X) in accordance with the present invention are the following:

| Reference-number | Chemical Name | Structure |
|---|---|---|
| BIO1151 | Ethyl-carbamic acid (1R,2S,5R)-2-isopropyl-5-methyl-cyclohexyl ester | 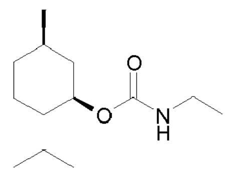 |
| BIO1378 | Ethyl-carbamic acid (1S,2R,5S)-2-isopropyl-5-methyl-cyclohexyl ester | 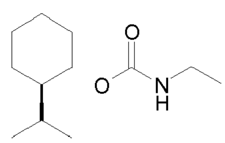 |
| BIO1461 | Ethyl-carbamic acid (1S,2S,5R)-2-isopropyl-5-methyl-cyclohexyl ester | 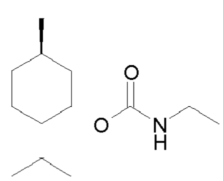 |
| BIO1155 | (3-Methoxy-propyl )-carbam ic acid (1R, 2S,5R)-2-isopropyl-5-methyl-cyclohexyl ester | 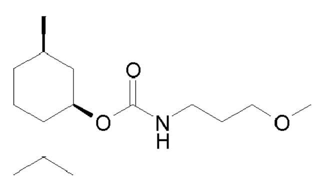 |

(continued)

| Reference-number | Chemical Name | Structure |
|---|---|---|
| BIO1339 | (3-Methoxy-propyl)-carbamic acid (1S,2R, 5S)-2-isopropyl-5-methyl-cyclohexyl ester | 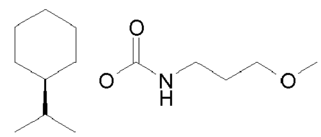 |
| BIO1460 | (3-Methoxy-propyl)-carbamic acid (1S,2S, 5R)-2-isopropyl-5-methyl-cyclohexyl ester | 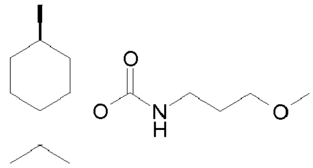 |
| BIO1267 | Butyl-carbamic acid (1R,2S,5R)-2-isopropyl-5-methyl-cyclohexyl ester | 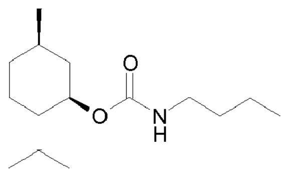 |
| BIO1860 | Butyl-carbamic acid (1S,2R,5S)-2-isopropyl-5-methyl-cyclohexyl ester | 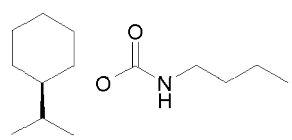 |
| BIO1268 | (3-Isopropoxy-propyl)-carbamic acid (1R, 2S,5R)-2-isopropyl-5-methyl-cyclohexyl ester | 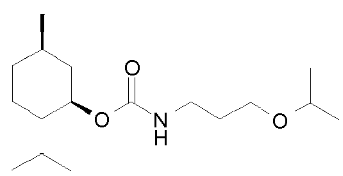 |
| BIO1271 | Hexyl-carbamic acid (1 R,2S,5R)-2-isopropyl-5-methyl-cyclohexyl ester | 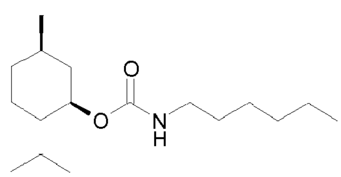 |

(continued)

| Reference-number | Chemical Name | Structure |
|---|---|---|
| BIO1159 | Isobutyl-carbamic acid (1R,2S,5R)-2-isopropyl-5-methyl-cyclohexyl ester | 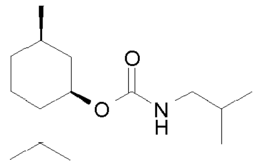 |
| BIO1301 | Methyl-carbamic acid (1 S,2R,5S)-2-isopropyl-5-methyl-cyclohexyl ester | 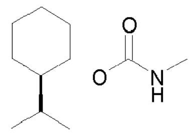 |
| BIO1571 | Benzo[1,3]dioxol-5-ylmethyl-carbamic acid (1R,2S,5R)-2-isopropyl-5-methyl-cyclohexyl ester | 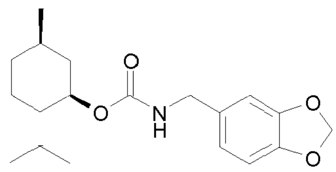 |
| BIO1580 | Phenyl-carbamic acid (1 R,2S,5R)-2-isopropyl-5-methyl-cyclohexyl ester | 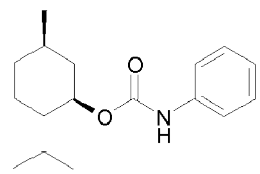 |
| BIO1185 | Methyl-carbamic acid (1 R,2S,5R)-2-isopropyl-5-methyl-cyclohexyl ester | 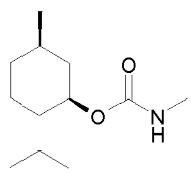 |
| BIO1272 | Isopropyl-carbamic acid (1 R,2S,5R)-2-isopropyl-5-methyl-cyclohexyl ester | 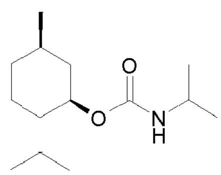 |

(continued)

| Reference-number | Chemical Name | Structure |
|---|---|---|
| BIO1336 | (2-Methoxy-ethyl)-carbamic acid (1 R,2S, 5R)-2-isopropyl-5-methyl-cyclohexyl ester | 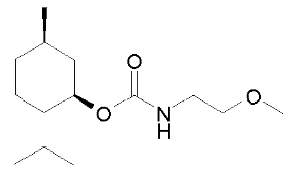 |
| BIO1338 | (2-Hydroxy-ethyl)-carbamic acid (1 R,2S, 5R)-2-isopropyl-5-methyl-cyclohexyl ester | 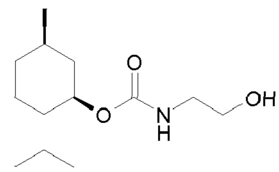 |
| BIO1662 | (6-Hydroxy-hexyl)-carbamic acid (1 R,2S, 5R)-2-isopropyl-5-methyl-cyclohexyl ester | 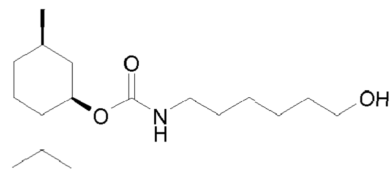 |
| BIO1699 | Cyclohexylmethyl-carbamic acid (1 R,2S, 5R)-2-isopropyl-5-methyl-cyclohexyl ester | 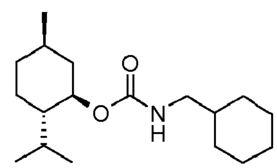 |
| BIO1702 | (Tetrahydro-furan-2-ylmethyl)-carbamic acid (1R,2S,5R)-2-isopropyl-5-methyl-cyclohexyl ester | 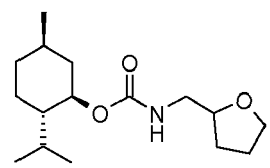 |
| BIO1266 | Cyclohexyl-carbamic acid (1 R,2S,5R)-2-isopropyl-5-methyl-cyclohexyl ester | 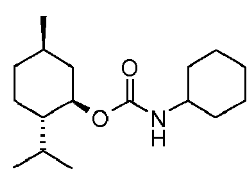 |

(continued)

| Reference-number | Chemical Name | Structure |
|---|---|---|
| BIO1632 | (2-Ethoxy-phenyl)-carbamic acid (1 R,2S, 5R)-2-isopropyl-5-methyl-cyclohexyl ester | 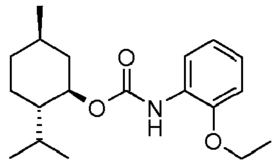 |
| BIO1633 | (2-Acetyl-phenyl)-carbamic acid (1 R,2S, 5R)-2-isopropyl-5-methyl-cyclohexyl ester | 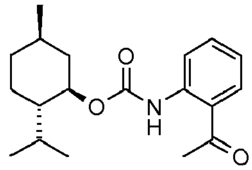 |
| BIO1634 | ((1R,2S,5R)-2-Isopropyl-5-methyl-cyclohexyloxycarbonylamino)-benzoic acid methyl ester | 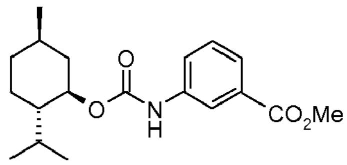 |
| BIO1695 | Benzyl-carbamic acid (1 R,2S,5R)-2-isopropyl-5-methyl-cyclohexyl ester | 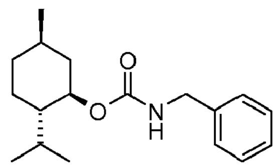 |
| BIO1553 | Diethyl-carbamic acid (1 R,2S,5R)-2-isopropyl-5-methyl-cyclohexyl ester | 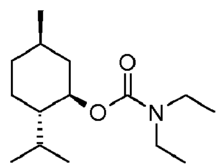 |
| BIO1635 | (2-Methoxy-phenyl)-methyl-carbamic acid (1R,2S,5R)-2-isopropyl-5-methyl-cyclohexyl ester | 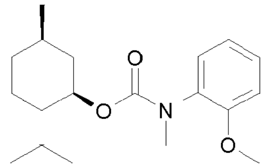 |

(continued)

| Reference-number | Chemical Name | Structure |
|---|---|---|
| BIO1340 | Carbonic acid (1S,2R,5S)-2-isopropyl-5-methyl-cyclohexyl ester 3-((1R,2S,5R)-2-isopropyl-5-methyl-cyclohexyloxycarbonylamino)-propyl ester | 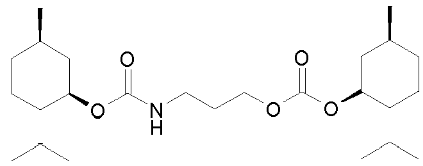 |
| | Carbonic acid (1R,2S,5R)-2-isopropyl-5-methyl-cyclohexyl ester 3-((1R,2S,5R)-2-isopropyl-5-methyl-cyclohexyloxycarbonylamino)-propyl ester | 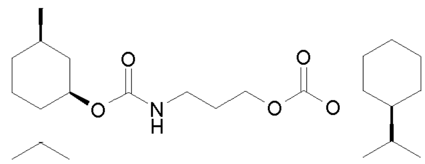 |

The (preferred) compounds of formula (M-X), in particular those explicitly listed above, were particularly active regarding the anti-ageing effects to be achieved in the context of the present invention.

In another preferred embodiment, preferred compounds of formulae (I), (Carb-I), (Carb-II) and (Carb-II-R1H) are those in which X, Y and Z independently of one another denote C1-C4-alkyl or C2-C4-alkenyl.

Such cyclohexyl carbamates are derived from trisubstituted cyclohexanols, thus to compounds of formula (I) in which A denotes

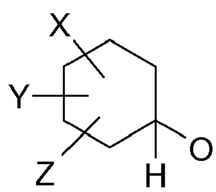

,

wherein X, Y and Z have the meaning given above.

Preferably, X, Y and Z independently of one another denote C1-C4-alkyl, more preferably methyl, isopropyl or tert.-butyl. In a preferred embodiment, at least one substituent of X, Y or Z denotes methyl.

More preferably, X, Y and Z independently of one another denote methyl or isopropyl, most preferably X, Y and Z each denote methyl, in particular A denotes

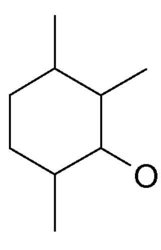

or

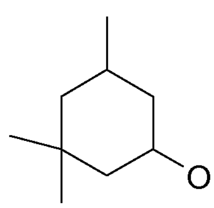

.

Also preferred cyclohexyl carbamates are derived from trisubstituted cyclohexanols are those wherein X denotes methyl and Y and Z together form a radical (a bridge) with 3 carbon atoms.

Among the compounds of formula (I) derived from bicyclic cyclohexanols, it was found that those wherein A denotes

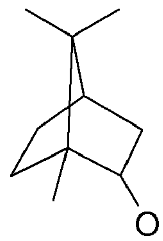

(i.e. borneyl or isoborneyl) were particularly active, in particular those of formula (Carb-II-R1H).

Particularly preferred cyclohexyl carbamates of formula (Carb-II-R1H), derived from trisubstituted cyclohexanols, are the following:

| Reference-number | Chemical Name | Structure | **CyO-N-Code** |
|---|---|---|---|
| BIO1701 | (2-Methoxy-phenyl)-carbamic acid 2,3,6-trimethyl-cyclohexyl ester | 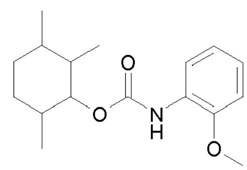 | **BM73** |
| BIO1617 | Butyl-carbamic acid 2,3,6-trimethyl-cyclohexyl ester | 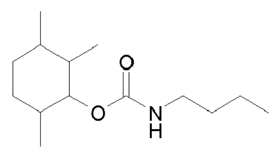 | **BM5** |
| BIO1850 | Hexyl-carbamic acid 3,3,5-trimethyl-cyclohexyl ester | 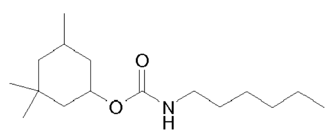 | **BU13** |
| BIO1703 | (2-Methoxy-phenyl)-carbamic acid 3,3,5-trimethyl-cyclohexyl ester | 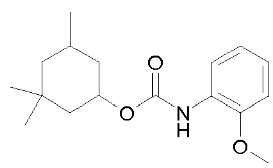 | **BU73** |
| BIO1616 | Butyl-carbamic acid 3,3,5-trimethyl-cyclohexyl ester | 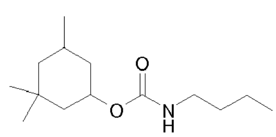 | **BU5** |
| BIO1844 | sec-Butyl-carbamic acid 3,3,5-trimethyl-cyclohexyl ester | 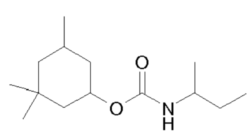 | **BU7** |

(continued)

| Reference-number | Chemical Name | Structure | **CyO-N-Code** |
|---|---|---|---|
| BIO1572 | Ethyl-carbamic acid 3,3,5-trimethyl-cyclohexyl ester | 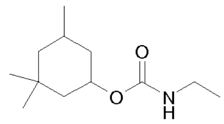 | **BU2** |
| BIO1573 | Ethyl-carbamic acid 1,7,7-trimethyl-bicyclo[2.2.1]hept-2-yl ester | 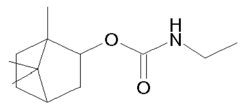 | **TQ2** |
| BIO1574 | (3-Methoxy-propyl)-carbamic acid 3,3,5-trimethyl-cyclohexyl ester | 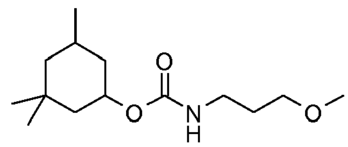 | **BU109** |
| BIO1642 | (2-Hydroxy-phenyl)-carbamic acid 3,3,5-trimethyl-cyclohexyl ester | 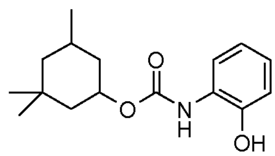 | **BU111** |

The (preferred) compounds of formula (I) derived from trisubstituted cyclohexanols, in particular those explicitly listed above, were particularly active regarding the effects to be achieved in the context of the present invention.

The following compounds of formula (I) are particularly preferred since these were among the most active and effective compounds tested:

BIO1151, BIO1155, BIO1266, BIO1267, BIO1271, BIO1272, BIO1336, BIO1338, BIO1339, BIO1378, BIO1460, BIO1461, BIO1551, BIO1561, BIO1571, BIO1574, BIO1580, BIO1615, BIO1617, BIO1632, BIO1633, BIO1634, BIO1643, BIO1685, BIO1690, BIO1694, BIO1695, BIO1699, BIO1703, BIO1707, BIO1741, BIO1822, BIO1823, BIO1824, BIO1840, BIO1841, BIO1845, BIO1850, BIO1851 and BIO1860.

The compounds of formula (I) of the present invention may generally be obtained by procedures well-known in chemical synthesis. For example, reaction of

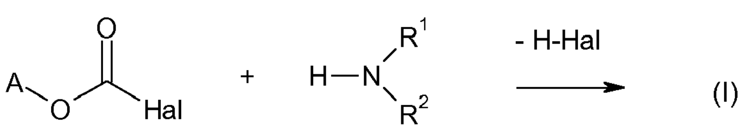


or

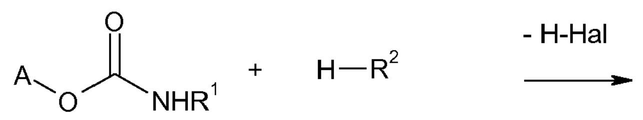


(I)

wherein
A, $R^1$ and $R^2$ denote a (preferred) radical as defined hereinabove, preferably $R^1$ denotes H, and Hal denotes a halide, preferably chloride or bromide.

In order to facilitate the dehydrohalogenation step and the formation of a compound of formula (I) it is preferred to carry out said reaction in the presence of a base, preferably a tertiary amine.

The preferred compounds of formula (I) wherein $R^1$ denotes H may preferably be obtained by reacting a cyclohexanol of formula A-H with a corresponding isocyanate O=C=N-$R^2$, as illustrated in the following reaction scheme:

$$A\text{-}H + O{=}C{=}N\text{-}R^2 \rightarrow (I)$$

wherein A and $R^2$ denote a (preferred) radical as defined hereinabove. The reactions may be conducted in the absence or in the presence of an inert solvent.

The present invention also relates to a (preferably topical) cosmetic or pharmaceutical composition, comprising

(a) one, two or more (preferably of the preferred) compounds of formula (I) as defined herein and/or a cosmetically or pharmaceutically acceptable salt thereof, preferably in an amount having an anti-ageing effect (as defined herein), and

(b) one or more further anti-ageing actives suitable for cosmetic or pharmaceutical application which are not compounds of formula (I), preferably in an amount having an anti-ageing effect (as defined herein),
and

(c) optionally one or more cosmetically or pharmaceutically acceptable carriers.

Thus, in a (preferably topical) cosmetic or pharmaceutical composition according to the present invention the amount of the one, two or more (preferably of the preferred) compounds of formula (I) as defined herein (component (a), above) alone and/or the amount of the one or more further active anti-ageing actives (component (b), above) alone may not be sufficient to exhibit an anti-ageing effect. However, the total amount, i.e. the sum, of components (a) and (b) in a composition according to the present invention is sufficient to exhibit an anti-ageing effect, in particular to effect prolongation of the life span of a cell, preferably of a human skin cell and/or a human neuronal cell, by inhibition of cellular senescence.

As already indicated above, in preferred embodiments, the amount of the one, two or more (preferably of the preferred) compounds of formula (I) as defined herein (component (a), above) alone and/or the amount of the one or more further anti-ageing actives (component (b), above) alone in a composition according to the present invention are sufficient to exhibit an anti-ageing effect, in particular to effect prolongation of the life span of a cell, preferably of a human skin cell and/or a human neuronal cell, by inhibition of cellular senescence.

Preferred composition in accordance with the present invention are those wherein one, a plurality of or all the further anti-ageing actives of component (b) are selected from the following groups (b-1) to (b-8):

(b-1) one or more agents selected from the group consisting of antioxidants,
and/or

(b-2) one or more agents selected from the group consisting of substances which absorb or reflect UV radiation, preferably UV-filters (UV-absorbers) for cosmetic purposes, in particular for skin-protecting purposes,
and/or

(b-3) one or more skin moisturizing agents, preferably selected from the group consisting of alkane diols or alkane triols comprising 3 to 12 carbon atoms,
and/or

(b-4) one or more agents selected from the group consisting of further SIRT1 activators, and/or

(b-5) one or more agents selected from the group consisting of further proteasome activators, and/or

(b-6) one or more agents selected from the group consisting of glycosaminoglycans (GAGs) and substances stimulating the synthesis of glycosaminoglycans, and/or

(b-7) one or more agents selected from the group consisting of matrix-metalloproteinase (MMP) inhibitors, and/or

(b-8) one or more agents selected from the group consisting of substances stimulating the formation of collagen, in particular in skin tissue.

For use in the conventional manner for cosmetics and pharmaceuticals, the compounds of formula (I) are applied, preferably to human skin, in an adequate quantity.

Particular advantages are offered here by preparations, preferably cosmetic and dermatological preparations, which contain one or more compounds of formula (I) and additionally act as a sun protection means, thereby providing a preparation which protects the skin from ultraviolet radiation.

A cosmetic or pharmaceutical, preferably topical, preparation according to the invention containing a) one or more compounds of formula (I) and one or more anti-ageing actives selected from the above mentioned group of component (b) allows to achieve an overall higher, i.e. more pronounced, anti-ageing action. Said more pronounced anti-ageing action is, at least partly, based on synergistic effects.

A cosmetic or pharmaceutical, preferably topical, preparation according to the invention containing a) one or more compounds of formula (I) and one or more anti-ageing actives selected from the above mentioned group of component (b), in particular of component (b-1), (b-2), (b-6), (b-7) and/or (b-8), more preferably of component (b-1) and/or (b-2), have shown to exhibit particularly improved efficacy, in particular faster and/or stronger anti-ageing activity. In many cases a more than additive, often synergistic, anti-ageing activity was observed.

As mentioned above, reactive oxygen species damage the proteasome resulting in an impaired activity thereof.

The combination of one or more compounds of formula (I) and one or more antioxidants of component (b-1) is particularly beneficial because antioxidants additionally protect the proteasome from reactive oxygen species.

The combination of one or more compounds of formula (I) and one or more UV filters of component (b-2) is particularly beneficial because UV light is one major source for reactive oxygen species. Particular advantageouse are cosmetic, dermatological and/or pharmaceutical preparations according to the invention which additionally include one or more UV filters (UV absorbers) and which thus act as compositions with one or more anti-ageing effects and additionally as a sunscreen, overall resulting in a higher, improved anti-ageing activity. Moreover, such a composition shows improved activity in achieving a more even skin tone and in reducing age spots.

Furthermore, compounds of formula (I) prevent photo-ageing and present a new concept of solar protection; particularly in synergy with UV-filters and/or antioxidants, preferably in combination with one or more UV-filters and one or more antioxidants, in particular through restoring of the proteasome after UV irradiation.

As already mentioned above, MMP-1 cleaves collagen, resulting in the degradation of collagen. The activation of the proteasome by one or more of the compounds of formula (I) according to the present invention results in a reduced level of MMP-1 and thus in less collagen degradation. Said anti-ageing effects are more pronounced when one or more matrix-metalloproteinase (MMP) inhibitors of component (b-7) are used, thereby overall resulting in a higher, improved anti-ageing activity.

A cosmetic or pharmaceutical, preferably topical, preparation according to the invention containing a) one or more compounds of formula (I) support the de novo synthesis of collagen, resulting in higher levels of collagen (in the skin tissue), thereby inhibiting wrinkle formation. Said anti-wrinkle effect is improved, i.e. more pronounced, when a composition according to the present invention in addition to one or more of the compounds of formula (I) comprises

- one or more agents selected from the group consisting of glycosaminoglycans (GAGs) and substances stimulating the synthesis of glycosaminoglycans of component (b-6), in particular hyaluronic acid, and/or

- one or more matrix-metalloproteinase (MMP) inhibitors of component (b-7), and/or

- one or more collagen stimulating substances of component (b-8).

A composition (preparation), preferably a topical composition, according to the present invention preferably contains one or more compounds of formula (I) (including all stereoisomers, enantiomers, diastereomers, cis/trans-isomers and epimers, without taking into account possible counterions) in a total amount of 0.001 - 30% by weight, more preferably 0.01 - 20% by weight, even more preferably 0.01 - 5% by weight, particularly preferably 0.05 - 3% by weight and most preferably 0.1 - 2% by weight, in each case based on the total weight of the preparation (composition).

In the context of the present invention an effective amount of compounds, preferably of the preferred compounds, of formula (I) relates to a total amount of one, two or more compounds, preferably of the preferred compounds, of formula (I) having an anti-ageing effect on a human skin cell and/or a human neuronal cell.

The compounds of formula (I) can easily be incorporated in these concentrations in common cosmetic or dermatological formulations (preparations) such as pump sprays, aerosol sprays, creams, ointments, tinctures, lotions and the like.

The cosmetic, dermatological or pharmaceutical preparations according to the invention can be produced by conventional processes known per se, such that one or more compounds of formula (I) are incorporated into (topical) cosmetic, dermatological or pharmaceutical products which can have a conventional composition and which in addition to the effects mentioned hereinbefore or hereinafter can also be used for the treatment, care and cleansing of the skin or hair.

For use, topical cosmetic, dermatological or pharmaceutical preparations according to the invention or for use according to the invention comprising formula (I) are generally applied to the skin in an adequate amount in the conventional manner for topical cosmetic, dermatological or pharmaceutical products.

Preferred cosmetic or pharmaceutical, preferably topical, preparations according to the present invention comprise the further anti-ageing actives of component (b) according to groups (b-1) to (b-8) in the following respective amounts:

- the total quantity of antioxidants of component (b-1) is in the range of from 0.001 to 10 wt.%, preferably in the range of from 0.01 to 5 wt.%, more preferably in the range of from 0.05 to 3 wt.%,
  and/or

- the total quantity of UV filter substances (UV absorbers) of component (b-2) is in the range of from from 0.01% to 40% by weight, preferably in the range of from 0.1 % to 30% by weight, more preferably in the range of from 0.2 to 20 % by weight, even more preferably in the range of from 0.5% to 15% by weight, in particular in the range of from 1.0 to 10% by weight,
  and/or

- the total quantity of skin moisturizing agents of component (b-3) is in the range of from 0.1 to 30 wt.%, preferably in the range of from 0.25 to 20 wt.%, more preferably in the range of from 0.5 to 10 wt.%, even more preferably in the range of from 1 to 5 wt.%,
  and/or

- the total quantity of SIRT1 activators of component (b-4) is in the range of from 0.001 to 15 wt.%, preferably in the range of from 0.01 to 10 wt.%, more preferably in the range of from 0.05 to 5 wt.%,
  and/or

- the total quantity of further proteasome activators of component (b-5) is in the range of from 0.01 to 15 wt.%, preferably in the range of from 0.05 to 10 wt.%, more preferably in the range of from 0.1 to 5 wt.%,
  and/or

- the total quantity of glycosaminoglycans and substances stimulating the synthesis of glycosaminoglycans of component (b-6) is in the range of from 0.01 to 10 wt.%, preferably in the range of from 0.05 to 5 wt.%, more preferably in the range of from 0.1 to 3 wt. %,
  and/or

- the total quantity of matrix-metalloproteinase (MMP) inhibitors of component (b-7) is in the range of from 0.01 to 5 wt.%, preferably in the range of from 0.01 to 3 wt.%, more preferably in the range of from 0.05 to 2 wt.%,
  and/or

- the total quantity of substances stimulating the formation of collagen of component (b-8) is in the range of from 0.01 to 5 wt.%, preferably in the range of from 0.01 to 3 wt.%, more preferably in the range of from 0.05 to 2 wt.%,
  in each case based on the total weight of the preparation (composition).

In the context of the present text, in case a substance has antioxidant properties as well as skin lightening properties, said substance is considered as antioxidant active of component (b-1), in particular for quantitative assessments.

In the context of the present text, in case a substance has UV filter properties as well as antioxidant properties, said substance is considered as UV filter of component (b-2), in particular for quantitative assessments.

In the context of the present text, in case a substance has skin moisturizing properties as well as properties corresponding to those of component (b-6), said substance is considered as skin moisturizing agent of component (b-3), in particular for quantitative assessments.

In the context of the present text, in case a substance is a SIRT1 activator of component (b-4) and additionally has one or more further properties selected from the group consisting of those of components (b-5) to (b-8), said substance is considered as SIRT1 activator (b-4), in particular for quantitative assessments.

In the context of the present text, in case a substance is a substance stimulating the synthesis of glycosaminoglycans of component (b-6) and additionally has one or more further properties selected from the group consisting of those of components (b-7) or (b-8), said substance is considered as active of component (b-6), in particular for quantitative assessments.

In the context of the present text, in case a substance is a matrix-metalloproteinase (MMP) inhibitor of component (b-7) and additionally a substance stimulating the formation of collagen of component (b-8), said substance is considered as active of component (b-7), in particular for quantitative assessments.

Preferred cosmetic or pharmaceutical, preferably topical, preparations according to the present invention comprise one, two, three or more antioxidants of component (b-1), selected from the group consisting of:

amino acids (preferably glycine, histidine, tyrosine, tryptophane) and derivatives thereof, imidazoles (preferably urocanic acid) and derivatives thereof, peptides, preferably D,L-carnosine, D-carnosine, L-carnosine and derivatives thereof (preferably anserine), carnitine, creatine, matrikine peptides (preferably lysyl-threonyl-threonyl-lysyl-serine) and palmitoylated pentapeptides, carotenoids, carotenes (preferably alpha-carotene, beta-carotene, lycopene) and derivatives thereof, lipoic acid and derivatives thereof (preferably dihydrolipoic acid), aurothioglucose, propyl thiouracil and other thiols (preferably thioredoxine, glutathione, cysteine, cystine, cystamine and glycosyl, N-acetyl, methyl, ethyl, propyl, amyl, butyl and lauryl, palmitoyl, oleyl, gamma-linoleyl, cholesteryl, glyceryl and oligoglyceryl esters thereof) and salts thereof, dilauryl thiodipropionate, distearyl thiodipropionate, thiodipropionic acid and derivatives thereof (preferably esters, ethers, peptides, lipids, nucleotides, nucleosides and salts) and sulfoximine compounds (preferably buthionine sulfoximines, homocysteine sulfoximine, buthionine sulfones, penta-, hexa-, heptathionine sulfoximine) in very small tolerated doses (e.g. pmol to$\mu$ mol/kg), also (metal) chelators (preferably alpha-hydroxy fatty acids, palmitic acid, phytic acid, lactoferrin, alpha-hydroxy acids (preferably citric acid, lactic acid, malic acid), humic acid, bile acid, bile extracts, tannins, bilirubin, biliverdin, EDTA, EGTA and derivatives thereof), unsaturated fatty acids and derivatives thereof (preferably gamma-linolenic acid, linoleic acid, oleic acid), folic acid and derivatives thereof, ubiquinone and derivatives thereof, ubiquinol and derivatives thereof, vitamin C and derivatives (preferably ascorbyl palmitate, Mg ascorbyl phosphate, ascorbyl acetate, ascorbyl glucoside), tocopherols and derivatives (preferably vitamin E acetate), vitamin A and derivatives (vitamin A palmitate) and coniferyl benzoate of benzoic resin, rutinic acid and derivatives thereof, flavonoids and glycosylated precursors thereof, in particular quercetin and derivatives thereof, preferably alpha-glucosyl rutin, rosmarinic acid, carnosol, carnosolic acid, resveratrol, caffeic acid and derivatives thereof, sinapic acid and derivatives thereof, ferulic acid and derivatives thereof, curcuminoids, chlorogenic acid and derivatives thereof, retinoids, preferably retinyl palmitate, retinol or tretinoin, ursolic acid, levulinic acid, butyl hydroxytoluene, butyl hydroxyanisole, nordihydroguaiac acid, nordihydroguaiaretic acid, trihydroxybutyrophenone, uric acid and derivatives thereof, mannose and derivatives thereof, zinc and derivatives thereof (preferably ZnO, $ZnSO_4$), selenium and derivatives thereof (preferably selenium methionine), superoxide dismutase, stilbenes and derivatives thereof (preferably stilbene oxide, trans-stilbene oxide) and the derivatives (salts, esters, ethers, sugars, nucleotides, nucleosides, peptides and lipids) of these cited active ingredients which are suitable according to the invention or extracts or fractions of plants having an antioxidant effect, preferably green tea, rooibos, honeybush, grape, rosemary, sage, melissa, thyme, lavender, olive, oats, cocoa, ginkgo, ginseng, liquorice, honeysuckle, sophora, pueraria, pinus, citrus, Phyllanthus emblica or St. John's wort, grape seeds, wheat germ, Phyllanthus emblica, coenzymes, preferably coenzyme Q10, plastoquinone and menaquinone.

Preferred actives of component (b-1) are selected from the group consisting of vitamin A, tocopherol, tocopheryl acetate, vitamin C and ubiquinone.

Advantageous UV filters and inorganic light protection pigments are mentioned in WO 2005/123101. UV absorbers particularly suitable for combination are also mentioned in WO 2005/123101.

Advantageously, these preparations contain at least one UVA filter and/or at least one UVB filter and/or at least one inorganic pigment. The preparations may be present here in various forms such as are conventionally used for sun

protection preparations. Thus, they may be in form of a solution, an emulsion of the water-in-oil type (W/O) or of the oil-in-water type (O/W) or a multiple emulsion, for example of the water-in-oil-in-water type (W/O/W), a gel, a hydrodispersion, a solid stick or else an aerosol.

In a further preferred embodiment a formulation according to the invention contains a total amount of sunscreen agents, i.e. in particular UV filters and/or inorganic pigments (UV filtering pigments) such that the formulation according to the invention has a light protection factor of greater than or equal to 2 (preferably greater than or equal to 5). Such formulations according to the invention are particularly suitable for protecting the skin and hair.

The formulations according to the invention advantageously contain at least one UV-A filter and/or at least one UV-B filter and/or a broadband filter and/or at least one inorganic pigment. Formulations according to the invention preferably contain at least one UV-B filter or a broadband filter, more particularly preferably at least one UV-A filter and at least one UV-B filter.

Preferred cosmetic or pharmaceutical, preferably topical, preparations according to the present invention comprise one, two, three or more UV-filters of component (b-2), selected from the group consisting of:

UV absorbers from the class comprising 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

The compounds according to the invention or for use according to the invention having formula (I) are particularly preferably combined with water-soluble UV filters, in a preferred embodiment with phenylene bis-benzimidazyl tetrasulfonic acid disodium salt (Neo Heliopan®AP) and/or 2-phenylbenzimidazole sulfonic acid (Neo Heliopan®Hydro).

In addition, it is advantageous to combine compounds of formula (I) with active ingredients which penetrate into the skin and protect the skin cells from inside against sunlight-induced damage such as skin ageing, skin inflammation and skin cancer. Preferred respective ingredients, so called arylhydrocarbon receptor antagonists, are described in WO 2007/128723, incorporated herein by reference. Preferred is 2-benzylidene-5,6-dimethoxy-3,3-dimethylindan-1-one.

The UV filters cited below which can be used within the context of the present invention are preferred but naturally are not limiting.

UV filters which are preferably combined with one or more compounds of formula (I) in a preparation according to the present invention are selected from the group consisting of

- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan®HMS)
- salicylic acid-2-ethylhexyl ester (Neo Heliopan®OS)
- triethanolamine salicylate
- 4-isopropyl benzyl salicylate
- anthranilic acid menthyl ester (Neo Heliopan®MA)
- diisopropyl cinnamic acid ethyl ester
- p-methoxycinnamic acid-2-ethylhexyl ester (Neo Heliopan®AV)
- diisopropyl cinnamic acid methyl ester

- p-methoxycinnamic acid isoamyl ester (Neo Heliopan®E 1000)
- p-methoxycinnamic acid diethanolamine salt
- p-methoxycinnamic acid isopropyl ester
- 2-phenylbenzimidazole sulfonic acid and salts (Neo Heliopan®Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan®MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb®HEB)
- benzylidene malonate polysiloxane (Parsol®SLX)
- glyceryl ethylhexanoate dimethoxycinnamate
- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1'-oxy)-1,3,5-triazine) (Uvinul®T150)

Broadband filters which are preferably combined with one or more compounds of formula (I) in a preparation according to the present invention are selected from the group consisting of

- 2-ethylhexyl-2-cyano-3,3-diphenyl acrylate (Neo Heliopan®303)
- ethyl-2-cyano-3,3'-diphenyl acrylate
- 2-hydroxy-4-methoxybenzophenone (Neo Heliopan®BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone

- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl®XL)
- 2,2'-methylene bis-(6-(2H-benzotriazol-2-yl)-4-1,1,3,3-tetramethylbutyl) phenol) (Tinosorb®M)
- 2,4-bis-[4-(2-ethylhexyloxy)-2-hydroxyphenyl]-1,3,5-triazine
- 2,4-bis-[{(4-(2-ethylhexyloxy)-2-hydroxy} phenyl]-6-(4-methoxyphenyl)-1,3,5-triazine (Tinosorb®S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[{4-(2-ethylhexyloxy)-2-hydroxy}phenyl]-6-[4-(2-methoxyethyl carbonyl) phenylamino]-1,3,5-triazine
- 2,4-bis-[{4-(3-(2-propyloxy)-2-hydroxypropyloxy)-2-hydroxy}phenyl]-6-[4-(2-ethylcarboxyl) phenylamino]-1,3,5-triazine
- 2,4-bis-[{4-(2-ethyl hexyloxy)-2-hydroxy}phenyl]-6-(1-methyl pyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy} phenyl]-6-(4-methoxyphenyl)-1,3,5-triazine
- 2,4-bis-[[4-(1',1',1',3',5',5',5'-heptamethylsiloxy-2"-methylpropyloxy)-2-hydroxy} phenyl]-6-(4-methoxyphenyl)-1,3,5-triazine

UV-A filters filters which are preferably combined with one or more compounds of formula (I) in a preparation according to the present invention are selected from the group consisting of

- 4-isopropyl dibenzoyl methane
- terephthalylidene dibornane sulfonic acid and salts (Mexoryl®SX)
- 4-t-butyl-4'-methoxydibenzoyl methane (avobenzone) / (Neo Heliopan®357)
- phenylene bis-benzimidazyl tetrasulfonic acid disodium salt (Neo Heliopan®AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul® A Plus)
- indanylidene compounds in accordance with DE 100 55 940 (= WO 02/38537)

UV filters which are more preferably combined with one or more compounds of formula (I) in a preparation according to the present invention are selected from the group consisting of

- p-aminobenzoic acid
- 3-(4'-trimethylammonium) benzylidene bornan-2-one methyl sulfate
- salicylic acid homomenthyl ester (Neo Heliopan®HMS)
- 2-hydroxy-4-methoxybenzophenone (Neo Heliopan®BB)
- 2-phenylbenzimidazole sulfonic acid (Neo Heliopan®Hydro)
- terephthalylidene dibornane sulfonic acid and salts (Mexoryl®SX)

- 4-tert-butyl-4'-methoxydibenzoyl methane (Neo Heliopan®357)
- 3-(4'-sulfo)benzylidene bornan-2-one and salts
- 2-ethylhexyl-2-cyano-3,3-diphenyl acrylate (Neo Heliopan®303)
- N-[(2 and 4)-[2-(oxoborn-3-ylidene) methyl]benzyl] acrylamide polymer
- p-methoxycinnamic acid-2-ethylhexyl ester (Neo Heliopan®AV)
- p-aminobenzoic acid ethyl ester (25 mol) ethoxylated (INCI name: PEG-25 PABA)
- p-methoxycinnamic acid isoamyl ester (Neo Heliopan®E1000)
- 2,4,6-trianilino-(p-carbo-2'-ethylhexyl-1'-oxy)-1,3,5-triazine (Uvinul®T150)
- phenol, 2-(2H-benzotriazol-2-yl)-4-methyl-6-(2-methyl-3(1,3,3,3-tetramethyl-1-(trimethylsilyl)oxy)disiloxyanyl) propyl) (Mexoryl®XL)
- 4,4'-[(6-[4-(1,1-dimethyl)aminocarbonyl) phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan®MBC)
- 3-benzylidene camphor
- salicylic acid-2-ethylhexyl ester (Neo Heliopan®OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb®M)
- phenylene bis-benzimidazyl tetrasulfonic acid disodium salt (Neo Heliopan®AP)
- 2,4-bis-[{(4-(2-ethylhexyloxy)-2-hydroxy}phenyl]-6-(4-methoxyphenyl)-1,3,5-triazine (Tinosorb®S)
- benzylidene malonate polysiloxane (Parsol®SLX)
- menthyl anthranilate (Neo Heliopan®MA)
- 2-(4-diethylamino-2-hydroxybenzoyl) benzoic acid hexyl ester (Uvinul® A Plus)
- indanylidene compounds in accordance with DE 100 55 940 (= WO 02/38537).

Advantageous inorganic light protection pigments are finely dispersed metal oxides and metal salts which are also mentioned in WO 2005/123101. The total quantity of inorganic pigments, in particular hydrophobic inorganic micropigments in the finished cosmetic preparation according to the present invention is advantageously from 0.1 to 30% by weight, preferably 0.5 to 10.0% by weight, in each case based on the total weight of the preparation.
Furthermore, particulate UV filters or inorganic pigments, which can optionally be hydrophobed, can be used, such as the oxides of titanium ($TiO_2$) zinc (ZnO), iron ($Fe_2O_3$), zirconium ($ZrO_2$) silicon ($SiO_2$) manganese (e.g. MnO), aluminium ($Al_2O_3$), cerium (e.g. $Ce_2O_3$) and/or mixtures thereof.
Preferred cosmetic or pharmaceutical, preferably topical, preparations according to the present invention comprise one, two, three or more skin moisturizing agents of component (b-3), selected from the group consisting of:

alkane diols or alkane triols comprising 3 to 12 carbon atoms, preferably $C_3$-$C_{10}$-alkane diols and $C_3$-$C_{10}$-alkane triols.

More preferably the skin moisturizing agents of component (b-3) are selected from the group consisting of: glycerol, 1,2-propylene glycol, 1,2-butylene glycol, 1,3-butylene glycol, 1,2-pentanediol, 1,2-hexanediol, 1,2-octanediol and 1,2-decanediol.

Preferred cosmetic or pharmaceutical, preferably topical, preparations according to the present invention comprise one, two, three or more further SIRT1 activators of component (b-4), selected from the group consisting of:

Resveratrol, Resveratrol derivatives, in particular Hydroxyresveratrol, Butein, Piceatannol, Isoliquiritigenin, Fisetin, Quercetin, Deoxyrhapontin, Rhapontin, Trihydroxychalcone, Pentahydroxychalcone, Chalcone, Tetrahydroxyflavone, Dihydroxyflavone, Kaempferol, Hydroxyapigenin, Apigenin, Myrecitin, Gossypetin, Morin, Daidzein, Genistein, Naringenin, Catechin, Epicatechin, Gallocatechin, Epigallocatechin, Hinokitiol, Ergothioneine, Ambroxol, Trolox, Dipyridamole, Suramin, Camptothecin, Coumestrol, Pinosylvin, SIRT1-activators as described in US 2005/0136537, incorporated herein by reference regarding the SIRT1-activators disclosed therein, Prosveltyl (Silab) comprising Nelumbo nucifera leaf extract, Longevicell (Silab) comprising hydrolyzed Myrtus communis leaf extract, Sepivinol (Seppic) comprising polyphenols from wine, Orsirtine (ISP Vincience) comprising Oryza Sativa (rice) extract, peptides, in particular Kluyveromyces peptide, Dynachondrine ISR (ISP Vincience) comprising hydrolyzed soy protein, Sirpetide (ISP Vincience) comprising Heptapeptide-6, a synthetic peptide consisting of asparagine, aspartic acid, glutamic acid, glycine, leucine and tyrosine and Matrispondin (Sederma SAS) comprising Palmitoyl Tetrapeptide-10 (a synthetic peptide existing of lysine, threonine and phenylalanine) and ACB Sirtuin Complex (Active Concepts) comprising Saccharomyces/Podophyllum peltatum ferment filtrate.

Preferred actives of component (b-4) are selected from the group consisting of hydroxyresveratrol, resveratrol, Myrtus communis leaf extract and palmitoyl tetrapeptide-10.

Preferred cosmetic or pharmaceutical, preferably topical, preparations according to the present invention comprise one, two, three or more further proteasome activators of component (b-5), selected from the group consisting of:

Phaeodactylum tricornutum, Palmitoyl Isoleucin, Olea Europaea (olive) leaf extract, Hydrolyzed Candida Saitoana Extract (Silab) and Plankton extract from Scenedesmus from Sahel (Biotech Marine).

Preferred actives of component (b-5) are selected from the group consisting of oleuropein, Phaeodactylum tricornutum extract and palmitoyl isoleucin.

Preferred cosmetic or pharmaceutical, preferably topical, preparations according to the present invention comprise one, two, three or more substances stimulating the synthesis of glycosaminoglycans of component (b-6), selected from the group consisting of:

hyaluronic acid, Subliskin (Sederma, INCI: Sinorhizobium Meliloti Ferment Filtrate, Cetyl Hydroxyethylcellulose, Lecithin), Hyalufix (BASF, INCI: Water, Butylene Glycol, Alpinia galanga leaf extract, Xanthan Gum, Caprylic/Capric Triglyceride), Stimulhyal (Soliance, INCI: Calcium ketogluconate), Syn-Glycan (DSM, INCI: Tetradecyl Aminobutyroylvalylaminobutyric Urea Trifluoroacetate, Glycerin, Magnesium chloride), Kalpariane (Biotech Marine), DC Upregulex (Distinctive Cosmetic Ingredients, INCI: Water, Butylene Glycol, Phospholipids, Hydrolyzed Sericin), glucosamine, N-acetyl glucosamine, retinoids, preferably retinol and vitamin A, Arctium lappa fruit extract, Eriobotrya japonica extract, Genkwanin, N-Methyl-L-serine and soy protein hydrolysate.

Preferred actives of component (b-6) are selected from the group consisting of hyaluronic acid, retinol, retinyl palmitate, Alpinia galanga leaf extract and tetradecyl aminobutyroylvalylaminobutyric urea trifluoroacetate.

Preferred preparations, preferably cosmetic preparations, according to the invention containing one or more compounds of formula (I) preferably additionally contain one or more active ingredients which prevent a breakdown of the connective tissue. Active ingredients are advantageous here which inhibit matrix-metallo-proteinases (MMPs). These enzymes are in a position to break down macromolecules of the extra-cellular matrix (ECM) of the connective tissue, also including the collagens, proteolytically. In particular the matrix-metallo-proteinase-1 (MMP-1), matrix-metallo-proteinase-2 (MMP-2) and matrix-metallo-proteinase-9 (MMP-9) are responsible for the breakdown of the connective tissue of the skin.

In order to counteract the breakdown of the connective tissue, the combination of active ingredients, which encourage the formation of collagen in the tissue (collagen stimulants), is furthermore advantageous in preferred cosmetic preparations according to the invention containing one or more compounds of formula (I). Preferably, one or more compounds of component (b-7) and/or component (b-8) are used in this respect.

Preferred cosmetic or pharmaceutical, preferably topical, preparations according to the present invention comprise one, two, three or more matrix-metalloproteinase inhibitors of component (b-7), selected from the group consisting of:

Ursolic acid, retinyl palmitate, propyl gallate, precocenes, 6-hydroxy-7-methoxy-2,2-dimethyl-1 (2H)-benzopyran, 3,4-dihydro-6-hydroxy-7-methoxy-2,2-dimethyl-1 (2H)-benzopyran, benzamidine hydrochloride, the cysteine proteinase inhibitors N-ethylmalemide and epsilon-amino-n-caproic acid of the serinprotease inhibitors: phenyl-methylsufonylfluoride, collhibin (company Pentapharm; INCI: hydrolysed rice protein), oenotherol (company Soliance; INCI: propylene glycol, aqua, Oenothera biennis root extract, ellagic acid and ellagitannins, for example from pomegranate), phosphoramidone hinokitiol, EDTA, galardin, EquiStat (company Collaborative Group; apple fruit extract, soya seed extract, ursolic acid, soya isoflavones and soya proteins), sage extracts, MDI (company Atrium; INCI: glycosaminoglycans), fermiskin (company Silab/Mawi; INCI: water and lentinus edodes extract), actimp 1.9.3 (company Expanscience/Rahn; INCI: hydrolysed lupine protein), lipobelle soyaglycone (company Mibelle; INCI: alcohol, polysorbate 80, lecithin and soy isoflavones), extracts from green and black tea and further plant extracts, which are listed in WO 02/069992 (see tables 1-12 there, incorporated herein by reference), proteins or glycoproteins from soya, hydrolysed proteins from rice, pea or lupine, plant extracts which inhibit MMPs, preferably extracts from shitake mushrooms, extracts from the leaves of the Rosaceae family, sub-family Rosoideae, quite particularly extracts of blackberry leaf (preferably as described in WO 2005/123101 A1, incorporated herein by reference).

Preferred actives of component (b-7) are selected from the group consisting of retinyl palmitate, ursolic acid, genistein and daidzein.

Preferred cosmetic or pharmaceutical, preferably topical, preparations according to the present invention comprise one, two, three or more substances stimulating the formation of collagen of component (b-8), selected from the group consisting of:

ascorbic acid and derivatives, retinol, retinol derivatives, magnesium ascorbyl phosphate, plant extracts, preferably extracts of aloe and centella species, peptidic materials and their derivatives, preferably carnitine, carnosine, creatine, matrikine peptides (preferably lysyl-threonyl-threonyl-lysyl-serine), palmitoylated pentapeptides (preferably Matrixyl™ from Sederma) or the oligopeptide with the trade name Vincipeptide (company Vincience/France), asiatic acid, madecassic acid, madecassoside, asiaticoside, extracts of Centella asiatica, niacinamide, astaxanthine, glucans, preferably from yeast or oats, soya extracts and soya isoflavones, preferably genistein and daidzein, rutin, chrysin, morin, betel nut alkaloids, forskolin, betulinic acid, extracts of Plantago species, TGF-beta, extracts from Ginkgo biloba, glutamine, glycolic acid, and mixtures of aloe vera extract, raspberry extract and magnesium ascorbyl phosphate.

Preferred actives of component (b-8) are selected from the group consisting of carnitine, carnosine and magnesium ascorbyl phosphate.

Further, a preferred cosmetic or pharmaceutical, preferably topical, preparation according to the present invention contains as component (b) one, a plurality of or all the further anti-ageing actives selected from groups (b-1) to (b-8) consisting of:

(b-1) vitamin A, tocopherol, tocopheryl acetate, vitamin C and ubiquinone;

(b-2) organic UV absorbers from the class of 4-aminobenzoic acid and 4-aminobenzoic acid derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives, indole derivatives;

(b-3) $C_3$-$C_{10}$-alkane diols and $C_3$-$C_{10}$-alkane triols, preferably glycerol, 1,2-propylene glycol, 1,2-butylene glycol, 1,3-butylene glycol, 1,2-pentanediol, 1,2-hexanediol, 1,2-octanediol, 1,2-decanediol;

(b-4) hydroxyresveratrol, resveratrol, Myrtus communis leaf extract, palmitoyl tetrapeptide-10;

(b-5) oleuropein, Phaeodactylum tricornutum extract, palmitoyl isoleucin;

(b-6) hyaluronic acid, retinol, retinyl palmitate, Alpinia galanga leaf extract, tetradecyl aminobutyroylvalylaminobutyric urea trifluoroacetate;

(b-7) retinyl palmitate, ursolic acid, genistein, daidzein;

(b-8) carnitine, carnosine and magnesium ascorbyl phosphate.

In case vitamin A is used as component (b-1) or as constituent of component (b-1), the total amount thereof preferably is in the range of from 0.1 to 3 wt.%, based on the total weight of the preparation.
In case vitamin E and/or its acetate are used as component (b-1) or as constituent(s) of component (b-1), the total amount thereof preferably is in the range of from 0.1 to 2 wt.%, based on the total weight of the preparation.
In case vitamin C is used as component (b-1) or as constituent of component (b-1), the total amount thereof preferably is in the range of from 0.01 to 3 wt.%, based on the total weight of the preparation.
In case ubiquinone is used as component (b-1) or as constituent of component (b-1), the total amount thereof preferably is in the range of from 0.001 to 0.1 wt.%, based on the total weight of the preparation.
In case Hydroxyresveratrol is used as component (b-4) or as constituent of component (b-4), the total amount thereof preferably is in the range of from 0.01 to 0.5 wt.%, based on the total weight of the preparation.
In case Resveratrol is used as component (b-4) or as constituent of component (b-4), the total amount thereof preferably is in the range of from 0.001 to 0.2 wt.%, based on the total weight of the preparation.
In case Myrtus communis leaf extract is used as component (b-4) or as constituent of component (b-4), the total amount thereof preferably is in the range of from 0.1 to 4 wt.%, based on the total weight of the preparation.
In case Palmitoyl Tetrapeptide-10 is used as component (b-4) or as constituent of component (b-4), the total amount thereof preferably is in the range of from 0.01 to 3 wt.%, based on the total weight of the preparation.
In case Oleuropein is used as component (b-5) or as constituent of component (b-5), the total amount thereof preferably is in the range of from 0.1 to 10 wt.%, based on the total weight of the preparation.
In case Phaeodactylum tricornutum extract is used as component (b-5) or as constituent of component (b-5), the total amount thereof preferably is in the range of from 0.1 to 3 wt.%, based on the total weight of the preparation.
In case Palmitoyl Isoleucin is used as component (b-5) or as constituent of component (b-5), the total amount thereof preferably is in the range of from 0.1 to 2 wt.%, based on the total weight of the preparation.
In case Hyaluronic acid is used as component (b-6) or as constituent of component (b-6), the total amount thereof preferably is in the range of from 0.01 to 3 wt.%, based on the total weight of the preparation.
In case Retinol is used as component (b-6) or as constituent of component (b-6), the total amount thereof preferably is in the range of from 0.01 to 1 wt.%, based on the total weight of the preparation.
In case Retinyl palmitate is used as component (b-6) or as constituent of component (b-6), the total amount thereof preferably is in the range of from 0.01 to 1 wt.%, based on the total weight of the preparation.
In case Alpinia galanga leaf extract is used as component (b-6) or as constituent of component (b-6), the total amount thereof preferably is in the range of from 0.1 to 5 wt.%, based on the total weight of the preparation.
In case Tetradecyl Aminobutyroylvalylaminobutyric Urea Trifluoroacetate is used as component (b-6) or as constituent of component (b-6), the total amount thereof preferably is in the range of from 0.1 to 3 wt.%, based on the total weight of the preparation.
In case Retinyl palmitate is used as component (b-7) or as constituent of component (b-7), the total amount thereof preferably is in the range of from 1 to 3 wt.%, based on the total weight of the preparation.
In case Ursolic acid is used as component (b-7) or as constituent of component (b-7), the total amount thereof preferably is in the range of from 0.01 to 1 wt.%, based on the total weight of the preparation.
In case Genistein is used as component (b-7) or as constituent of component (b-7), the total amount thereof preferably is in the range of from 0.01 to 2 wt.%, based on the total weight of the preparation.
In case Daidzein is used as component (b-7) or as constituent of component (b-7), the total amount thereof preferably is in the range of from 0.01 to 2 wt.%, based on the total weight of the preparation.
In case Carnitine is used as component (b-8) or as constituent of component (b-8), the total amount thereof preferably is in the range of from 0.05 to 0.2 wt.%, based on the total weight of the preparation.
In case Carnosine is used as component (b-8) or as constituent of component (b-8), the total amount thereof preferably is in the range of from 0.05 to 0.2 wt.%, based on the total weight of the preparation.
In case Magnesium ascorbyl phosphate is used as component (b-8) or as constituent of component (b-8), the total amount thereof preferably is in the range of from 0.01 to 3 wt.%, based on the total weight of the preparation.
A cosmetic or pharmaceutical, preferably topical, preparation according to the present invention may additionally comprise one or more active ingredients for skin lightening selected from the group consisting of:

kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), sulfur-containing molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid,

thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for skin lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract and/or grape extract.

Preferred skin lighteners as component (b) are kojic acid and phenylethyl resorcinol as tyrosinase inhibitors, beta- and alpha-arbutin, hydroquinone, nicotinamide, dioic acid, Mg ascorbyl phosphate and vitamin C and its derivatives, mulberry extract, Bengkoang extract, papaya extract, turmeric extract, nutgrass extract, licorice extract (containing glycyrrhizin), alpha-hydroxy-acids, 4-alkylresorcinols, 4-hydroxyanisole. These skin lighteners are preferred due to their very good activity, in particular in combination with one or more of the preferred or particularly preferred compounds of formula (I) according to the present invention.

Preparations according to the present invention including (a) one or more compounds of formula (I) and (b) one or more tyrosinase inhibitors have shown to exhibit particularly improved, in particular faster and/or stronger, activity, based on the modulation of two independent cellular mechanisms.

Preferred skin lightening actives to be combined with one or more compounds of formula (I) are tyrosinase inhibitors and are preferably selected from the group consisting of kojic acid and skin lightening resorcinol derivatives, preferably 4-alkylresorcinols, in particular 4-C3-C8-alkylresorcinols, and 4-(1-phenylethyl)1,3-dihydroxybenzene.

The total amount of the one or more skin lightening active ingredients suitable for cosmetic or pharmaceutical application which are not compounds of formula (I) is preferably in the range of from 0.01 to 30 wt.%, more preferably in the range of from 0.01 to 20 wt.%, particularly preferably in the range of from 0.01 to 5 wt.%, in each case based on the total weight of the preparation.

Cosmetic preparations preferred according to the invention can also contain anti-inflammatory and/or redness and/or itch ameliorating active ingredients. The compounds mentioned in WO 2005/123101 are advantageously used as anti-inflammatory or redness and/or itch ameliorating active ingredients.

The melanin production is often stimulated as a result of an inflammation, a process called postinflammatory hyperpigmentation. Skin insults that result in inflammation/irritation can induce postinflammatory hyperpigmentation. Among such insults are acne lesions, ingrown hairs, scratches, insect bites, and surfactant damage. One of the most common forms of postinflammatory hyperpigmentations is tanning following exposure to sunlight as a response to UV damage to skin. Although in the latter, there may not be visible erythema, histologically, such exposed skin has elevated inflammatory/irritant cell content, yielding a "subclinical" inflammatory/irritant process. Thus to prevent inflammation/irritation of the skin is beneficial regarding the inhibition of melanogenesis in the skin.

One further area of application of compounds of formula (I) and of preparations according to the present invention is the therapeutic treatment of melanin-induced pigmentation disorders such as hyperpigmentations (e.g. scar hyperpigmentations, post-traumatic drug-induced hyperpigmentations, post-inflammatory hyperpigmentations induced by phototoxic reactions, ephelides).

Reactive oxygen species, such as superoxide and nitric oxide, generated in damaged skin (e.g. resulting from UV exposure) or released as by-products from inflammatory cells are known stimulators of melanogenesis in melanocytes. In such a case it is important to maintain the cellular redox by the suppression of reactive oxygen species, and to boost anti-oxidative defenses for the prevention of melanogenesis.

Thus, preferred preparations, preferably cosmetic preparations, according to the invention containing one or more compounds of formula (I) preferably additionally contain

- one or more agents selected from the group of antioxidants, and
- one or more UV-absorbers, preferably selected from the preferred UV-filters of component (b-2), above, and
- optionally one or more agents selected from the group of anti-irritants and anti-inflammatory substances, and
- optionally one or more skin lightening agents.

The total amount of anti-irritants (one or more compounds) and anti-inflammatory substances (one or more compounds) in the preparations according to the invention is preferably 0.01 to 20 wt.%, particularly preferably 0.03 to 10 wt.%, in particular 0.05 to 5 wt.%, based on the total weight of the preparation.

Steroidal anti-inflammatory substances of the corticosteroid type, such as e.g. hydrocortisone, dexamethasone, dexamethasone phosphate, methyl prednisolone or cortisone, are advantageously used as anti-inflammatory active ingredients or active ingredients to relieve reddening and itching, the list of which can be extended by the addition of other steroidal anti-inflammatories. Non-steroidal anti-inflammatories can also be used. Examples which can be cited here are oxicams such as piroxicam or tenoxicam; salicylates such as aspirin, disalcid, solprin or fendosal; acetic acid derivatives such as diclofenac, fenclofenac, indomethacin, sulindac, tolmetin or clindanac; fenamates such as mefenamic, meclofenamic, flufenamic or niflumic; propionic acid derivatives such as ibuprofen, naproxen, benoxaprofen or pyrazoles such as phenylbutazone, oxyphenylbutazone, febrazone or azapropazone. Anthranilic acid derivatives, in particular avenanthramides described in WO 2004/047833, are preferred anti-itch ingredients in a composition according to the present invention. Alternatively, natural anti-inflammatory substances or substances to relieve reddening and itching can be used. Plant extracts, special highly active plant extract fractions and highly pure active substances isolated from plant extracts can be used. Particularly preferred are extracts, fractions and active substances from camomile, aloe vera, commiphora species, rubia species, echinacea species, willow, willowherb, oats, black and green tea, gingko, coffee, pepper, blackcurrant, tomato, vanilla, almonds, as well as pure substances such as inter alia bisabolol, apigenin-7-glucoside, boswellic acid, phytosterols, glycyrrhizinic acid, glabridin or licochalcone A.

In other preferred embodiments, a composition according to the present invention, comprises one or more actives providing a benefit for the skin, in particular skin irritation-reducing or skin-soothing agents, preferably selected from the group consisting of anti-inflammatory agents, compounds that alleviate itching and/or compounds that alleviate reddening which are suitable for cosmetic and/or dermatological applications, wherein the one or more actives are preferably selected from the groups consisting of:

- steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone; and/or

- natural or naturally occuring anti-inflammatory mixtures of substances or mixtures of substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea; preferably selected from the group consisting of extracts or fractions from camomile, Aloe vera, oats, calendula, arnica, honeysuckle, rosemary, witch hazel, ginger or Echinacea, and/or

- pure substances, preferably alpha-bisabolol, apigenin, apigenin-7-glucoside, gingerols, shogaols, gingerdiols, dehydrogingerdiones, paradols, natural or naturally occuring avenanthramides, preferably tranilast, avenanthramide A, avenanthramide B, avenanthramide C, non-natural or non-naturally occuring avenanthramides, preferably dihydroavenanthramide D, dihydroavenanthramide E, avenanthramide D, avenanthramide E, avenanthramide F, boswellic acid, phytosterols, glycyrrhizin, glabridin and licochalcone A; preferably selected from the group consisting of alpha-bisabolol, gingerols, shogaols, gingerdiols, dehydrogingerdiones, paradols, natural avenanthramides, non-natural avenanthramides, preferably dihydroavenanthramide D (as described in WO 2004/047833), boswellic acid, phytosterols, glycyrrhizin, and licochalcone A; and/or

Preferably a preparation according to the present invention comprises one or more actives selected from the groups consisting of:

- extracts or fractions from camomile, Aloe vera, oats, calendula, arnica, honeysuckle, rosemary, witch hazel, ginger or Echinacea; and/or

- alpha-bisabolol, gingerols, shogaols, gingerdiols, dehydrogingerdiones, paradols, natural avenanthramides, non-natural avenanthramides, preferably dihydroavenanthramide D, boswellic acid, phytosterols, glycyrrhizin, and licochalcone A; and/or

- allantoin, panthenol, lanolin, (pseudo-)ceramides [preferably Ceramide 2, hydroxypropyl bispalmitamide MEA, cetyloxypropyl glyceryl methoxypropyl myristamide, N-(1-hexadecanoyl)-4-hydroxy-L-proline (1-hexadecyl) ester, hydroxyethyl palmityl oxyhydroxypropyl palmitamide], glycosphingolipids, phytosterols, chitosan, mannose, lactose and β-glucans, in particular 1,3-1,4-β-glucan from oats.

When bisabolol is used in the context of the present invention it can be of natural or synthetic origin, and is preferably "alpha-bisabolol". Preferably, the bisabolol used is synthetically prepared or natural (-)-alpha-bisabolol and/or synthetic mixed-isomer alpha-bisabolol. If natural (-)-alpha-bisabolol is used, this can also be employed as a constituent of an essential oil or of a plant extract or of a fraction thereof, for example as a constituent of (fractions of) oil or extracts of camomile or of Vanillosmopsis (in particular Vanillosmopsis erythropappa or Vanillosmopsis arborea). Synthetic alpha-bisabolol is obtainable, for example, under the name "Dragosantol" from Symrise.

In case ginger extract is used in the context of the present invention, preferably extracts of the fresh or dried ginger root are used which are prepared by extraction with methanol, ethanol, iso-propanol, acetone, ethyl acetate, carbon dioxide ($CO_2$) hexane, methylene chloride, chloroform or other solvents or solvent mixtures of comparable polarity. The extracts are characterized by the presence of active skin irritation-reducing amounts of constituents such as e.g. gingerols, shogaols, gingerdiols, dehydrogingerdiones and/or paradols.

The (particularly) preferred compounds of formula (I) of the present invention are preferably used in the preferred compositions indicated hereinbefore or hereinafter.

The present invention further relates to novel compounds of formula (I) or a cosmetically acceptable salt of a compound of formula (I) or a mixture containing two or more of these compounds or the salts thereof, selected from the group consisting of:

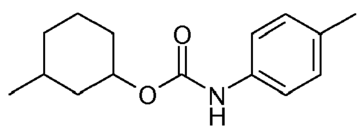
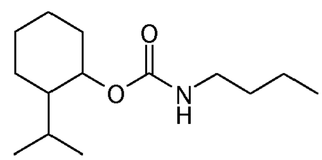

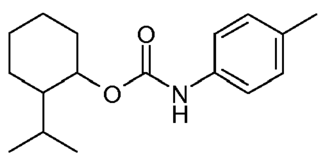
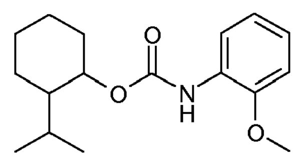

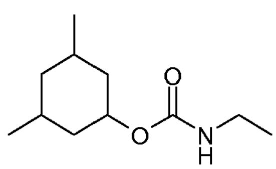
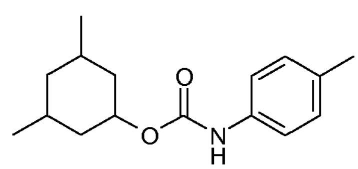

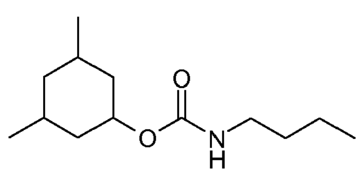
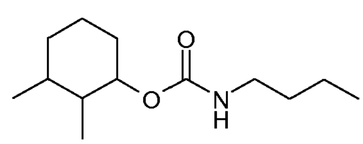

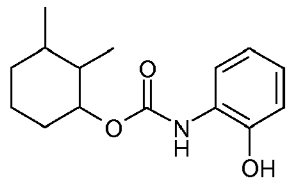
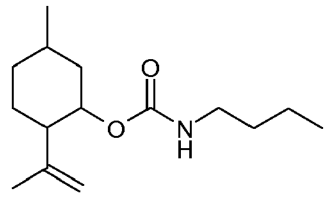
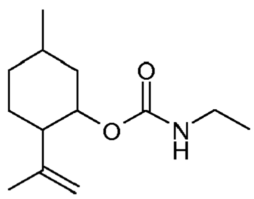
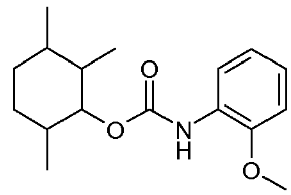
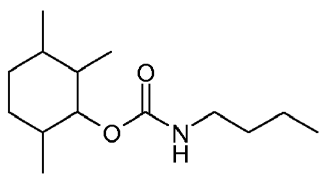
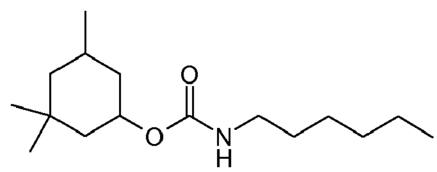
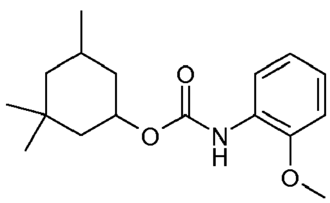
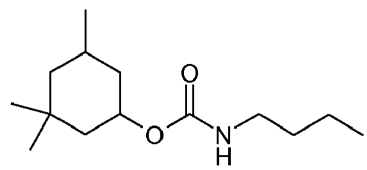
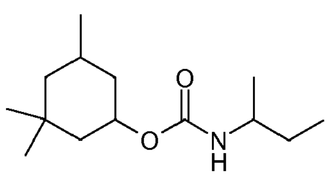
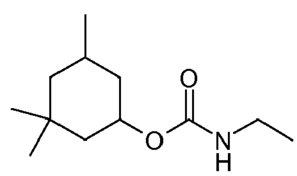
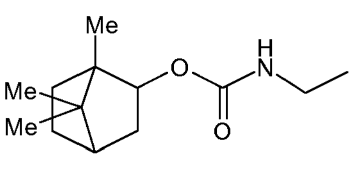
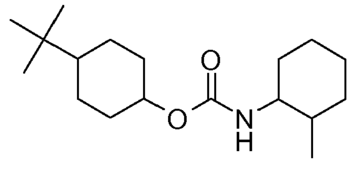

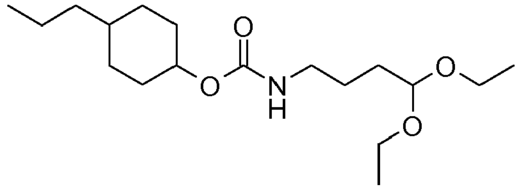

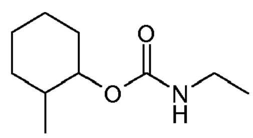

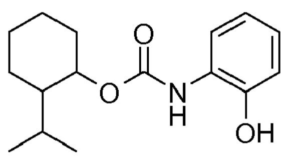

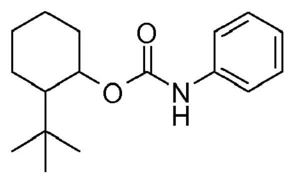

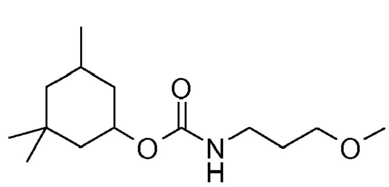

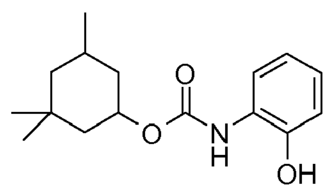

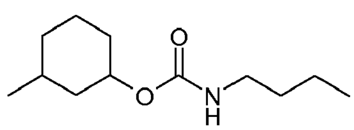

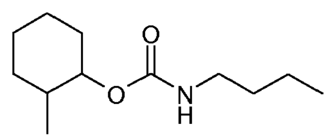

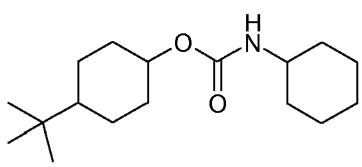

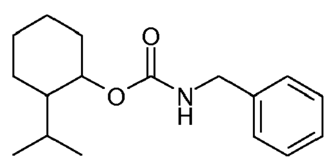

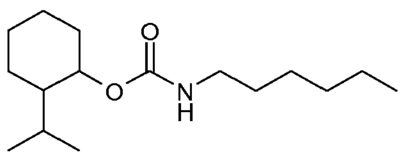

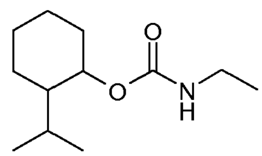

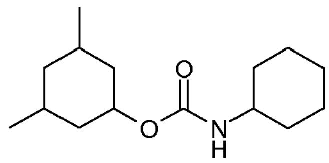

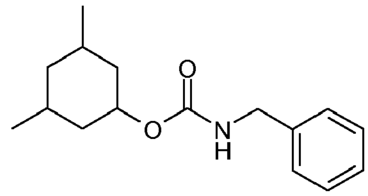

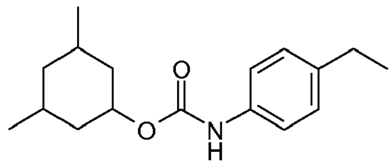

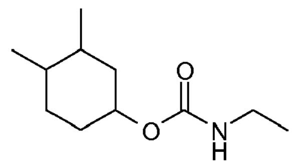

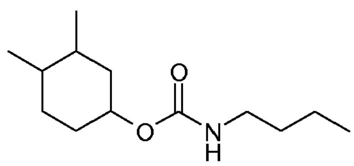

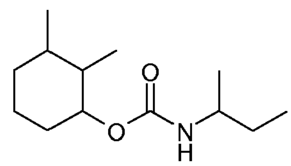

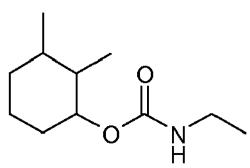

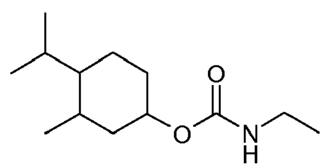

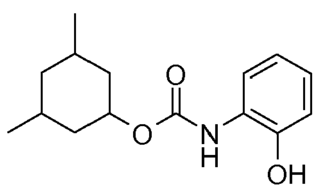

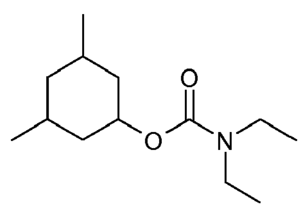

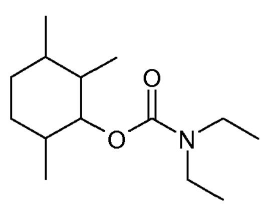

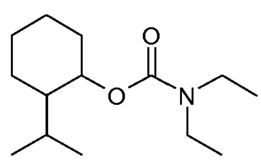

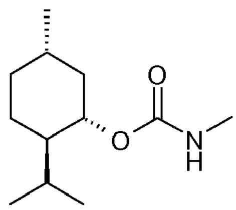

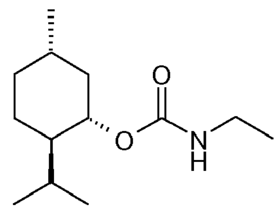

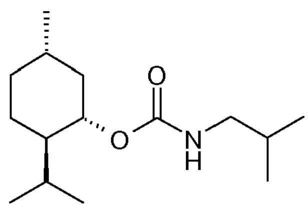

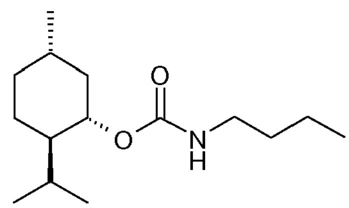

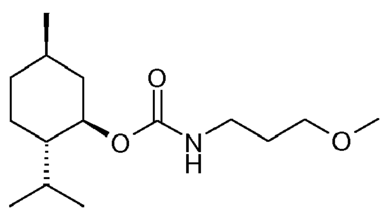

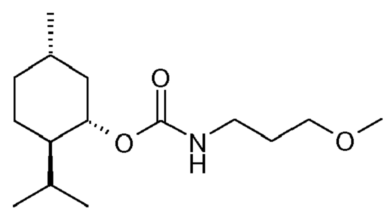

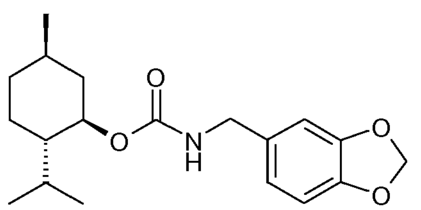

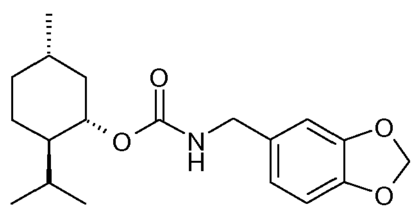

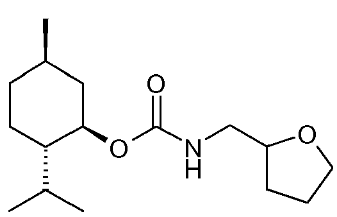

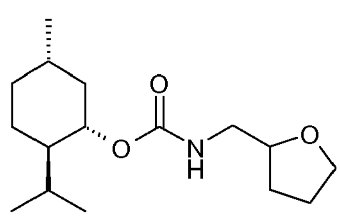

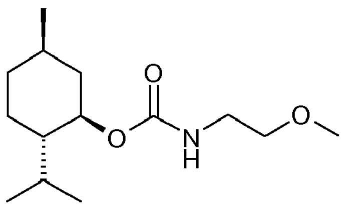

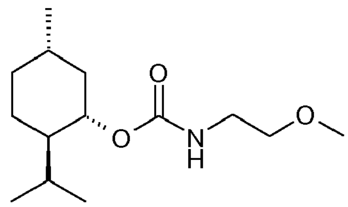

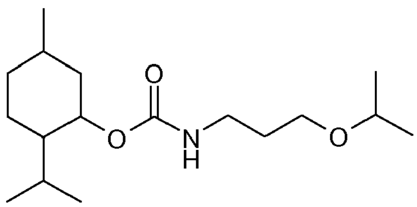

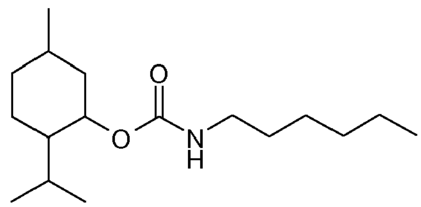

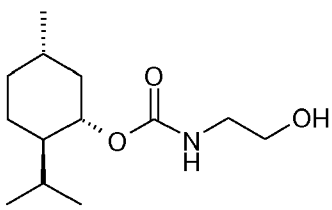

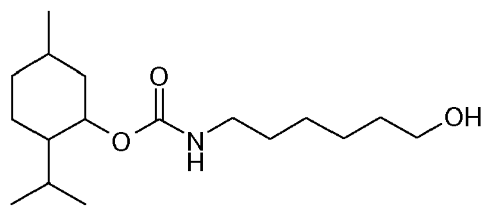

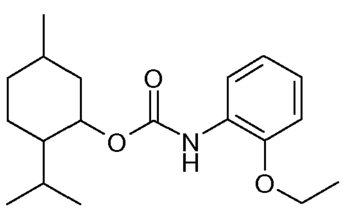

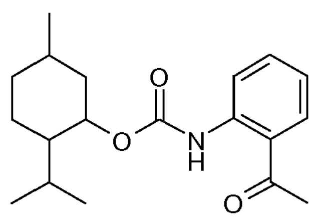

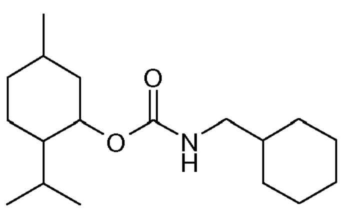

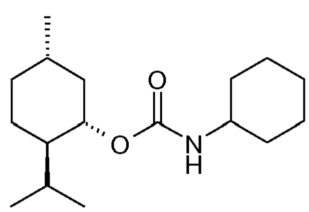

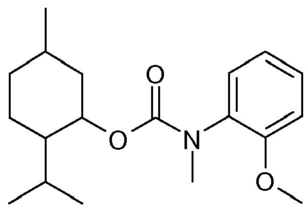

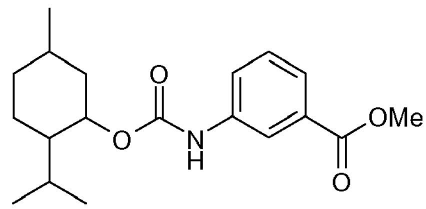

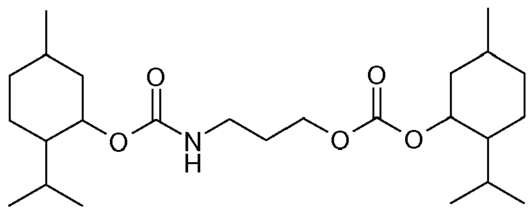

In some embodiments, for technical or non-technical reasons, as the case may be, from the compounds used in accordance with the present invention and/or compositions, preferably topical compositions, preferred the following compounds and their cosmetically acceptable salts are excluded:

compounds of formula (I), in particular the compounds of formula (Carb-II) and more specifically of formula (Carb-II-R1H) or of formula (M-X), in which $R^2$ denotes 3-Mephenyl, naphthyl, biphenyl, p-hydroxyphenyl, 2-(3,4-dihydroxyphenyl)ethyl, 3,4-dihydroxybenzyl, p-carboxyphenyl,
and/or

compounds of formula (I), in particular the compounds of formula (Carb-II) and more specifically of formula (Carb-II-R1H), in which $R^2$ denotes phenyl, methyl,
and/or

compounds of formula (I), in particular the compounds of formula (Carb-II) and more specifically of formula (Carb-II-R1H) or of formula (M-X), in which $R^2$ contains one, several or all of the following groups:

-COOH in alpha-position of N,

=CH2,

a carbon-carbon triple bond,

-COOR in alpha- or beta-position of N, wherein R is a C1-C4-alkyl-radical,

-O(CO)-Ph in alpha-position of N, and/or

a 3,4-dihydroxyphenyl - group, preferably a dihydroxyphenyl - group.

The (particularly) preferred compounds of formula (I) of the present invention are preferably used in the preferred compositions indicated hereinbefore or hereinafter.

The (particularly) preferred aspects and embodiments mentioned hereinbefore or hereinafter relating to compounds of formula (I) or compositions (preparations) comprising one or more compounds of formulae (I), (Carb-II), (Carb-II-R1H) and (M-X) according to the present invention also apply to (particularly) preferred aspects and embodiments, uses and methods in accordance with the present invention.

The present invention further relates to a method for the cosmetic

- proteasomal clearance in a cell, preferably in a human skin cell and/or a human neuronal cell, and/or
- stimulation of activity and/or expression of one or more sirtuin proteins (sirtuins), preferably of SIRT1, and/or
- prevention, treatment or reduction of a skin ageing effect, in particular wrinkles, age spots, photo-ageing, cellular senescence and/or inflammation-induced ageing, preferably in a human skin cell and/or a human neuronal cell, and/or
- regulation of the skin tone, and/or
- prevention, treatment or reduction of oxidative stress, and/or
- inhibition of UV-induced erythema,
  comprising the following step:
  - application, preferably topical application, of a cosmetically effective amount of a compound of formula (I) or a cosmetically acceptable salt of a compound of formula (I) or a mixture containing two or more of these compounds or the salts thereof as defined herein or of a cosmetic composition as defined herein.

A further aspect of the present invention is the use of a compound of formula (I) or a pharmaceutically acceptable salt of a compound of formula (I) or a mixture containing two or more of these compounds or the salts thereof as defined herein for

- preventing or treating an age-associated disease, more preferably for preventing or treating a neurodegenerative disease, in particular Alzheimer’s disease, Parkinson’s disease and/or Huntington’s disease,
  and/or
- preventing, treating or reducing a skin ageing effect, in particular age spots, hyper-pimentation, photo-ageing and/or inflammation-induced ageing,
  and/or
- increasing the insulin sensitivity.

The present invention further relates to a compound of formula (I) or a pharmaceutically acceptable salt of a compound of formula (I) or a mixture containing two or more of these compounds or the salts thereof as defined herein as a drug, preferably as active for

- preventing or treating an age-associated disease, more preferably for preventing or treating a neurodegenerative disease, in particular Alzheimer’s disease, Parkinson’s disease and/or Huntington’s disease,
  and/or
- preventing, treating or reducing a skin ageing effect, in particular age spots, hyper-pimentation, photo-ageing and/or inflammation-induced ageing,
  and/or
- increasing the insulin sensitivity,
  and/or
  the therapeutic prolongation of the life span of a cell, preferably of a human skin cell and/or a human neuronal cell, by inhibition of cellular senescence, in particular by
- proteasomal clearance in a cell, and/or
- stimulation or increase of proteasome activity and/or proteasome expression, and/or
- stimulation of activity and/or expression of one or more sirtuin proteins (sirtuins), preferably of SIRT1, and/or
- reduction of proinflammatory cytokines, and/or

- prevention, treatment or reduction of oxidative stress, and/or
- induction of cellular antioxidant capacity, and/or
- stimulation of cellular energy metabolism, and/or
- stimulation of mitochondrial protein expression, and/or
- inhibition of UV-induced erythema, and/or
- inhibition of DNA-damage and/or induction of DNA-damage repair systems, and/or
- induction of cellular proliferation, and/or
- reduction and/or degradation of damaged proteins in a cell, preferably in a human skin cell and/or a human neuronal cell.

The present invention further relates to a pharmaceutical composition comprising a pharmaceutically active amount of one or more compounds of formula (I) as defined herein, preferably for

- preventing or treating an age-associated disease, more preferably for preventing or treating a neurodegenerative disease, in particular Alzheimer's disease, Parkinson's disease and/or Huntington's disease,
  and/or
- preventing, treating or reducing a skin ageing effect, in particular age spots, hyper-pimentation, photo-ageing and/or inflammation-induced ageing,
  and/or
- increasing the insulin sensitivity.
  Further, the present invention also relates to a method for treating a disease, preferably for
- preventing or treating an age-associated disease, more preferably for preventing or treating a neurodegenerative disease, in particular Alzheimer's disease, Parkinson's disease and/or Huntington's disease,
  and/or
- preventing, treating or reducing a skin ageing effect, in particular age spots, hyper-pimentation, photo-ageing and/or inflammation-induced ageing,
  and/or
- increasing the insulin sensitivity,
  comprising the following step:
- application, preferably topical application, of a pharmaceutically effective amount of a compound of formula (I) or a pharmaceutically acceptable salt of a compound of formula (I) or a mixture containing two or more of these compounds or the salts thereof as defined herein or of a pharmaceutical composition as defined herein.

The present invention also relates to a cosmetic or therapeutic method for the prolongation of the life span of a cell, preferably of a human skin cell and/or a human neuronal cell, by inhibition of cellular senescence, in particular by

- proteasomal clearance in a cell, and/or
- stimulation or increase of proteasome activity and/or proteasome expression, and/or
- stimulation of activity and/or expression of one or more sirtuin proteins (sirtuins), preferably of SIRT1,
  comprising the steps of:
  - provision of one or more compounds of formula (I) or a cosmetically or pharmaceutically acceptable salt thereof, or of a cosmetic or pharmaceutical composition according to the present invention,

- application, preferably topical application, of the one or more compounds of formula (I) or of the composition to human skin in an effective amount,

said application preferably remaining for at least 10 minutes, more preferably for at least 30 minutes, most preferably for at least 60 minutes, on said skin ("leave-on product").

The present invention also relates to a cosmetic or therapeutic method for the

- prevention, treatment or reduction of skin ageing effects, in particular wrinkles, age spots, photo-ageing, hyperpigmentation and inflammation-induced ageing,
  and/or

- regulation of the skin tone leading to even skin tone,
  comprising the steps of:

  - provision of one or more compounds of formula (I) or a cosmetically or pharmaceutically acceptable salt thereof, or of a cosmetic or pharmaceutical composition according to the present invention,

  - application, preferably topical application, of the one or more compounds of formula (I) or of the composition to human skin in an effective amount,

wherein said application preferably remains on said skin for at least 10 minutes, more preferably for at least 30 minutes, most preferably for at least 60 minutes ("leave-on product").

Substances and auxiliaries which may additionally contain a preparation according to the invention containing one or more compounds of formula (I) are, for example:

preservatives, in particular those described in US 2006/0089413, antimicrobial agents, such as e.g. antibacterial agents or agents to treat yeast and mold, in particular those described in WO 2005/123101, antiacne and sebum reducing agents, in particular those described in WO 2008/046791, further compounds against ageing of the skin, in particular those described in WO 2005/123101 and US 2009/0232915, anti-cellulite agents, in particular those described in WO 2007/077541, anti-dandruff agents, in particular those described in WO 2008/046795, anti-irritants (ant-iinflammatory agents, irritation-preventing agents, irritation-inhibiting agents), in particular those described in WO 2007/042472 and US 2006/0089413, (further) antioxidants, in particular those described in WO 2005/123101, carrier materials, in particular those described in WO 2005/123101, chelating agents, in particular those described in WO 2005/123101, deodorizing agents and antiperspirants, in particular those described in WO 2005/123101, (further) moisture regulators (moisture-donating agents, moisturizing substance, moisture-retaining substances), in particular those described in WO 2005/123101, osmolytes, in particular those described in WO 2005/123101, compatible solutes, in particular those described in WO 01/76572 and WO 02/15868, proteins and protein hydrolysates, in particular those described in WO 2005/123101 and WO 2008/46676, skin-lightening agents, in particular those described in WO 2007/110415, cooling agents, in particular those described in WO 2005/123101, skin-cooling agents, in particular those described in WO 2005/123101, skin warming agents, in particular those described in WO 2005/123101, (further) UV-absorbing agents, in particular those described in WO 2005/123101, (further) UV filters, in particular those described in WO 2005/123101, benzylidene-beta-dicarbonyl compounds in accordance with WO 2005/107692 and alpha-benzoyl-cinnamic acid nitriles in accordance with WO 2006/015954, insect repellents, in particular those described in WO 2005/123101, plant parts, plant extracts, in particular those described in WO 2005/123101, vitamins, in particular those described in WO 2005/123101, emulsifiers, in particular those described in WO 2005/123101, gelling agents, in particular those described in WO 2005/123101, oils in particular those described in WO 2005/123101, waxes in particular those described in WO 2005/123101, fats in particular those described in WO 2005/123101, phospholipids, in particular those described in WO 2005/123101, saturated fatty acids and mono- or polyunsaturated fatty acids and $\alpha$-hydroxy acids and polyhydroxy-fatty acids and esters of saturated and/or unsaturated branched and/or unbranched alkane carboxylic acids, in particular those described in WO 2005/123101, surface-active substances (surfactants), in particular those described in WO 2005/123101, skin repair agents comprising cholesterol and/or fatty acids and/or ceramides and/or pseudoceramides, in particular those described in WO 2006/053912, dyestuffs and colorants and pigments, in particular those described in WO 2005/123101, aroma chemicals and flavors and fragrances, in particular those described in S. Arctander, Perfume and Flavor Chemicals, private publishing house, Montclair, N.J., 1969 and Surburg, Panten, Common Fragrance and Flavor Materials, 5th Edition, Wiley-VCH, Weinheim 2006, preferably those explicitly mentioned in US 2008/0070825, alcohols and polyols, in particular those described in WO 2005/123101, organic solvents, in particular those described in WO 2005/123101, silicones and silicone oils and silicone derivatives in particular those described

in WO 2008/046676, virucides, abrasives, astringents, antiseptic agents, antistatics, binders, buffers, cleansing agents, care agents, depilatory agents, softeners, enzymes, essential oils, in particular those described in US 2008/0070825, fibres, film-forming agents (e.g. polyvinyl pyrrolidones, chitosan or chitosan derivatives), fixatives, foam-forming agents, foam stabilizers, substances for preventing foaming, foam boosters, gel-forming agents, hair growth activators, hair growth inhibitors, hair care agents, hair-setting agents, hair-straightening agents, hair-smoothening, bleaching agents, strengthening agents, stain-removing agents, optically brightening agents, impregnating agents, dirt-repellent agents, friction-reducing agents, lubricants, opacifying agents, plasticizing agents, covering agents, polish, gloss agents, polymers in particular those described in WO 2008/046676, powders, peptides, mono-, di- and oligosaccharides, re-oiling agents, abrading agents, skin-soothing agents, skin-cleansing agents, skin care agents, skin-softening agents, skin-smoothing agents, nourishing agents, skin-warming agents, stabilizers, detergents, fabric conditioning agents, suspending agents, thickeners, yeast extracts, algae or microalgae extracts, animal extracts, liquefiers, color-protecting agents, and electrolytes.

In a preferred embodiment, a preparation according to the present invention comprises one or more compounds of formula (I) and one or more hair growth modulating actives, in particular one or more agents to stimulate hair growth.

Preferred agents to stimulate hair growth are selected from the group consisting of pyrimidine derivatives, in particular 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids, in particular caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters, in particular tocopheryl nicotinate, benzyl nicotinate and C1-C6 alkyl nicotinate, proteins, in particular the tripeptide Lys-Pro-Val, diphencypren, hormones, finasteride, dutasteride, flutamide, bicalutamide, pregnane derivatives, progesterone and its derivatives, cyproterone acetate, spironolactone and other diuretics, calcineurin inhibitors, in particular FK506 (Tacrolimus, Fujimycin) and its derivatives, Cyclosporin A and derivatives thereof, zinc and zinc salts, polyphenols, procyanidins, proanthocyanidins, phytosterols, in particular beta-sitosterol, biotin, eugenol, (±)-beta-citronellol, panthenol, glycogen, in particular from mussels, hydrolysates from rice, hydrolysates from wheat, and extracts from microorganisms, algae, microalgae or plants and plant parts, in particular of the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, licorice, grape, apple, barley and hops.

In another preferred embodiment, a preparation according to the present invention comprises one or more compounds of formula (I) and one or more agents to inhibit hair growth.

Preferred agents to inhibit hair growth are selected from the group consisting of activin, activin derivatives or activin agonists, ornithine decarboxylase inhibitors, in particular alpha-difluoromethylomithine or pentacyclic triterpenes, in particular ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, and extracts from microorganisms, algae, microalgae or plants and plant parts, in particular of the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum and Gymnema sylvestre.

Also advantageous are preparations according to the invention which are administered orally, for example in the form of tablets (for example film tablets), coated tablets, capsules (for example gelatin capsules), granulates, juices, solutions emulsions, micro emulsions, sprays or products which can be consumed orally in another form, or in the form of food, which, because of the compound(s) contained therein of formula (I) bring about "beauty from inside".

The following osmolytes may be a component of a preparation according to the invention: sugar alcohols (myo-inositol, mannitol, sorbitol), quaternary amines such as taurine, choline, betaine, betaine glycine, ectoine, diglycerol phosphate, phosphorylcholine, glycerophosphorylcholines, amino acids such as glutamine, glycine, alanine, glutamate, aspartate or proline, phosphatidylcholine, phosphatidylinositol, inorganic phosphates, and polymers of the cited compounds such as proteins, peptides, polyamino acids and polyols. Preferred osmolytes, which may be a component of a preparation according to the invention, are diglycerol phosphate and/or ectoine.

Preferred cosmetics carrier materials, which may be a component of a preparation according to the invention, are solid or liquid at 25°C and 1013 mbar (including highly viscous substances).

Preferred liquid carrier substances, which may be a component of a preparation according to the invention are selected from the group consisting of glycerol, 1,2-propylene glycol, 1,2-butylene glycol, 1,3-butylene glycol, ethanol, water and mixtures of two or more of said liquid carrier materials with water. Optionally, these preparations according to the invention may be produced using preservatives or solubilizers.

Preferred solid carrier materials, which may be a component of a preparation according to the invention are

hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

Furthermore, the preparations according to the invention may be present in encapsulated form, these preferably being encapsulated with a solid covering material, which is preferably selected from starches, degraded or chemically or physically modified starches (in particular dextrins and maltodextrins), gelatins, gum arabic, agar-agar, ghatti gum, gellan gum, modified and non-modified celluloses, pullulan, curdlan, carrageenans, alginic acid, alginates, pectin, inulin, xanthan gum and mixtures of two or more of said substances.

The solid covering material is preferably selected from gelatin (preferred are pork, beef, chicken and/or fish gelatins and mixtures thereof, preferably comprising at least one gelatin with a bloom value of greater than or equal to 200, preferably with a bloom value of greater than or equal to 240), maltodextrin (preferably obtained from maize (corn), wheat, tapioca or potato, preferred maltodextrins have a DE value of 10 - 20), modified cellulose (for example cellulose ether), alginates (for example Na-alginate), carrageenan (beta-, iota-, lambda- and/or kappa carrageenan), gum arabic, curdlan and/or agar-agar. Gelatin is preferably used, in particular, because of its good availability in different bloom values. Particularly preferred, especially for oral use are seamless gelatin or alginate capsules, the covering of which dissolves very rapidly in the mouth or bursts when chewing. Production may take place, for example, as described in EP 0 389 700, US 4,251,195, US 6,214,376, WO 03/055587 or WO 2004/050069.

Preferred cosmetic, dermatological or pharmaceutical preparations according to the present inventions are selected from the group of products for treatment, protecting, care and cleansing of the skin and/or hair or as a make-up product, preferably as a leave-on product (meaning that the one or more compounds of formula (I) stay on the skin and/or hair for a longer period of time, compared to rinse-off products, so that the anti-ageing action thereof is more pronounced).

The formulations according to the invention are preferably in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a solution, e.g. in oil (fatty oils or fatty acid esters, in particular $C_6$-$C_{32}$ fatty acid $C_2$-$C_{30}$ esters) or silicone oil, dispersion, suspension, creme, lotion or milk, depending on the production method and ingredients, a gel (including hydrogel, hydrodispersion gel, oleogel), spray (e.g. pump spray or spray with propellant) or a foam or an impregnating solution for cosmetic wipes, a detergent, e.g. soap, synthetic detergent, liquid washing, shower and bath preparation, bath product (capsule, oil, tablet, salt, bath salt, soap, etc.), effervescent preparation, a skin care product such as e.g. an emulsion (as described above), ointment, paste, gel (as described above), oil, balsam, serum, powder (e.g. face powder, body powder), a mask, a pencil, stick, roll-on, pump, aerosol (foaming, non-foaming or post-foaming), a deodorant and/or antiperspirant, mouthwash and mouth rinse, a foot care product (including keratolytic, deodorant), an insect repellent, a sunscreen, aftersun preparation, a shaving product, aftershave balm, pre- and aftershave lotion, a depilatory agent, a hair care product such as e.g. shampoo (including 2-in-1 shampoo, anti-dandruff shampoo, baby shampoo, shampoo for dry scalps, concentrated shampoo), conditioner, hair tonic, hair water, hair rinse, styling creme, pomade, perm and setting lotion, hair spray, styling aid (e.g. gel or wax), hair smoothing agent (detangling agent, relaxer), hair dye such as e.g. temporary direct-dyeing hair dye, semi-permanent hair dye, permanent hair dye, hair conditioner, hair mousse, eye care product, make-up, make-up remover or baby product.

It is also advantageous to administer the compounds having formula (I) in encapsulated form, e.g. in gelatine, wax materials, liposomes or cellulose capsules.

The formulations according to the invention are particularly preferably in the form of an emulsion, in particular in the form of a W/O, O/W, W/O/W, O/W/O emulsion, PIT emulsion, Pickering emulsion, emulsion with a low oil content, micro- or nanoemulsion, a gel (including hydrogel, hydrodispersion gel, oleogel), a solution e.g. in oil (fatty oils or fatty acid esters, in particular $C_6$-$C_{32}$ fatty acid $C_2$-$C_{30}$ esters)) or silicone oil, or a spray (e.g. pump spray or spray with propellant).

Auxiliary substances and additives can be included in quantities of 5 to 99 wt.%, preferably 10 to 80 wt.%, based on the total weight of the formulation. The amounts of cosmetic or dermatological auxiliary agents and additives and perfume to be used in each case can easily be determined by the person skilled in the art by simple trial and error, depending on the nature of the particular product.

The preparations can also contain water in a quantity of up to 99 wt.%, preferably 5 to 80 wt.%, based on the total weight of the preparation.

The one or more substances with a physiological cooling effect (cooling agents), which can be used in combination with one or more compounds of formula (I) according to the invention, are preferably selected here from the following list: menthol and menthol derivatives (for example L-menthol, D-menthol, racemic menthol, isomenthol, neoisomenthol, neomenthol) menthylethers (for example (l-menthoxy)-1,2-propandiol, (l-menthoxy)-2-methyl-1,2-propandiol, l-menthyl-methylether), menthylesters (for example menthylformiate, menthylacetate, menthylisobutyrate, menthyllactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or $N^{\alpha}$-(menthanecarbonyl)glycinethylester [WS5], as described in US 4,150,052, menthanecarboxylic acid-N-(4-cyanophenyl)amide or menthanecarboxylic acid-N-(4-cyanomethylphenyl)amide as described in WO 2005/049553, methanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (I-(-)-isopulegol, I-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(1-isopropyl)cyclohexane-carboxamide [WS12], oxamates (preferably those described in EP 2 033 688 A2).

The or the plurality of substances with a physiological cooling effect, which can be used in combination with one or more compounds of formula (I) according to the invention, are in particular preferably substances, which at least substantially cause a physiological cooling effect. Such preferred substances are: menthylethers (for example (I-menthoxy)-1,2-propandiol, (I-menthoxy)-2-methyl-1,2-propandiol), polar menthylesters (for example menthyllacetates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate), the semi-esters of menthols with a dicarboxylic acid or derivates thereof (for example mono-menthylsuccinate, mono-menthylglutarate, mono-menthylmalonate, O-menthyl succinic acid ester-N,N-(dimethyl) amide, O-menthyl succinic acid esteramide), not according to the invention, menthane carboxylic acid amides (for example menthane carboxylic acid-N-ethylamide [WS3], $N^{\alpha}$-(menthanecarbonyl)glycinethylester [WS5], menthane carboxylic acid-N-(4-cyanophenyl)amide, menthane carboxylic acid-N-(alkoxyalkyl)amides), menthone - derivatives (for example L-menthone glycerol ketal), 2,3-dimethyl-2-(2-propyl)-butyric acid derivates (for example 2,3-dimethyl-2-(2-propyl)-butyric acid-N-methylamide), pyrrolidone derivatives of cycloalkyldione derivatives (for example 3-methyl-2(1-pyrrolidinyl)-2-cyclopentene-1-one) or tetrahydropyrimidine-2-ones (for example iciline or related compounds, which are described in WO 2004/026840), 1R,2S,5R)-N-(4-Methoxyphenyl)-5-methyl-2-(1-isopropyl)cyclohexane-carboxamide [WS12], L-Menthyl N-methyl oxamate, L-menthyl N-ethyl oxamate (as described in EP 2 033 688).

The total quantity of substances having a physiological cooling effect (one or more compounds) in the preparations according to the invention preferably is in the range of from 0.05 - 5% by weight, more preferably in the range of from 0.1 - 3% by weight, in particular in the range of from 0.25 - 1.5% by weight, in each case based on the total weight of the cosmetic or pharmaceutical preparation.

Components which cause a hot, sharp, tingly or prickly feeling on the skin or on the mucous membranes, in particular flavours with a heat-producing effect and/or sharp tasting compounds (sharp substances) which may, apart from one or more compounds of formula (I), be a component of a preparation according to the invention, are mentioned in WO 2005/123101.

Further, combinations with compounds which reduce the hypersensitivity of skin nerves based on their action as TRPV1 antagonists, e.g. trans-4-tert-butyl cyclohexanol (as described in WO 2009/087242), or indirect modulators of TRPV1 by an activation of the $\mu$-receptor, e.g. acetyl tetrapeptide-15, are preferred.

The following anti-cellulite actives may be a component of a (preferably topical) preparation, preferably a cosmetic preparation, according to the invention: lipolysis stimulators like xanthines, in particular caffeine, extracts containing caffeine, or beta-adrenergic receptor agonists, for example synephrine and derivatives, and agents encouraging the activity of anti-cellulite agents, for example agents which stimulates and/or depolarises C nerve fibres such as capsaicin or vanillyl-nonylamid and derivatives thereof or extracts containing one or more of these substances like extracts obtainable from various species of the genus Capsicum (such as Capsicum annum), and compounds stimulating the microcirculation or draining, preferably selected from the group consisting of butcher's broom extract or its active component ruscogenin, horse chestnut extract or its active component escin, ivy extract and/or pineapple extract, (and) L-carnitine, coenzym A, isoflavonoides, soy extracts, conjugated linoleic acid (CLA). Preferably, anti-cellulite actives as a component of a preparation according to the invention are selected from the group consisting of caffeine, synephrine and/or L-carnitine.

Formulations according to the invention, in particular dermatological formulations, can also advantageously contain dyes and/or coloured pigments, particularly if they are intended for use in the area of decorative cosmetics. The dyes and coloured pigments can be selected from the corresponding positive list in the German cosmetics ordinance or the EU list of cosmetic colorants. In most cases they are identical to the dyes approved for foodstuffs. Advantageous

coloured pigments are for example titanium dioxide, mica, iron oxides (e.g. $Fe_2O_3$ $Fe_3O_4$, FeO(OH)) and/or tin oxide. Advantageous dyes are for example carmine, Berlin blue, chromium oxide green, ultramarine blue and/or manganese violet.

If the topical formulations according to the invention are intended for use in the facial area, it is convenient to choose as the dye one or more substances from the following group: 2,4-dihydroxyazobenzol, 1-(2'-chloro-4'-nitro-1'-phenylazo)-2-hydroxynaphthalene, Ceres red, 2-(4-sulfo-1-naphthylazo)-1-naphthol-4-sulfonic acid, calcium salt of 2-hydroxy-1,2'-azonaphthalene-1'-sulfonic acid, calcium and barium salts of 1-(2-sulfo-4-methyl-1-phenylazo)-2-naphthyl carboxylic acid, calcium salt of 1-(2-sulfo-1-naphthylazo)-2-hydroxynaphthalene-3-carboxylic acid, aluminium salt of 1-(4-sulfo-1-phenylazo)-2-naphthol-6-sulfonic acid, aluminium salt of 1-(4-sulfo-1-naphthylazo)-2-naphthol-3,6-disulfonic acid, 1-(4-sulfo-1-naphthylazo)-2-naphthol-6,8-disulfonic acid, aluminium salt of 4-(4-sulfo-1-phenylazo)-1-(4-sulfophenyl)-5-hydroxypyrazolone-3-carboxylic acid, aluminium and zirconium salts of 4,5-dibromofluorescein, aluminium and zirconium salts of 2,4,5,7-tetrabromofluorescein, 3',4',5',6'-tetrachloro-2,4,5,7-tetrabromofluorescein and its aluminium salt, aluminium salt of 2,4,5,7-tetraiodofluorescein, aluminium salt of quinophthalone disulfonic acid, aluminium salt of indigo disulfonic acid, red and black iron oxide (Colour Index Number (CIN): 77491 (red) and 77499 (black)), iron oxide hydrate (CIN: 77492), manganese ammonium diphosphate and titanium dioxide.

Also advantageous are oil-soluble natural dyes, such as e.g. paprika extracts, β-carotene or cochineal.

Also advantageous within the meaning of the present invention are dermatological formulations containing pearlescent pigments. The types of pearlescent pigment listed below are particularly preferred:

1. Natural pearlescent pigments, such as e.g.

   - "pearl essence" (guanine/hypoxanthine mixed crystals obtained from fish scales) and
   - "mother of pearl" (ground mussel shells)

2. Monocrystalline pearlescent pigments such as e.g. bismuth oxychloride (BiOCl)

3. Layered substrate pigments: e.g. mica / metal oxide

The basis for pearlescent pigments is formed for example by powdered pigments or castor oil dispersions of bismuth oxychloride and/or titanium dioxide and bismuth oxychloride and/or titanium dioxide on mica. The lustre pigment listed under CIN 77163, for example, is particularly advantageous.

The list of cited pearlescent pigments is naturally not intended to be limiting. Advantageous pearlescent pigments within the meaning of the present invention are obtainable in many ways known per se. For example, substrates other than mica can be coated with other metal oxides, such as e.g. silica and the like. $SiO_2$ particles coated with $TiO_2$ and $Fe_2O_3$ ("Ronaspheres"), for example, which are sold by Merck and are particularly suitable for the optical reduction of fine lines, are advantageous.

It can also be advantageous to dispense altogether with a substrate such as mica. Iron pearlescent pigments, which are produced without the use of mica, are particularly preferred. Such pigments are available from BASF, for example, under the trade name Sicopearl Copper 1000.

Particularly advantageous also are special effect pigments, which are available from Flora Tech under the trade name Metasomes Standard / Glitter in various colours (yellow, red, green, blue). Here the glitter particles are mixed with various auxiliary substances and dyes (for example the dyes with CIN 19140, 77007, 77289, 77491).

The dyes and pigments can be present both individually and mixed together and coated with one another, wherein different colour effects can generally be obtained by means of varying coating thicknesses. The total amount of dyes and colouring pigments is advantageously chosen from the range from e.g. 0.1 wt.% to 30 wt.%, preferably 0.5 to 15 wt.%, in particular 1.0 to 10 wt.%, based in each case on the total weight of the (cosmetic) formulations.

A combination with (metal)-chelating agents may also be advantageous in some preparations. (Metal)-chelating agents to be preferably used are the compounds mentioned in WO 2005/123101.

The one or more compounds of formula (I) may advantageously be used, in particular, in cosmetic and dermatological preparations in combination with insect repellents such as, for example, DEET, IR 3225, Dragorepel™ (Symrise GmbH & Co. KG).

The one or more compounds of formula (I) can advantageously be used in particular in cosmetic and dermatological preparations in combination with hair care agents and anti-dandruff active ingredients (for example climbazole, ketoconazole, piroctone oleamine, zinc-pyrithione).

The compounds of formula (I) can also advantageously be used in numerous cases in combination with one or more preservatives in preparations according to the invention. The preservatives mentioned in WO 2005/123101 are preferably selected here.

Preparations according to the invention, apart from one or more compounds of formula (I), may also contain plant extracts which can be used for cosmetic purposes. The plant extracts are preferably selected from the table of listed substances beginning on page 44 of the third edition of the handbook on the contents declaration of cosmetic agents, published by the Industrieverband Körperpflegemittel und Waschmittel e.V. (IKW), Frankfurt. The extracts mentioned in WO 2005/123101 are also particularly advantageous.

In preferred embodiments, a composition according to the present invention, comprises one or more cosmetically acceptable carriers selected from the group consisting of

(i) (alkane) diols having 3 to 10 carbon atoms, preferably selected from the group consisting of 1,2-propylene glycol, 2-methylpropane-1,3-diol, 1,2-butylene glycol, 1,3-butanediol, 1,2-pentanediol, 1,3-pentanediol, 1,5-pentanediol, 2,4-pentanediol, 2-methylpentane-2,4-diol, 1,2-hexanediol, 1,6-hexanediol, 1,2-octanediol, dipropylene glycol, preferably 1,2-butylene glycol, 1,2-pentanediol and/or dipropylene glycol, and/or

(ii-1) esters having 6 to 36 carbon atoms, preferably monoesters, diesters or triesters, preferably selected from the group consisting of diethyl phthalate, diethylhexyl 2,6-naphthalate, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 3,5,5-trimethylhexyl 3,5,5-trimethylhexanoate, 2-ethylhexyl isononanoate, 2-ethylhexyl 3,5,5-trimethylhexanoate, 2-ethylhexyl 2-ethylhexanoate, 2-ethylhexyl palmitate, 2-ethylhexyl laurate, 2-hexyldecyl stearate, cetearyl ethylhexanoate, stearyl heptanoate, stearyl caprylate, 2-octyldodecyl palmitate, oleyl oleate, oleyl erucate, erucyl oleate, erucyl erucate, 2-ethylhexyl isostearate, isotridecyl isononanoate, 2-ethylhexyl cocoate, $C_{12-15}$-alkyl benzoates, cetyl palmitate, triethyl citrate, triacetin (triacetyl citrate), benzyl benzoate, benzyl acetate, vegetable oils (preferably olive oil, sunflower oil, soya oil, groundnut oil, rapeseed oil, almond oil, palm oil, coconut oil, palm kernel oil) and triglycerides, in particular glyceryl stearate, glyceryl triisononanoate, glyceryl laurate or triglycerides with identical or different C6 to C10 fatty acid radicals (so-called medium-chain triglycerides, in particular caprylic/capric triglyceride, like glyceryl tricaprylate, glyceryl tricaprate),_and/or

(ii-2) branched and unbranched alkyl or alkenyl alkohols, preferably selected from the group consisting of decanol, decenol, octanol, octenol, dodecanol, dodecenol, octadienol, decadienol, dodecadienol, oleyl alcohol, ricinoleyl alcohol, erucyl alcohol, stearyl alcohol, isostearyl alcohol, cetyl alcohol, lauryl alcohol, myristyl alcohol, arachidyl alcohol, linoleyl alcohol, linolenyl alcohol, hexyldecanol, octyldodecanol (in particular 2-octyl-1-dodecanol) and cetearyl alcohol and behenyl alcohol, and/or

(ii-3) branched and unbranched hydrocarbons and waxes, cyclic or linear silicone oils and dialkyl ethers having 6 to 24 carbon atoms, preferably selected from the group consisting of jojoba oil, isoeicosane, dicaprylyl ether, mineral oil, petrolatum, squalane, squalene, cyclomethicone, decamethylcyclopentasiloxane, undecamethylcyclotrisiloxane, polydimethylsiloxane and poly(methyl-phenyl siloxane.

Formulations according to the invention can also contain preservatives. The following can be used as preservatives: formaldehyde, glutardialdehyde, parabens (e.g. methyl, ethyl, propyl and butyl paraben), dibromodicyanobutane, imidazolidinyl ureas ("Germall"), isothiazolinones ("Kathon"), methyl chlorothiazolidine, methyl thiazolidine, organic acids (e.g. benzoic acid, sorbic acid, salicylic acid) and salts and esters thereof, propionic acid and formic acid and salts thereof, glycols (e.g. propylene glycol, 1,2-dihydroxyalkanes), plant-based preservative aids such as e.g. lantadin A, caryophyllene, hesperidin, diosmin, phellandrene, pigenin, quercetin, hypericin, aucubin, diosgenin, plumbagin, corlilagin and the like.

The cosmetic or therapeutic, preferably topical, preparations according to the invention also preferably contain antimicrobial active ingredients. Suitable antimicrobial actives are:

Aryl- or aryloxy-substituted, unbranched or monoalkyl- and polyalkyl-branched saturated or mono- to pentaunsaturated (up to five double or triple bonds, also mixed ene/ine compounds) fatty alcohols, fatty aldehydes and fatty acids having chain lengths of $C_2$ to $C_{40}$.

Aryl- or aryloxy-substituted, unbranched or monoalkyl- and polyalkyl-branched saturated or mono- to pentaunsaturated (up to five double or triple bonds, also mixed ene/ine compounds) alkane diols, dialdehydes and dicarboxylic acids having chain lengths of $C_2$ to $C_{40}$, particularly preferably chain lengths of $C_4$ to $C_{12}$.

Mono- and oligoglycerides (up to 4 glycerol units) of aryl- or aryloxy-substituted unbranched or monoalkyl- and polyalkyl-branched saturated or mono- to pentaunsaturated (up to five double or triple bonds, also mixed ene/ine compounds) fatty alcohols (mono- and oligoglycerol monoalkyl ethers), fatty acids (mono- and oligoglycerol

monoalkyl esters), alkanediols (mono- and oligoglycerol monoalkyl ethers; bis(mono-/oligoglyceryl)alkyl diethers) and dicarboxylic acids (mono- and oligoglycerol monoalkyl esters; bis(mono-/oligoglyceryl) alkyl diesters) having chain lengths of $C_2$ to $C_{40}$.

Fatty acid esters of unbranched or monoalkyl- and polyalkyl-branched saturated or mono- to pentaunsaturated (up to five double or triple bonds, also mixed ene/ine compounds), optionally also aryl- or aryloxy-substituted, carboxylic acids having chain lengths of $C_2$ to $C_{40}$ with unbranched or monoalkyl- and polyalkyl-branched saturated or mono- to pentaunsaturated (up to five double or triple bonds, also mixed ene/ine compounds), optionally also aryl- or aryloxy-substituted, monohydric to hexahydric fatty alcohols having chain lengths of $C_2$ to $C_{40}$.

Plant and animal fatty acid cuts, containing unbranched or monoalkyl- and polyalkyl-branched saturated or mono- to pentaunsaturated (up to five double or triple bonds, also mixed ene/ine compounds) fatty alcohols, fatty aldehydes and fatty acids having chain lengths of $C_2$ to $C_{40}$ (e.g. coconut fatty acids, palm kernel fatty acids, wool wax acids).

Mono- and oligoglycerides of lanolin, of lanolin alcohols and lanolic acids (e.g. glyceryl lanolate, neocerite), glycyrrhetic acid and derivatives (e.g. glycyrrhetinyl stearate), natural and synthetic cardenolides (e.g. digitoxin, dogoxin, digoxygenin, gitoxygenin, strophanthin and strophanthidin), natural and synthetic bufadienolides (e.g. scillaren A, scillarenin and bufotalin), sapogenins and steroid sapogenins (e.g. amyrins, oleanolic acid, digitonin, gitogenin, tigogenin and diosgenin), steroid alkaloids of plant and animal origin (e.g. tomatidin, solanin, solanidin, conessin, batrachotoxin and homobatrachotoxin).

Mono- and polyhalogenated nitriles, dinitriles, trinitriles or tetranitriles.

Mono- and oligohydroxy fatty acids having chain lengths of $C_2$ to $C_{24}$ (e.g. lactic acid, 2-hydroxypalmitic acid), oligomers and/or polymers thereof and plant and animal raw materials containing these.

Acyclic terpenes: terpene hydrocarbons (e.g. ocimene, myrcene), terpene alcohols (e.g. geraniol, linalool, citronellol), terpene aldehydes and ketones (e.g. citral, pseudoionone, beta-ionone); monocyclic terpenes: terpene hydrocarbons (e.g. terpinene, terpinolene, limonene), terpene alcohols (e.g. terpineol, thymol, menthol), terpene ketones (e.g. pulegone, carvone); bicyclic terpenes: terpene hydrocarbons (e.g. carane, pinane, bornane), terpene alcohols (e.g. borneol, isoborneol), terpene ketones (e.g. camphor); sesquiterpenes: acyclic sesquiterpenes (e.g. farnesol, nerolidol), monocyclic sesquiterpenes (e.g. bisabolol), bicyclic sesquiterpenes (e.g. cadinene, selinene, vetivazulene, guajazulene), tricyclic sesquiterpenes (e.g. santalene), diterpenes (e.g. phytol), tricyclic diterpenes (e.g. abietic acid), triterpenes (squalenoids; e.g. squalene), tetraterpenes.

Ethoxylated, propoxylated or mixed ethoxylated/propoxylated cosmetic fatty alcohols, fatty acids and fatty acid esters having chain lengths of $C_2$ to $C_{40}$ with 1 to 150 E/O and/or P/O units.

Antimicrobial peptides and proteins having an amino acid value from 4 to 200, e.g. Skin Antimicrobial Peptides (SAPs), Lingual Antimicrobial Peptides (LAPs), human beta-defensins (in particular h-BD1 and h-BD2), lactoferrins and hydrolysates thereof and peptides obtained therefrom, Bactericidal/Permeability Increasing Proteins [BPIs], Cationic Microbial Proteins [CAPs], lysozyme.

Very suitable carbohydrates or "carbohydrate derivatives", which in the interests of brevity can also be included under the term "carbohydrates", are compounds containing sugars and substituted sugars or sugar groups. The sugars include in particular also the deoxy and dideoxy forms, N-acetyl galactosamine-, N-acetyl glucosamine- and sialic acid-substituted derivatives as well as sugar esters and ethers. Preference is given to

a) monosaccharides, including in particular pentoses and hexoses,

b) disaccharides, including in particular sucrose, maltose, lactobiose,

c) oligosaccharides, including in particular the tri- and tetrasaccharides, and

d) polysaccharides, including in particular starch, glycogen, cellulose, dextran, tunicin, inulin, chitin, in particular chitosans, chitin hydrolysates, alginic acid and alginates, plant gums, body mucosa, pectins, mannans, galactans, xylans, araban, polyoses, chondroitin sulfates, heparin, hyaluronic acid and glycosaminoglycanes, hemicelluloses, substituted cellulose and substituted starch, in particular the hydroxyalkyl-substituted polysaccharides in each case.

Amylose, amylopectin, xanthan, alpha-, beta- and gamma-dextrin are particularly suitable. The polysaccharides can consist of e.g. 4 to 1,000,000, in particular 10 to 100,000, monosaccharides. Chain lengths are preferably chosen in each case which ensure that the active ingredient is soluble in or can be incorporated into the particular formulation.

Sphingolipids such as sphingosine; N-monoalkylated sphingosines; N,N-dialkylated sphingosines; sphingosine-1-phosphate; sphingosine-1-sulfate; psychosine (sphingosine-beta-D-galactopyranoside); sphingosyl phosphoryl cholin; lysosulfatides (sphingosyl galactosyl sulfate; lysocerebroside sulfate); lecithin; sphingomyelin; sphinganine.

So-called "natural" antibacterial active ingredients can also be used, most of which are essential oils. Typical oils having an antibacterial action are, for example, oils of aniseed, lemon, orange, rosemary, wintergreen, clove, thyme, lavender, hops, citronella, wheat, lemongrass, cedarwood, cinnamon, geranium, sandalwood, violet, eucalyptus, peppermint, gum benzoin, basil, fennel, menthol and Ocmea origanum, Hydastis carradensis, Berberidaceae daceae, Ratanhiae or Curcuma longa.

Important substances having an antimicrobial action which can be found in essential oils are for example anethol, catechol, camphene, carvacrol, eugenol, eucalyptol, ferulic acid, farnesol, hinokitiol, tropolone, limonene, menthol, methyl salicylate, thymol, terpineol, verbenone, berberine, curcumin, caryophyllene oxide, nerolodol, geraniol.

Mixtures of the cited active systems or active ingredients and active ingredient combinations containing these active ingredients can also be used.

The amount of antimicrobial active ingredients in the formulations is preferably 0.01 to 20 wt.%, based on the total weight of the formulations, particularly preferably 0.05 to 10 wt.%.

In another preferred embodiment a topical, preferably cosmetic, preparation according to the present invention additionally comprises one or more fragrance materials, preferably having a Clog P value of at least 3, preferably of at least 4, more preferably of at least 5. Suitable fragrance materials are mentioned in S. Arctander, Perfume and Flavor Chemicals, Vol. I and II, Montclair, N. J., 1969, self-published or H. Surburg and J. Panten, Common Fragrance and Flavor Materials, 5th. Ed., Wiley-VCH, Weinheim 2006, particularly those explicitly mentioned in US 2008/0070825.

Preparations according to the present invention advantageously comprise a total amount of 0.1 to 5 wt.%, preferably 0.2 to 4 wt.%, more preferably 0.25 to 3 wt.%, even more preferably 0.3 - 2.5 wt.%, of the one or more (preferred) fragrance materials, in each case based on the total weight of the preparation.

In a further preferred embodiment a preparation, preferably a cosmetic leave-on product, according to the present invention additionally comprises one or more of fragrance materials having a boiling point of 250°C or greater (at 1013 mbar). The total amount of fragrance materials having a boiling point of 250°C or greater (at 1013 mbar) preferably is at least 10 wt.%, more preferably at least 20 wt.%, based on the total amount of fragrance materials present in a preparation according to the present invention.

More preferably the fragrance materials, preferably having a boiling point of 250°C or greater at 1013 mbar, are selected from (here in some cases the normal industrial product names and registered trademarks of various firms are given):

alpha-amyl cinnamic aldehyde, alpha-hexyl cinnamic aldehyde, 2-phenoxyethylisobutyrate (Phenirat), methyl dihydrojasmonate [preferably with a content of cis-isomers of > 60 by weight (Hedione, Hedione HC)], 4,6,6,7,8,8-hexamethyl-1,3,4,6,7,8-hexahydrocyclopenta[g]benzopyran (Galaxolide), benzylsalicylate, 2-methyl-3-(4-tert-butyl-phenyl)propanal (Lilial), 4,7-methano-3a,4,5,6,7,7a-hexahydro-5-indenyl acetate and/or 4,7-methano-3a, 4,5,6,7,7a-hexahydro-6-indenyl acetate (Herbaflorat), styrallyl acetate (1-phenylethyl acetate), octahydro-2,3,8,8-tetramethyl-2-acetonaphthone and/or 2-acetyl-1,2,3,4,6,7,8-octahydro-2,3,8,8-tetramethylnaphthaline (Iso E Super), hexylsalicylate, 4-tert.-butylcyclohexyl acetate (Oryclon), 2-tert.-butylcyclohexyl acetate (Agrumex HC), alpha-ionone (4-(2,2,6-trimethyl-2-cyclohexen-1-yl)-3-buten-2-one), 4-(4-hydroxy-4-methylpentyl)-3-cyclohexene carboxaldehyde (Lyral), (E)-and/or (Z)-3-methylcyclopentadec-5-enone (Muscenone), 15-pentadec-11-enolide and/or 15-pentadec-12-enolide (Globalide), 15-cyclopentadecanolide (Macrolide), 1-(5,6,7,8-tetrahydro-3,5,5,6,8,8-hexamethyl-2-naphthalenyl)ethanone (Tonalide), ethylene brassylate, 2-ethyl-4-(2,2,3-trimethyl-3-cyclopenten-1-yl)-2-buten-1-ol (Sandranol), alpha-Santalol, 2,2-dimethyl-3-(3-methylphenyl)-propanol (Majantol), allyl heptanoate, 4-methylacetophenone, (4aR,5R,7aS,9R)-octahydro-2,2,5,8,8,9a-hexamethyl-4H-4a,9-methanoazuleno(5,6-d)-1,3-dioxol) (Ambrocenide), Timberol (1-(2,2,6-trimethylcyclohexyl)hexan-3-ol), benzylacetone, methyl cinnamate, 3a, 6,6,9a-tetramethyldodecahydronaphtho[2,1-b]furan (Ambroxid).

Cosmetic or pharmaceutical preparations containing one or more compounds of formula (I) may, in particular if crystalline or microcrystalline solid bodies such as, for example, inorganic micropigments are to be incorporated in the preparations, according to the invention also contain anionic, cationic, non-ionic and/or amphoteric surfactants mentioned in WO 2005/123101.

Anionic surfactants generally have carboxylate, sulfate or sulfonate groups as functional groups. In aqueous solution they form negatively charged organic ions in the acid or neutral environment. Cationic surfactants are almost exclusively characterised by the presence of a quaternary ammonium group. In aqueous solution they form positively

charged organic ions in the acid or neutral environment. Amphoteric surfactants contain both anionic and cationic groups and therefore behave in aqueous solution in the same way as anionic or cationic surfactants, depending on the pH. They have a positive charge in a strongly acid environment and a negative charge in an alkaline environment. In the neutral pH range, by contrast, they are zwitterionic. Polyether chains are typical of non-ionic surfactants. Non-ionic surfactants do not form ions in the aqueous medium.

A. Anionic surfactants

Anionic surfactants which can advantageously be used are acyl amino acids (and salts thereof), such as

- acyl glutamates, for example sodium acyl glutamate, di-TEA-palmitoyl aspartate and sodium caprylic/capric glutamate,

- acyl peptides, for example palmitoyl-hydrolysed milk protein, sodium cocoyl-hydrolysed soya protein and sodium/potassium cocoyl-hydrolysed collagen,

- sarcosinates, for example myristoyl sarcosin, TEA-lauroyl sarcosinate, sodium lauroyl sarcosinate and sodium cocoyl sarcosinate,

- taurates, for example sodium lauroyl taurate and sodium methyl cocoyl taurate,

- acyl lactylates, lauroyl lactylate, caproyl lactylate

- alaninates
  carboxylic acids and derivatives, such as
  for example lauric acid, aluminium stearate, magnesium alkanolate and zinc undecylenate,

- ester carboxylic acids, for example calcium stearoyl lactylate, laureth-6 citrate and sodium PEG-4 lauramide carboxylate,

- ether carboxylic acids, for example sodium laureth-13 carboxylate and sodium PEG-6 cocamide carboxylate,
  phosphoric acid esters and salts, such as e.g. DEA-oleth-10-phosphate and dilaureth-4 phosphate,
  sulfonic acids and salts, such as

- acyl isothionates, e.g. sodium / ammonium cocoyl isothionate,

- alkyl aryl sulfonates,

- alkyl sulfonates, for example sodium cocomonoglyceride sulfate, sodium $C_{12-14}$ olefin sulfonate, sodium lauryl sulfoacetate and magnesium PEG-3 cocamide sulfate,

- sulfosuccinates, for example dioctyl sodium sulfosuccinate, disodium laureth sulfosuccinate, disodium lauryl sulfosuccinate and disodium undecylenamido MEA sulfosuccinate
  and
  sulfuric acid esters, such as

- alkyl ether sulfate, for example sodium, ammonium, magnesium, MIPA, TIPA laureth sulfate, sodium myreth sulfate and sodium $C_{12-13}$ pareth sulfate,

- alkyl sulfates, for example sodium, ammonium and TEA lauryl sulfate.

B. Cationic surfactants

Cationic surfactants which can advantageously be used are

- alkyl amines,

- alkyl imidazoles,

- ethoxylated amines and

- quaternary surfactants.
  $RNH_2CH_2CH_2COO^-$ (where pH=7)
  $RNHCH_2CH_2COO$- $B^+$ (where pH=12) $B^+$ = any cation, e.g. $Na^+$

- esterquats

Quaternary surfactants contain at least one N atom, which is covalently bonded to 4 alkyl or aryl groups. This leads to a positive charge, regardless of the pH. Alkyl betaine, alkyl amidopropyl betaine and alkyl amidopropyl hydroxysulfaine are advantageous. The cationic surfactants used can also preferably be chosen from the group of quaternary ammonium compounds, in particular benzyl trialkyl ammonium chlorides or bromides, such as benzyl dimethylstearyl ammonium chloride for example, also alkyl trialkyl ammonium salts, for example cetyl trimethyl ammonium chloride or bromide, alkyl dimethyl hydroxyethyl ammonium chlorides or bromides, dialkyl dimethyl ammonium chlorides or bromides, alkyl amide ethyl trimethyl ammonium ether sulfates, alkyl pyridinium salts, for example lauryl or cetyl pyrimidinium chloride, imidazoline derivatives and compounds having a cationic character such as amine oxides, for example alkyl dimethyl amine oxides or alkyl aminoethyl dimethyl amine oxides. Cetyl trimethyl ammonium salts are particularly advantageously used.

C. Amphoteric surfactants

Amphoteric surfactants which can advantageously be used are

- acyl/dialkyl ethylene diamine, for example sodium acyl amphoacetate, disodium acyl amphodipropionate, disodium alkyl amphodiacetate, sodium acyl amphohydroxypropyl sulfonate, disodium acyl amphodiacetate and sodium acyl amphopropionate,

- N-alkyl amino acids, for example aminopropyl alkyl glutamide, alkyl aminopropionic acid, sodium alkyl imidodipropionate and lauroamphocarboxyglycinate.

D. Non-ionic surfactants

Non-ionic surfactants which can advantageously be used are

- alcohols,

- alkanolamides, such as cocamides MEA/DEA/MIPA,

- amine oxides, such as cocamidopropylamine oxide,

- esters produced by esterification of carboxylic acids with ethylene oxide, glycerol, sorbitan or other alcohols,

- ethers, for example ethoxylated/propoxylated alcohols, ethoxylated/ propoxylated esters, ethoxylated/propoxylated glycerol esters, ethoxylated/propoxylated cholesterols, ethoxylated/propoxylated triglyceride esters, ethoxylated/ propoxylated lanolin, ethoxylated/propoxylated polysiloxanes, propoxylated POE ethers and alkyl polyglycosides such as lauryl glucoside, decyl glycoside and cocoglycoside,

- sucrose esters, ethers,

- polyglycerol esters, diglycerol esters, monoglycerol esters,

- methyl glucose esters, esters of hydroxy acids.

The use of a combination of anionic and/or amphoteric surfactants with one or more non-ionic surfactants is also advantageous.
The surface-active substance (surfactant) or the combination of surface-active substances can be present in the formulations according to the invention in a concentration of between 1 and 98 wt.%, based on the total weight of the formulations.

Cosmetic (e.g. dermatological) or pharmaceutical formulations according to the invention containing one or more compounds according to the invention or for use according to the invention having formula (I) can also take the form of emulsions.

The oil phase of preparations according to the invention, which contain one or more compounds of formula (I) may advantageously be selected from the substance groups mentioned in WO 2005/123101.

The oil phase (lipid phase) in the formulations according to the invention (in particular topical cosmetic formulations) can advantageously be selected from the following group of substances:

- mineral oils (advantageously paraffin oil), mineral waxes

- fatty oils, fats, waxes and other natural and synthetic fat bodies, preferably esters of fatty acids with low C-number alcohols, for example with isopropanol, propylene glycol or glycerol, or esters of fatty alcohols with low C-number alkanoic acids or with fatty acids;

- alkyl benzoates (e.g. mixtures of n-dodecyl, n-tridecyl, n-tetradecyl or n-pentadecyl benzoate);

- cyclic or linear silicone oils such as dimethyl polysiloxanes, diethyl polysiloxanes, diphenyl polysiloxanes and mixed forms thereof.

(Natural or synthetic) esters are advantageously used, in particular (a) esters of saturated and/or unsaturated branched and/or unbranched alkane carboxylic acids having a chain length of 3 to 30 C atoms and saturated and/or unsaturated, branched and/or unbranched alcohols having a chain length of 3 to 30 C atoms, (b) esters of aromatic carboxylic acids and saturated and/or unsaturated, branched and/or unbranched alcohols having a chain length of 3 to 30 C atoms. Preferred ester oils are isopropyl myristate, isopropyl palmitate, isopropyl stearate, isopropyl oleate, n-butyl stearate, n-hexyl laurate, n-decyl laurate, n-decyl oleate, isooctyl stearate, isononyl stearate, isononyl isononanoate, 3,5,5-trimethylhexyl-3,5,5-trimethylhexanoate, 2-ethylhexyl isononanoate, 2-ethylhexyl-3,5,5-trimethylhexanoate, 2-ethylhexyl-2-ethylhexanoate, cetearyl-2-ethylhexanoate, 2-ethylhexyl palmitate, 2-ethylhexyl laurate, 2-hexyldecyl stearate, 2-octyldecyl palmitate, 2-octyldodecyl palmitate, oleyl oleate, oleyl erucate, erucyl oleate, erucyl erucate and synthetic, semisynthetic and natural mixtures of such esters, e.g. jojoba oil.

The oil phase can also advantageously be chosen from the group consisting of branched and unbranched hydrocarbons and hydrocarbon waxes, silicone oils, dialkyl ethers, the group consisting of saturated or unsaturated, branched or unbranched alcohols, and of fatty acid triglycerides, in particular the triglycerol esters of saturated and/or unsaturated, branched and/or unbranched alkane carboxylic acids having a chain length of 8 to 24, in particular 12 to 18 C atoms. The fatty acid triglycerides can advantageously be selected from the group of synthetic, semisynthetic and natural oils, e.g. triglycerides of capric or caprylic acid, apricot kernel oil, avocado oil, cottonseed oil, borage seed oil, thistle oil, groundnut oil, gamma-oryzanol, rosehip seed oil, hemp oil, hazelnut oil, blackcurrant seed oil, coconut oil, cherry kernel oil, salmon oil, flax oil, maize oil, macadamia nut oil, almond oil, evening primrose oil, mink oil, olive oil, palm oil, palm kernel oil, pecan nut oil, peach kernel oil, pistachio nut oil, rapeseed oil, rice bran oil, castor oil, safflower oil, sesame oil, soya oil, sunflower oil, teatree oil, grape seed oil or wheat germ oil, and the like. Any blends of such oil and wax components can also advantageously be used. In some cases it is also advantageous to use waxes, for example cetyl palmitate, as the sole lipid component of the oil phase, the oil phase advantageously being chosen from the group consisting of 2-ethylhexyl isostearate, octyl dodecanol, isotridecyl isononanoate, isoeicosane, 2-ethylhexyl cocoate, $C_{12-15}$-alkyl benzoate, caprylic-capric acid triglyceride and dicaprylyl ether. Mixtures of $C_{12-15}$-alkyl benzoate and 2-ethylhexyl isostearate, mixtures of $C_{12-15}$-alkyl benzoate and isotridecyl isononanoate and mixtures of $C_{12-15}$-alkyl benzoate, 2-ethylhexyl isostearate and isotridecyl isononanoate are particularly advantageous. The hydrocarbons paraffin oil, squalane and squalene can also advantageously be used. The oil phase can advantageously also have a content of cyclic or linear silicone oils or consist entirely of such oils, it being preferable, however, to use an additional content of other oil phase components along with the silicone oil or silicone oils. Cyclomethicone (e.g. decamethyl cyclopentasiloxane) can advantageously be used as the silicone oil. Other silicone oils can also advantageously be used, however, for example undecamethyl cyclotrisiloxane, polydimethyl siloxane and poly(methylphenyl siloxane). Mixtures of cyclomethicone and isotridecyl isononanoate and of cyclomethicone and 2-ethylhexyl isostearate are also particularly advantageous.

The aqueous phase of formulations according to the invention (in particular topical cosmetic formulations) in the form of an emulsion can advantageously include: alcohols, diols or polyols having a low C number, and ethers thereof, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, also alcohols having a low C number, e.g. ethanol, isopropanol, 1,2-propanediol, glycerol and in particular one or more thickeners, which can advantageously be chosen from the group comprising silicon

dioxide, aluminium silicates such as e.g. bentonites, polysaccharides or derivatives thereof, e.g. hyaluronic acid, guar gum, xanthan gum, hydroxypropyl methyl cellulose, or allulose derivatives, particularly advantageously from the group of polyacrylates, preferably a polyacrylate from the group of so-called carbopols, for example type 980, 981, 1382, 2984, 5984 carbopols, either individually or in combination, or from the group of polyurethanes, also alpha- or beta-hydroxy acids, preferably lactic acid, citric acid or salicylic acid, also emulsifiers, which can advantageously be selected from the group of ionic, non-ionic, polymeric, phosphate-containing and zwitterionic emulsifiers.

Formulations according to the invention in the form of an emulsion advantageously include one or more emulsifiers. O/W emulsifiers, for example, can advantageously be chosen from the group of polyethoxylated or polypropoxylated or polyethoxylated and polypropoxylated products, e.g.:

- fatty alcohol ethoxylates,
- ethoxylated wool wax alcohols,
- polyethylene glycol ethers having the general formula $R\text{-}O\text{-}(\text{-}CH_2\text{-}CH_2\text{-}O\text{-})_n\text{-}R'$,
- fatty acid ethoxylates having the general formula $R\text{-}COO\text{-}(\text{-}CH_2\text{-}CH_2\text{-}O\text{-})_n\text{-}H$,
- etherified fatty acid ethoxylates having the general formula

  $R\text{-}COO\text{-}(\text{-}CH_2\text{-}CH_2\text{-}O\text{-})_n\text{-}R'$,

- esterified fatty acid ethoxylates having the general formula

  $R\text{-}COO\text{-}(\text{-}CH_2\text{-}CH_2\text{-}O\text{-})_n\text{-}C(O)\text{-}R'$,

- polyethylene glycol glycerol fatty acid esters,
- ethoxylated sorbitan esters,
- cholesterol ethoxylates,
- ethoxylated triglycerides,
- alkyl ether carboxylic acids having the general formula

  $R\text{-}COO\text{-}(\text{-}CH_2\text{-}CH_2\text{-}O\text{-})_n\text{-}OOH$, where n represents a number from 5 to 30,

- polyoxyethylene sorbitol fatty acid esters,
- alkyl ether sulfates having the general formula $R\text{-}O\text{-}(\text{-}CH_2\text{-}CH_2\text{-}O\text{-})_n\text{-}SO_3\text{-}H$,
- fatty alcohol propoxylates having the general formula $R\text{-}O\text{-}(\text{-}CH_2\text{-}CH(CH_3)\text{-}O\text{-})_n\text{-}H$,
- polypropylene glycol ethers having the general formula

  $R\text{-}O\text{-}(\text{-}CH_2\text{-}CH(CH_3)\text{-}O\text{-})_n\text{-}R'$,

- propoxylated wool wax alcohols,
- etherified fatty acid propoxylates $R\text{-}COO\text{-}(\text{-}CH_2\text{-}CH(CH_3)\text{-}O\text{-})_n\text{-}R'$,
- esterified fatty acid propoxylates having the general formula

  $R\text{-}COO\text{-}(\text{-}CH_2\text{-}CH(CH_3)\text{-}O\text{-})_n\text{-}C(O)\text{-}R'$,

- fatty acid propoxylates having the general formula

  $R\text{-}COO\text{-}(\text{-}CH_2\text{-}CH(CH_3)\text{-}O\text{-})_n\text{-}H$,

- polypropylene glycol glycerol fatty acid esters,
- propoxylated sorbitan esters,
- cholesterol propoxylates,
- propoxylated triglycerides,
- alkyl ether carboxylic acids having the general formula

  $R\text{-}O\text{-}(\text{-}CH_2\text{-}CH(CH_3)\text{-}O\text{-})_n\text{-}CH_2\text{-}COOH$,

- alkyl ether sulfates or the acids on which these sulfates are based having the general formula $R\text{-}O\text{-}(\text{-}CH_2\text{-}CH(CH_3)\text{-}O\text{-})_n\text{-}SO_3\text{-}H$,
- fatty alcohol ethoxylates/propoxylates having the general formula $R\text{-}O\text{-}X_n\text{-}Y_m\text{-}H$,

- polypropylene glycol ethers having the general formula R-O-$X_n$-$Y_m$-R',
- etherified fatty acid propoxylates having the general formula R-COO-$X_n$-$Y_m$-R',
- fatty acid ethoxylates/propoxylates having the general formula R-COO-$X_n$-$Y_m$-H.

Particularly advantageously according to the invention the polyethoxylated or polypropoxylated or polyethoxylated and polypropoxylated O/W emulsifiers used are chosen from the group of substances having HLB values of 11 to 18, most particularly advantageously having HLB values of 14.5 to 15.5, if the O/W emulsifiers have saturated R and R' radicals. If the O/W emulsifiers have unsaturated R and/or R' radicals, or if isoalkyl derivatives are present, the preferred HLB value of such emulsifiers can also be lower or higher.

It is advantageous to choose the fatty alcohol ethoxylates from the group of ethoxylated stearyl alcohols, cetyl alcohols, cetyl stearyl alcohols (cetearyl alcohols). Particularly preferred are:

polyethylene glycol (n) stearyl ether (steareth-n) where n = 13-20,

polyethylene glycol (n) cetyl ether (ceteth-n) where n = 13-20,

polyethylene glycol (n) isocetyl ether (isoceteth-n) where n = 13-20,

polyethylene glycol (n) cetyl stearyl ether (ceteareth-n) where n = 13-20,

polyethylene glycol (m) isostearyl ether (isosteareth-m) where m = 12-20,

polyethylene glycol (k) oleyl ether (oleth-k) where k = 12-15,

polyethylene glycol (12) lauryl ether (laureth-12),

polyethylene glycol (12) isolauryl ether (isolaureth-12).

It is also advantageous to choose the fatty acid ethoxylates from the following group:

polyethylene glycol (n) stearate where n = 20-25,

polyethylene glycol (m) isostearate where m = 12-25,

polyethylene glycol (k) oleate where k = 12-20.

Sodium laureth-11 carboxylate can advantageously be used as the ethoxylated alkyl ether carboxylic acid or its salt. Sodium laureth 1-4 sulfate can advantageously be used as the alkyl ether sulfate. Polyethylene glycol (30) cholesteryl ether can advantageously be used as the ethoxylated cholesterol derivative. Polyethylene glycol (25) soya sterol has also proved itself.

Polyethylene glycol (60) evening primrose glycerides can advantageously be used as ethoxylated triglycerides.

It is also advantageous to choose the polyethylene glycol glycerol fatty acid esters from the group comprising polyethylene glycol (n) glyceryl laurate where n = 20-23, polyethylene glycol (6) glyceryl caprate/caprinate, polyethylene glycol (20) glyceryl oleate, polyethylene glycol (20) glyceryl isostearate, polyethylene glycol (18) glyceryl oleate/cocoate.

It is likewise beneficial to choose the sorbitan esters from the group comprising polyethylene glycol (20) sorbitan monolaurate, polyethylene glycol (20) sorbitan monostearate, polyethylene glycol (20) sorbitan monoisostearate, polyethylene glycol (20) sorbitan monopalmitate, polyethylene glycol (20) sorbitan monooleate.

The following can be used as advantageous W/O emulsifiers: fatty alcohols having 8 to 30 carbon atoms, monoglycerol esters of saturated and/or unsaturated, branched and/or unbranched alkane carboxylic acids having a chain length of 8 to 24, in particular 12 to 18 C atoms, diglycerol esters of saturated and/or unsaturated, branched and/or unbranched alkane carboxylic acids having a chain length of 8 to 24, in particular 12 to 18 C atoms, monoglycerol ethers of saturated and/or unsaturated, branched and/or unbranched alcohols having a chain length of 8 to 24, in particular 12 to 18 C atoms, diglycerol ethers of saturated and/or unsaturated, branched and/or unbranched alcohols having a chain length of 8 to 24, in particular 12 to 18 C atoms, propylene glycol esters of saturated and/or unsaturated, branched and/or unbranched alkane carboxylic acids having a chain length of 8 to 24, in particular 12 to 18 C atoms, and sorbitan esters of saturated and/or unsaturated, branched and/or unbranched alkane carboxylic acids having a chain length of 8 to 24, in particular 12 to 18 C atoms.

Particularly advantageous W/O emulsifiers are glyceryl monostearate, glyceryl monoisostearate, glyceryl monomyristate, glyceryl monooleate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprinate, glyceryl monocaprylate.

The formulations according to the invention (in particular cosmetic, including dermatological formulations) can contain deodorants, i.e. active ingredients having a deodorising and perspiration-inhibiting action. These include, for example, odour maskers, such as the common perfume constituents, antiperspirants based on aluminium, zirconium or zinc salts, odour absorbers, for example the layered silicates described in DE-P 40 09 347, in particular montmorillonite, kaolinite, nontronite, saponite, hectorite, bentonite, smectite, and also zinc salts of ricinoleic acid, for example. They also include bactericidal or bacteriostatic deodorising substances, such as e.g. hexachlorophene, 2,4,4'-trichloro-2'-hydroxydiphenyl ether (Irgasan), 1,6-di-(4-chlorophenylbiguanido)hexane (chlorhexidine), 3,4,4'-trichlorocarbanilide, and the active agents described in the laid-open patent specifications DE-37 40 186, DE-39 38 140, DE-42 04 321, DE-42 29 707, DE-42 29 737, DE-42 37 081, DE-43 09 372, DE-43 24 219 and containing cation-active substances, such as e.g. quaternary ammonium salts and odour absorbers such as e.g. Grillocin® (combination of zinc ricinoleate and various additives) or triethyl citrate, optionally in combination with ion-exchange resins.

The amount of deodorising and/or antiperspirant active ingredients in the formulations is preferably 0.01 to 20 wt.%, based on the total weight of the formulations, particularly preferably 0.05 to 10 wt.%.

Preferred embodiments and further aspects of the present invention emerge from the attached patent claims and the following examples.

The examples describe the invention in more detail, without limiting the area of protection of the claims. Unless stated otherwise, all the data, in particular amounts and percentages, relate to the weight.

## Examples 1: Synthesis of compounds of formula (I)

### Examples 1.1: Di-substituted cyclohexyl carbamates of formula (Carb-II-R1H)

### Example 1.1.1: sec-Butyl-carbamic acid 2,3-dimethyl-cyclohexyl ester (BIO1845)

50.7 g (0.5 mol) sec.-Butyl isocyanate were placed in a 500 ml vessel at room temperature and 64.1 g (0.5 mol) 2,3-Dimethylcyclohexanol were added. The reaction mixture was heated for 5 h to 150 ˚C, cooled and subsequently 100 ml water were added. After refluxing for one hour the solution was cooled down, the phases separated and extracted once with MTBE (methyl tert.-butyl ether). The raw product was purified by distillation to yield 60.2 g product as a mixture of isomers with a purity of 99.6%.

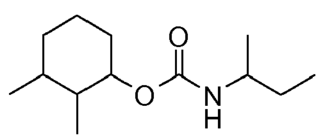

main signals of isomer mixture:

$^{1}$H-NMR (400 MHz, $CDCl_3$, TMS): δ= 4.66 (m, H), 4.41 (m, H), 3.62 (m, H), 2.06 (m, H), 1.68 (m, H), 1.44 (m, 2 H), 1.12 (d, 6.6 Hz, 3 H), 0.91 (t, 7.3 Hz, 3 H), 0.89 (d, 6.9 Hz, 3 H), 0.80 (d, 7.0 Hz, 3 H) ppm.

$^{13}$C-NMR (400 MHz, $CDCl_3$, TMS): δ= 156.2 (s), 76.2 (d), 48.2 (d), 37.8 (d), 34.2 (d), 30.0 (t), 20.8 (q), 19.1 (q), 10.3 (q), 6.1 (q) ppm.

MS (EI, major isomer): *m/z* = 227 (not detected), 198 (27), 111 (71), 95 (20), 81 (18), 69 (100), 55 (37), 44 (94), 41 (31).

The following cyclohexyl carbamates were produced analogously to the methodology of BIO1845 as described in example 1.1.1 or BIO1824 as described in example 1.3.1, below. The cyclohexyl carbamates were obtained in comparable yields and purities (generally > 99%, depending on the structure as a mixture of stereoisomers):

**Example 1.1.2: (2-Hydroxy-phenyl)-carbamic acid 2,3-dimethyl-cyclohexyl ester (BIO1643)**

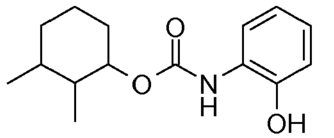

main signals of isomer mixture:

$^1$H-NMR (400 MHz, $CDCl_3$, TMS): δ = 7.93 (m, H), 7.15 (d, 7.5 Hz, H), 7.05 (d,d,7.7 Hz, 8.1 Hz, H), 6.97 (d, 8.1 Hz, H), 6.87 (d,d, 7.3 Hz, 7.7 Hz, H), 6.7 (m, H), 4.81 (t,d, 4.5 Hz, 11.6 Hz, H), 1.10-2.18 (m, 8 H), 0.92 (d, 6.9 Hz, 3 H), 0.86 (d, 7.1 Hz, 3 H) ppm.

$^{13}$C-NMR (400 MHz, $CDCl_3$, TMS): δ = 155.4 (s), 147.2 (s), 125.6 (d), 125.4 (s), 121.2 (d), 120.9 (d), 118.6 (d), 78.6 (d), 37.3 (d), 34.6 (t), 34.2 (d), 27.2 (t), 25.4 (t), 19.1 (q), 6.2 (q) ppm.

MS (EI): *m*/*z* = 263 (15), 153 (100), 135 (15), 110 (21), 109 (64), 95 (9), 81 (9), 69 (51), 55 (27), 41 (11).

**Example 1.1.3: Ethyl-carbamic acid 3,5-dimethyl-cyclohexyl ester (BIO1561)**

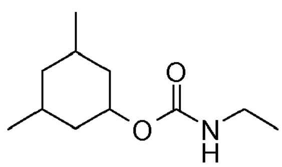

main signals of isomer mixture:

$^1$H-NMR (400 MHz, $CDCl_3$, TM): δ = 4.61 (m, 2 H), 3.20 (m, 2 H), 1.96 (d, 11.9 Hz, 2 H), 1.60 (d, 14.4 Hz, H), 1.52 (m, 2 H), 1.13 (t, 7.2 Hz, 3 H), 0.92 (d, 6.6 Hz, 6 H), 0.80-1.05 (m, 2 H), 0.53 (q, 11.9 Hz, H) ppm.

$^{13}$C-NMR (400 MHz, $CDCl_3$, TMS): δ = 156.3 (s), 73.1 (d), 43.1 (t), 40.5 (t), 40.5 (t), 38.4 (t), 30.6 (d), 30.6 (d), 22.2 (q), 22.2 (q), 15.3 (q) ppm.

MS (EI, major isomer): *m*/*z* = 199 (not detected), 127 (4), 95 (41), 90 (100), 69 (65), 55 (39), 41 (62), 29 (26).

**Example 1.1.4: p-Tolyl-carbamic acid 3,5-dimethyl-cyclohexyl ester (BIO1822)**

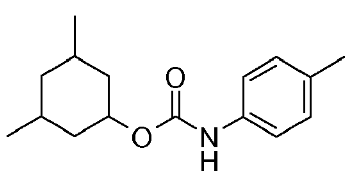

main signals of isomer mixture:

$^1$H-NMR (400 MHz, $CDCl_3$, TMS): δ = 7.25 (d, 8.1 Hz, 2 H), 7.10 8d, 8.3 Hz, 2 H), 6.45 (m, H), 4.71 (t,t, 4.4 Hz, 11.3 Hz, H), 2.30 (s, 3 H), 2.03 (d, 12.0 Hz, 2 H), 1,62 (d, 14.1Hz, H), 1.55 (m, 2 H), 0.97 (q, 11.3 Hz, 2 H), 0.94 (d, 6.6 Hz, 6 H), 0.56 (d,t, 11.5 Hz, 12.6 Hz, H) ppm.

$^{13}$C-NMR (400 MHz, $CDCl_3$, TMS): δ = 153.4 (s), 135.5 (s), 132.8 (s), 129.5 (d), 129.5 (d), 118.7 (d), 118.7 (d), 73.9 (d), 43.0 (t), 40.3 (t), 40.3 (t), 30.6 (d), 30.6 (d), 22.2 (q), 22.2 (q), 20.7 (q) ppm.

MS (EI): *m/z* = 262 (5), 261 (24), 151 (100), 107 (72), 106 (20), 69 (45), 55 (20), 41 (11).

**Example 1.1.5: n-Butyl-carbamic acid 3,5-dimethyl-cyclohexyl ester (BIO1840)**

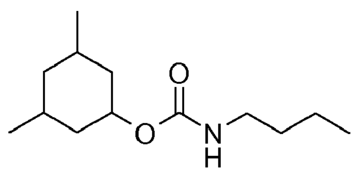

main signals of isomer mixture:

$^1$H-NMR (400 MHz, $CDCl_3$, TM): δ = 4.58 (m, 2 H), 3.14 (q, 6.3 Hz, 2 H), 1.94 (d, 11.7 Hz, 2 H), 1.58 (d, 12.6 Hz, H), 1.40-1.54 (m, 4 H), 1.32 (m, 2 H), 0.90 (d, 6.5 Hz, 6 H), 0.90 (t, 7.2 Hz, 3 H), 0.89 (q, 11.8 Hz, 2 H), 0.50 (q, 12.0 Hz, H) ppm.

$^{13}$C-NMR (400 MHz, $CDCl_3$, TMS): δ = 156.4 (s), 73.1 (d), 43.1 (t), 40.6 (t), 40.5 (t), 40.5 (t), 32.1 (t), 30.6 (d), 30.6 (d), 22.2 (q), 22.2 (q), 19.9 (t), 13.7 (q) ppm.

MS (EI): *m/z* = 227 (1), 184 (1), 118 (100), 111 (43), 95 (28), 69 (77), 55 (28), 41 (29), 30 (19).

**Example 1.1.6: Phenyl-carbamic acid 3,5-dimethyl-cyclohexyl ester (BIO1685)**

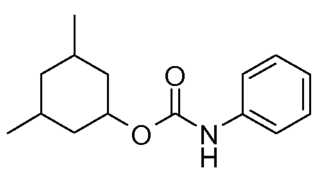

main signals of isomer mixture:

$^1$H-NMR (400 MHz, $CDCl_3$, TMS): δ = 7.37 (d, 7.9 Hz, 2 H), 7.30 (m, 2 H), 7.05 (m, H), 6.53 (m, H), 4.72 (t,t, 4.3 Hz, 11.4 Hz, H), 2.04 (d, 11.7 Hz, 2 H), 1.63 (d, 12.5 Hz, H), 1.55 (m, 2 H), 0.94 (q, 11.7 Hz, 2 H), 0.94 (d, 6.5 Hz, 6 H), 0.56 (d,t, 11.6 Hz, 12.6 Hz, H) ppm.

$^{13}$C-NMR (400 MHz, $CDCl_3$, TMS): δ = 153.2 (s), 138.1 (s), 129.0 (d), 129.0 (d), 123.2 (d), 118.6 (d), 118.6 (d), 74.0 (d), 43.0 (t), 40.3 (t), 40.3 (t), 30.6 (d), 30.6 (d), 22.1 (q), 22.1 (q) ppm.

MS (EI): *m/z* = 248 (3), 247 (15), 137 (29), 111 (29), 95 (34), 93 (84), 69 (100), 55 (47), 41 (35).

**Example 1.1.7: n-Butyl-carbamic acid 2-isopropenyl-5-methyl-cyclohexyl ester (BIO1615)**

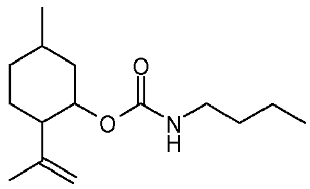

main signals of isomer mixture:

$^1$H-NMR (400 MHz, $CDCl_3$, TMS): δ = 4.73 (m, 2 H), 4.65 (d,t, 4.3 Hz, 10.9 Hz, H), 4.54 (m, H), 3.13 (d,t, 6.0 Hz, 6.0 Hz, 2 H), 2.07 (m, 2 H), 1.63-1.73 (m, 2 H), 1.69 (t, 1.2 Hz, 3 H), 1.56 (m, H), 1.26-1.49 (m, 5 H), 0.87-1.02 (m, 2 H), 0.92 (d, 6.5 Hz, 3 H), 0.91 (t, 7.2 Hz, 3 H) ppm.

$^{13}$C-NMR (400 MHz, $CDCl_3$, TM): δ = 156.4 (s), 146.8 (s), 111.5 (t), 73.8 (d), 51.0 (d), 41.0 (t), 40.6 (t), 34.2 (t), 32.1 (t), 31.4 (d), 30.7 (t), 22.0 (q), 19.9 (t), 19.5 (q), 13.7 (q) ppm.

MS (EI, major isomer): *m/z* = 254 (1), 253 (4), 136 (100), 118 (87), 107 (35), 93 (40), 81 (56), 67 (20), 57 (20), 41 (32), 29 (10).

**Example 1.1.8: Ethyl-carbamic acid 2-isopropenyl-5-methyl-cyclohexyl ester (BIO1551)**

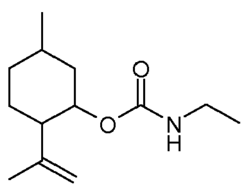

main signals of isomer mixture:

$^1$H-NMR (400 MHz, $CDCl_3$, TMS): δ = 4.74 (m, 2 H), 4.65 (d,t, 4.3 Hz, 10.9 Hz, H), 4.58 (m, H), 3.18 (m, 2 H), 1.87-2.11 (m, 2 H), 1.48-1.75 (m, 3 H), 1.69 (t, 1.2 Hz, 3 H), 1.39 (m, H), 1.10 (t, 7.2 Hz, 3 H), 0.82 -1.03 (m, 2 H), 0.92 (d, 6.6 Hz, 3 H) ppm.

$^{13}$C-NMR (400 MHz, $CDCl_3$, TM): δ = 156.3 (s), 146.6 (s), 111.5 (t), 73.7 (d), 51.0 (d), 41.1 (t), 35.7 (t), 34.2 (t), 31.4 (d), 30.7 (t), 22.1 (q), 19.5 (q), 15.2 (q) ppm.

MS (EI, major isomer): *m/z* = 226 (1), 225(2), 136(100), 121 (58), 107 (37), 90 (62), 81 (48), 69 (19), 55 (21), 41 (21), 29 (20).

**Example 1.1.9: n-Butyl-carbamic acid 2,3-dimethyl-cyclohexyl ester (BIO1842)**

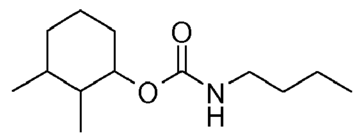

main signals of isomer mixture:

$^{1}H$-NMR (400 MHz, $CDCl_3$, TMS): $\delta$ = 4.66 (m, 2 H), 3.17 (q, 6.3 Hz, 2 H), 2.05 (m, H), 1.24-1.80 (m, 11 H), 0.92 (t, 7.3 Hz, 3 H), 0.89 (d, 6.6 Hz, 3 H), 0.79 (d, 6.9 Hz, 3 H) ppm.

$^{13}C$-NMR (400 MHz, $CDCl_3$, TMS): $\delta$ = 156.8 (s), 76.3 (d), 40.6 (t), 37.4 (d), 34.8 (t), 34.2 (d), 32.2 (t), 27.4 (t), 25.6 (t), 20.0 (t), 19.1 (q), 13.8 (q), 6.1 (q) ppm.

MS (EI, major isomer): *m/z* = 227(<1), 118(100), 111 (49), 110 (88), 95 (53), 81 (55), 69 (83), 57 (35), 55 (54), 41 (35).

**Example 1.1.10: Cyclohexyl-carbamic acid 3,5-dimethyl-cyclohexyl ester (BIO1743)**

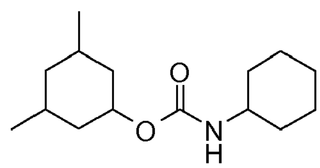

main signals of isomer mixture:

$^{1}H$-NMR (400 MHz, $CDCl_3$, TMS): $\delta$ = 4.59 (m, H), 4.47 (m, H), 3.47 (m, H), 1.94 (m, 2 H), 1.69 (d, 13.5 Hz, H), 1.52 (m, 2 H), 1.00-1.40 (m, 10 H), 0.92 (d, 6.5 Hz, 6 H), 0.86 (m, 2 H), 0.52 (q, 11.9 Hz, H) ppm.

$^{13}C$-NMR (400 MHz, $CDCl_3$, TMS): $\delta$ = 156.8 (s), 73.0 (d), 49.1 (d), 43.1 (t), 40.5 (t), 40.5 (t). 33.5 (t), 33.5 (t), 30.6 (d), 30.6 (d), 25.5 (t), 25.5 (t), 24.8 (t), 22.2 (q), 22.2 (q) ppm.

MS (EI, minor isomer): *m/z* = 253 (2), 144 (100), 111 (26), 110 (33), 95 (61), 82 (32), 69 (96), 56 (59), 55 (71), 41 (50).

MS (EI, major isomer): *m/z* = 253 (3), 144 (91), 111 (47), 110 (11), 95 (32), 82 (31), 69 (100), 56 (66), 55 (60), 41 (42).

**Example 1.1.11: Benzyl-carbamic acid 3,5-dimethyl-cyclohexyl ester (BIO1745)**

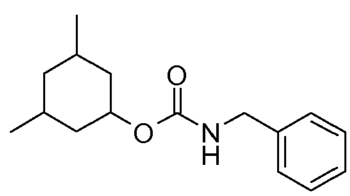

main signals of isomer mixture:

$^1$H-NMR (400 MHz, $CDCl_3$, TMS): δ = 7.34 (m, 2 H), 7.28 (m, 2 H), 7.27 (m, H), 4.92 (m, H), 4.65 (t,t, 4.3 Hz, 11.4 Hz, H), 4.36 (d, 5.5 Hz, 2 H), 1.98 (d, 12.1 Hz, 2 H), 1.60 (d, 14.5 Hz, H), 1.52 (m, 2 H), 0.92 (d, 6.5 Hz, 6 H), 0.86 (m, 2 H), 0.53 (q, 11.9 Hz, H) ppm.

$^{13}$C-NMR (400 MHz, $CDCl_3$, TMS): δ = 156.3 (s), 138.7 (s), 128.6 (d), 128.6 (d), 127.5 (d), 127.4 (d), 127.4 (d), 73.6 (d), 45.0 (t), 43.0 (t), 40.4 (t), 40.4 (t), 30.6 (d), 30.6 (d), 22.2 (q), 22.2 (q) ppm.

MS (EI, minor isomer): *m/z* = 261 (2), 151 (100), 150 (93), 106 (29), 95 (72), 91 (59), 69 (55), 55 (43), 41 (31).

MS (EI, major isomer): *m/z* = 261 (2), 151 (78), 150 (100), 106 (24), 95 (38), 91 (55), 69 (63), 55 (37), 41 (26).

**Example 1.1.12: (4-Ethyl-phenyl)-carbamic acid 3,5-dimethyl-cyclohexyl ester (BIO1823)**

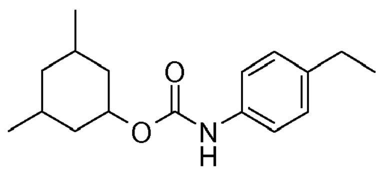

ααα , main signals of isomer mixture:

$^1$H-NMR (400 MHz, $CDCl_3$, TMS): δ = 7.27 (m, 2 H), 7.13 (m, 2 H), 6.46 (s, H), 4.17 (t,t 4.3 Hz, 11.4 Hz, H), 2.60 (q, 7.6 Hz, 2 H), 2.03 (d, 12.1 Hz, 2 H), 1.62 (d, 14.4 Hz, H), 1.55 (m, 2 H), 1.21 (t, 7.6 Hz, 3 H), 0.94 (d, 6.5 Hz, 6 H), 0.94 (q, 11.9 Hz, 2 H), 0.56 (q, 12.0 Hz, H) ppm.

$^{13}$C-NMR (400 MHz, $CDCl_3$, TMS): δ = 153.4 (s), 137.8 (s), 137.2 (s), 128.0 (d), 128.0 (d), 118.5 (d), 118.5 (d), 72.7 (d), 42.9 (t), 40.4 (t), 40.4 (t), 30.4 (d), 30.4 (d), 27.9 (t), 22.1 (q), 22.1 (q), 15.9 (q) ppm.

MS (EI, minor isomer): *m/z* = 275(22), 165 (100), 150 (26), 132(21), 121 (41), 111 (11), 106 (42), 69 (52), 55 (22), 41 (13).

**Example 1.1.13: Ethyl-carbamic acid 3,4-dimethyl-cyclohexyl ester (BIO1582)**

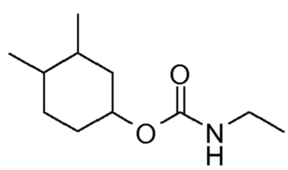

main signals of isomer mixture:

$^1$H-NMR (400 MHz, $CDCl_3$, TMS): δ = 4.60 (m, 2 H), 3.20 (d,q, 5.2 Hz, 7.2 Hz, 2 H), 0.95-2.10 (m, 8 H), 1.14 (t, 7.2 Hz, 3 H), 0.83 (d, 6.9 Hz, 3 H), 0.88 (d, 6.4 Hz, 3 H) ppm.

$^{13}$C-NMR (400 MHz, $CDCl_3$, TMS): δ = 156.2 (s), 73.7 (d), 35.7 (t), 34.5 (t), 33.3 (d), 31.9 (d), 30.7 (t), 26.3 (t), 19.2 (q), 15.3 (q), 11.9(q)ppm.

MS (EI, major isomer): *m/z* = 200 (<1), 127 (4), 110 (20), 95 (21), 90 (100), 81 (20), 69 (49), 55 (22), 41 (16).

**Example 1.1.14: Ethyl-carbamic acid 2,3-dimethyl-cyclohexyl ester (BIO1581)**

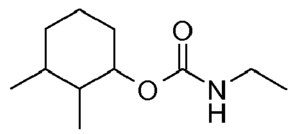

main signals of isomer mixture:

$^1$H-NMR (400 MHz, $CDCl_3$, TMS): δ = 4.60-4.72 (m, 2 H), 3.21 (d,q, 5.3 Hz, 7.2 Hz, 2 H), 2.05 (m, H), 1.25-1.81 (m, 7 H), 1.14 (t, 7.2 Hz, 3 H), 0.89 (d, 6.7 Hz, 3 H), 0.79 (d, 7.2 Hz, 3 H) ppm.

$^{13}$C-NMR (400 MHz, $CDCl_3$, TMS): δ = 156.2 (s), 76.3 (d), 37.5 (d), 35.7 (t), 34.8 (t), 34.2 (d), 27.4 (t), 25.6 (t), 19.1 (q), 15.3 (q), 6.2 (q) ppm.

MS (EI, major isomer): *m/z* = 200 (<1), 199 (<1), 127 (3), 110 (100), 95 (62), 90 (74),81 (67), 69 (65), 55 (37), 41 (24).

**Example 1.1.15: Ethyl-carbamic acid 4-isopropyl-3-methyl-cyclohexyl ester (BIO1560)**

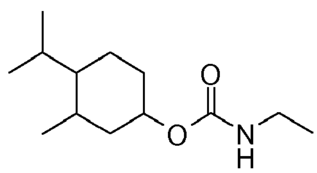

main signals of isomer mixture:

$^1$H-NMR (400 MHz, $CDCl_3$, TMS): δ = 4.78 (t,t, 4.2 Hz, 11.2 Hz, H), 4.61 (m, H), 0.81-2.20 (m, 9 H), 1.12 (t, 7.2 Hz, 3 H), 0.89 (d, 7.0 Hz, 6 H), 0.87 (d, 6.6 Hz, 3 H) ppm.

$^{13}$C-NMR (400 MHz, $CDCl_3$, TMS): δ = 156.4 (s), 70.0 (d), 46.8 (d), 39.6 (t), 35.7 (t), 32.8 (t), 30.1 (d), 26.7 (d), 23.2 (t), 21.6 (q), 21.6 (q), 15.1 (q), 12.6 (q) ppm.

MS (EI): *m/z* = 227 (not detected), 123 (9), 109 (4), 95 (55), 90 (100), 83 (19), 69 (16), 55 (21), 41 (15).

**Examples 1.2: Unsubstituted cyclohexyl carbamates of formula (Carb-II-R1H)**

**Example 1.2.1: Phenyl-carbamic acid cyclohexyl ester (BIO1741)**

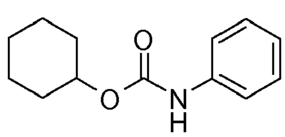

$^{1}$H-NMR (400 MHz, $CDCl_3$, TMS): δ = 7.38 (m, 2 H), 7.30 (m, 3 H), 7.05 (m, H), 6.51 (m, H), 4.76 (t,t, 3.9Hz, 9.0 Hz, H), 1.94 (m, 2 H), 1.75 (m, 2 H), 1.56 (m, H), 1.42 (m, 4 H), 1.27 (m, H) ppm.

$^{13}$C-NMR (400 MHz, $CDCl_3$, TMS): δ = 153.2 (s), 138.1 (s), 129.0 (d), 129.0 (d), 123.2 (d), 118.5 (d), 118.5 (d), 73.7 (d), 31.9 (t), 31.9 (t), 25.4 (t), 23.8 (t), 23.8 (t) ppm.

MS (EI): *m/z* = 220 (4), 219 (25), 137 (59), 132 (15), 119 (30), 93 (100), 83 (54), 67 (24), 55 (83), 41 (40).

**Examples 1.3: Mono-substituted cyclohexyl carbamates of formula (Carb-II-R1H)**

**Example 1.3.1: p-Tolyl-carbamic acid 2-isopropyl-cyclohexyl ester (BIO1824)**

75.6 g (0.56 mol) of para-tolylisocyanate were placed with 500 ml toluene in a one litre vessel and subsequently 73.4 g (0.51 mol) of 2-isopropylcyclohexanol were added. The reaction mixture was heated to reflux for 6 hours. After cooling to room temperature 50 g of water were added and the mixture was refluxed for one more hour. After phase separation the solvent was stripped off and the crude product recrystallized from 235 g of n-heptane. The product (79.8 g) was obtained in form of off-white crystals in 99.2 % purity. This corresponds to a theoretical yield of 56 %.

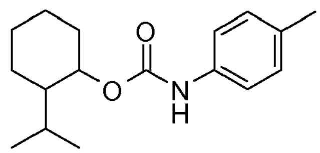

main signals of isomer mixture:

$^{1}$H-NMR (400 MHz, $CDCl_3$, TMS): δ = 7.28 (m, 2 H), 7.10 (m, 2 H), 6.50 (m, H), 5.19 (m, H); 2.30 (s, 3 H), 2.07 (m, H), 1.70-1.81 (m, 2 H), 1.22-1.55 (m, 6 H), 1.07 (m, H), 0.92 (d, 6.7 Hz, 3 H), 0.90 (d, 6.7 Hz, 3 H) ppm.

$^{13}$C-NMR (400 MHz, $CDCl_3$, TMS): δ = 153.4 (s), 135.6 (s), 132.7 (s), 129.5 (d), 129.5 (d), 118.6 (d), 118.6 (d), 71.7 (d), 47.2 (d), 30.9 (t), 29.5 (d), 26.0 (t), 25.1 (t), 20,8 (q), 20.7 (q), 20.7 (q), 20.4 (t) ppm.

MS (EI): *m/z* = 276 (5), 275 (30), 151 (89), 125 (17), 107 (100), 83 (32), 69 (74), 57 (21), 41 (18).

**Example 1.3.2: n-Butyl-carbamic acid 2-isopropyl-cyclohexyl ester (BIO1841)**

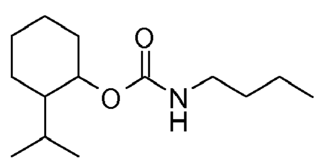

main signals of isomer mixture:

$^{1}$H-NMR (400 MHz, $CDCl_3$, TMS): δ = 5.06 (m, H), 4.65 (m, H), 3.17 (q, 6.4 Hz, 2 H), 2.01 (t, 13.8 Hz, H), 1.74 (m, H), 1.68 (d, 10.3 Hz, H), 1.41-1.53 (m, 3 H), 1.35 (m, 2 H), 1.26 (m, H), 1.24 (m, H), 1.02 (m, H), 0.93 (t, 7.3 Hz, 3 H), 0.90 (d, 6.5 Hz, 6 Hz) ppm.

$^{13}$C-NMR (400 MHz, $CDCl_3$, TMS): δ = 156.5 (s), 70.8 (d), 47.2 (d), 40.7 (t), 32.2 (t), 31.1 (t), 29.5 (d), 26.1 (t), 25.0 (t), 20.8 (q), 20.7 (q), 20.5 (t), 19.9 (t), 13.8 (q) ppm.

MS (EI, major isomer): $m/z$ = 241 (<1), 198 (2), 124 (84), 118 (100), 109 (36), 99 (26), 81 (64), 69 (71), 57 (61), 41 (37).

MS (EI, minor isomer): $m/z$ = 241 (<1), 198 (1), 124 (100), 118 (97), 109 (41), 99 (12), 81 (65), 69 (71), 57 (61), 41 (41).

**Example 1.3.3: (2-Methoxy-phenyl)-carbamic acid 2-isopropyl-cyclohexyl ester (BIO1744)**

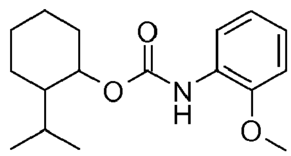

main signals of isomer mixture:

$^1$H-NMR (400 MHz, $CDCl_3$, TMS): δ = 8.12 (m, H), 7.18 (d, 7.4 Hz, H), 6.98 (m, H), 6.95 (m, H), 6.85 (m, H), 5.21 (m, H), 3.88 (s, 3 H), 2.04-2.15 (m, 2 H), 1.15-1.82 (m, 7 H), 1.08 (m, H), 0.92 (d, 6.9 Hz, 3 H), 0.92 (d, 7.0 Hz, 3 H) ppm.

$^{13}$C-NMR (400 MHz, $CDCl_3$, TMS): δ = 153.3 (s), 147.4 (s), 128.0 (s), 122.5 (d), 121.1 (d), 118.0 (d), 109.9 (d), 71.6 (d), 55.6 (q), 47.2 (d), 30.9 (t), 29.4 (d), 26.0 (t), 25.1 (t), 20.8 (q), 20.8 (q), 20.4 (t) ppm.

MS (EI, major isomer): $m/z$ = 292 (3), 291 (21), 167 (45), 123 (100), 108 (46), 81 (46), 69 (87), 55 (29), 41 (36).

MS (EI, minor isomer): $m/z$ = 292 (3), 291 (21), 167 (43), 123 (100), 108 (38), 81 (35), 69 (76), 55 (25), 41 (32).

**Example 1.3.4: (2-Methyl-cyclohexyl)-carbamic acid 4-tert-butyl-cyclohexyl ester BIO1690)**

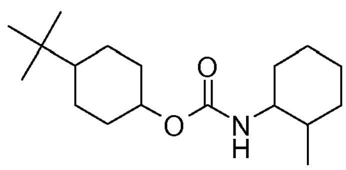

main signals of isomer mixture:

$^1$H-NMR (400 MHz, $CDCl_3$, TMS): δ = 4.50 (m, 2 H), 3.15 (m, H), 2.05 (m, 2 H), 1.79 (m, 2 H), 1.72 (m, 2 H), 0.99-1.38 (m, 12 H), 0.94 (d, 6.4 Hz, 3 H), 0.85 (s, 9 H) ppm.

$^{13}$C-NMR (400 MHz, $CDCl_3$, TMS): δ = 156.0 (s), 73.7 (d), 55.8 (d), 47.2 (d), 38.9 (d), 34.4 (t), 34.1 (t), 34.1 (t), 32.6 (t), 32.6 (t), 32.3 (s), 27.6 (q), 27.6 (q), 27.6 (q), 25.5 (t), 25.5 (t), 25.5 (t), 19.1 (q) ppm.

MS (EI): $m/z$ = 296 (1), 295 (3), 238 (1), 158 (100), 139 (32), 96 (58), 83 (30), 70 (17), 57 (56), 41 (17).

**Example 1.3.5: (2-Hydroxy-phenyl)-carbamic acid 2-isopropyl-cyclohexyl ester (BIO1646)**

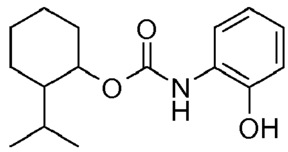

main signals of isomer mixture:

$^1$H-NMR (400 MHz, $CDCl_3$, TMS): δ = 8.00 (m, H), 7.19 (d, 7.8 Hz, H), 7.04 (d, d, 7.3 Hz, 8.1 Hz, H), 6.96 (d, 8.1 Hz, 1H), 6.88 (d,d, 7.3 Hz, 7.9 Hz, H), 6.81 (m, H), 5.22 (m, H), 2.08 (d, 13.1 Hz, H), 1.35-1.55 (m, 4 H), 1.30 m, 2 H), 1.09 (m, H), 0.93 (d, 6.5 Hz, 3 H), 0.91 (d, 6.5Hz, 3H) ppm.

$^{13}$C-NMR (400 MHz, $CDCl_3$, TMS): δ = 155.4 (s), 147.3 (s), 125.7 (d), 125.4 (s), 121.3 (d), 129.9 (d), 118.8 (d), 73.4 (d), 47.1 (d), 30.8 (t), 29.4 (d), 25.9 (t), 25.0 (t), 20.8 (q), 20.7 (q), 20.3 (t) ppm.

MS (EI): *m/z* = 278 (1), 277 (5), 153 (100), 124 (20), 109 (82), 83 (34), 69 (84), 55 (29), 41 (27).

**Example 1.3.6: Phenyl-carbamic acid 2-tert-butyl-cyclohexyl ester (BIO1740)**

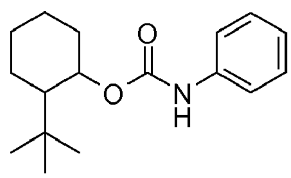

main signals of isomer mixture:

$^1$H-NMR (400 MHz, $CDCl_3$, TMS): δ = 7.41 (m, 2 H), 7.30 (m, 2 H), 7.05 (m, H), 6.57 (m, H), 5.32 (m, H), 2.04 (d, 13.6 Hz, H), 1.84 (d, 12.7 Hz, H), 1.67 (d, 12.7 Hz, H), 1.21-1.56 (m, 5 H), 1.17 (d, 12.6 Hz, H), 0.91 (s, 9 H) ppm.

$^{13}$C-NMR (400 MHz, $CDCl_3$, TMS): δ = 153.0 (s), 138.2 (s), 129.0 (d), 129.0 (d), 123.2 (d), 11.8 (d), 118.5 (d), 72.0 (d), 50.2 (d), 32.6 (s), 31.8 (t), 28.5 (q), 28.5 (q), 28.5 (q), 26.6 (t), 22.3 (t), 20.7 (t) ppm.

MS (EI, major isomer): *m/z* = 276 (1), 275 (6), 123 (32), 93 (71), 83 (39), 67 (18), 57 (100), 41 (25).

MS (EI, minor isomer): *m/z* = 276 (1), 275 (7), 123 (25), 93 (69), 83 (34), 67 (22), 57 (100), 41 (30).

**Example 1.3.7: p-Tolyl-carbamic acid 3-methyl-cyclohexyl ester (BIO1825)**

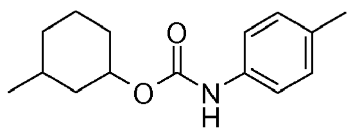

main signals of isomer mixture:

$^1$H-NMR (400 MHz, $CDCl_3$, TMS): δ = 7.25 (m, 2 H), 7.09 (m, 2 H), 6.51 (m, H), 4.67 (t,t, 4.3 Hz, 11.2 Hz, H), 2.30 (s, 3 H), 2.04 (d, 12.2 Hz, 2 H), 1.78 (d, 13.3 Hz, H), 1.63 (d, 13.1 Hz, H), 1.51 (m, H), 1.35 (t,q, 3.6 Hz, 13.1 Hz, H), 1.22 (d,d,d, 11.2 Hz, 11.9 Hz, 13.3 Hz, H), 0.99 (d,t, 11.5 Hz, 12.0 Hz, H), 0.93 (d, 6.5 Hz, 3 H), 0.82 (d,t, 11.9 Hz, 12.9 Hz, H) ppm.

$^{13}$C-NMR (400 MHz, $CDCl_3$, TMS): δ = 153.3 (s), 135.5 (s), 132.7 (s), 129.5 (d), 129.5 (d), 118.7 (d), 118.7 (d), 74.1 (d), 40.9 (t), 34.0 (t), 31.9 (t), 31.4 (d), 24.0 (t), 22.3 (q), 20.7 (q) ppm.

MS (EI): *m/z* = 248 (7), 247 (45), 151 (100), 133 (13), 107 (71), 106 (22), 97 (33), 55 (64), 41(11).

**Example 1.3.8: n-Butyl-carbamic acid 3-methyl-cyclohexyl ester (BIO1821)**

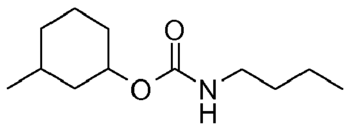

main signals of isomer mixture:

$^1$H-NMR (400 MHz, $CDCl_3$, TMS): δ = 4.57 (m, 2 H), 3.16 (m, 2 H), 1.98 (d, 11.4 Hz, H), 1.75 (d, 13.5 Hz, H), 1.61 (d, 13.0 Hz, H), 1.47 (m, 2 H), 1.34 (m, 2 H), 1.25-1.56 (m, 3 H), 1.16 (m, H), 0.93 (m, H), 0.92 (t, 7.3 Hz, 3 H), 0.92 (d, 7.0 Hz, 3 H), 0.80 (q, 12.6 Hz, H) ppm.

$^{13}$C-NMR (400 MHz, $CDCl_3$, TMS): δ = 156.3 (s), 73.4 (d), 41.0 (t), 40.6 (t), 34.1 8t), 32.1 (t), 32.0 (t), 31.4 (d9, 24.0 (t), 22.3 (q), 19.9 (t), 13.7 (q) ppm.

MS (EI, minor isomer): *m/z* = 214 (1), 213 (1), 170 (2), 126 (2), 118 (100), 97 (32), 81 (14), 55 (31), 41 (10), 30 (14).

MS (EI, major isomer): *m/z* = 214 (1), 213 (2), 170 (3), 126 (9), 118 (100), 97 (45), 81 (11), 55 (36), 41 (11), 30 (12).

**Example 1.3.9: Cyclohexyl-carbamic acid 4-tert-butyl-cyclohexyl ester (BIO1747)**

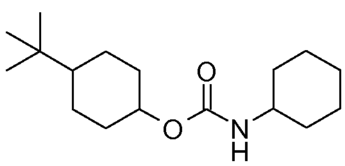

main signals of isomer mixture:

$^1$H-NMR (400 MHz, $CDCl_3$, TMS): δ = 4.44-4.54 (m, 2 H), 3.47 (m, H), 2.05 (d, 12.3 Hz, 2 H), 1.92 (d, 12.3 Hz, 2 H), 1.79 (d, 13.2 Hz, 2 H), 1.69 (d, 13.2 Hz, 2 H), 1.59 (d, 12.8 Hz, H), 0.95-1.46 (m, 9 H), 0.97 (d, 11.7 Hz, H), 0.85 (s, 9 H) ppm.

$^{13}$C-NMR (400 MHz, $CDCl_3$, TMS): δ = 155.5 (s), 73.7 (d), 49.6 (d), 47.2 (d), 33.5 (t), 33.5 (t), 32.6 8t), 32.6 (t), 32.3 (s), 27.6 (q), 27.6 (q), 27.6 (q), 25.5 (t), 25.5 (t), 25.5 (t), 24.8 (t) ppm.

MS (EI, minor isomer): *m/z* = 281 (not detected), 144 (84), 83 (25), 82 (36), 67 (31), 57 (100), 56 (24), 41 (32).

MS (EI, major isomer): *m/z* = 281 (<1), 144 (93), 83 (33), 82 (32), 67 (33), 57 (100), 56 (36), 41 (31).

**Example 1.3.10: Benzyl-carbamic acid 2-isopropyl-cyclohexyl ester (BIO1748)**

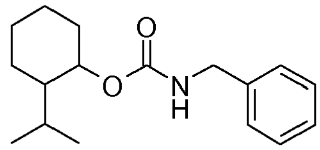

main signals of isomer mixture:

$^1$H-NMR (400 MHz, $CDCl_3$, TMS): δ = 7.34 (m, 2 H), 7.28 (m, 2 H), 7.27 (m, H), 5.12 (m, H), 4.93 (m, H), 4.38 (m, 2 H), 2.03 (m, H), 1.64-1.76 (m, 2 H), 0.99-1.52 (m, 7 H), 0.89 (d, 6.3 Hz, 6 H) ppm.

$^{13}$C-NMR (400 MHz, $CDCl_3$, TMS): δ = 156.5 (s), 138.8 (s), 128.6 (d), 18.6 (d), 127.5 (d), 127.5 (d), 127.4 (d), 71.3 (d), 47.2 (d), 45.0 (t), 31.0 (t), 29.5 (t), 26.0 (d), 25.0 (t), 20.8 (q), 20.8 (q), 20.4 (t) ppm.

MS (EI, major isomer): *m/z* = 275 (<1), 151 (59), 150 (44), 124 (44), 109 (34), 106 (35), 91 (53), 81 (100), 69 (56), 55 (26), 41 (31).

MS (EI, minor isomer): *m/z* = 275 (<1), 151 (64), 150 (65), 124 (50), 109 (39), 106 (30), 91 (69), 81 (100), 69 (60), 55 (30), 41 (42).

**Example 1.3.11: n-Hexyl-carbamic acid 2-isopropyl-cyclohexyl ester (BIO1851)**

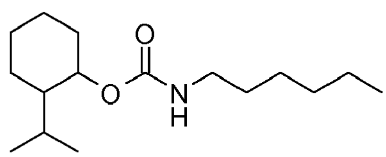

main signals of isomer mixture:

$^1$H-NMR (400 MHz, $CDCl_3$, TMS): δ = 5.06 (m, H), 4.61 8m, H), 3.15 (m, 2 H), 1.99 (d, 13.5 Hz, H), 1.64-1.78 (m, 2 H), 1.47 (m, 4 H), 1.19-1.40 (m, 10 H), 1.02 (m, H), 0.90 (d, 7.0 Hz, 6 H), 0.89 (t, 6.6 Hz, 3 H) ppm.

$^{13}$C-NMR (400 MHz, $CDCl_3$, TM): δ = 156.6 (s), 70.7 (d), 47.3 (d), 41.0 (t), 31.6 (t), 31.1 (t), 31.0 (t), 29.5 (d), 26.5 (t), 26.1 (t), 25.0 (t), 22.6 (t), 20.8 (q), 20.8 (q), 20.5 (t), 14.0 (q) ppm.

MS (EI, major isomer): m/z= 269 (<1), 146 (95), 124 (86), 109 (34), 81 (59), 69 (100), 57 (45), 43 (52), 41 (40).

MS (EI, minor isomer): m/z = 269 (<1), 146 (90), 124 (97), 109 (38), 81 (54), 69 (100), 57 (44), 43 (52), 41 (44).

**Examples 1.4: Tri-substituted cyclohexyl carbamates of formula (Carb-II-R1H)**

**Example 1.4.1: n-Butyl-carbamic acid 2,3,6-trimethyl-cyclohexyl ester (BIO1617)**

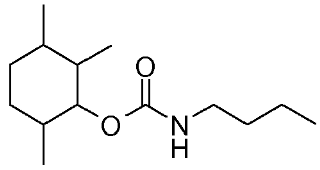

main signals of isomer mixture:

$^1$H-NMR (400 MHz, $CDCl_3$, TMS): $\delta$ = 4.72 (m, H), 4.15 (t, 10.2 Hz, H), 3.18 (m, 2 H), 1.92 (m, H), 1.49 (m, 2 H), 1.35 (m, 2 H), 0.92-1.78 (m, 6 H), 0.85-0.95 (m, 12 H) ppm.

$^{13}$C-NMR (400 MHz, $CDCl_3$, TMS): $\delta$ = 157.4 (s), 82.7 (d), 40.7 (t), 40.6 (d), 36.6 (d), 34.6 (t), 32.9 (t), 32.2 (t), 20.0 (q), 19.9 (d), 19.9 (t), 18.6 (q), 15.1 (q), 13.8 (q) ppm.

MS (EI, major): m/z = 241 (8), 198 (5), 124 (73), 118 (100), 109 (39), 95 (31), 82 (31), 69 (65), 55 (23), 41 (22).

**Example 1.4.2: (2-Methoxy-phenyl)-carbamic acid 2,3,6-trimethyl-cyclohexyl ester (BI01701)**

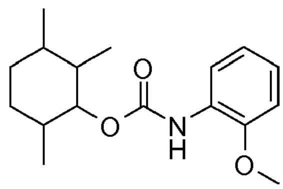

main signals of isomer mixture:

$^1$H-NMR (400 MHz, $CDCl_3$, TMS): $\delta$ = 8.13 (m, H), 7.24 (m, H), 6.92-7.00 (m, 2 H), 6.85 (m, H), 4.29 (t, 10.0 Hz, H), 3.86 (s, 3 H), 0.99-1.76 (m, 7 H), 0.94 (d, 6.4 Hz, 6 H), 0.94 (d, 6.6 Hz, 3 H) ppm.

$^{13}$C-NMR (400 MHz, $CDCl_3$, TMS): $\delta$ = 154.0 (s), 147.5 (s), 128.1 (s), 122.4 (d), 121.1 (d), 118.0 (d), 109.9 (d), 83.4 (d), 55.6 (q), 44.4 (d), 38.1 (d), 37.8 (d), 34.6 (t), 32.9 (t), 20.0 (q), 18.6 (q), 15.2 (q) ppm.

MS (EI, major isomer): m/z = 291 (50), 190 (5), 167 (55), 150 (12), 123 (100), 108 (25), 83 (19), 69 (57), 55 (21), 41 (14).

**Example 1.4.3: sec-Butyl-carbamic acid 3,3,5-trimethyl-cyclohexyl ester (BIO1844)**

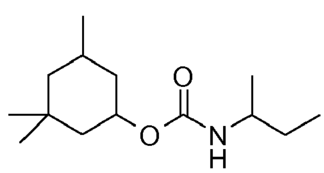

main signals of isomer mixture:

$^1$H-NMR (400 MHz, $CDCl_3$, TMS): $\delta$ = 4.75 (t,t, 4.1 Hz, 11.5 Hz, H), 4.38 (m, H), 3.60 (m, H), 2.00 (d, 11.4 Hz, H), 1.70 (d, 12.3 Hz, H), 1.66 (m, H), 1.43 (m, 2 H), 1.31 (d, 13.2 Hz, H), 1.10 (d, 6.8 Hz, 3 H), 1.04 (m, H), 0.93 (s, 6 H), 0.89 (t, 7.5 Hz, 3 H), 0.89 (d, 6.5 Hz, 3 H), 0.80 (m, H), 0.76 (t, 12.5 HZ, H) ppm.

$^{13}$C-NMR (400 MHz, $CDCl_3$, TMS): δ = 155.8 (s), 71.1 (d), 48.2 (d), 47.6 (t), 44.4 (t), 41.0 (t), 33.1 (q), 32.2 (s), 30.0 (t), 27.1 (d), 25.6 (q), 22.3 (q), 20.7 (q), 10.3 (q) ppm.

MS (EI, major isomer): m/z = 241 (not detected), 226 (<1), 212 (38), 168 (28), 125 (35), 109 (23), 83 (39), 69 (100), 57 (31), 44 (86), 41 (32).

**Example 1.4.4: n-Butyl-carbamic acid 3,3,5-trimethyl-cyclohexyl ester (BIO1616)**

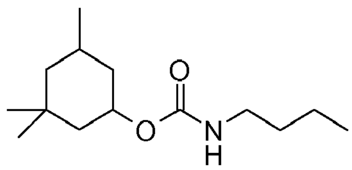

main signals of isomer mixture:

$^{1}$H-NMR (400 MHz, $CDCl_3$, TMS): δ = 4.77 (t,t, 4.1 Hz, 11.6 Hz, H), 4.64 (m, H), 3.16 (q, 6.3 Hz, 2 H), 2.01 (d, 11.6 Hz, H), 1.63-1.75 (m, 3 H), 1.47 (m, 2 H), 1.29-1.39 (m, 3 H), 1.03 (m, H), 0.94 (s, 6 H), 0.92 (t, 7.3 Hz, 3 H), 0.90 (d, 6.5 Hz, 3 H), 0.71-0.85 (m, 2 H) ppm.

$^{13}$C-NMR (400 MHz, $CDCl_3$, TMS): δ = 156.4 (s), 71.2 (d), 47.6 (t), 44.4 (t), 40.9 (t), 40.6 (t), 33.1 (q), 32.2 (t), 32.1 (s), 27.1 (d), 25.5 (q), 22.3 (q), 19.9 (t), 13.7 (q) ppm.

MS (EI): m/z = 242 (<1), 241 (<1), 125 (17), 118 (100), 109 (36), 83 (29), 69 (57), 57 (18), 55 (17), 41 (21).

**Example 1.4.5: (2-Methoxy-phenyl)-carbamic acid 3,3,5-trimethyl-cyclohexyl ester (BIO1703)**

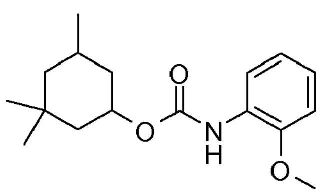

main signals of isomer mixture:

$^{1}$H-NMR (400 MHz, $CDCl_3$, TMS): δ = 8.09 (m, H), 7.17 (m, H), 6.96 (m, 2 H), 6.84 (m, H), 4.89 (t,t, 4.4 Hz, 11.6 Hz, H), 3.85 (s, 3 H), 2.08 (d, 12.0 Hz, H), 1.78 (d, 12.1 Hz, H), 1.73 (m, H), 1.35 (d, 13.2 Hz, H), 1.14 (t, 12.0 Hz. H), 0.97 (s, 3 H), 0.96 (s, 3 H), 0.92 (d, 6.5 Hz, 3 H), 0.90 (m, H), 0.80 (t, 12.7 Hz, H) ppm.

$^{13}$C-NMR (400 MHz, $CDCl_3$, TMS): δ = 153.2 (s), 147.5 (s), 127.9 (s), 122.5 (d), 121.1 (d), 118.1 (d), 109.9 (d), 71.9 (d), 55.6 (q), 47.6 (t), 44.3 (t), 40.8 (t), 33.1 (q), 32.3 (s), 27.1 (d), 25.5 (q), 22.3 (q) ppm.

MS (EI): m/z= 292 (12), 291 (62), 167 (53), 123 (100), 108 (31), 83 (18), 69 (52), 55 (17), 41 (19).

**Example 1.4.6: n-Hexyl-carbamic acid 3,3,5-trimethyl-cyclohexyl ester (BIO1850)**

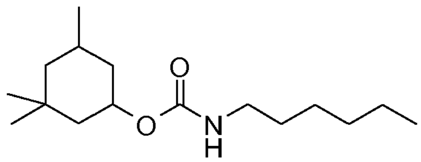

main signals of isomer mixture:

$^1$H-NMR (400 MHz, $CDCl_3$, TMS): δ = 4.77 (t, 11.5 Hz, H), 4.62 (m, H), 3.15 (q, 6.5 Hz, 2 H), 2.00 (d, 11.4 Hz, H), 1.62-1.75 (m, 2 H), 1.47 (m, 2 H), 1.24-1.35 (m, 8 H), 1.04 (m, H), 0.94 (s, 6 H), 0.90 (d, 6.4 Hz, 3 H), 0.88 (t, 6.9 Hz, 3 H), 0.76 (m, H) ppm.

$^{13}$C-NMR (400 MHz, $CDCl_3$, TMS): δ = 156.4 (s), 71.2 (d), 47.6 (t), 44.5 (t), 41.0 (t), 41.0 (t), 33.1 (q), 32.2 (s), 31.5 (t), 30.0 (t), 27.1 (d), 26.4 (t), 25.6 (q), 22.6 (t), 22.3 (q), 14.0 (q) ppm.

MS (EI, minor isomer): m/z= 270 (<1), 269 (1), 146 (100), 125 (16), 109 (35), 83 (36), 69 (82), 55 (23), 41 (32), 30 (24).

MS (EI, major isomer): m/z= 270 (<1), 269 (1), 146 (100), 125 (28), 109 (34), 83 (39), 69 (89), 55 (23), 41 (28), 30 (24).

**Example 1.4.7: Ethyl-carbamic acid 1,7,7-trimethyl-bicvclor2.2.1]hept-2-yl ester (BIO1573)**

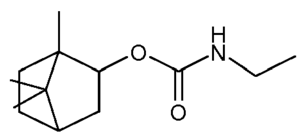

main signals of isomer mixture:

$^1$H-NMR (400 MHz, $CDCl_3$, TMS): δ = 4.83 (d,d,d, 10.0 Hz, 3.4 Hz, 2.0 Hz, H), 4.63 (m, H), 3.22 (d,q, 5.9 Hz, 7.2 Hz, 2H), 2.33 (m, H), 1.88 (m, H), 1.73 (m, H), 1.66 (m, H), 1.17-1.32 (m, 2 H), 1.15 (t, 7.2 Hz, 3 H), 1.01 (m, H), 0.90 (s, 3 H), 0.86 (s, 3 H), 0.84 (s, 3 H) ppm.

$^{13}$C-NMR (400 MHz, $CDCl_3$, TMS): δ = 157.0 (s), 79.9 (d), 48.7 (s), 47.8 (s), 44.9 (d), 36.9 (t), 35.8 (t), 28.1 (t), 27.1 (t), 19.8 (q), 18.8 (q), 15.3 (q), 13.5 (q) ppm.

MS (EI): m/z = 226 (2), 225 (12), 136 (49), 121 (34), 108 (21), 95 (100), 55 (12), 41 (19), 29 (13).

**Example 1.4.8: (3-Methoxy-propyl)-carbamic acid 3,3,5-trimethyl-cyclohexyl ester (BIO1574)**

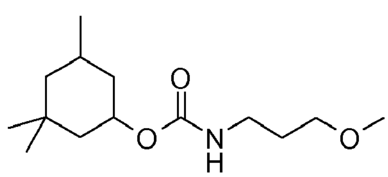

main signals of isomer mixture:

$^1$H-NMR (400 MHz, $CDCl_3$, TMS): δ = 4.94 (m, H), 4.76 (t,t, 4.3 Hz, 11.6 Hz, H), 3.35 (t, 5.9 Hz, 2 H), 3.33 (s, 3 H), 3.27 (q, 5.9 Hz, 2 H), 2.00 (d, 12.3 Hz, H), 1.76 (q, 6.2 Hz, 2 H), 1.62-1.74 (m, 2 H), 1.32 (d, 13.1 Hz, H), 1.03 (m, H), 0.94 (s, 6 H), 0.90 (d, 6.6 Hz, 3 H), 0.78 (m, 2 H) ppm.

$^{13}$C-NMR (400 MHz, $CDCl_3$, TMS): δ = 156.4 (s), 71.3 (d), 71.1 (t), 58.7 (q), 47.6 (t), 44.5 (t), 41.0 (t), 39.0 (t), 33.1 (q), 32.2 (s), 29.7 (t), 27.1 (d), 25.6 (q), 22.3 (q) ppm.

MS (EI, minor isomer): m/z = 257 (2), 242 (1), 109 (100), 101 (35), 90 (48), 69 (96), 55 (39), 45 (39), 41 (63), 30 (37).

MS (EI, major isomer): m/z = 257 (3), 242 (1), 109 (65), 101 (34), 83 (53), 69 (100), 55 (33), 45 (44), 41 (57), 30 (33).

**Example 1.4.9: (2-Hydroxy-phenyl)-carbamic acid 3,3,5-trimethyl-cyclohexyl ester (BIO1642)**

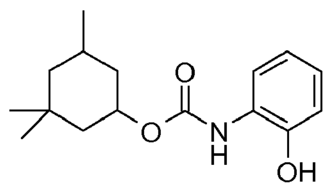

main signals of isomer mixture:

$^1$H-NMR (400 MHz, $CDCl_3$, TMS): δ = 7.90 (m, H), 7.20 (d, 8.0 Hz, H), 7.03 (d,d, 8.1 Hz, 7.3 Hz, H) 6.95 (d, 8.1 Hz, H), 6.86 (d,d, 7.2 Hz, 7.9 Hz, H), 6.80 (m, H), 4.89 (t, t, 4.4 Hz, 11.6 Hz, H), 2.08 (d, 11.8 Hz, H), 1.65-1.83 (m, 3 H), 1.36 (d, 13.2 Hz, H), 1.15 (t, 12.0 Hz, H), 0.97 (s, 3 H), 0.96 (s, 3 H), 0.93 (d, 6.6 Hz, 3 H), 0.80 (t, 12.7 Hz, H) ppm.

$^{13}$C-NMR (400 MHz, $CDCl_3$, TMS): δ = 155.2 (s), 147.2 (s), 125.6 (s), 125.5 (d), 121.2 (d), 120.8 (d), 118.5 (d), 73.5 (d), 47.5 (t), 44.2 (t), 40.6 (t), 22.0 (q), 32.3 (s), 27.1 (d), 25.5 (q), 22.3 (q) ppm.

MS (EI): m/z= 278 (2), 277 (9), 153 (100), 109 (83), 83 (22), 69 (75), 55 (25), 41 (29).

**Example 1.4.10: Ethyl-carbamic acid 3,3,5-trimethyl-cyclohexyl ester (BIO1572)**

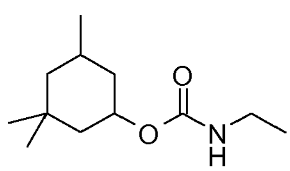

$^1$H-NMR (400 MHz, $CDCl_3$, TMS): δ = 0.70-0.87 (m, 2 H), 0.90 (d, J = 6.5 Hz, 3 H), 0.94 (s, 6 H), 0.95-1.16 (m, 2 H), 1.13 (t, J = 7.2 Hz, 3 H), 1.29-1.36 (m, 1 H), 1.62-1.76 (m, 1 H), 2.01 (d, br., J = 11 Hz, 1 H), 3.14-3.25 (m, 2 H), 4.57 (s, br., 1 H), 4.71-4.83 (m, 1 H) ppm.

$^{13}$C-NMR (400 MHz, $CDCl_3$, TMS): δ = 15.30 ($CH_3$), 22.32 ($CH_3$), 25.56 ($CH_3$), 27.10 (CH), 32.23 (C), 33.07 ($CH_3$), 35.72 ($CH_2$), 40.95 ($CH_2$), 44.46 ($CH_2$), 47.61 ($CH_2$), 71.23 (CH), 156.28 (CO) ppm.

MS (EI): m/z = 214 (1), 141 (4), 124 (12), 109 (52), 95 (9), 90 (100), 83 (19), 69 (34), 55 (11).

**Examples 1.5: N,N-Dialkyl cyclohexyl carbamates of formula (Carb-II)**

**Example 1.5.1: Diethyl-carbamic acid 2,3,6-trimethyl-cyclohexyl ester (BIO1692)**

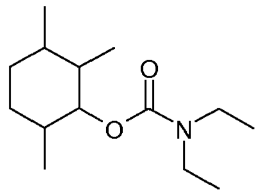

4.27 g (30 mmol) of 2,3,6-trimethylcyclohexanol were placed with 110 ml dichlormethane in a 250 ml vessel at room temperature and 3.08 g (39 mmol) of pyridine were added. The reaction mixture was cooled to 0 ˚C and 3.56 g (12 mmol) of triphosgene in 15 ml dichlormethane were added dropwise. After five minutes 2.37 g (30 mmol) pyridine were added. Subsequently, 2.19 g (30 mmol) of diethylamine in 15 ml dichlormethane were added dropwise, the resulting mixture was allowed to come to ambient temperature and then quenched with water. After separation of the phases, the water phase was extracted once with dichlormethane and the combined organic phases were concentrated. The raw product was purified by distillation and column chromatography to yield 1.5 g of the desired product as a mixture of isomers with a purity of 99.4%.
Main signals of isomer mixture:

$^1$H-NMR (400 MHz, $CDCl_3$, TMS): δ = 4.48 (t, 10.3 Hz, H), 3.29 (q, 4.8 Hz, 4 H), 1.92 (m, H), 1.70 (m, H), 1.44-1.65 (m, 4 H), 1.35 (m, H), 1.12 (t, 7.2 Hz, 6 H), 0.91 (d, 7.4 Hz, 6 H), 0.89 (d, 6.9 Hz, 3 H) ppm.

$^{13}$C-NMR (400 MHz, $CDCl_3$, TMS): δ = 156.5 (s), 79.4 (d), 41.8 (t), 41.0 (t), 40.6 (d), 39.0 (d), 34.6 (d), 32.6 (t), 28.7 (t), 18.5 (q), 15.9 (q), 14.4 (q), 13.6 (q), 12.9 (q) ppm.

MS (EI, major isomer): m/z = 242 (2), 241 (0), 124 (21), 118 (100), 100 (16), 83 (22), 69 (56), 55 (13), 41 (10).

**Example 1.5.2: Diethyl-carbamic acid 2-isopropyl-cyclohexyl ester (BIO1694)**

BIO1694 was produced analogously to the methodology as described for BIO1692 in example 1.5.1 and obtained in comparable yield and puritiy (> 99%) as a mixture of stereoisomers.

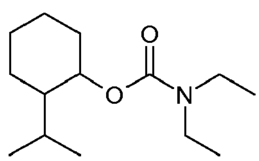

main signals of isomer mixture:

$^1$H-NMR (400 MHz, $CDCl_3$, TMS): δ = 5.10 (m, H), 3.28 (m, 4 H), 2.01 (m, H), 1.66-1.81 (m, 3 H), 0.99-1.52 (m, 6 H), 1.13 (t, 7.1 Hz, 6 H), 0.90 (d, 6.7 Hz, 3 H), 0.89 (d, 6.7 Hz, 3 H) ppm.

$^{13}$C-NMR (400 MHz, $CDCl_3$, TMS): δ = 155.6 (s), 70.9 (d), 47.6 (d), 41.3 (t), 41.3 (t), 31.0 (t), 29.7 (d), 26.2 (t), 25.5 (t), 20.9 (q), 20.7 (q), 20.7 (t), 14.1 (q), 14.1 (q) ppm.

MS (EI): m/z = 242 (2), 241 (8), 124 (82), 118 (100), 100 (28), 83 (48), 69 (97), 57 (29), 41 (17).

**Examples 1.6: Synthesis of menthyl carbamates of formula (M-X)**

**Example 1.6.1: n-Butyl-carbamic acid (1R,2S,5R)-2-isopropyl-5-methyl-cyclohexyl ester (BIO1267)**

66.6 g of menthyl chloroformate (70% in toluene) were added to a mixture of 16.6 g of pyridine and 21.9 g n-butylamine in 100 mL toluene at 0˚C over a period of 50 minutes. After stirring for 12 hours at room temperature, 100 mL of 2M HCl and subsequently 50 mL water were added, the phases separated and the water phase discarded. After washing with saturated $NaHCO_3$-solution and water the organic phase was dried and evaporated to yield 55.1 g of crude product which was recrystallized from 30 g n-heptane to give 34.5 g of the analytically pure product as white crystals.

$^1$H-NMR (400 MHz, $CDCl_3$, TMS): δ = 4.57 (m, H), 4.54 (d,t, 4.1 Hz, 10.8 Hz, H), 3.16 (m, 2 H), 2.04 (d, 11.7 Hz, H), 1.92 (d,q,q, 2.3 Hz, 6.9 Hz, 6.9 Hz, H), 1.66 (m, 2 H), 1.47 (m, 3 H), 1.24-1.39 (m, 3 H), 1.06 (m, H), .081-0.99 (m, 2 H), 0.92 (t, 7.3 Hz, 3 H), 0.90 (d, 6.5 Hz, 3 H), 0.89 (d, 7.0 Hz, 3 H), 0.79 (d, 6.9 Hz, 3 H) ppm.

$^{13}$C-NMR (400 MHz, $CDCl_3$, TMS): δ = 156.5 (s), 74.3 (d), 47.8 (d), 41.6 (t), 40.7 (t), 34.4 (t), 32.1 (t), 31.4 (d), 26.3 (d), 23.6 (t), 22.1 (q), 20.8 (q), 19.9 (t), 16.5 (q), 13.7 (q) ppm.

MS (EI): m/z= 255 (<1), 254 (<1), 138 (83), 118 (100), 95 (88), 83 (83), 69 (33), 55 (53), 41 (39), 29 (20).

**Example 1.6.2: Ethyl-carbamic acid (1R,2S,5R)-2-isopropyl-5-methyl-cyclohexyl ester (BIO1151)**

58.7 g of menthyl chloroformate (80% in toluene) were added to a mixture of 16.6 g of pyridine and 150 mL ethylamine (2M solution in THF) in 100 mL toluene at 0˚C over a period of 30 minutes. After stirring for 12 hours at room temperature, 100 mL of 2M HCl and subsequently 50 mL water were added, the phases separated and the water phase discarded. After washing with saturated $NaHCO_3$-solution and water the organic phase was dried and evaporated to yield 47.1 g of crude product which was recrystallized from 82.4 g n-heptane to give 24.4 g of the analytically pure product as white crystals.

$^1$H-NMR (400 MHz, $CDCl_3$, TMS): δ = 4.54 (d,t, 4.3 Hz, 10.8 Hz, H), 4.54 (m, H), 3.20 (q, 6.9 Hz, 2 H), 2.05 (m, H), 1.92 (d,q,q, 2.7 Hz, 7.0 Hz, 7.0 Hz, H), 1.61-1.71 (m, 2 H), 1.48 (m, H), 1.30 (m, H), 1.13 (t, 7.2 Hz, 3 H), 1.06 (m, H), 0.82-0.99 (m, 2 H), 0.90 (d, 6.6 Hz, 3 H), 0.89 (d, 7.0 Hz, 3 H), 0.79 (d, 7.0 Hz, 3 H) ppm.

$^{13}$C-NMR (400 MHz, $CDCl_3$, TMS): δ = 156.4 (s), 74.3 (d), 47.5 (d), 41.5 (t), 35.8 (t), 34.4 (t), 31.4 (d), 26.3 (d), 23.6 (t), 22.1 (q), 20.8 (q), 16.5 (q), 15.3 (q) ppm.

MS (EI): m/z = 228 (<1), 227 (not detected), 138 (82), 123 (42), 95 (100), 90 (71), 81 (75), 71 (49), 55 (49), 41 (52), 29 (33).

The menthyl carbamates according to examples 1.6.3 to 1.6.20 were produced analogously to the methodology as described in example 1.6.1 and example 1.6.2.

**Example 1.6.3: Cyclohexyl-carbamic acid (1R,2S,5R)-2-isopropyl-5-methyl-cyclohexyl ester (BIO1266)**

$^1$H-NMR (400 MHz, $CDCl_3$, TMS): δ = 4.54 (d,t, 4.0 Hz, 11.0 Hz, H), 4.47 (m, H), 3.46 (m, H), 2.04 (d, 12.0 Hz, H), 1.87-1.97 (m, 3 H), 1.56-1.74 (m, 5 H), 1.48 (m, H), 1.33 (m, 3 H), 1.11 (m, 4 H), 0.93 (m, H), 0.90 (d, 6.6 Hz, 3 H), 0.89 (d, 7.0 Hz, 3 H), 0.84 (m, H), 0.79 (d, 7.0 Hz, 3 H) ppm.

$^{13}$C-NMR (400 MHz, $CDCl_3$, TMS): δ = 155.7 (s), 74.1 (d), 49.7 (d), 47.5 (d), 41.6 (t), 34.4 (t), 33.5 (t), 33.5 (t), 31.4 (d), 26.3 (d), 25.6 (t), 24.8 (t), 24.8 (t), 23.6 (t), 22.1 (q), 20.8 (q), 16.5 (q) ppm.

MS (EI): m/z= 282 (<1), 281 (<1), 144 (87), 138 (65), 95 (49), 83 (100), 69 (37), 55 (67), 41 (36).

**Example 1.6.4: (2-Ethoxy-phenyl)-carbamic acid (1R,2S,5R)-2-isopropyl-5-methyl-cyclohexyl ester (BIO1632)**

$^1$H-NMR (400 MHz, $CDCl_3$, TMS): δ = 8.11 (m, H), 7.18 (m, H), 6.94 (m, 2 H), 6.84 (m, H), 4.69 (d,t, 4.4 Hz, 10.8 Hz, H), 4.09 (q, 7.0 Hz, 2 H), 2.12 (d, 12.1 Hz, H), 2.00 (d,q,q, 2.8 Hz, 7.0 Hz, H), 1.70 (m, 2 H), 1.54 (m, H), 1.46 (t, 7.0 Hz, 3 H), 1.41 (m, H), 1.09 (m, H), 1.04 (d,t, 11.1 Hz, 12.1 Hz, H), 0.92 (d, 7.0 Hz, 3 H), 0.92 (d, 6.5 Hz, 3 H), 0.88 (m, H), 0.82 (d, 6.9 Hz, 3 H) ppm.

$^{13}$C-NMR (400 MHz, $CDCl_3$, TMS): δ = 153.3 (s), 146.7 (s), 128.0 (s), 122.4 (d), 121.0 (d), 118.1 (d), 110.9 (d), 74.9 (d), 64.1 (t), 47.3 (d), 41.4 (t), 34.3 (t), 31.4 (d), 26.2 (d), 23.5 (t), 22.1 (q), 20.8 (q), 16.4 (q), 14.9 (q) ppm.

MS (EI): m/z = 320 (6), 319 (31), 181 (67), 137 (100), 108 (40), 83 (86), 69 (35), 55 (51), 41 (24).

**Example 1.6.5: (2-Acetyl-phenyl)-carbamic acid (1R,2S,5R)-2-isopropyl-5-methyl-cyclohexyl ester (BIO1633)**

$^1$H-NMR (400 MHz, $CDCl_3$, TMS): δ = 11.1 (m, H), 8.51 (d, 8.6 Hz, H), 7.87 (d, 8.0 Hz, H), 7.53 (d,d, 8.5 Hz, 7.2 Hz, H), 7.05 (d,d, 7.2 Hz, 8.0 Hz, H), 4.65 (d,t, 4.4 Hz, 10.8 Hz, H), 2.66 (s, 3 H), 2.10 (d, 11.9 Hz, H), 1.99 (d,q,q, 2.7 Hz, 6.9 Hz, 6.9 Hz, H), 1.69 (m, 2 H), 1.52 (m, H), 1.43 (t, 10.9 Hz, H), 1.09 (m, H), 1.06 (d,t, 11.1 Hz, 12.0 Hz, H), 0.92 (d, 6.5 Hz, 3 H), 0.91 (d, 7.0 Hz, 3 H), 0.88 (m, H), 0.81 (d, 6.9 Hz, 3 H) ppm.

$^{13}$C-NMR (400 MHz, $CDCl_3$, TMS): δ = 202.3 (s), 153.8 (s), 141.7 (s), 135.0 (d), 131.7 (d), 121.4 (s), 121.1 (d), 119.2 (d), 75.1 (d), 47.1 (d), 41.2 (t), 34.3 (t), 31.5 (d), 28.6 (q), 26.2 (d), 23.6 (t), 22.1 (q), 20.8 (q), 16.5 (q) ppm.

MS (EI): m/z= 318 (2), 317 (11), 135 (100), 120 (25), 83 (83), 69 (31), 55 (45), 43 (27).

**Example 1.6.6: Benzyl-carbamic acid (1R,2S,5R)-2-isopropyl-5-methyl-cyclohexyl ester (BIO1695)**

$^1$H-NMR (400 MHz, $CDCl_3$, TMS): δ = 7.34 (m, 3 H), 7.28 (m, 2 H), 7.27 (m, H), 4.89 (m, H), 4.59 (d,t, 4.4 Hz, 10.9 Hz, H), 4.37 (m, 2 H), 2.07 (d, 12.1 Hz, H), 1.93 (m, H), 1.66 (m, 2 H), 1.49 8m, H), 1.31 (t,t, 3.0 Hz, 10.8 Hz, H), 1.06 (m, H), 0.96 (d,t, 11.0 Hz, 12.0 Hz, H), 0.90 (d, 6.6 Hz, 3 H), 0.89 (d, 7.1 Hz, 3 H), 0.85 (m, H), 0.80 (d, 7.1 Hz, 3 H) ppm.

$^{13}$C-NMR (400 MHz, $CDCl_3$, TMS): δ = 156.5 (s), 138.8 (s), 128.6 (d), 18.6 (d), 127.5 (d), 127.4 (d), 127.4 (d), 74.8 (d), 47.4 (d), 45.0 (t), 41.5 (t), 34.3 (t), 31.4 (d), 26.3 (d), 23.6 (t), 22.1 (q), 20.8 (q), 16.5 (q) ppm.

MS (EI): m/z= 290 (<1), 289 (1), 150 (100), 138 (27), 123 (11), 106 (10), 91 (37), 69 (16), 55 (21), 41 (13).

**Example 1.6.7: Cyclohexylmethyl-carbamic acid (1R,2S,5R)-2-isopropyl-5-methyl-cyclohexyl ester (BIO1699)**

$^1$H-NMR (400 MHz, $CDCl_3$, TMS): δ = 4.61 (m, H), 4.54 (d,t, 4.3 Hz, 10.8 Hz, H), 3.00 (m, 2 H), 2.04 (d), 12.1 Hz, H), 1.92 (d,q,q, 2.5 Hz, 7.0 Hz, 7.0 Hz, H), 1.69 (m, 7 H), 1.46 (m, 2 H), 0.81-1.33 (m, 9 H), 0.85 (d, 6.6 Hz, 3 H), 0.89 (d, 7.0 Hz, 3 H), 0.79 (d, 7.0 Hz, 3 H) ppm.

$^{13}$C-NMR (400 MHz, $CDCl_3$, TMS): δ = 156.6 (s), 74.3 (d), 47.5 (d), 47.2 (t), 41.6 (t), 38.3 (d), 34.4 (t), 31.4 (d), 30.7 (t), 30.7 (t), 26.4 (t), 26.3 (d), 25.9 (t), 25.8 (t), 23.6 (t), 22.1 (q), 20.8 (q), 16.5 (q) ppm.

MS (EI): m/z= 296 (<1), 295 (<1), 158 (100), 138 (95), 123 (17), 95 (42), 83 (57), 69 (18), 55 (39), 41 (21).

**Example 1.6.8: (Tetrahydro-furan-2-ylmethyl)-carbamic acid (1R,2S,5R)-2-isopropyl-5-methyl-cyclohexyl ester (BIO1702)**

main signals of isomer mixture:

$^1$H-NMR (400 MHz, $CDCl_3$, TMS): δ = 4.94 (m, H), 4.54 (t, 9.9 Hz, H), 3.96 (m, H), 3.85 (t,d, 6.5 Hz, 8.4 Hz, H), 3.74 (d,d,d, 2.6 Hz, 6.8 Hz, 8.2 Hz, H), 3.42 (m, H), 3.15 (d,d,d, 5.4 Hz, 6.8 Hz, 12.1 Hz, H), 2.03 (d, 12.1 Hz, H), 1.93

(m, 4 H), 1.66 (m, 2 H), 1.56 (m, H), 1.48 (m, H), 1.30 (t, 11.4 Hz, H), 1.06 (m, H), 0.94 (d,t, 10.9 Hz, 12.0 Hz, H), 0.90 (d, 6.6 Hz, 3 H), 0.89 (d, 7.0 Hz, 3 H), 0.84 (m, H), 0.79 (d, 6.9 Hz, 3 H) ppm.

$^{13}$C-NMR (400 MHz, $CDCl_3$, TMS): δ = 156.7 (s), 78.0 (d), 74.5 (d), 68.1 (t), 47.4 (d), 44.7 (t), 41.5 (t), 34.3 (t), 31.3 (d), 28.4 (t), 26.2 (d), 25.9 (t), 23.6 (t), 22.1 (q), 20.8 (q), 16.5 (q) ppm.

MS (EI): m/z = 285 (<1), 284 (1), 139 (33), 102 (18), 83 (46), 71 (100), 55 (21), 43 (25).

**Example 1.6.9: (2-Methoxy-ethyl)-carbamic acid (1R,2S,5R)-2-isopropyl-5-methyl-cyclohexyl ester (BIO1336)**

$^{1}$H-NMR (400 MHz, $CDCl_3$, TMS): δ = 4.96 (m, H), 4.54 (d,t, 4.1 Hz, 10.8 Hz, H), 3.45 (t, 4.9 Hz, 2 H), 3.36 (s, 3 H), 3.36 (m, 2 H), 2.04 (d, 12.8 Hz, H), 1.93 (d,q,q, 2.6 Hz, 7.0 Hz, 7.0 Hz, H), 1.66 (m, 2 H), 1.48 (m, H), 1.30 (t, 11.5 Hz, H), 1.06 (m, H), 0.95 (m, H), 0,90 (d, 6.5 Hz, 3 H), 0.89 (d, 7.0 Hz, 3 H), 0.85 (m, H), 0.79 (d, 7.0 Hz, 3 H) ppm.

$^{13}$C-NMR (400 MHz, $CDCl_3$, TMS): δ = 156.5 (s), 74.6 (d), 71.5 (t), 58.8 (q), 47.4 (d), 41.5 (t), 40.7 (t), 34.3 (t), 31.4 (d), 26.2 (d), 23.6 (t), 22.1 (q), 20.8 (q), 16.5 (q) ppm.

MS (EI): m/z= 258 (<1), 257 (1), 119 (37), 95 (45), 83 (100), 76 (25), 69 (39), 55 (45), 41 (28), 30 (20).

**Example 1.6.10: (6-Hydroxy-hexyl)-carbamic acid (1R,2S,5R)-2-isopropyl-5-methyl-cyclohexyl ester (BIO1662)**

$^{1}$H-NMR (400 MHz, $CDCl_3$, TMS): δ = 4.54 (m, 2 H), 3.64 (t, 6.6 Hz, 2 H), 3.17 (m, 2 H), 2.04 (d, 12.3 Hz, H), 1.91 (d,q,q, 2.5 Hz, 6.9 Hz, 6.9 Hz, H), 1.66 (m, 2 H), 1.24-1.61 (m, 11 H), 1.06 (m, H), 0.93 (m, H), 0.90 (d, 6.6 Hz, 3 H), 0.89 (d, 7.0 Hz, 3 H), 0.86 (m, H), 0.79 (d, 6.9 Hz, 3 H) ppm.

$^{13}$C-NMR (400 MHz, $CDCl_3$, TMS): δ = 156.6 (s), 74.3 (d), 62.5 (t), 47.4 (d), 41.5 (t), 40.7 (t), 34.3 (t), 32.6 (t), 31.4 (d), 30.0 (t), 26.4 (t), 26.3 (d), 25.3 (t), 23.5 (t), 22.1 (q), 20.8 (q), 16.5 (q) ppm.

MS (EI): m/z= 299 (<1), 138 (30), 123 (31), 95 (82), 81 (83), 71 (74), 55 (90), 41 (100), 31 (45).

**Example 1.6.11: (3-Methoxy-propyl)-carbamic acid (1R,2S,5R)-2-isopropyl-5-methyl-cyclohexyl ester (BIO1155)**

$^{1}$H-NMR (400 MHz, $CDCl_3$, TMS): δ = 4.96 (m, H), 4.54 (d,t, 4.1 Hz, 10.7 Hz, H), 3.45 (t, 6.0 Hz, 2 H), 3.33 (s, 3 H), 3.28 (d,t, 6.0 Hz, 6.0 Hz, 2 H), 2.04 (d, 11.5 Hz, H), 1.92 (d,q,q, 2.4 Hz, 7.0 Hz, 7.0 Hz, H), 1.78 (t,t, 6.3 Hz, 6.3 Hz, 2 H), 1.66 (m, 2 H), 1.48 (m, H), 1.29 (t, 11.1 Hz, H), 1.06 (m, H), 0.81-0.99 (m, 2 H), 0.90 (d, 6.5 Hz, 3 H), 0.89 (d, 7.0 Hz, 3 H), 0.79 (d, 7.0 Hz, 3 H) ppm.

$^{13}$C-NMR (400 MHz, $CDCl_3$, TM): δ = 156.5 (s), 74.3 (d), 71.1 (t), 58.7 (q), 47.4 (d), 41.5 (t), 39.0 (t), 34.4 (t), 31.4 (d), 29.7 (t), 26.3 (d), 23.6 (t), 22.1 (q), 20.8 (q), 16.5 (q) ppm.

MS (EI): m/z = 271 (1), 138 (50), 101 (36), 95 (86), 90 (72), 83 (100), 71 (58), 55 (57), 41 (42), 30 (19).

**Example 1.6.12: (3-Isopropoxy-propyl)-carbamic acid (1R,2S,5R)-2-isopropyl-5-methyl-cyclohexyl ester (BIO1268)**

$^{1}$H-NMR (400 MHz, $CDCl_3$, TMS): δ = 5.01 (m, H), 4.53 (d,t, 4.1 Hz, 10.8 Hz, H), 3.55 (q,q, 6.0 Hz, 6.0 Hz, H), 3.48 (t, 5.8 Hz, 2 H), 3.28 (d,t, 6.3 Hz, 6.3 Hz, 2 H), 2.04 (d, 11.6 Hz, 1.92 (d,q,q, 2.4 Hz, 7.0 Hz, 7.0 Hz, H), 1.75 (t,t, 6.2 Hz, 6.2 Hz, 2 H), 1.66 (m, 2 H), 1.48 (m, H), 1.29 (m, H), 1.15 (d, 6.1 Hz, 6 H), 1.06 (m, H), 0.81-0.99 (m, 2 H), 0.90 (d, 6,5 Hz, 3 H), 0.89 (d, 7.0 Hz, 3 H), 0.79 (d, 7.0 Hz, 3 H) ppm.

$^{13}$C-NMR (400 MHz, $CDCl_3$, TMS): δ = 156.5 (s), 74.3 (d), 71.6 (d), 66.5 (t), 47.4 (d), 41.5 (t), 39.3 (t), 34.4 (t), 31.4 (d), 30.0 (t), 26.4 (d), 23.7 (t), 22.1 (q), 22.1 (q), 22.1 (q), 20.8 (q), 16.6 (q) ppm.

MS (EI): m/z= 300 (<1), 299 (<1), 118 (93), 102 (100), 95 (42), 83 (84), 57 (64), 43 (42).

**Example 1.6.13: Hexyl-carbamic acid (1R,2S,5R)-2-isopropyl-5-methyl-cyclohexyl ester (BIO1271)**

$^{1}$H-NMR (400 MHz, $CDCl_3$, TMS): δ = 4.57 (m, H), 4.54 (d,t, 4.2 Hz, 10.8 Hz, H), 3.15 (m, 2 H), 2.04 (m, H), 1.92 (d,q,q, 2.3 Hz, 7.0 Hz, 7.0 Hz, H), 1.66 (m, 2 H), 1.41-1.55 (m, 4 H), 1.24-1.36 (m, 6 H), 1.06 (m, H), 0.81-0.99 (m, 2 H), 0.90 (d, 6.5 Hz, 3 H), 0.89 (t, 7.0 Hz, 3 H), 0.89 (d, 7.0 Hz, 3 H), 0.79 (d, 7.0 Hz, 3 H) ppm.

$^{13}$C-NMR (400 MHz, $CDCl_3$, TMS): δ = 156.5 (s), 74.3 (d), 47.5 (d), 41.6 (t), 41.0 (t), 34.4 (t), 31.5 (t), 31.4 (d), 30.0 (t), 26.4 (t), 26.3 (d), 23.6 (t), 22.6 (t), 22.1 (q), 20.8 (q), 16.5 (q), 14.0 (q) ppm.

MS (EI): m/z= 284 (<1), 283 (<1), 146 (86), 138 (79), 95 (70), 83 (100), 69 (35), 55 (53), 43 (41), 30 (20).

**Example 1.6.14: Isopropyl-carbamic acid (1R,2S,5R)-2-isopropyl-5-methyl-cyclohexyl ester (BIO1272)**

$^{1}$H-NMR (400 MHz, $CDCl_3$, TMS): δ = 4.53 (d,t, 3.9 Hz, 10.6 Hz, H), 4.39 (m, H), 3.78 (m, H), 2.03 (d, 12.0 Hz, H), 1.90 (d,q,q, 2.5 Hz, 7.0 Hz, 7.0 Hz, H), 1.64 (m, 2 H), 1.47 (m, H), 1.27 (m, H), 1.13 (d, 6.6 Hz, 6 H), 1.04 (m, H), 0.80-0.98 (m, 2 H), 0.88 (d, 6.5 Hz, 3 H), 0.88 (d, 7.0 Hz, 3 H), 0.78 (d, 6.9 Hz, 3 H) ppm.

$^{13}$C-NMR (400 MHz, $CDCl_3$, TMS): δ 155.7 (s), 74.2 (d), 47.5 (d), 42.9 (d), 41.6 (t), 34.4 (t), 31.4 (d), 26.3 (d), 23.6 (t), 23.1 (q), 23.1 (q), 22.1 (q), 20.8 (q), 16.5 (q) ppm.

MS (EI): m/z= 242 (<1), 241 (<1), 226 (4), 138 (94), 104 (97), 95 (97), 83 (100), 69 (42), 55 (64), 43 (53), 29 (12).

**Example 1.6.15: Isobutyl-carbamic acid (1R,2S,5R)-2-isopropyl-5-methyl-cyclohexyl ester (BIO1159)**

$^{1}$H-NMR (400 MHz, $CDCl_3$, TMS): δ = 4.64 (m, H), 4.54 (d,t, 4.2 Hz, 10.8 Hz, H), 3.00 (m, 2 H), 2.04 (d, 11.9 Hz, H), 1.92 (d,q,q, 2.7 Hz, 7.0 Hz, 7.0 Hz, H), 1.75 (m, H), 1.66 (m, 2 H), 1.48 (m, H), 1.29 (t, 11.6 Hz, H), 1.06 (m, H), 0.81-0.99 (m, 2 H), 0.91 (d, 6.7 Hz, 6 H), 0.90 (d, 7.1 Hz, 3 H), 0.90 (d, 6.5 Hz, 3 H), 0.79 (d, 7.0 Hz, 3 H) ppm.

$^{13}$C-NMR (400 MHz, $CDCl_3$, TMS): δ = 156.6 (s), 74.3 (d), 48.3 (t), 47.5 (d), 41.5 (t), 34.4 (t), 31.4 (d), 28.9 (d), 26.4 (d), 23.6 (t), 22.1 (q), 20.8 (q), 19.9 (q), 19.9 (q), 16.5 (q) ppm.

MS (EI): m/z = 255 (<1), 212(1), 138(71), 118 (62), 95 (47), 83 (100), 69(29), 57 (41), 41 (28), 30 (26).

**Example 1.6.16: Methyl-carbamic acid (1S,2R,5S)-2-isopropyl-5-methyl-c-clohexyl ester (BIO1301)**

$^{1}$H-NMR (400 MHz, $CDCl_3$, TMS): δ = 4.57 (m, H), 4.52 (d,t, 4.4 Hz, 10.7 Hz, H), 2.76 (d, 4.9 Hz, 3 H), 2.02 (d, 11.5 Hz, H), 1.90 (d,q,q, 2.5 Hz, 7.0 Hz, 7.0 Hz, H), 1.64 (m, 2 H), 1.46 (m, H), 1.27 (t, 11.0 Hz, H), 1.04 (m, H), 0.79-0.96 (m, 2 H), 0.88 (d, 6,5 Hz, 3 H), 0.87 (d, 7.0 Hz, 3 H), 0.77 (d, 7.0 Hz, 3 H) ppm.

$^{13}$C-NMR (400 MHz, $CDCl_3$, TMS): δ = 157.1 (s), 74.4 (d), 47.4 (d), 41.5 (t), 34.3 (t), 31.4 (d), 27.5 (q), 26.2 (d), 23.5 (t), 22.1 (q), 20.8 (q), 16.5 (q) ppm.

MS (EI): m/z = 214 (<1), 213 (not detected), 138 (72), 123 (38), 95 (100), 81 (81), 76 (43), 67 (29), 55 (48), 41 (33), 29 (12).

**Example 1.6.17: (2-Hydroxy-ethyl)-carbamic acid (1R,2S,5R)-2-isopropyl-5-methyl-cyclohexyl ester (BIO1338)**

$^1$H-NMR (400 MHz, $CDCl_3$, TMS): δ = 5.01 (m, H), 4.55 (d, t, 4.0 Hz, 10.7 Hz, H), 3.73 (m, 2 H), 3.34 (m, 2 H), 2.31 (m, 3 H), 2.05 (d, 11.9 Hz, H), 1.92 (d,q,q, 2.7 Hz, 7.0 Hz, 7.0 Hz, H), 1.67 (m, 2 H), 1.48 (m, H), 1.31 (t, 11.9 Hz, H), 1.06 (m, H), 0.79-1.01 (m, 2 H), 0.90 (d, 6.6 Hz, 3 H), 0.89 (d, 7.0 Hz, 3 H), 0.79 (d, 7.0 Hz, 3 H) ppm.

$^{13}$C-NMR (400 MHz, $CDCl_3$, TMS): δ = 157.4 (s), 74.9 (d), 62.3 (t), 47.3 (d), 43.4 (t), 41.4 (t), 34.3 (t), 31.4 (d), 26.2 (d), 23.5 (t), 22.1 (q), 20.8 (q), 16.4 8q) ppm.

MS (EI): m/z= 243 (<1), 138 (57), 106 (40), 95 (65), 83 (100), 69 (40), 55 (57), 41 (38).

**Example 1.6.18: Benzo[1,3]dioxol-5-ylmethyl-carbamic acid (1R,2S,5R)-2-isopropyl-5-methyl-cyclohexyl ester (BIO1571)**

$^1$H-NMR (400 MHz, $CDCl_3$, TMS): δ = 6.78 (m, H), 6.76 (d, 7.9 Hz, H), 6.73 (d, 7.9 Hz, H), 4.87 (m, H), 4.58 (d,t, 4.4 Hz, 10.8 Hz, H), 4.27 (m, 2 H); 2.06 (d, 11.9 Hz, H), 1.92 (m, H), 1.62-1.71 (m, 2 H), 1.49 (m, H), 1.30 (t, 12.0 Hz, H), 1.06 (m, H), 0.81-0.99 (m, 2 H), 0.90 (d, 6.6 Hz, 3 H), 0.88 (d, 7.0 Hz, 3 H), 0.80 (d, 7.0 Hz, 3 H) ppm.

$^{13}$C-NMR (400 MHz, $CDCl_3$, TMS): δ = 156.4 (s), 147.9 (s), 146.9 (s), 132.7 (s), 120.7 (d), 108.2 (d), 108.1 (d), 101.0 (t), 74.8 (d), 47.4 (d), 44.9 (t), 41.5 (t), 34.3 (t), 31.4 (d), 26.3 (d), 23.5 (t), 22.1 (q), 20.8 (q), 16.5 (q) ppm.

MS (EI): m/z = 334 (2), 333 (9), 150 (15), 135 (30), 95 (11), 83 (16), 69 (11), 55 (17), 41 (12).

**Example 1.6.19: Phenyl-carbamic acid (1R,2S,5R)-2-isopropyl-5-methyl-cyclohexyl ester (BIO1580)**

$^1$H-NMR (400 MHz, $CDCl_3$, TMS): δ = 7.39 (m, 2 H), 7.30 (m, 2 H), 7.04 (m, H), 6.55 (m, H), 4.66 (d,t, 4.4 Hz, 10.9 Hz, H), 2.11 (d, 12.0 Hz, H), 1.97 (d,q,q, 2.7 Hz, 6.9 Hz, 6.9 Hz, H), 1.69 (m, 2 H), 1.52 (m, H), 1.37 (d,d, 10.9 Hz, 12.5 Hz, H), 1.09 (m, H), 1.01 (d,t, 10.8 Hz, 11.9 Hz, H), 0.92 (d, 6.5 Hz, 3 H), 0.91 (d, 7.0 Hz, 3 H), 0.88 (m, H), 0.81 (d, 6.9 Hz, 3 H) ppm.

$^{13}$C-NMR (400 MHz, $CDCl_3$, TMS): δ = 153.3 (s), 138.2 (s), 129.0 (d), 129.0 (d), 123.2 (d), 118.4 (d), 118.4 (d), 75.1 (d), 47.4 (d), 41.4 (t), 34.3 (t), 31.4 (d), 26.3 (d), 23.5 (t), 22.0 (q), 20.8 (q), 16.4 (q) ppm.

MS (EI): m/z= 276 (4), 275 (21), 137 (44), 119 (25), 93 (93), 83 (100), 69 (33), 55 (40), 41 (23).

**Example 1.6.20: Methyl-carbamic acid (1R,2S,5R)-2-isopropyl-5-methyl-cyclohexyl ester (BIO1185)**

$^1$H-NMR (400 MHz, $CDCl_3$, TMS): δ = 4.57 (m, H), 4.52 (d, t, 4.4 Hz, 10.7 Hz, H), 2.76 (d, 4.9 Hz, 3 H), 2.02 (d, 11.5 Hz, H), 1.90 (d,q,q, 2.5 Hz, 7.0 Hz, 7.0 Hz, H), 1.64 (m, 2 H), 1.46 (m, H), 1.27 (t, 11.0 Hz, 11.0 Hz, H), 1.04 (m, H), 0.91 (m, H), 0.87 (d, 6.5 Hz, 3 H), 0.87 (d, 7.0 Hz, 3 H), 0.82 (m, H), 0.77 (d, 7.0 Hz, 3 H) ppm.

$^{13}$C-NMR (200 MHz, $CDCl_3$, TM): δ = 157.1 (s), 74.4 (d), 47.4 (d), 41.5 (t), 34.3 (t), 31.4 (d), 27.5 (q), 26.2 (d), 23.5 (t), 22.1 (q), 20.8 (q), 16.5 (q) ppm.

MS (EI): m/z = 213 (not detected), 198 (0), 138 (62), 123 (34), 95 (100), 81 (79), 76 (49), 55 (52), 41 (50).

**Example 1.6.21: Diethyl-carbamic acid (1R,25,5R)-2-isopropyl-5-methyl-cyclohexyl ester (BIO1553)**

30 mmol of l-menthol were placed with 110 ml dichlormethane in a 250 ml vessel at room temperature and 3.08 g (39 mmol) of pyridine were added. The reaction mixture was cooled to 0 ˚C and 3.56 g (12 mmol) of triphosgene

in 15 ml dichlormethane were added dropwise. After five minutes 2.37 g (30 mmol) pyridine were added. Subsequently, 2.19 g (30 mmol) of diethylamine in 15 ml dichlormethane were added dropwise, the resulting mixture was allowed to come to ambient temperature and then quenched with water. After separation of the phases, the water phase was extracted once with dichlormethane and the combined organic phases were concentrated. The raw product was purified by distillation and column chromatography to yield 1.5 g of the desired product as a mixture of isomers with a purity of 99.4%.
Main signals of isomer mixture:

$^1$H-NMR (400 MHz, $CDCl_3$, TMS): δ = 4.57 (d,t, 4.4 Hz, 10.8 Hz, H), 3.26 (m, 4 H), 2.06 (d, 12.0 Hz, H), 1.94 (d,q, q, 2.8 Hz, 7.0 Hz, 7.0 Hz, H), 1.69 (m, H), 1.65 (m, H), 1.49 (m, H), 1.36 (d,d,t, 3.0 Hz, 10.8 Hz, 12.4 Hz, H), 1.11 (t, 7.1 Hz, 6 H), 1.07 (m, H), 0.95 (d,d, 10.9 Hz, 12.1 Hz, H), 0.90 (d, 7.0 Hz, 3 H), 0.90 (d, 6.6 Hz, 3 H), 0.86 (m, H), 0.79 (d, 6.9 Hz, 3 H) ppm.

$^{13}$C-NMR (400 MHz, $CDCl_3$, TMS): δ = 155.8 (s), 74.6 (d), 47.5 (d), 41.6 (t), 40.9 (t), 40.9 (t), 34.4 (t), 31.4 (d), 26.3 (d), 23.5 (t), 22.1 (q), 20.9 (q), 16.4 (q), 13.9 (q), 13.9 (q) ppm.

MS (EI): m/z = 255 (2), 138 (82), 118 (100), 95 (50), 83 (93), 69 (67), 55 (63), 41 (31), 29 (32).

**Example 2: SIRT1 assay**

NHDF cells (normal human dermal fibroblasts) were seeded in 96-well plates. After 24 h cultivation at 37 °C and 5% $CO_2$ in DMEM (Dulbecco's Modified Eagle Medium), cells were treated with test substances for another 48 h. After washing the cells with PBS (phosphate buffered saline), the cells were fixed with paraformaldehyde and permeabilized. Then they were washed again and blocked with BSA (bovine serum albumen), followed by incubation with secondary antibody. After extensive washing, the fluorescence is measured in a microplate reader and fluorescence images were recorded on a fluorescence microscope with an attached closed-circuit display camera.
The stimulation of SIRT1 expression was calculated by:

$$\text{Stimulation of SIRT1 expression [\%]} = \left( \frac{\text{RFU test substance - RFU background}}{\text{RFU control - RFU background}} \text{ x } 100 \right) - 100$$

RFU test substance = relative fluorescent units of the wells with test substance, stained completely
RFU control = relative fluorescent units of the wells without test substance, stained completely
RFU background = relative fluorescent units of the wells without test substance, stained only with secondary antibody.

Table Example 2: Stimulation of SIRT1 expression by the individual substances (mean values of at least 2 independent tests)

| | test substance | concentration [μM] | Stimulation |
|---|---|---|---|
| BIO1841 | Butyl-carbamic acid 2-isopropyl-cyclohexyl ester tested as following mixture of isomeres:<br>79% Butyl-carbamic acid (1R*,2R*)-2-isopropyl-cyclohexyl ester<br>20% Butyl-carbamic acid (1R*,2S*)-2-isopropyl-cyclohexyl ester | 30 | 14% |

(continued)

| | test substance | concentration [μM] | Stimulation |
|---|---|---|---|
| BIO1823 | (4-Ethyl-phenyl)-carbamic acid 3,5-dimethyl-cyclohexyl ester tested as following mixture of isomers: 76.7% (4-Ethyl-phenyl)-carbamic acid (1alpha,3alpha,5alpha)-3,5-dimethyl-cyclohexylester + (4-Ethyl-phenyl)-carbamic acid (1alpha*,3alpha*, 5beta*)-3,5-dimethyl-cyclohexylester 23.1 % (4-Ethyl-phenyl)-carbamic acid (1alpha,3beta,5beta)-3,5-dimethyl-cyclohexylester | 30 | 66% |
| BIO1845 | sec-Butyl-carbamic acid 2,3-dimethyl-cyclohexyl ester | 30 | 52% |
| BIO1617 | Butyl-carbamic acid 2,3,6-trimethyl-cyclohexyl ester | 30 | 24% |
| BIO1267 | Butyl-carbamic acid (1R,2S, 5R)-2-isopropyl-5-methyl-cyclohexyl ester | 30 | 29% |
| BIO1271 | Hexyl-carbamic acid (1R,2S, 5R)-2-isopropyl-5-methyl-cyclohexyl ester | 30 | 66% |
| BIO1580 | Phenyl-carbamic acid (1R, 2S,5R)-2-isopropyl-5-methyl-cyclohexyl ester | 30 | 117% |
| BIO1860 | Butyl-carbamic acid (1S,2R, 5S)-2-isopropyl-5-methyl-cyclohexyl ester | 30 | 47% |

**Example 3: Proteasome activity**

Examples 3.1 and 3.2 describe the measurement of proteasome activity of BIO1272.

| | | |
|---|---|---|
| BIO1272 | Isopropyl-carbamic acid (1 R,2S,5R)-2-isopropyl-5-methyl-cyclohexyl ester | 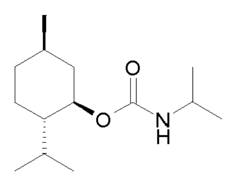 |

**Example 3.1: Measurement of proteasome activity using isolated proteasome**

The method used herein to investigate the proteasome stimulating activity of a substance is based on the measurement of the 20S proteasome core activity. To detect this activity, the isolated 20S proteasome reacts with an oligopeptide, which is bound to a fluorochrome. With its chymotrypsin-like proteolytic activity, the proteasome degrades

the oligopeptide and liberates the fluorochrome. For this assay 4-methyl-coumaryl-amide (MCA) was used as fluorochrome. Liberation of MCA correlates linearly with the proteasome activity. MCA wass detected using an excitation of 360 nm and an emission of 460 nm.

For interpretation, the measured proteasome activities / intensities of fluorescence were compared with pure MCA standards at distinct concentrations.

Two different controls were used for each assay. One "ordinary" control (C) which consisted of isolated proteasome alone, and a DMSO-control (DC). DC was necessary because all substances were diluted in DMSO. Proteasome activity was calculated relative to DMSO control (0 mM).

**Table Example 3.1: Measurement of proteasome activity of BIO1272 using isolated** proteasome (mean values of 3 independent tests)

| Concentration BIO1272 [mM] | 0 mM | 5mM | 10mM | 20mM |
|---|---|---|---|---|
| Relative proteasome activity [%] | 100 % | 125 % | 210 % | 250 % |

**Example 3.2: Measurement of proteasome activity using cell lysates**

To determine proteasome activity in cell lysate, human keratinocyte cell lines (HaCaT cells) were seeded out in Petri-dishes (area: 22.1 $cm^2$) and harvested when they reached confluence. Isolation of the pure cell lysate included cell washing, incubation and homogenization in lysis buffer (250mM sucrose, 25mM HEPES (4-(2-hydroxyethyl)-1-piperazineethanesulfonic acid), 10mM $MgCl_2$*6H2O, 1.3mM EDTA) and final cell lysis with liquid nitrogen. Determination of proteasome activity is based on the liberation of MCA and the corresponding fluorescence like described above in example 3.1. Proteasome activity was calculated relative to DMSO control (0 mM).

Table Example 3.2: Measurement of proteasome activity of BIO1272 using cellular extracts (mean values of 3 independent tests)

| **Concentration BIO1272 [mM]** | 0 mM | 5mM | 10mM | 20mM |
|---|---|---|---|---|
| **Relative proteasome activity [%]** | 100 % | 105 % | 120 % | 140 % |

**Formulation examples: "Compound of list A"**

Unless indicated otherwise in the respective formulation example, each compound from the following List A was formulated separately into each single formulation of the formulation examples **K1** - **K9** and **F1 - F10** given below.

**List A:**

BIO1151, BIO1155, BIO1266, BIO1267, BIO1271, BIO1272, BIO1336, BIO1338, BIO1339, BIO1378, BIO1460, BIO1461, BIO1551, BIO1561, BIO1571, BIO1574, BIO1580, BIO1615, BIO1617, BIO1632, BIO1633, BIO1634, BIO1643, BIO1685, BIO1690, BIO1694, BIO1695, BIO1699, BIO1703, BIO1707, BIO1741, BIO1822, BIO1823, BIO1824, BIO1840, BIO1841, BIO1845, BIO1850, BIO1851 and BIO1860.

Additionally, several formulations were produced including mixtures of two, three of four different compounds selected from list A. In such a case, the amount used in the formulation example refers to the sum of the compounds selected from list A used therein.

In case two different compounds of list A were used as a mixture in the formulation examples given herein, generally the ratio by weight of the two compounds was chosen in the range of from 10 : 1 to 1 : 10, preferably in the range of from 5 : 1 to 1 : 5, more preferably in the range of from 3 : 1 to 1 : 3.

In formulation examples **K1 - K3** and **K5 - K9** the following two perfume oils PFO1 and PF02 were each used as fragrance (DPG = dipropylene glycol).

Perfume oil PFO1 with rose smell

| Component / NAME | Parts by weight |
|---|---|
| Acetophenone, 10% in DPG | 10.00 |
| n-Undecanal | 5.00 |
| Aldehyde C14, so-called (peach aldehyde) | 15.00 |
| Allylamyl glycolate, 10% in DPG | 20.00 |
| Amyl salicylate | 25.00 |
| Benzyl acetate | 60.00 |
| Citronellol | 80.00 |
| d-Limonene | 50.00 |
| Decenol trans-9 | 15.00 |
| Dihydromyrcenol | 50.00 |
| Dimethylbenzylcarbinyl acetate | 30.00 |
| Diphenyloxide | 5.00 |
| Eucalyptol | 10.00 |
| Geraniol | 40.00 |
| Nerol | 20.00 |
| Geranium oil | 15.00 |
| Hexenol cis-3, 10% in DPG | 5.00 |
| Hexenyl salicylate cis-3 | 20.00 |
| Indole, 10% in DPG | 10.00 |
| Alpha-lonone | 15.00 |
| Beta-lonone | 5.00 |
| Lilial® (2-methyl-3-(4-tert-butyl-phenyl)propanal) | 60.00 |
| Linalool | 40.00 |
| Methylphenyl acetate | 10.00 |
| Phenylethyl alcohol | 275.00 |
| Styrolyl acetate | 20.00 |
| Terpineol | 30.00 |
| Tetrahydrolinalool | 50.00 |
| Cinnamyl alcohol | 10.00 |
| Total: | 1,000.00 |

Perfume oil PFO2 with white blossom and musk smell

| Component / NAME | Parts by weight |
|---|---|
| Benzyl acetate | 60.00 |

(continued)

| Component / NAME | Parts by weight |
|---|---|
| Citronellyl acetate | 60.00 |
| Cyclamenaldehyde (2-methyl-3-(4-isopropylphenyl)propanal | 20.00 |
| Dipropylene glycol (DPG) | 60.00 |
| Ethyllinalool | 40.00 |
| Florol (2-isobutyl-4-methyltetrahydro-2H-pyran-4-ol) | 30.00 |
| Globanone® [(E/Z)-8-cyclohexadecen-1-one] | 180.00 |
| Hedione® (methyldihydrojasmonate) | 140.00 |
| Hexenyl salicylate, cis-3 | 10.00 |
| Vertocitral (2,4-dimethyl-3-cyclohexenecarboxaldehyde) | 5.00 |
| Hydratropaaldehyde, 10% in DPG | 5.00 |
| Isodamascone (1-(2,4,4-trimethyl-2-cyclohexen-1-yl)-2-buten-1-one, 10% in DPG | 5.00 |
| Isomuscone (cyclohexadecanone) | 40.00 |
| Jacinthaflor (2-methyl-4-phenyl-1,3-dioxolane) | 10.00 |
| Cis-jasmone, 10% in DPG | 20.00 |
| Linalool | 50.00 |
| Linalyl acetate | 30.00 |
| Methyl benzoate, 10% in DPG | 25.00 |
| para-Methyl cresol, 10% in DPG | 10.00 |
| Nerol | 20.00 |
| Phenylpropylaldehyde | 5.00 |
| 2-Phenylethyl alcohol | 82.00 |
| Tetrahydrogeraniol | 13.00 |
| 2,2-Dimethyl-3-cyclohexyl-1-propanol | 80.00 |
| Total: | 1,000.00 |

**Formulation examples K1 - K9:**

Formulations according to the invention with compositions according to Table 1

**K1 = Sun Protection Gel (SPF 6)**
K2 = Sun Protection Lotion SPF 24 (UVA/UVB Balance)
K3 = Tinted Anti Ageing Balm, SPF 15
K4 = Eye Contour Emulsion, SPF 15
K5 = Skin Soothing Night Cream O/W
K6 = Anti Wrinkle Night Cream W/O
K7 = Anti Wrinkle Ampoule
K8 = Anti Wrinkle Gel
K9 = Body Oil

Table 1: Compositions of formulations according to the invention (Examples **K1 - K9**)

| | | K1 | K2 | K3 | K4 | K5 | K6 | K7 | K8 | K9 |
|---|---|---|---|---|---|---|---|---|---|---|
| **Skin Lightening Ingredients** | | | | | | | | | | |
| **Compound of list A** | | 0.1 | 0.02 | 0.01 | 0.05 | 0.1 | 0.7 | 0.02 | 0.02 | 0.2 |
| (-) alpha Bisabolol nat. | Bisabolol | | 0.1 | | 0.2 | | | | | |
| Abil 350 | Dimethicone | | | 2 | | | | | | |
| Actipone® Laminaria SaccharinaGW | Glycerin, Water (Aqua), Laminaria Saccharina Extract | | | | | 1 | | | | |
| Aloe Vera Gel Conc.10:1 | Aloe Barbadensis Leaf Juice | | 1 | | | | | | | |
| Aluminium Stearate | Aluminium Stearate | | | | | | 1.2 | | | |
| Amaze XT | Dehydroxanthan Gum | 1.4 | | | | | | | | |
| Avocado Oil | Persea Gratissima (Avocado) Oil | | | | | | | | | 2 |
| Betulin 90% (1079) | Betulin | | | | 0.15 | | | | | |
| Biotive® L-Arginine | Arginine | 3.2 | 0.5 | 0.6 | 0.9 | | | | | |
| Biotive® Troxerutin | Troxerutin | | 0.5 | 0.5 | | | | | | |
| Carbopol ETD 2020 | Acrylates/C10-30 Alkyl Acrylate Cross-polymer | 0.2 | | | | | | | 0.7 | |
| Carbopol ETD 2050 | Carbomer | | | 0.2 | | 0.2 | | | | |
| Carbopol Ultrez-21 | Acrylates/C10-30 Alkyl Acrylate Cross-polymer | | | | 0.5 | | | | | |
| Corapan TQ | Diethylhexyl 2,6 Naphtalate | | | | 3 | | | | | |
| Cutina GMS V | Glyceryl Stearate | | | | | 2 | | | | |
| Cutina PES | Pentaerythrityl Distearate | | | 2 | | | | | | |
| Dermacryl AQF | Acrylates Copolymer | | 2 | | | | | | | |
| Dipropylene Glycol | Dipropylene Glycol | | | | | | | | 7 | |
| Dow Corning 193 surfactant | PEG-12 Dimethicone | 1 | | | | | | | 2 | |
| Dow Corning 246 fluid | Cyclohexasiloxane | | 3 | | | 1 | | | | |
| D-Panthenol 75 L | Panthenol | | | | | | | 1 | | |

(continued)

| | | K1 | K2 | K3 | K4 | K5 | K6 | K7 | K8 | K9 |
|---|---|---|---|---|---|---|---|---|---|---|
| **Skin Lightening Ingredients** | | | | | | | | | | |
| Dracorin® CE | Glyceryl Stearate/Citrate | | | | | 3 | | | | |
| Dracorin® GOC | Glyceryl Oleate Citrate, Caprylic Capric Triglyceride | | | | 1.5 | | | | | |
| Drago-Beta-Glucan | Water (Aqua), Butylene Glycol, Glycerin, Avena Sativa (Oat) Kernel Extract | | | | | | | | 1 | |
| DragoCalm® | Water, Glycerin, Avena Sativa (Oat Kernel Extract) | | | | | | | 1 | | |
| Dragocide® Liquid | Phenoxyethanol, Methylparaben, Ethylparaben, Butylparaben, Propylparaben, Isobutylparaben | | | | | 0.8 | 0.8 | | | |
| Dragoderm® | Glycerin, Triticum Vulgare (Wheat) Gluten, Water (Aqua) | | | | | 2 | | | | |
| Dragosan W/O P | Sorbitan Isostearate, Hydrogenated Castor Oil, Ceresin, Beeswax (Cera Alba) | | | | | | 8 | | | |
| Dragosantol® 100 | Bisabolol | | | 0.1 | | | 0.2 | | | |
| Dragosine® | Carnosine | 0.2 | | | | | | 0.2 | 0.2 | |
| Dragoxat® 89 | Ethylhexyl Isononanoate | | 2 | 5 | | 4 | 7 | | | 10 |
| EDTA B | Tetrasodium EDTA | | | | | | | | 0.2 | |
| EDTA BD | Disodium EDTA | | 0.1 | 0.1 | 0.1 | | | | | |
| Emulsiphos® | Potassium Cetyl Phosphate, Hydrogenated Palm Glycerides | | 2 | 2 | | | | | | |
| Ethanol | Ethanol | 10 | | | | | | | | |
| Extrapo-ne®Ginkgo Biloba | Propylene Glycol, Water (Aqua), Ginkgo Biloba Leaf Extract, Glucose, Lactic Acid | | | | | 1 | | | | |
| Food Color Brown E172+E171 Powder | Color | | | 2 | | | | | | |

(continued)

| | | K1 | K2 | K3 | K4 | K5 | K6 | K7 | K8 | K9 |
|---|---|---|---|---|---|---|---|---|---|---|
| **Skin Lightening Ingredients** | | | | | | | | | | |
| Fragrance | Parfum | 0.1 | 0.2 | 0.3 | | 0.4 | 0.3 | 0.1 | 0.1 | 0.5 |
| Frescolat® MGA | Menthone Glycerin Acetal | | | | | | | 0.1 | | |
| Frescolat® ML | Menthyl Lactate | | | | | | | | 0.1 | |
| Fruitapone® Orange B | Propylene Glycol, Water (Aqua), Citric Acid, Citrus Aurantium Dulcis (Orange) Juice, Trideceth-9, Bisabolol | | | | | | | | 1 | |
| Glycerin | Glycerin | 2.5 | 3 | | | 5 | 3 | | 4 | |
| Hydrolite®-5 | Pentylene Glycol | 3 | 2 | | 5 | | | | 4.5 | |
| Hydroviton®-24 | Water, Pentylene Glycol, Glycerin, Lactic Acid, Sodium Lactate, Serine, Urea, Sorbitol, Sodium Chloride, Allantoin | | | | | 1 | 1 | | | |
| Iso Adipat | Diisopropyl Adipate | | | | 1 | | | | | |
| Isodragol® | Triisononanoin | | 2 | | | | | | | 13 |
| Jojoba Oil | Simmondsia Chinensis (Jojoba) Seed Oil | 1 | | | | | 2 | | | |
| Keltrol CG RD | Xanthan Gum | | 0.4 | 0.2 | 0.2 | 0.1 | | 0.05 | | |
| Lanette 16 | Cetyl Alcohol | | 1 | | | | | | | |
| Lanette O | Cetearyl Alcohol | | 0.5 | | | 3 | | | | |
| Lara Care A-200 | Galactoarabinan | | 0.3 | | | | | | | |
| Macadamia Nut Oil | Macadamia Ternifolia Seed Oil | | | | | | | | | 0.5 |
| Magnesium Sulfate | Magnesium Sulfate | | | | | | 0.7 | | | |
| Mineral Oil | Mineral Oil | | | | | | 8 | | | 49.6 5 |
| Neo Heliopan® 303 | Octocrylene | | 10 | 4 | | | | | | |
| Neo Heliopan® 357 | Butylmethoxy-dibenzoylmthane | | 3 | 2 | 3 | | | | | |

(continued)

| | | K1 | K2 | K3 | K4 | K5 | K6 | K7 | K8 | K9 |
|---|---|---|---|---|---|---|---|---|---|---|
| **Skin Lightening Ingredients** | | | | | | | | | | |
| Neo Heliopan® AP | Disodium Phenyl Dibenzimidazole Tetrasulfonate | 3 | | | | | | | | |
| Neo Heliopan® AP, 15%sol., neutralized with Biotive® L-Arginine | Aqua, Disodium Phenyl Dibenzimidazole Tetrasulfonate, Arginin | | 6.7 | 6.7 | | | | | | |
| Neo Heliopan® E 1000 | Isoamyl p.Methoxycinn amate | | 1 | | | | | | | |
| Neo Heliopan® HMS | Homosalate | | 5 | | 5 | | | | | |
| Neo Heliopan® Hydro, 20%sol., neutralized with Biotive® L-Arginine | Aqua, Phenyl-benzimidazole Sulphonic Acid, Arginin | | 10 | 10 | 10 | | | | | |
| Neo Heliopan® MBC | 4-Methylbenzylidene Camphor | 1 | | | | | | | | |
| Neo Heliopan® OS | Ethylhexyl Salicylate | | | 3 | 5 | | | | | |
| Neutral Oil | Caprylic/Capric Triglyceride | | | | | 6 | | | | |
| Ozokerite Wax 2389 | Ozokerite | | | | | | 2 | | | |
| PCL-Liquid 100 | Cetearyl Ethylhexanoate | | | 2 | 2 | 4 | 5 | | | 21 |
| PCL-Solid | Stearyl Heptanoate, Stearyl Caprylate | | | | | 3 | | | | |
| Phytoconcentrole® Shea Butter | Glycine Soja (Soybean) Oil, Butyrospermum Parkii (Shea Butter) | | | | | | | | | 0.5 |
| Silcare Silicone 41 M65 | Stearyl Dimethicone | | 1 | | | | | | | |
| Sodium Chloride | Sodium Chloride | | | | | | | | | |
| Sodium Hydroxide 10% sol. | Sodium Hydroxide | | | | | 0.9 | | | 3.3 | |
| Solubilizer | PEG-40Hydrogenate d Castor Oil, Trideceth-9, Propylene Glycol, Water (Aqua) | | | | | | | 1.5 | 0.8 | |
| SymCalmin® | Pentylene Glycol, Butylene Glycol, Hydroxyphenyl Propamido-benzoic Acid | | | | | 1 | | | | |
| SymClariol® | Decylene Glycol | | | 0.5 | | | | | | |

(continued)

| | | K1 | K2 | K3 | K4 | K5 | K6 | K7 | K8 | K9 |
|---|---|---|---|---|---|---|---|---|---|---|
| **Skin Lightening Ingredients** | | | | | | | | | | |
| SymDiol® 68 | 1,2 Hexanediol, Caprylyl Glycol | 0.6 | | | | | | 1 | 0.5 | 1 |
| SymGlucan® | Water (Aqua) Glycerin, Beta Glucan | | 2 | | 2 | 1 | | 5 | | |
| SymHe-lios®1031 | Benzylidene Dimethoxydimethylindanone | | | 0.5 | 0.5 | | | | | |
| SymMatrix® | Maltodextrin, Rubus Fruticosus (Blackberry) Leaf Extract | | | | | 0.5 | | | | |
| SymMollient® L | Neopentyl Glycol Diisononanoate | | | | 2 | | | | | |
| SymMollient® S | Cetearyl Nonanoate | | | | 1 | | | | | |
| SymMollient® W/S | Trideceth-9, PEG-5 Isononanoate | | | | | | | 2 | | |
| SymRelief® | Bisabolol, Zingiber Officinale (Ginger) Root Extract | | 0.1 | | | 0.2 | | | 0.1 | 0.1 |
| SymRepair® | Hexyldecanol, Bisabolol, Cetylhydroxy-proline Palmitamide, Stearic Acid, Brassica Campestris (Rapeseed Sterols) | | | 1 | | | 3 | | | 1 |
| SymSitive®1609 | Pentylene Glycol, 4-t-Butylcyclohexanol | | | | | 0.5 | | | | |
| SymVital® | Aloe Barbadensis Leaf Juice Powder, Magnesium Ascorbyl Phosphate, Rubus Idaeus (Raspberry) Leaf Extract | 0.5 | | | | | | 0.1 | 0.5 | |
| Tapioca Pure | Tapioca Starch | | 5 | | | | | | | |
| Tegosoft PC41 | Polyglyceryl-4 Caprate | | | | | | | | 0.5 | |
| Tegosoft TN | C12-15 Alkyl Benzoate | | | | 5 | | | | | |
| Vitamin A Palmitate | Retinyl Palmitate | | | | | | | | | 0.05 |
| Vitamin E acetate | Tocopherol Acetate | | 0.5 | 0.5 | 0.5 | | 0.2 | | | 0.5 |
| Water, demin. | Water (Aqua) | ad10 0 | ad10 0 | ad10 0 | ad100 | ad10 0 | ad10 0 | ad10 0 | ad100 | |

**Examples: F1 - F10: Orally consumable use examples ["Beauty from Inside"]**

**Example F1: Fruit gums**

| | % by weight |
|---|---|
| Water | Ad 100 |
| Saccharose | 34.50 |
| Glucose syrup, DE 40 | 31.89 |
| Iso Syrup C* Tru Sweet 01750 (Cerestar GmbH) | 1.50 |
| Gelatin 240 Bloom | 8.20 |
| Yellow and red food colourants | 0.01 |
| Citric acid | 0.20 |
| **Compound of list A** | 0.075 |

**Example F2: Hard boiled candy**

| | I (% by weight) | II (% by weight) |
|---|---|---|
| Sugar (Saccharose) | Ad 100 | Ad 100 |
| High fructose corn syrup | 41.00 | 41.00 |
| Maltose | 3.00 | 3.00 |
| Palm kernel oil | 0.90 | 0.90 |
| Citric acid | 0.30 | 0.30 |
| Ginger extract | 0.40 | - |
| Ginseng extract | - | 0.40 |
| Blue colourant | 0.01 | 0.01 |
| **Compound of list A** | 0.10 | 0.25 |
| Honey | - | 1.50 |
| Honey flavour | - | 0.30 |

**Example F3: Gelatin capsules suitable for direct consumption**

| | % by weight | | |
|---|---|---|---|
| | I | II | III |
| Gelatin shell: | | | |
| Glycerin | 2.014 | 2.014 | 2.014 |
| Gelatin 240 Bloom | 7.91 | 7.91 | 7.91 |
| Aspartame | 0.05 | - | - |
| Sucralose | 0.035 | 0.050 | 0.070 |

(continued)

| | % by weight | | |
|---|---|---|---|
| | I | II | III |
| Allura Red (red colourant) | 0.006 | 0.006 | 0.006 |
| Brilliant Blue (blue colourant) | 0.005 | 0.005 | 0.005 |
| | | | |
| Core composition: | | | |
| Plant oil triglyceride (coconut oil fraction) | to 100 | to 100 | to 100 |
| Flavour G | 9.95 | 12.0 | 12.0 |
| **Compound of list A** | 0.07 | 0.20 | 0.50 |

Flavour G had the following composition here (in wt.%): 0.1% neotam powder, 29.3% peppermint oil arvensis, 29.35% peppermint piperta oil Willamette, 2.97% sucralose, 2.28% triacetin, 5.4% diethyl tartrate, 12.1% peppermint oil yakima, 0.7% ethanol, 3.36% 2-hydroxyethylmenthyl carbonate, 3.0% 2-hydroxypropylmenthyl carbonate, 5.77% D-limonene, 5.67% L-menthyl acetate.

The gelatin capsules I, II, III suitable for direct consumption were produced according to WO 2004/050069 and in each case had a diameter of 5 mm and the weight ratio of the core material to the shell material was 90:10. The capsules in each case opened in the mouth within less than 10 seconds and dissolved completely within less than 50 seconds.

**Example F4: Tablets in round tablet form**

| | % by weight | | |
|---|---|---|---|
| | I | II | **III** |
| Magnesium stearate | 0.9 | 0.9 | 0.9 |
| Citric acid | 0.2 | 0.2 | 0.2 |
| **Compound of list A** | 0.05 | 0.20 | 0.50 |
| Dextrose | to 100 | to 100 | to 100 |

**Example F5: Chewing gum (with sugar and sugar-free)**

| | % by weight | |
|---|---|---|
| | I | II |
| Chewing gum base | 21.0 | 30.0 |
| Glycerin | 0.5 | 1.0 |
| Menthol spearmint eucalyptus flavour P1 | 1.0 | 1.4 |
| Glucose syrup | 16.5 | - |
| Powder sugar | to 100 | - |
| **Compound of list A** | 0.15 | 0.20 |
| Sorbitol (in powder form) | | to 100 |
| Palatinit | | 9.5 |

(continued)

| | % by weight | |
|---|---|---|
| | I | II |
| Xylitol | | 2.0 |
| Mannitol | | 3.0 |
| Aspartame | | 0.1 |
| Acesulfame K | | 0.1 |
| Emulgum (emulsifier) | | 0.3 |
| Sorbitol 70%, in water | | 14.0 |

Flavour P1 had the following composition (in wt.%):

0.05% isobutyraldehyde, 0.05% 3-octanol, 0.05% dimethylsulfide, 0.1% trans-2-hexanal, 0.1% cis-3-hexanol, 0.1% natural 4-terpineol, 0.1% isopulegol, 0.2% natural piperiton, 0.3% linalool, 1.0% isoamylalcohol, 1.0% isovaleraldehyde, 2.5% natural alpha-pinene, 2.5% natural beta-pinene, 8.0% eucalyptol, 7.0% I-menthyl acetate, 12.0% I-menthone, 5.0% isomenthone, 20.5% I-carvone, 39.45% I-menthol.

The following table relates to **Examples F6 -F10:**

Example F6 = Instant drink powder
Example F7 = Instant drink powder, sugar-free
Example F8 = Carbonated lemonade (soft drink)
Example F9 = Soya fruit drink
Example F10 = Reduced-fat yoghourt

| | % by weight | | | | |
|---|---|---|---|---|---|
| | **F6** | **F7** | **F8*** | **F9** | **F10** |
| **Compound of list A** | 0.50 | 0.70 | 0.10 | 0.05 | 0.20 |
| Sugar (Saccharose) | to 100 | | | | |
| Citric acid | 4.00 | 33.33 | 0.2 | | |
| Trisodiumcitrate | 0.26 | | | | |
| Tricalciumphosphate | 0.22 | | | | |
| Ascorbic acid (Vitamin C) | 0.24 | 0.44 | | | |
| Opacifier and Titanium dioxide (E 171) | 0.20 | | | | |
| Xanthan gum (E 415) | 0.072 | | | | |
| Sodiumcarboxymethylc ellulose (E 467) | 0.064 | | | | |
| Pectin (E 440) | 0.04 | | | | |
| Spray-dried pineapple flavour, contains yellow colourant tartrazine | 0.40 | | | | |
| Spray-dried raspberry flavour, contains red colorant | | 11.50 | | | |
| Lemon-lime flavour | | | 0.01 | | |
| D-Limonene | | | 0.005 | | |
| Maltodextrin (powder) | | to 100 | | | |
| Aspartame | | 3.30 | | | |
| Saccharose | | | 8.0 | 6.0 | 5.0 |

(continued)

| | % by weight | | | | |
|---|---|---|---|---|---|
| | **F6** | **F7** | **F8*** | **F9** | **F10** |
| Hesperetin (1% by weight in 1,2-propyleneglycol) | | | 0.05 | | |
| Ethylhydroxymethyl furanone | | | 0.01 ppb | | |
| Vanilla flavour | | | | 0.10 | 0.125 |
| Vanillin | | | 15 ppb | | |
| Maltol | | | 350 ppb | | |
| 2,5-dimethyl-4-hydroxy-2H-furan-3-one | | | 3 ppb | | |
| 1,2-Propylene glycol | | | 0.1 | | |
| Mixture of fruit juice concentrates | | | | 45.0 | |
| Soya powder | | | | 5.0 | |
| Yoghurt (1.5% by weight fat) | | | | | to 100 |
| Water | | | to 100 | to 100 | |
| * Carbon dioxide is added after filling into bottles. | | | | | |

## Claims

**1.** Use of a compound of formula (I) or a cosmetically or pharmaceutically acceptable salt of a compound of formula (I) or a mixture containing two or more of these compounds or the salts thereof

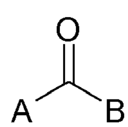

(I)

wherein A denotes

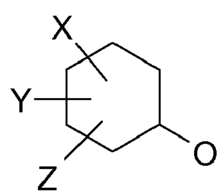

,

wherein X, Y and Z independently of one another denote hydrogen, C1-C4-alkyl or C2-C4-alkenyl,
wherein optionally two of the radicals X, Y and Z are covalently bonded to one another under formation of a bicyclic ring system, in such a bicyclic ring system two of the radicals X, Y and Z together preferably form a radical having 1 to 4 carbon atoms, preferably a hydrocarbon radical having 1 to 3 carbon atoms,
B denotes $NR^1R^2$, wherein
$R^1$ denotes hydrogen or an organic radical having 1 to 14 carbon atoms,
$R^2$ denotes an organic radical having 1 to 14 carbon atoms,

and
wherein optionally $R^1$ and $R^2$ are covalently bonded to one another, preferably so that B is a 3 to 8 membered ring, for the cosmetic (non-therapeutic) or therapeutic prolongation of the life span of a cell, preferably of a human skin cell and/or a human neuronal cell, by inhibition of senescence of said cell, in particular by

- proteasomal clearance, and/or
- stimulation or increase of proteasome activity and/or proteasome expression, and/or
- stimulation of activity and/or expression of one or more sirtuin proteins, preferably of SIRT1.

**2.** Use according to claim 1, wherein the compound of formula (I) is used

(i) for the cosmetic prevention, treatment or reduction of skin ageing effects, in particular wrinkles, age spots, photo-ageing and inflammation-induced ageing,
and/or
(ii) as anti-ageing active, preferably as cosmetic anti-ageing active, in particular as anti-wrinkle active, as active against inflammation-induced ageing, as active against photo-ageing and/or as skin tone regulator,
and/or
for the cosmetic (non-therapeutic) or therapeutic

- increase of insulin sensitivity, and/or
- reduction of proinflammatory cytokines, and/or
- prevention, treatment or reduction of oxidative stress, and/or
- induction of cellular antioxidant capacity, and/or
- stimulation of cellular energy metabolism, and/or
- stimulation of mitochondrial protein expression, and/or
- inhibition of UV-induced erythema, and/or
- inhibition of DNA-damage and/or induction of DNA-damage repair systems, and/or
- induction of cellular proliferation, and/or
- reduction and/or degradation of damaged proteins in a cell, preferably in a human skin cell and/or a human neuronal cell, and/or
- inhibition of collagen-degradation, preferably by reducing the MMP-level in the skin.

**3.** Use according to claim 1 or 2, wherein $R^1$ denotes hydrogen and/or wherein A denotes

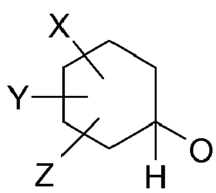

,

wherein X, Y and Z have the meaning given in claim 1.

**4.** Use according to any one of the claims 1 to 3, wherein $R^2$ denotes an organic radical having 1 to 12 carbon atoms, preferably an organic radical having 1 to 10 carbon atoms, more preferably an organic radical having 1 to 8 carbon atoms.

**5.** Use according to any one of the claims 1 to 4, wherein A denotes

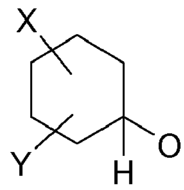,

preferably

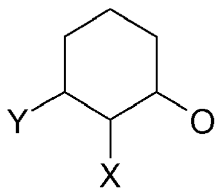, 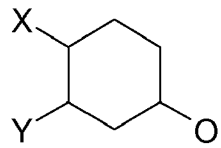 or 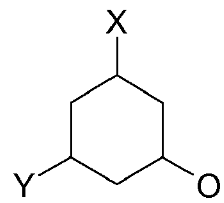,

wherein X and Y independently of one another denote C1-C4-alkyl or C2-C4-alkenyl, preferably C1-C4-alkyl.

**6.** Use according to claim 5, wherein X and Y independently of one another denote methyl, iso-propyl or tert.-butyl, and wherein preferably A denotes

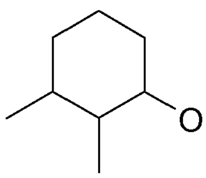, 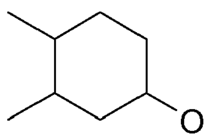, 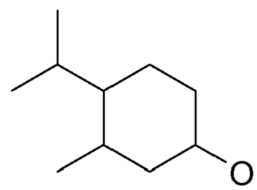,

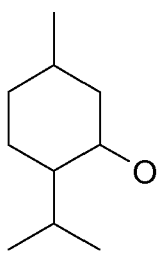, 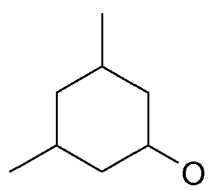 or 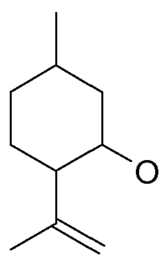.

**7.** Use according to any one of the claims 1 to 4, wherein A denotes

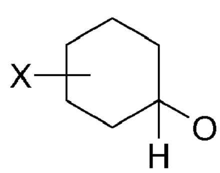,

wherein X denotes C1-C4-alkyl or C2-C4-alkenyl, preferably C1-C4-alkyl, more preferably methyl, iso-propyl or

tert.-butyl.

**8.** Use according to one of the claims 1 to 4, wherein A denotes

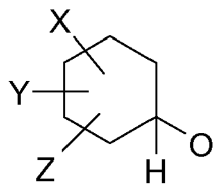

,

preferably

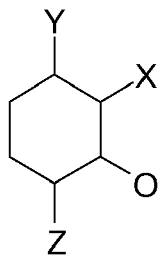

,

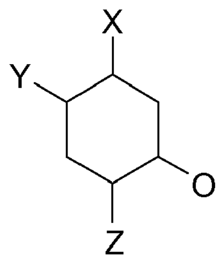

or

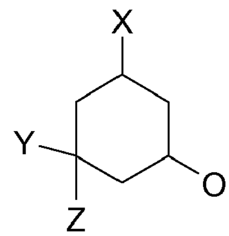

wherein X, Y and Z independently of one another denote C1-C4-alkyl or C2-C4-alkenyl, preferably C1-C4-alkyl, preferably methyl, iso-propyl or tert.-butyl.

**9.** Cosmetic or pharmaceutical, preferably topical, composition, comprising

(a) one, two or more compounds of formula (I) as defined in one of claims 1 to 8 and/or a cosmetically or pharmaceutically acceptable salt thereof, preferably in an amount having an anti-ageing effect on human skin, and
(b) one or more further anti-ageing actives suitable for cosmetic or pharmaceutical application which are not compounds of formula (I), preferably in an amount having an anti-ageing effect on human skin,
and
(c) optionally one or more cosmetically or pharmaceutically acceptable carriers.

**10.** Composition according to claim 9, wherein one, a plurality of or all the further anti-ageing actives of component (b) are selected from the following groups (b-1) to (b-8):

(b-1) one or more agents selected from the group consisting of antioxidants,
and/or
(b-2) one or more agents selected from the group consisting of substances which absorb or reflect UV radiation, preferably for cosmetic purposes, in particular for skin-protecting purposes,
and/or
(b-3) one or more skin moisturizing agents, preferably selected from the group consisting of alkane diols or alkane triols comprising 3 to 12 carbon atoms,
and/or
(b-4) one or more agents selected from the group consisting of further SIRT1 activators, and/or
(b-5) one or more agents selected from the group consisting of further proteasome activators,
and/or
(b-6) one or more agents selected from the group consisting of glycosaminoglycans and substances stimulating the synthesis of glycosaminoglycans,
and/or
(b-7) one or more agents selected from the group consisting of matrix-metalloproteinase (MMP) inhibitors,
and/or

(b-8) one or more agents selected from the group consisting of substances stimulating the formation of collagen.

**11.** Composition according to claim 9 or 10, wherein

- the total quantity of compounds of formula (I) is in the range of from 0.001 to 30 wt.%, preferably in the range of from 0.01 to 20 wt.%, more preferably in the range of from 0.01 to 5 wt.%.

**12.** Composition according to any one of claims 9 to 11, wherein

- the total quantity of antioxidants of component (b-1) is in the range of from 0.001 to 10 wt.%, preferably in the range of from 0.01 to 5 wt.%, more preferably in the range of from 0.05 to 3 wt.%,
and/or
- the total quantity of UV filter substances (UV absorbers) of component (b-2) is in the range of from from 0.01% to 40% by weight, preferably in the range of from 0.1 % to 30% by weight, more preferably in the range of from 0.2 to 20 % by weight, even more preferably in the range of from 0.5% to 15% by weight, in particular in the range of from 1.0 to 10% by weight,
and/or
- the total quantity of skin moisturizing agents of component (b-3) is in the range of from 0.1 to 30 wt.%, preferably in the range of from 0.25 to 20 wt.%, more preferably in the range of from 0.5 to 10 wt.%, even more preferably in the range of from 1 to 5 wt.%,
and/or
- the total quantity of SIRT1 activators of component (b-4) is in the range of from 0.001 to 15 wt.%, preferably in the range of from 0.01 to 10 wt.%, more preferably in the range of from 0.05 to 5 wt.%,
and/or
- the total quantity of further proteasome activators of component (b-5) is in the range of from 0.01 to 15 wt.%, preferably in the range of from 0.05 to 10 wt.%, more preferably in the range of from 0.1 to 5 wt.%,
and/or
- the total quantity of glycosaminoglycans and substances stimulating the synthesis of glycosaminoglycans of component (b-6) is in the range of from 0.01 to 10 wt.%, preferably in the range of from 0.05 to 5 wt.%, more preferably in the range of from 0.1 to 3 wt.%,
and/or
- the total quantity of matrix-metalloproteinase (MMP) inhibitors of component (b-7) is in the range of from 0.01 to 5 wt.%, preferably in the range of from 0.01 to 3 wt.%, more preferably in the range of from 0.05 to 2 wt.%,
and/or
- the total quantity of substances stimulating the formation of collagen of component (b-8) is in the range of from 0.01 to 5 wt.%, preferably in the range of from 0.01 to 3 wt.%, more preferably in the range of from 0.05 to 2 wt.%,
in each case based on the total weight of the composition.

**13.** Composition according to any one of claims 9 to 12, wherein one, a plurality of or all the further anti-ageing actives of component (b) are selected from the following groups (b-1) to (b-8) consisting of:

(b-1) vitamin A, tocopherol, tocopheryl acetate, vitamin C and ubiquinone;
(b-2) organic UV absorbers from the class of 4-aminobenzoic acid and 4-aminobenzoic acid derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives;
(b-3) $C_3$-$C_{10}$-alkane diols and $C_3$-$C_{10}$-alkane triols, preferably glycerol, 1,2-propylene glycol, 1,2-butylene glycol, 1,3-butylene glycol, 1,2-pentanediol, 1,2-hexanediol, 1,2-octanediol, 1,2-decanediol;
(b-4) hydroxyresveratrol, resveratrol, Myrtus communis leaf extract, palmitoyl tetrapeptide-10;
(b-5) oleuropein, Phaeodactylum tricornutum extract, palmitoyl isoleucin;
(b-6) hyaluronic acid, retinol, retinyl palmitate, Alpinia galanga leaf extract, tetradecyl aminobutyroylvalylaminobutyric urea trifluoroacetate;
(b-7) retinyl palmitate, ursolic acid, genistein, daidzein;
(b-8) carnitine, carnosine and magnesium ascorbyl phosphate.

**14.** Compound or a cosmetically or pharmaceutically acceptable salt thereof, selected from the group consisting of:

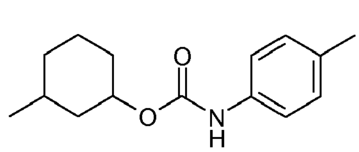
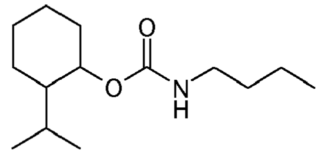
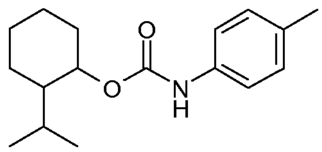
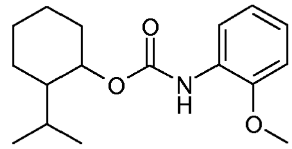
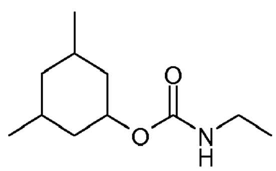
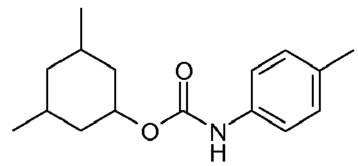
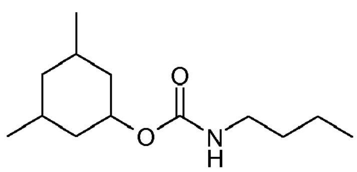
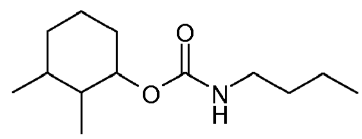
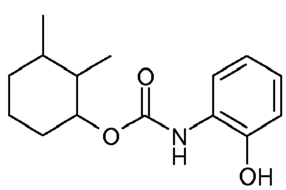
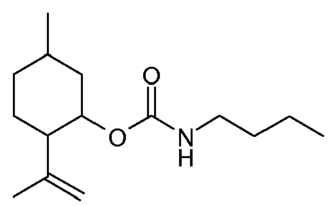
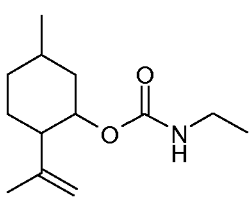
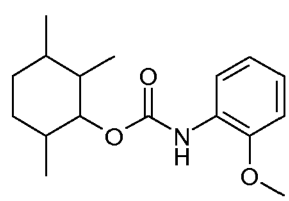

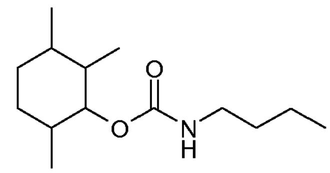

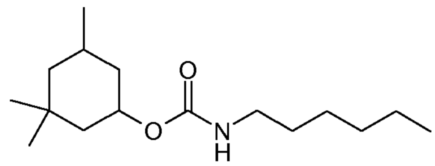

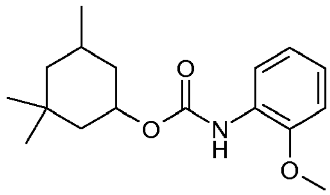

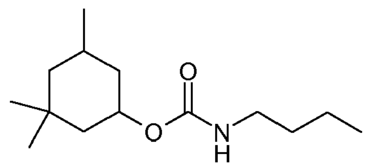

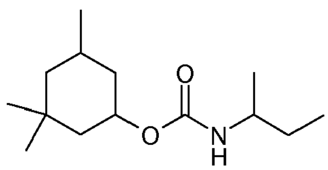

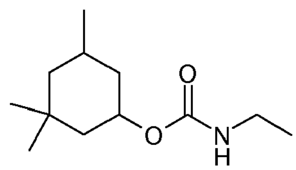

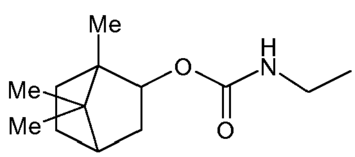

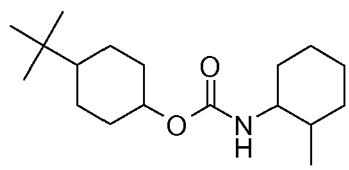

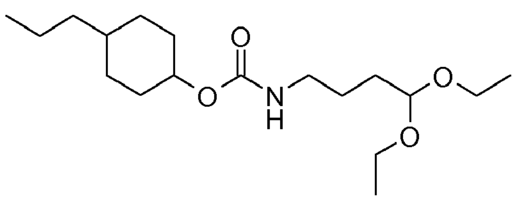

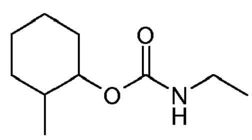

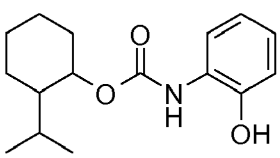

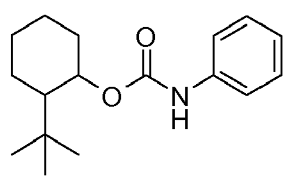

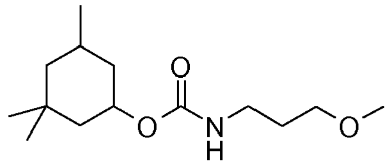

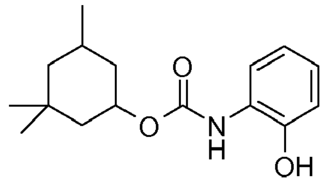

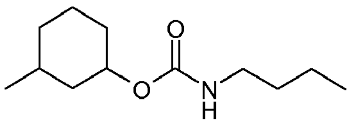

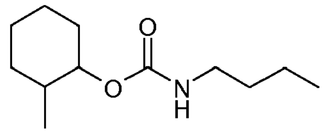

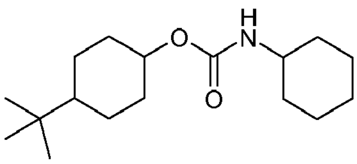

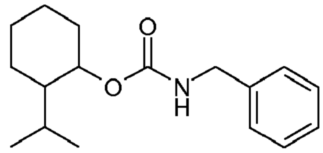

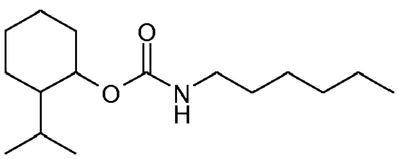

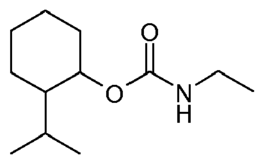

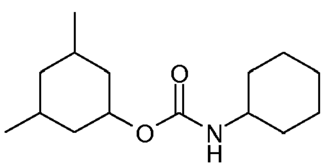

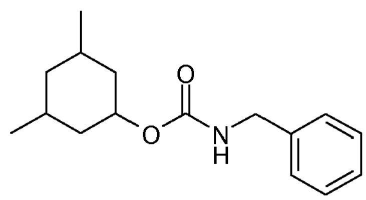

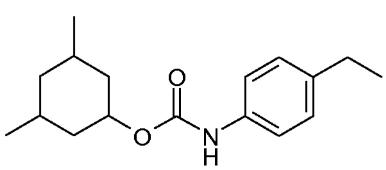

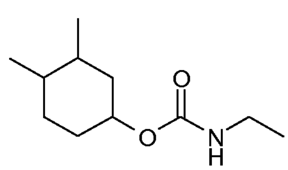

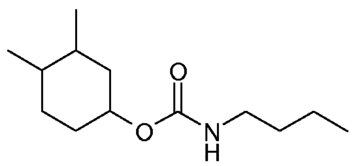
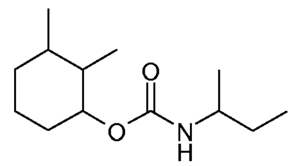
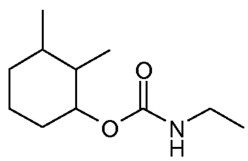
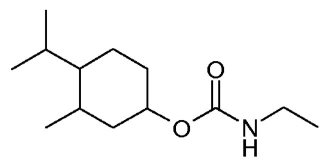
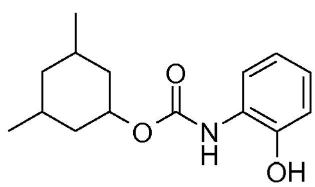
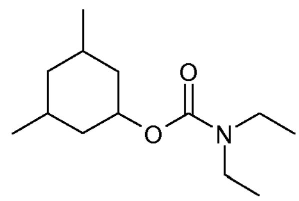
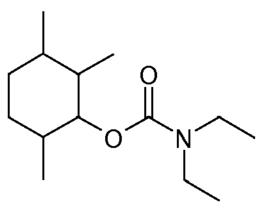
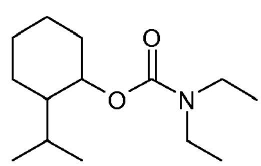
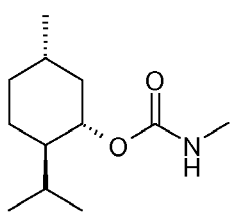
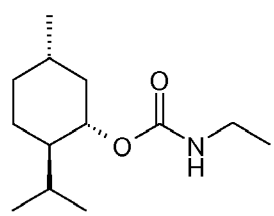
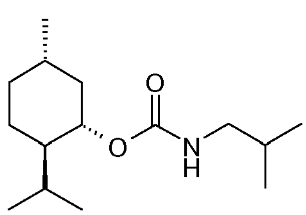
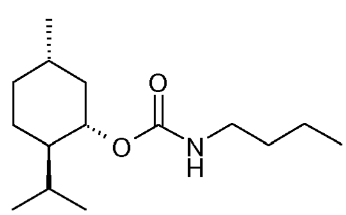

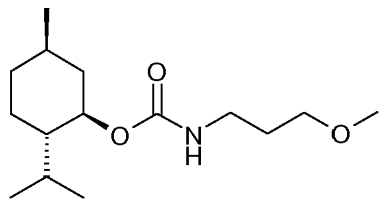
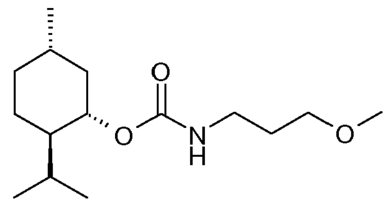
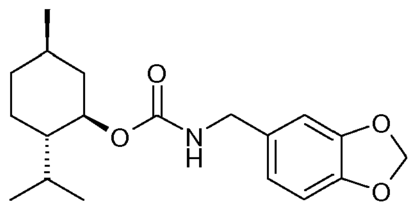
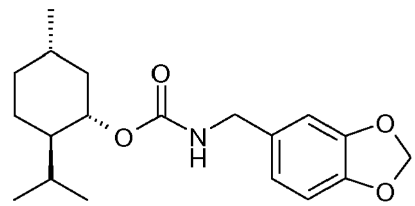
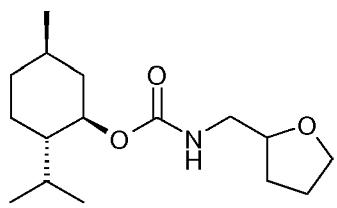
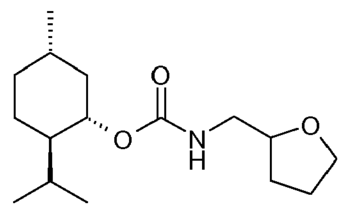
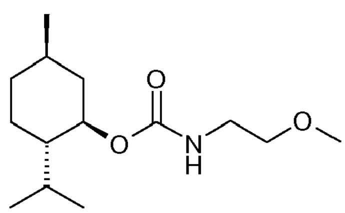
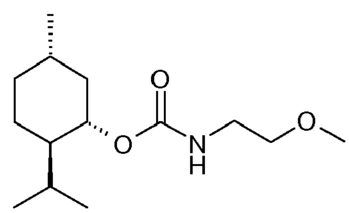
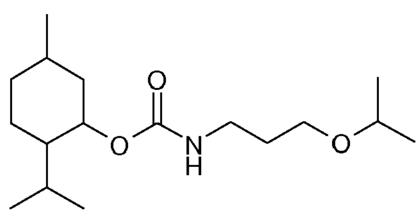
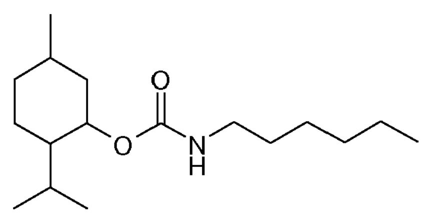

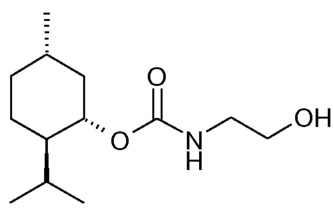

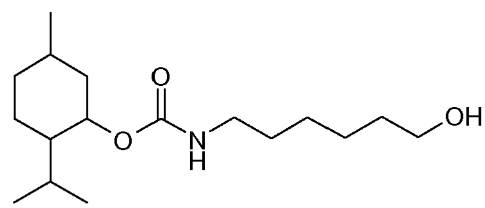

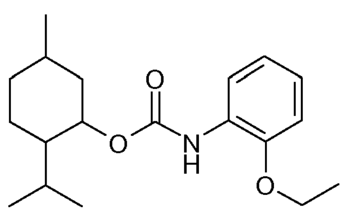

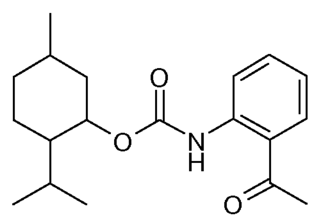

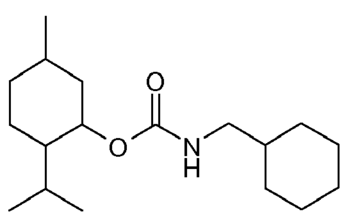

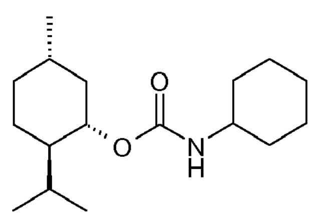

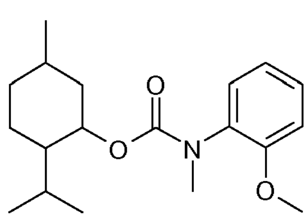

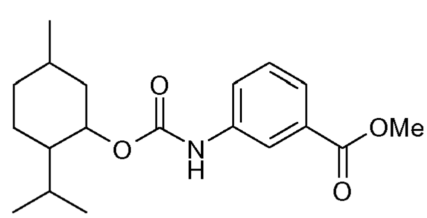

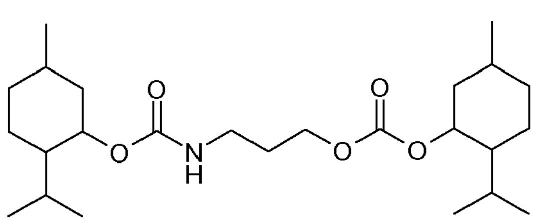

**15.** Method for the cosmetic

- proteasomal clearance in a cell, preferably in a human skin cell and/or a human neuronal cell, and/or
- stimulation of activity and/or expression of one or more sirtuin proteins (sirtuins), preferably of SIRT1, and/or
- prevention, treatment or reduction of a skin ageing effect, in particular wrinkles, age spots, photo-ageing, cellular senescence and/or inflammation-induced ageing, preferably in a human skin cell and/or a human neuronal cell, and/or
- regulation of the skin tone, and/or
- prevention, treatment or reduction of oxidative stress, and/or
- inhibition of UV-induced erythema,

comprising the following step:
- application of a cosmetically effective amount of a compound of formula (I) or a cosmetically acceptable salt of a compound of formula (I) or a mixture containing two or more of these compounds or the salts thereof as defined in any one of claims 1 to 8 or 14 or of a cosmetic composition as defined in any one of claims 9 to 13.

**16.** Compound of formula (I) or a pharmaceutically acceptable salt of a compound of formula (I) or a mixture containing two or more of these compounds or the salts thereof as defined in any one of claims 1 to 8 or 14 as a drug, preferably for

- preventing or treating an age-associated disease, more preferably for preventing or treating a neurodegenerative disease, in particular Alzheimer's disease, Parkinson's disease and/or Huntington's disease,
and/or
- preventing, treating or reducing a skin ageing effect, in particular age spots, hyper-pimentation, photo-ageing and/or inflammation-induced ageing,
and/or
- increasing the insulin sensitivity,
and/or

the therapeutic prolongation of the life span of a cell, preferably of a human skin cell and/or a human neuronal cell, by inhibition of cellular senescence, in particular by
- proteasomal clearance in a cell, and/or
- stimulation or increase of proteasome activity and/or proteasome expression, and/or
- stimulation of activity and/or expression of one or more sirtuin proteins (sirtuins), preferably of SIRT1, and/or
- reduction of proinflammatory cytokines, and/or
- prevention, treatment or reduction of oxidative stress, and/or
- induction of cellular antioxidant capacity, and/or
- stimulation of cellular energy metabolism, and/or
- stimulation of mitochondrial protein expression, and/or
- inhibition of UV-induced erythema, and/or
- inhibition of DNA-damage and/or induction of DNA-damage repair systems, and/or
- induction of cellular proliferation, and/or
- reduction and/or degradation of damaged proteins in a cell, preferably in a human skin cell and/or a human neuronal cell.

**17.** Pharmaceutical composition comprising a pharmaceutically active amount of one or more compounds of formula (I) as defined in any one of claims 1 to 8 or 14, preferably for

- preventing or treating an age-associated disease, more preferably for preventing or treating a neurodegenerative disease, in particular Alzheimer's disease, Parkinson's disease and/or Huntington's disease,
and/or
- preventing, treating or reducing a skin ageing effect, in particular age spots, hyper-pimentation, photo-ageing and/or inflammation-induced ageing,
and/or
- increasing the insulin sensitivity.

Europäisches Patentamt
European Patent Office
Office européen des brevets

# PARTIAL EUROPEAN SEARCH REPORT

under Rule 62a and/or 63 of the European Patent Convention. This report shall be considered, for the purposes of subsequent proceedings, as the European search report

Application Number
EP 10 16 3832

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/02071 A2 (UNILEVER PLC [GB]; UNILEVER NV [NL]; LEVER HINDUSTAN LTD [IN]) 10 January 2002 (2002-01-10)<br>* claim 2; table 5; compound C *<br>----- | 9-13, 15-17 | INV.<br>A61K8/42<br>A61Q19/08<br>C07C271/34 |
| X | DE 42 26 043 A1 (HAARMANN & REIMER GMBH [DE]) 10 February 1994 (1994-02-10)<br>* claim 1; example 6 *<br>----- | 9-17 | |
| X | US 2009/311401 A1 (LEY JAKOB PETER [DE] ET AL) 17 December 2009 (2009-12-17)<br>* paragraph [0136]; claim 10 *<br>----- | 9-17 | |
| X | US 2006/063764 A1 (GAUTSCHI MARKUS [CH]) 23 March 2006 (2006-03-23)<br>* claims 1-4; examples 1-3 *<br>----- | 9-13, 15-17 | |
| X | WO 2009/144179 A1 (SYMRISE GMBH & CO KG [DE]; HILLERS STEPHAN [DE]; OERTLING HEIKO [DE];) 3 December 2009 (2009-12-03)<br>* examples *<br>-----<br>-/-- | 9-13, 15-17 | TECHNICAL FIELDS SEARCHED (IPC)<br>A61K<br>A61Q<br>C07C |

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

1

| Place of search | Date of completion of the search | Examiner |
|---|---|---|
| Munich | 7 October 2011 | Miller, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04E07)

Europäisches Patentamt
European Patent Office
Office européen des brevets

# PARTIAL EUROPEAN SEARCH REPORT

Application Number
EP 10 16 3832

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | WO 97/21678 A1 (BAYER AG [DE]; ES SAYED MAZEN [DE]; YAMAMOTO MASARU [JP]; FROBEL KLAUS) 19 June 1997 (1997-06-19)<br>* page 9, lines 8-15; examples 1,4 *<br>----- | 9-13, 15-17 | |
| A | US 2004/136945 A1 (NIZARD CARINE [FR] ET AL) 15 July 2004 (2004-07-15)<br>* claims *<br>----- | 1-17 | |
| A | US 2008/255178 A1 (SCHRIMPF MICHAEL R [US] ET AL) 16 October 2008 (2008-10-16)<br>* claims 12-16 *<br>----- | 1-17 | |
| | | | TECHNICAL FIELDS SEARCHED (IPC) |

1

EPO FORM 1503 03.82 (P04C10)

Europäisches Patentamt
European Patent Office
Office européen des brevets

**INCOMPLETE SEARCH SHEET C**

Application Number
EP 10 16 3832

Claim(s) completely searchable:
-

Claim(s) searched incompletely:
1-17

Reason for the limitation of the search:

Claims 1-8 and 15-16 as filed are directed to subject-matter which is so unclear, broad (Article 84 EPC) and insufficiently disclosed (Article 83 EPC) that a meaningful search is not possible. The present application covers in the claims a huge variety of compounds and many possible cosmetic and therapeutic uses which are not even all clearly defined. The application as a whole is entirely speculative and sufficient support for all possible therapeutic and cosmetic uses is missing. The reasons are the following.
In examples 2 and 3 of the present application it is shown that a very limited number of eight compounds either stimulates the SIRT1 expression in a very specific cell medium or that one single compound influences the proteasomal activity.
It is simply not credible that with the same compound completely different treatments such as a treatment of Alzheimer or Parkinson can be achieved at the same time as a skin ageing effect such as a wrinkle reduction since first of all, completely different cells need to be targeted (Alzheimer or Parkinson: brain cells; wrinkle reduction: skin cells). Also the method of application is different, since a compound for treating Alzheimer or Parkinson will not be applied by using a simple skin cream. In addition the mechanism of action required for achieving the various desired results is completely different.
Thus, all different uses, effects and treatments mentioned in the claims have to be considered as a mere speculation.

The cosmetic and therapeutic uses are moreover not clearly defined, contrary to Article 84 EPC. In particular it is not clear what is meant by
a) "prolongation of a life span of a cell by inhibition of senescence of said cell", b) "proteasomal clearance", c) "increase of insulin sensitivity", d) "prevention, treatment or reduction of oxidative stress", e) "induction of cellular antioxidant capacity", f) "stimulation of cellular energy metabolism", g) "stimulation of mitochondrial protein expression", h) "inhibition of UV-induced erythema", i) "inhibition of DNA-damage and/or induction of DNA-damage repair systems", j) "induction of cellular proliferation", k) "reduction and/or degradation of damaged proteins in a cell", l) "inhibition of collagen-degradation" m) "preventing or treating an age-associated disease". A clear definition of all these speculative effects does not exist.
Moreover, it is not clear how these effects can be split in a therapeutic and cosmetic use. For example what is the difference between a cosmetic and therapeutic prolongation of a life span of a cell by inhibition of senescence of said cell?
Moreover it seems that some of the effects addressed by the present claims cannot even be achieved at all. A complete inhibition of senescence of a cell for example cannot be achieved, since any cell will eventually die and therefore will show a certain degree of senescence.

Europäisches Patentamt
European Patent Office
Office européen des brevets

INCOMPLETE SEARCH
SHEET C

Application Number
EP 10 16 3832

Inhibition of senescence would exclude ageing of thus would prevent the death of a cell. This is impossible, since no cell can live forever. Thus, the effect is unclear (Article 84 EPC) and impossible to achieve (Article 83 EPC).
In addition the present application does not indicate how the skilled person can measure any of the effects indicated above. So the skilled person is not informed by the present application what is exactly meant (Article 84 EPC) and how the intended effect (e.g. inhibition of senescence) is measured and evaluated (Article 83 EPC). So the skilled person can neither follow the various uses mentioned in the present application on purpose nor can he evaluate whether he works inside or outside the scope of the present claims.

Following the request of the applicant dated 23.12.2010 (main request) the scope of search has therefore been limited to the following part of the claims as filed:
As compounds addressed by independent claims 1, 9 and 14-17 as filed only compounds of formula M-X as indicated on page 98 of the application as filed have been taken into account.
For claims 1 and 16 only the effect "stimulation or increase of proteasome activity and/or proteasome expression" has been taken into account.
With respect to claim 2 only the effect for cosmetic uses as indicated in claim 2 under point (i) and (ii) has been considered. Claims 15-17 are interpreted in the same way as claim 2 (only prevention, treatment or reduction of skin ageing is taken into account).
The same limitation are valid for the subject-matter of the dependent claims.

## ANNEX TO THE EUROPEAN SEARCH REPORT ON EUROPEAN PATENT APPLICATION NO. EP 10 16 3832

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2011

| Patent document cited in search report | Publication date | Patent family member(s) | Publication date |
|---|---|---|---|
| WO 0202071 A2 | 10-01-2002 | AU 6752201 A | 14-01-2002 |
| | | CN 1446076 A | 01-10-2003 |
| | | JP 2004501950 A | 22-01-2004 |
| | | MX PA02012545 A | 10-04-2003 |
| | | WO 0202071 A2 | 10-01-2002 |
| DE 4226043 A1 | 10-02-1994 | AT 154587 T | 15-07-1997 |
| | | AU 4425693 A | 10-02-1994 |
| | | CA 2101790 A1 | 07-02-1994 |
| | | DE 4226043 A1 | 10-02-1994 |
| | | DE 59306774 D1 | 24-07-1997 |
| | | DK 0583651 T3 | 29-12-1997 |
| | | EP 0583651 A1 | 23-02-1994 |
| | | ES 2103045 T3 | 16-08-1997 |
| | | GR 3024499 T3 | 28-11-1997 |
| | | JP 4020980 B2 | 12-12-2007 |
| | | JP 4065881 B2 | 26-03-2008 |
| | | JP 6199740 A | 19-07-1994 |
| | | JP 2005232184 A | 02-09-2005 |
| US 2009311401 A1 | 17-12-2009 | CN 101611857 A | 30-12-2009 |
| | | EP 2135516 A1 | 23-12-2009 |
| | | US 2009311401 A1 | 17-12-2009 |
| US 2006063764 A1 | 23-03-2006 | AU 2003240349 A1 | 06-01-2004 |
| | | BR 0312156 A | 29-03-2005 |
| | | CN 1662141 A | 31-08-2005 |
| | | EP 1515610 A1 | 23-03-2005 |
| | | JP 2006504638 A | 09-02-2006 |
| | | MX PA04012203 A | 25-02-2005 |
| | | US 2006063764 A1 | 23-03-2006 |
| | | WO 2004000023 A1 | 31-12-2003 |
| WO 2009144179 A1 | 03-12-2009 | EP 2307357 A1 | 13-04-2011 |
| | | US 2011129432 A1 | 02-06-2011 |
| | | WO 2009144179 A1 | 03-12-2009 |
| WO 9721678 A1 | 19-06-1997 | AU 1094197 A | 03-07-1997 |
| | | WO 9721678 A1 | 19-06-1997 |
| US 2004136945 A1 | 15-07-2004 | AT 520391 T | 15-09-2011 |
| | | AU 2002251159 A1 | 21-10-2002 |
| | | CN 1499957 A | 26-05-2004 |
| | | EP 1372598 A2 | 02-01-2004 |
| | | FR 2822701 A1 | 04-10-2002 |
| | | HK 1063291 A1 | 26-05-2006 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 10 16 3832

This annex lists the patent family members relating to the patent documents cited in the above-mentioned European search report.
The members are as contained in the European Patent Office EDP file on
The European Patent Office is in no way liable for these particulars which are merely given for the purpose of information.

07-10-2011

| Patent document cited in search report | Publication date | Patent family member(s) | Publication date |
|---|---|---|---|
| | | JP 4180924 B2 | 12-11-2008 |
| | | JP 2004525160 A | 19-08-2004 |
| | | US 2004136945 A1 | 15-07-2004 |
| | | WO 02080876 A2 | 17-10-2002 |
| US 2008255178 A1 | 16-10-2008 | AT 509931 T | 15-06-2011 |
| | | CA 2679870 A1 | 02-10-2008 |
| | | CN 101641355 A | 03-02-2010 |
| | | EP 2129672 A1 | 09-12-2009 |
| | | ES 2363998 T3 | 22-08-2011 |
| | | JP 2010522201 A | 01-07-2010 |
| | | US 2008255178 A1 | 16-10-2008 |
| | | WO 2008118742 A1 | 02-10-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02080876 A
- US 4959393 A
- WO 2004105736 A
- JP 6072036 A
- US 5260474 A
- DE 2050087
- FR 1401219
- WO 2004089880 A
- EP 2135516 A
- WO 2004000023 A
- US 5703123 A
- DE 1300725
- US 6150415 A
- WO 2004033422 A
- EP 1284145 A
- WO 0198235 A
- WO 2004018398 A
- WO 2005123101 A
- WO 2007128723 A
- DE 10055940
- WO 0238537 A
- US 20050136537 A
- WO 02069992 A
- WO 2005123101 A1
- WO 2004047833 A
- US 20060089413 A
- WO 2008046791 A
- US 20090232915 A
- WO 2007077541 A
- WO 2008046795 A
- WO 2007042472 A
- WO 0176572 A
- WO 0215868 A
- WO 200846676 A
- WO 2007110415 A
- WO 2005107692 A
- WO 2006015954 A
- WO 2006053912 A
- US 20080070825 A
- WO 2008046676 A
- EP 0389700 A
- US 4251195 A
- US 6214376 B
- WO 03055587 A
- WO 2004050069 A
- US 4150052 A
- WO 2005049553 A
- WO 2004026840 A
- EP 2033688 A2
- EP 2033688 A
- WO 2009087242 A
- DE P4009347
- DE 3740186
- DE 3938140
- DE 4204321
- DE 4229707
- DE 4229737
- DE 4237081
- DE 4309372
- DE 4324219


### Non-patent literature cited in the description


- *J. Biol. Chem,* vol. 284 (44), 30076-30086
- *Cosmetics & Toiletries,* 1996, vol. 111 (5), 43-51
- *Bioorganic & Medicinal Chemistry Letters,* 2005, vol. 15 (9), 2209-2213
- *Organic Preparations and Procedures International,* 2004, vol. 36 (2), 141-149
- *Doklady - Akademiya Nauk Azerbaidzhanskoi SSR,* 1980, vol. 36 (2), 63-66
- *Journal of Agricultural and Food Chemistry,* 1967, vol. 15 (6), 1022-1029
- Seriya Khimicheskaya. *Izvestiya Akademii Nauk SSSR,* 1966, 922-924
- *Collection of Czechoslovak Chemical Communications,* 1965, vol. 30 (2), 585-598599-604
- Annales Pharmaceutiques Francaises. 1958, vol. 16, 408-13
- *Journal of Organic Chemistry,* 1958, vol. 23, 1590-1591
- *Azarbaycan Neft Tasarrufati,* 1933, 66-75
- *J. Org. Chem.,* 1981, vol. 46, 2804-2806
- *Biochemical Pharmacology,* 1961, vol. 8, 179-191
- *Synthetic Communications,* 2001, vol. 31 (24), 3759-3773
- *Journal of Medicinal Chemistry,* 1983, vol. 26 (9), 1215-18

- *Journal of Chromatography,* 1982, vol. 239, 227-31
- *Ecotoxicology and Environmental Safety,* 2008, vol. 71 (3), 889-894
- *Chirality,* 2010, vol. 22 (2), 267-274
- *Synthesis,* 1989, 131-132
- *J. Org. Chem.,* 1999, 7921-7928
- *Polish Journal of Chemistry,* 1996, vol. 70 (3), 310-19
- *Advanced Synthesis & Catalysis,* 2008, vol. 350 (9), 1235-1240
- *Organic & Biomolecular Chemistry,* 2005, vol. 3 (15), 2741-2749
- *Angewandte Chemie,* 1982, vol. 94 (9), 709-710
- Common Fragrance and Flavor Materials. Wiley-VCH, 2001, 52-55
- **S. ARCTANDER.** Perfume and Flavor Chemicals. private publishing house, 1969
- **SURBURG, PANTEN.** Common Fragrance and Flavor Materials. Wiley-VCH, 2006
- **S. ARCTANDER.** *Perfume and Flavor Chemicals,* 1969, vol. I, II
- **H. SURBURG ; J. PANTEN.** Common Fragrance and Flavor Materials. Wiley-VCH, 2006